# EUROPEAN PATENT SPECIFICATION

(11) **EP 4 045 483 B1**
(45) Date of publication and mention of the grant of the patent: **11.09.2024**
(21) Application number: 20790279.2
(22) Date of filing: 13.10.2020
(51) Int. Cl.: C07D 209/82, C09K 11/06, C07D 409/12, C07D 405/12, H10K 50/00

(54) **MATERIALS FOR ORGANIC ELECTROLUMINESCENT DEVICES**
MATERIALIEN FÜR ORGANISCHE ELEKTROLUMINESZENTE VORRICHTUNGEN
MATÉRIAUX POUR DISPOSITIFS ÉLECTROLUMINESCENTS ORGANIQUES

(30) Priority: 17.10.2019 EP 19203899
(43) Date of publication of application: 24.08.2022
(73) Proprietor: Merck Patent GmbH, 64293 Darmstadt (DE)
(72) Inventor: MUJICA-FERNAUD, Teresa, 64293 DARMSTADT (DE); KANG, Hyeon-Hui, Pyeongtaek 459-822 (KR); SEO, Ha-Na, Pyeongtaek 459-822 (KR); TUFFIN, Rachel, NG9 2JR Nottingham (GB); JUNG, Il, Pyeongtaek 459-822 (KR)
(74) Representative: Merck Patent Association
(86) International application number: PCT/EP2020/078701
(87) International publication number: WO 2021/074106

(56) References cited:
- WO-A1-2013/060418
- WO-A1-2017/102063
- CN-A- 105 175 313
- JP-A- 2018 127 409
- US-A1- 2016 225 998
- DATABASE WPI Week 201642, Derwent World Patents Index; AN 2016-00340S
- DATABASE WPI Week 201857, Derwent World Patents Index; AN 2018-63507V

## Description

The present application relates to an organic electroluminescent device (OLED) comprising an organic compound of a formula (1) defined hereinafter.

Electronic devices in the context of this application are understood to mean what are called organic electronic devices, which contain organic semiconductor materials as functional materials. More particularly, these are understood to mean OLEDs.

The construction of OLEDs in which organic compounds are used as functional materials is common knowledge in the prior art. In general, the term OLEDs is understood to mean electronic devices which have one or more layers comprising organic compounds and emit light on application of electrical voltage.

In electronic devices, especially OLEDs, there is great interest in improving the performance data, especially lifetime, efficiency and operating voltage. In these aspects, it has not yet been possible to find any entirely satisfactory solution. Furthermore, for use in electronic devices, there is interest in finding functional materials which have excellent material properties, in particular a high refractive index, because this is required for certain applications of the material in an OLED. Further material properties of high interest are a wide band gap, a high HOMO energy level (low ionization potential), a high glass transition temperature, a high thermal stability and a high charge mobility.

A great influence on the performance data of electronic devices is possessed by layers having a hole-transporting function, for example hole-injecting layers, hole transport layers, electron blocking layers and also emitting layers. For use in these layers, there is a continuous search for new materials having hole-transporting properties.

Hole-transport materials used in the hole-transport layer or in the hole-injection layer are, in particular, triarylamine derivatives which frequently contain at least one triarylamino group and at least one carbazole group. These compounds are frequently derived from diarylamino-substituted triphenylamines (TPA type), from diarylamino-substituted biphenyl derivatives (TAD type) or combinations of these base compounds. Furthermore, for example, use is made of fluorene or spirobifluorene derivatives which are substituted by one to four diarylamino groups (for example in accordance with EP 676461, US 7,714,145 or EP2814906). Compounds comprising a spirobifluorene group, a carbazole group and a diarylamino group are also known from the prior art (for example in KR2015-0051662).

In the context of studies of novel materials for use in OLEDs, it is found that specific compounds having the structural element of fluorene or spirobifluorene, combined with the structural elements of carbazole and an arylamine directly bonded to the carbazole moiety, are excellent functional materials for electronic devices. They are particularly useful as materials with a hole transporting function, for example for use in hole transporting layers, electron blocking layers and emitting layers. OLED compounds comprising carbazole moieties and triarylamines are disclosed in the prior art, for example, in CN 105 175 313, JP 2018 127409, US 2016/225998, WO 2017/102063 and WO 2013/060418.

When used in electronic devices, in particular in OLEDs, they lead to excellent results in terms of lifetime, operating voltage and quantum efficiency of the devices. The compounds also have one or more properties selected from high refractive index, wide band gap, very good hole-conducting properties, very good electron-blocking properties, high glass transition temperature, high oxidation stability, good solubility, high thermal stability, and low sublimation temperature.

The present application thus provides an organic electroluminescent device comprising two, three or four hole-transporting layers between the anode and the emitting layer, where two of the hole-transporting layers comprise a compound of a formula (1), 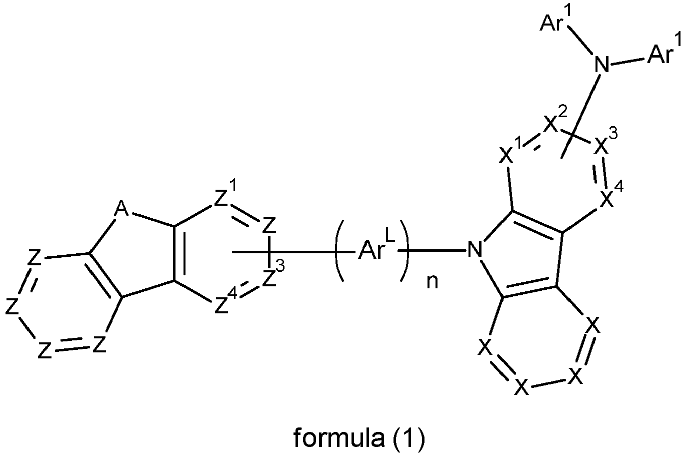 where the following applies to the symbols and indices used:
- A: stands for a group of formula (A-1) or (A-2), 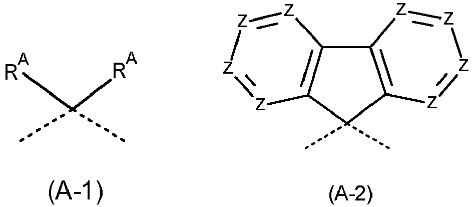 where the dashed bonds represent the bonds to the rest of the formula (1);
Z stands, on each occurrence, identically or differently, for CR^{Z};
Z¹, Z³, Z⁴ correspond to Z; with the proviso that, when n ≥ 1, then the group Ar^{L} is bonded to one of the group Z¹, Z³, Z⁴, which stands for C in this case and when n = 0, then the group Ar^{L} is absent and the carbazole moiety represented in formula (1) is directly bonded via its nitrogen atom to one of the groups Z¹, Z³, Z⁴, which stands for C in this case;
X stands, on each occurrence, identically or differently, for CR^{X};
X¹, X², X³, X⁴ correspond to X; with the proviso that the group -NAr¹Ar¹ is bonded to one of the groups X¹, X², X³, X⁴, which stands for C in this case;
Ar^{L} stands on each occurrence, identically or differently, for an aromatic ring system having 6 to 40 aromatic ring atoms and which may be substituted by one or more R radicals, or for an heteroaromatic ring systems having 5 to 40 aromatic ring atoms, which may be substituted by one or more R radicals;
Ar¹ stands on each occurrence, identically or differently, for an aromatic ring system having 6 to 60 aromatic ring atoms and which may be substituted by one or more R radicals, or for an heteroaromatic ring systems having 5 to 60 aromatic ring atoms, which may be substituted by one or more R radicals;
R^{A}, R^{X}, R^{Z} stand on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, a straight-chain alkyl, alkoxy or thioalkyl group having 1 to 40 C atoms or branched or cyclic alkyl, alkoxy or thioalkyl groups having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals; where two adjacent radicals R^{A}, two adjacent radicals R^{X} and/or two adjacent radicals R^{Z} may form a mono- or polycyclic, aliphatic ring system or aromatic ring system, which may be substituted by one or more radicals R;
R stands on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R')₃, B(OR')₂, OSO₂R', a straight-chain alkyl, alkoxy or thioalkyl group having 1 to 40 C atoms or branched or cyclic alkyl, alkoxy or thioalkyl groups having 3 to 40 C atoms, each of which may be substituted by one or more radicals R', where in each case one or more non-adjacent CH₂ groups may be replaced by R'C=CR', C≡C, Si(R')₂, Ge(R')₂, Sn(R')₂, C=O, C=S, C=Se, P(=O)(R'), SO, SO₂, O, S or CONR' and where one or more H atoms may be replaced by D, F, Cl, Br, I, CN or NO₂, an aromatic or heteroaromatic ring system having 5 to 60 aromatic ring atoms, which may in each case be substituted by one or more radicals R', or an aryloxy group having 5 to 60 aromatic ring atoms, which may be substituted by one or more radicals R', where two adjacent radicals R may form a mono- or polycyclic, aliphatic ring system or aromatic ring system, which may be substituted by one or more radicals R';
Ar is on each occurrence, identically or differently, an aromatic or heteroaromatic ring system having 5 to 24 aromatic ring atoms, which may in each case also be substituted by one or more radicals R';
R' stands on each occurrence, identically or differently, for H, D, F, Cl, Br, I, CN, a straight-chain alkyl, alkoxy or thioalkyl group having 1 to 20 C atoms or branched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, Cl, Br or I, or an aromatic or heteroaromatic ring system having 5 to 24 C atoms; and
n is 0, 1, 2 or 3
and where a compound of formula (1) is used in combination with a p-dopant in the hole-transport layer.

The case of n = 0 means that the group Ar^{L} which is indexed with n is not present, and the nitrogen atom is directly connected to one of the groups Z¹, Z³ or Z⁴. The case of n = 2, 3, 4 or 5 means that 2, 3, 4, or 5 groups Ar^{L} are present in a chain, having the structure -Ar^{L}-Ar^{L}- for the case of n = 2, having the structure -Ar^{L}-Ar^{L}-Ar^{L}- for the case of n = 3, having the structure -Ar^{L}-Ar^{L}-Ar^{L}-Ar^{L}- for the case of n = 4, and having the structure -Ar^{L}-Ar^{L}-Ar^{L}-Ar^{L}-Ar^{L}-for the case of n = 5.

Furthermore, the following definitions of chemical groups apply for the purposes of the present application:
Adjacent radicals in the sense of the present invention are radicals which are bonded to atoms which are linked directly to one another or which are bonded to the same atom.

An aryl group in the sense of this invention contains 6 to 60 aromatic ring atoms; a heteroaryl group in the sense of this invention contains 5 to 60 aromatic ring atoms, at least one of which is a heteroatom. The hetero atoms are preferably selected from N, O and S. This represents the basic definition. If other preferences are indicated in the description of the present invention, for example with respect to the number of aromatic ring atoms or the heteroatoms present, these apply.

An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quinoline or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aryloxy group in accordance with the definition of the present invention is taken to mean an aryl group, as defined above, which is bonded via an oxygen atom. An analogous definition applies to heteroaryloxy groups.

An aromatic ring system in the sense of this invention contains 6 to 60 C atoms in the ring system. A heteroaromatic ring system in the sense of this invention contains 5 to 60 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an sp³-hybridised C, Si, N or O atom, an sp²-hybridised C or N atom or an sp-hybridised C atom. Thus, for example, systems such as 9,9'-spirobifluorene, 9,9'-diarylfluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5 - 60 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphthacene, pentacene, benzopyrene, biphenyl, biphenylene, terphenyl, terphenylene, quaterphenyl, fluorene, spirobifluorene, dihydrophenanthrene, dihydropyrene, tetrahydropyrene, cis- or trans-indenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

For the purposes of the present invention, a straight-chain alkyl group having 1 to 40 C atoms or a branched or cyclic alkyl group having 3 to 40 C atoms or an alkenyl or alkynyl group having 2 to 40 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl.

An alkoxy or thioalkyl group having 1 to 40 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoro-methylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

The formulation that two radicals may form a ring with one another is, for the purposes of the present application, intended to be taken to mean, inter alia, that the two radicals are linked to one another by a chemical bond. This is illustrated by the following schemes:

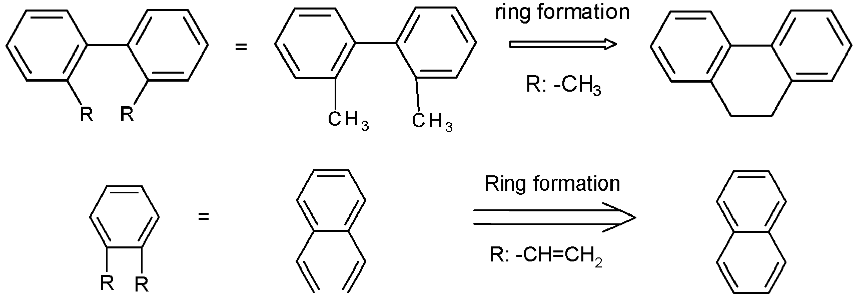

Furthermore, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

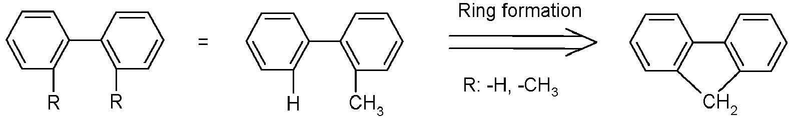

Preferably, Ar^{L} stands on each occurrence, identically or differently, for an aromatic ring system having 6 to 30, more preferably 6 to 25, particularly preferably 6 to 18 aromatic ring atoms and which may be substituted by one or more R radicals, or for an heteroaromatic ring systems having 5 to 30, more preferably 5 to 25, particularly preferably 5 to 18 aromatic ring atoms, which may be substituted by one or more R radicals.

More preferably, Ar^{L} is selected, identically or differently, from benzene, biphenyl, terphenyl, naphthalene, fluorene, indenofluorene, spirobifluorene, triazine, benzoquinoline, benzoquinazoline, dibenzofuran, dibenzothiophene, and carbazole, where each of the above-mentioned groups may be substituted by one or more radicals R.

Particularly preferably, Ar^{L} is selected from benzene, biphenyl and fluorene, most preferably from benzene, where the groups may be substituted by one or more radicals R. The compounds with the definition of Ar^{L} as given above are particularly well suitable for use in a hole transporting layer or an electron blocking layer. When used in such layers, they contribute to excellent OLED device data.

Examples of suitable groups (Ar^{L})ₙ are listed in the following table, where the dotted lines are the bonds to the rest of the compound:

| | | |
|---|---|---|
| 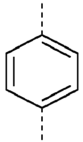 | 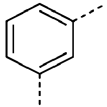 | 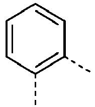 |
| Ar^{L}-1 | Ar^{L}-2 | Ar^{L}-3 |
| 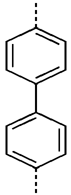 | 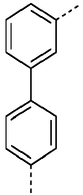 | 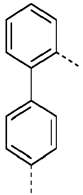 |
| Ar^{L}-4 | Ar^{L}-5 | Ar^{L}-6 |
| 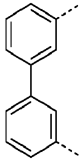 | 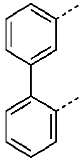 | 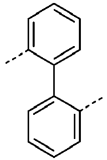 |
| Ar^{L}-7 | Ar^{L}-8 | Ar^{L}-9 |
| 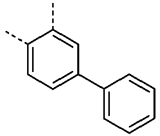 | 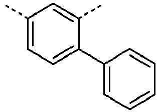 | 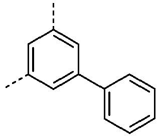 |
| Ar^{L}-10 | Ar^{L}-11 | Ar^{L}-12 |
| 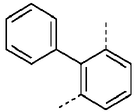 | 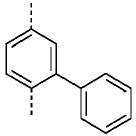 | 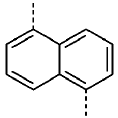 |
| Ar^{L}-13 | Ar^{L}-14 | Ar^{L}-15 |
| 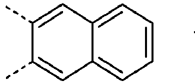 | 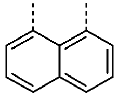 | 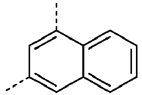 |
| Ar^{L}-16 | Ar^{L}-17 | Ar^{L}-18 |
| 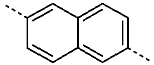 | 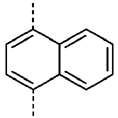 | 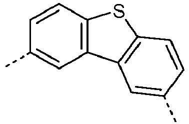 |
| Ar^{L}-19 | Ar^{L}-20 | Ar^{L}-21 |
| 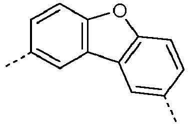 | 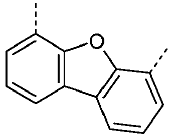 | 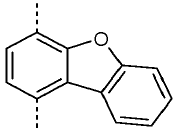 |
| Ar^{L}-22 | Ar^{L}-23 | Ar^{L}-24 |
| 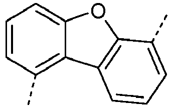 | 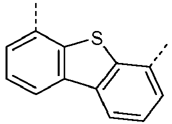 | 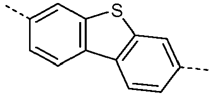 |
| Ar^{L}-25 | Ar^{L}-26 | Ar^{L}-27 |
| 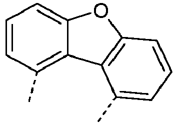 | 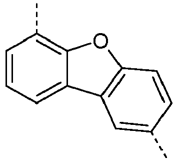 | 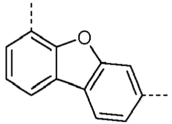 |
| Ar^{L}-28 | Ar^{L}-29 | Ar^{L}-30 |
| 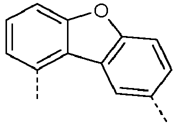 | 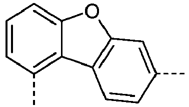 | 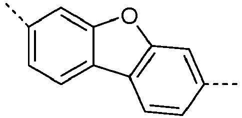 |
| Ar^{L}-31 | Ar^{L}-32 | Ar^{L}-33 |
| 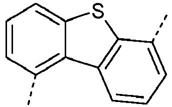 | 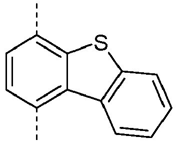 | 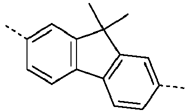 |
| Ar^{L}-34 | Ar^{L}-35 | Ar^{L}-36 |
| 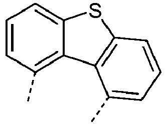 | 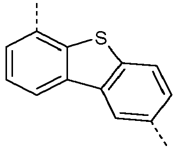 | 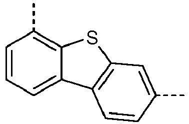 |
| Ar^{L}-37 | Ar^{L}-38 | Ar^{L}-39 |
| 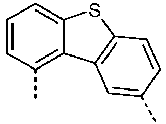 | 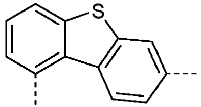 | 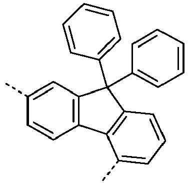 |
| Ar^{L}-40 | Ar^{L}-41 | Ar^{L}-42 |
| 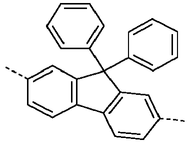 | 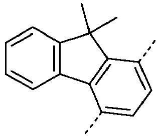 | 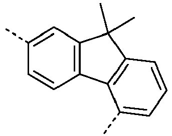 |
| Ar^{L}-43 | Ar^{L}-44 | Ar^{L}-45 |
| 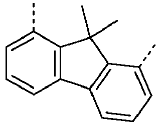 | 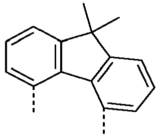 | 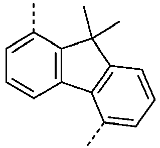 |
| Ar^{L}-46 | Ar^{L}-47 | Ar^{L}-48 |
| 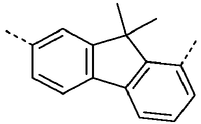 | 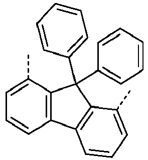 | 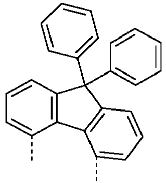 |
| Ar^{L}-49 | Ar^{L}-50 | Ar^{L}-51 |
| 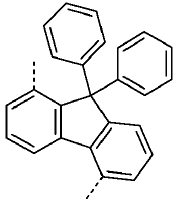 | 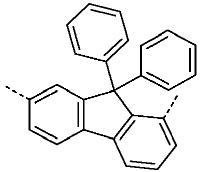 | 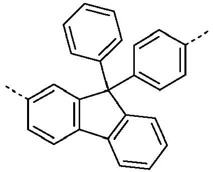 |
| Ar^{L}-52 | Ar^{L}-53 | Ar^{L}-54 |
| 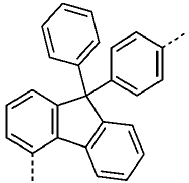 | 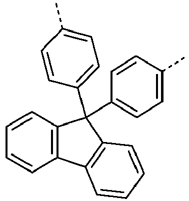 | 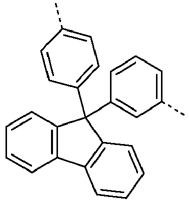 |
| Ar^{L}-55 | Ar^{L}-56 | Ar^{L}-57 |
| 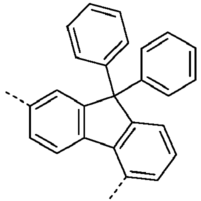 | 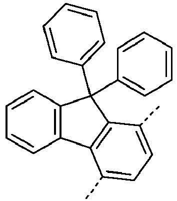 | 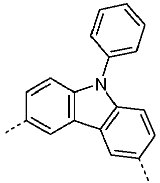 |
| Ar^{L}-58 | Ar^{L}-59 | Ar^{L}-60 |
| 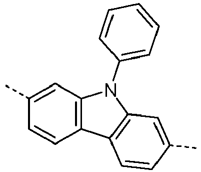 | 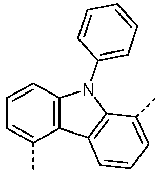 | 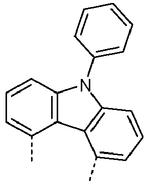 |
| Ar^{L}-61 | Ar^{L}-62 | Ar^{L}-63 |
| 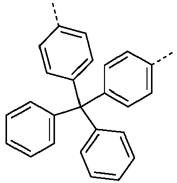 | 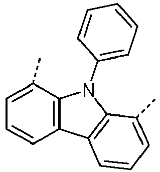 | 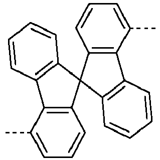 |
| Ar^{L}-64 | Ar^{L}-65 | Ar^{L}-66 |
| 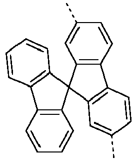 | 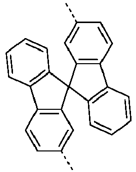 | 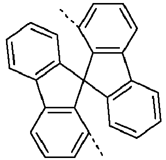 |
| Ar^{L}-67 | Ar^{L}-68 | Ar^{L}-69 |
| 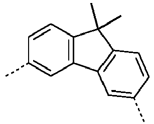 | 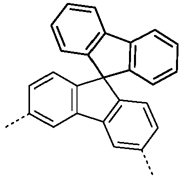 | 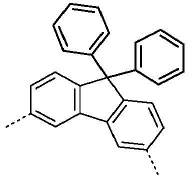 |
| Ar^{L}-70 | Ar^{L}-71 | Ar^{L}-72 |

where the groups Ar^{L}-1 to Ar^{L}-72 may be substituted at any free position by a radical R.

Among the groups Ar^{L}-1 to Ar^{L}-72, the groups Ar^{L}-1, Ar^{L}-2 and Ar^{L}-3 are preferred.

In accordance with a preferred embodiment, the compounds of formula (1) are selected from the compounds of formulae (2), (3) and (4), 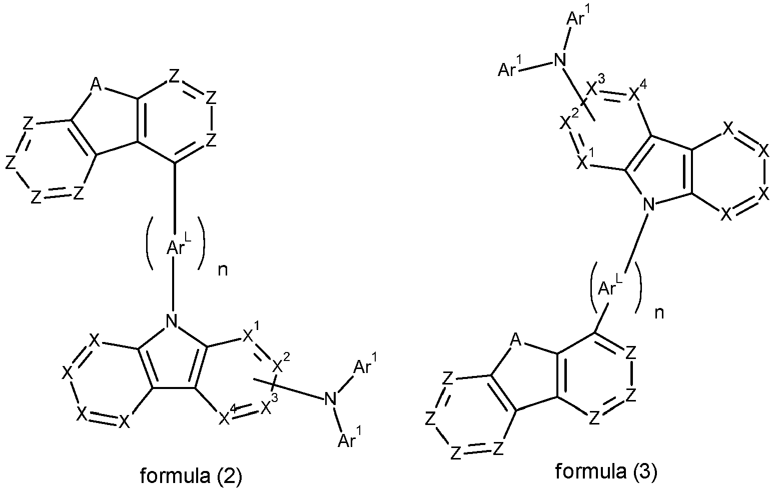 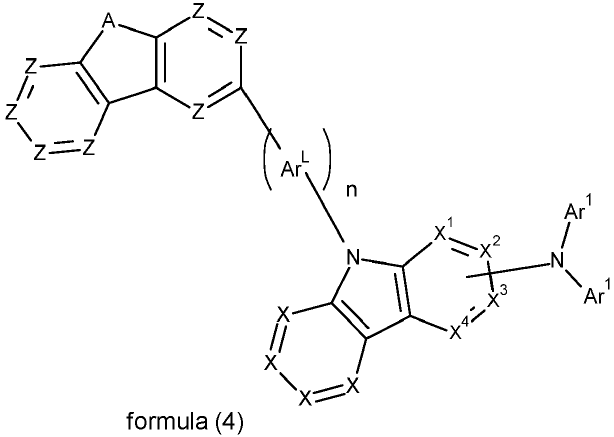 where the symbols have the same meaning as above.

The index n is preferably, identically or differently on each occurrence 0, 1, 2 or 3, particularly preferably 0 or 1.

In accordance with a preferred embodiment, the index n is 0. As mentioned above, when n is 0, the group Ar^{L} is absent so that the carbazole moiety represented in formula (1) is directly bonded via its nitrogen atom to one of the groups Z¹, Z³, Z⁴, which stands for C.

In accordance with another preferred embodiment, the index n is 1.

In accordance with a very preferred embodiment, the compounds of formula (1) are selected from the compounds of formulae (2-1) to (4-2), 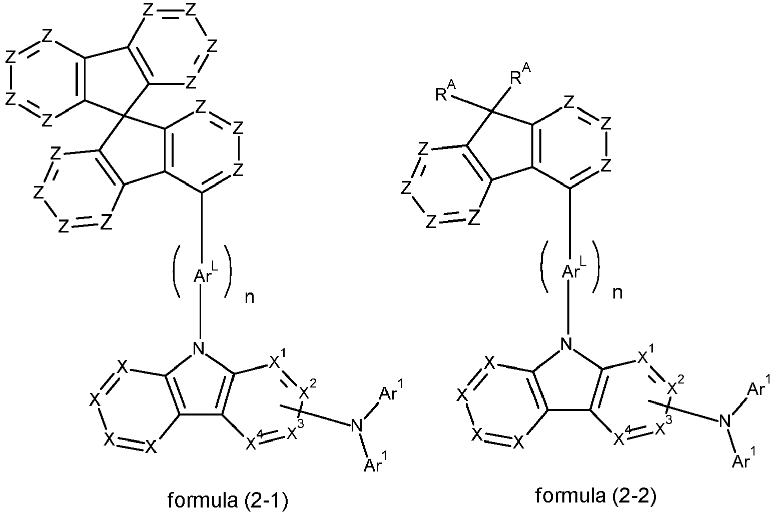 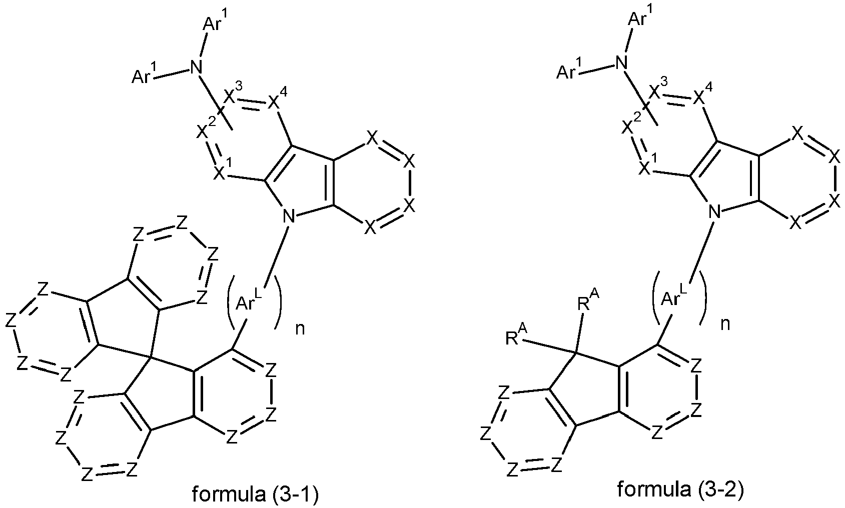 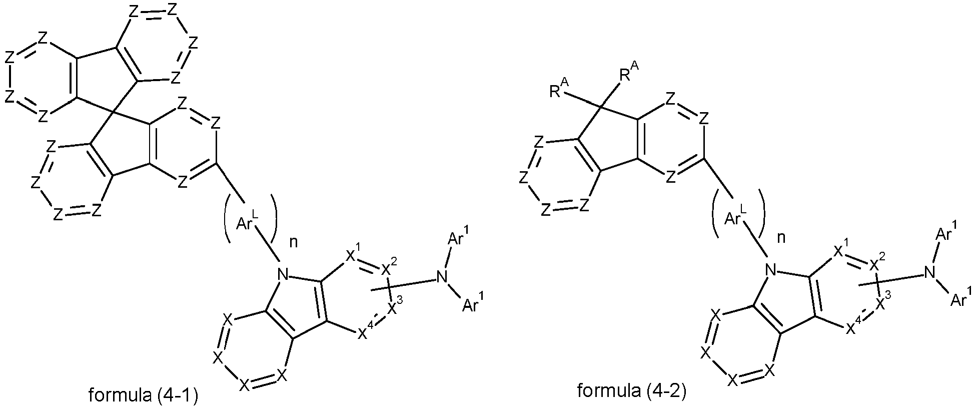 where the symbols have the same meaning as above.

Among the compounds of formulae (2-1) to (4-2), the compounds of formulae (2-1) and (2-2) are preferred, the compounds of formula (2-1) are more preferred.

In accordance with a particularly preferred embodiment, the compounds of formula (1) are selected from the compounds of formulae (2-1-1) to (4-2-2), 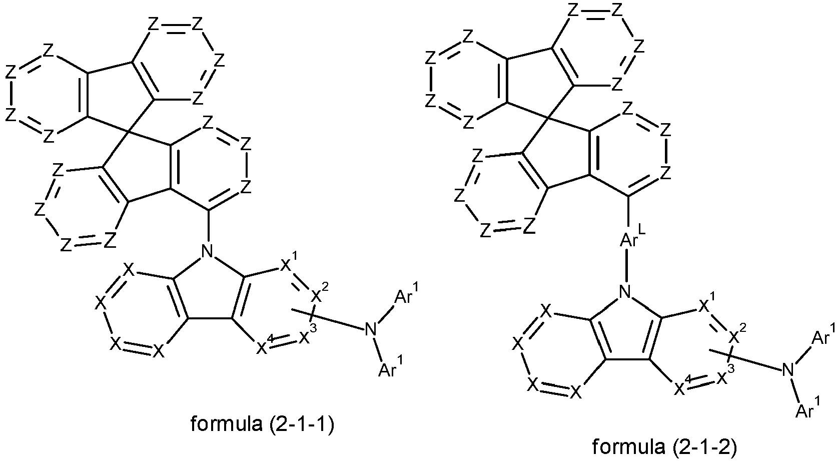 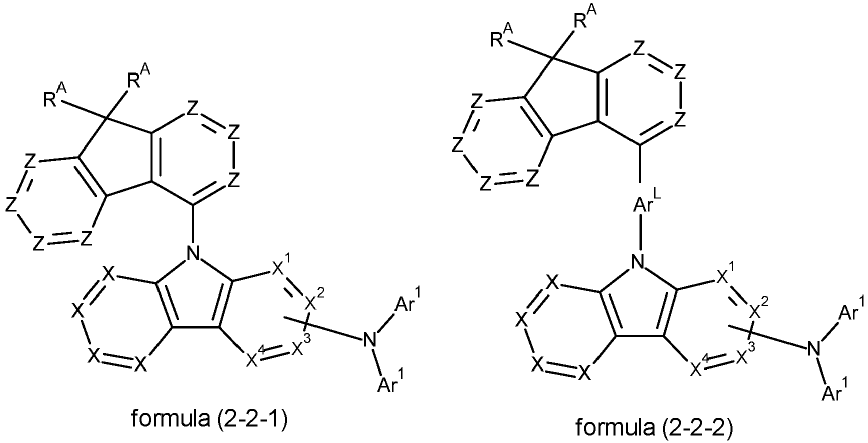 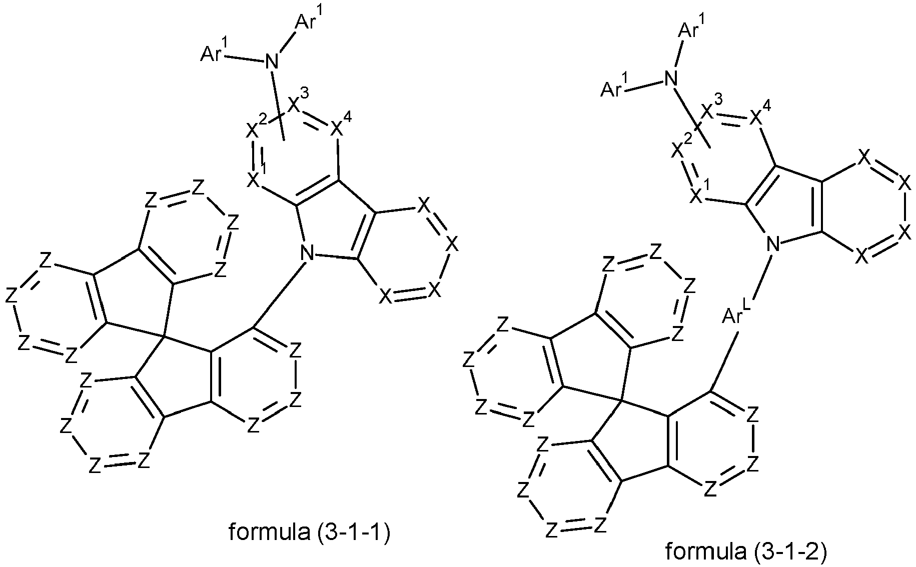 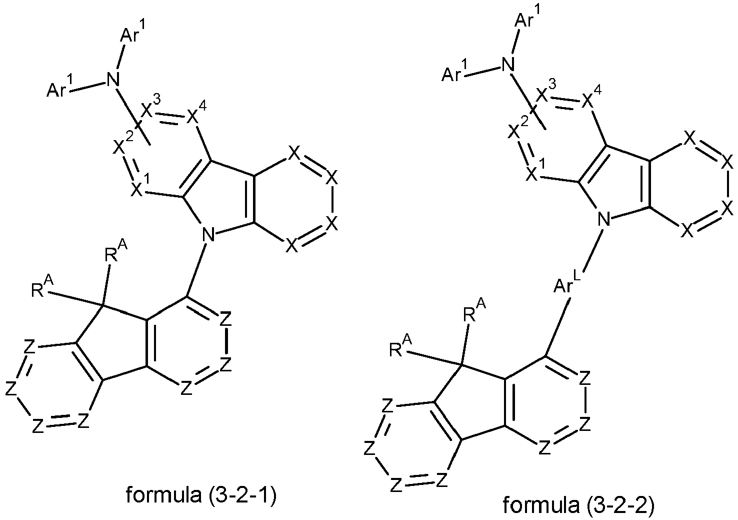 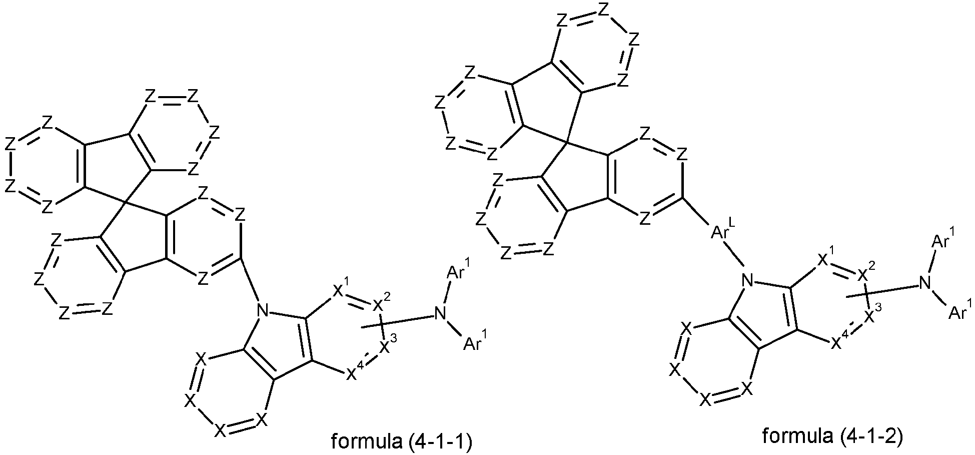 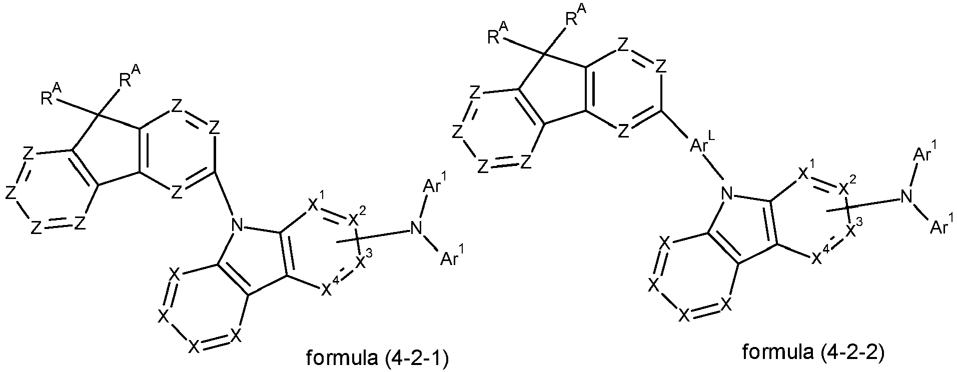 where the symbols and indices have the same meaning as above.

Among the compounds of formulae (2-1-1) to (4-2-2), the compounds of formulae (2-1-1), (2-1-2), (2-2-1) and (2-2-2) are preferred, the compounds of formulae (2-1-1) and (2-2-1) are more preferred, the compounds of formulae (2-1-1) are particularly more preferred.

In accordance with a particularly preferred embodiment, the compounds of formula (1) are selected from the compounds of formulae (2-1-1-1) to (4-2-2-4), 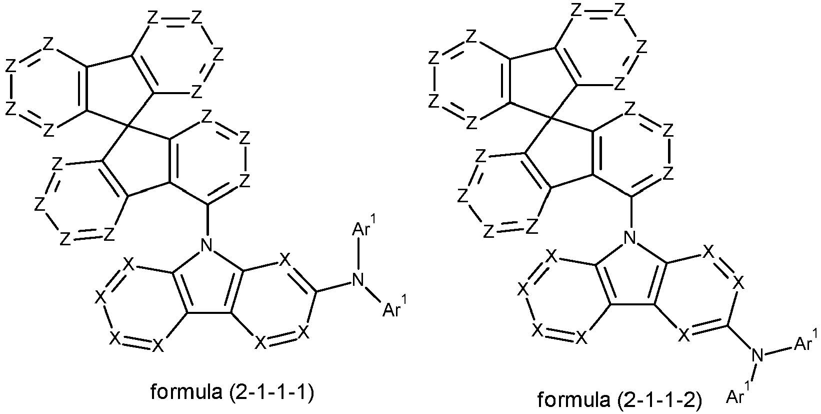 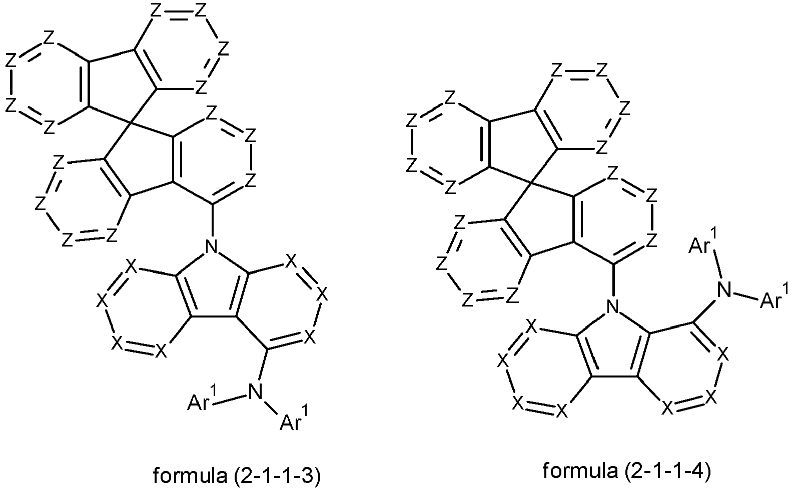 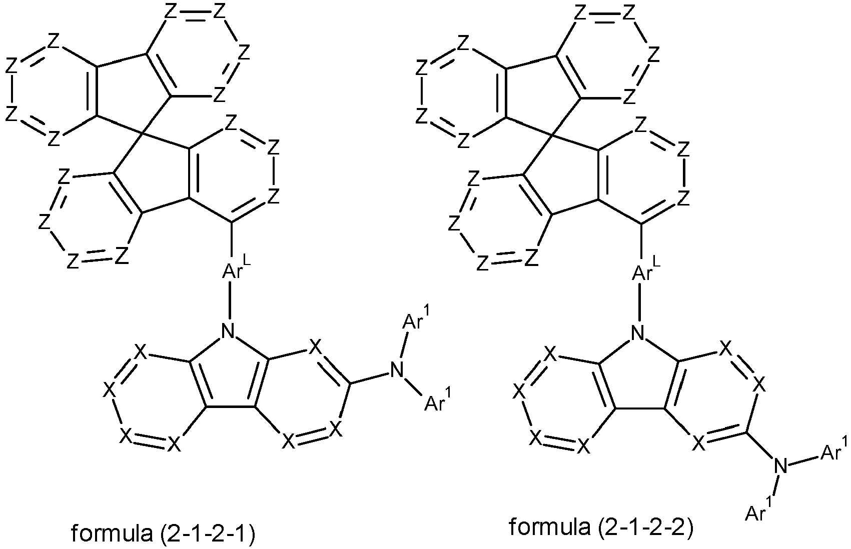 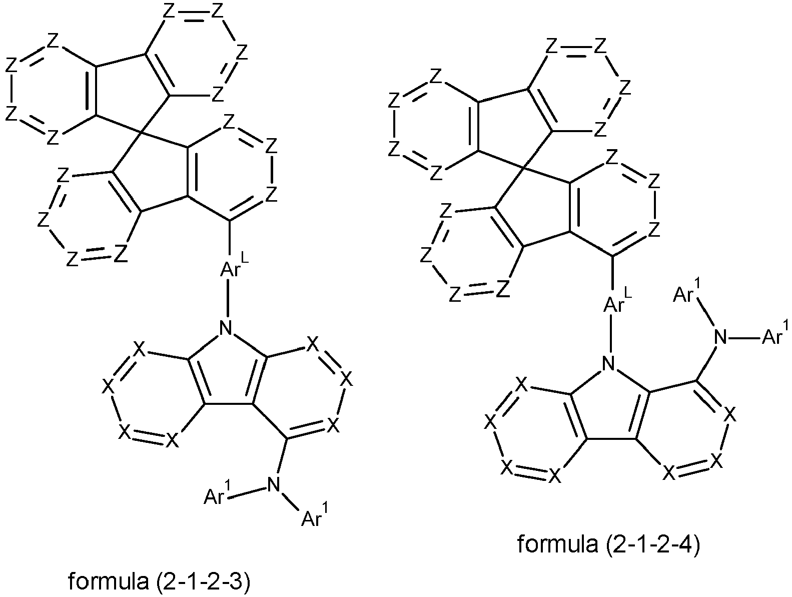 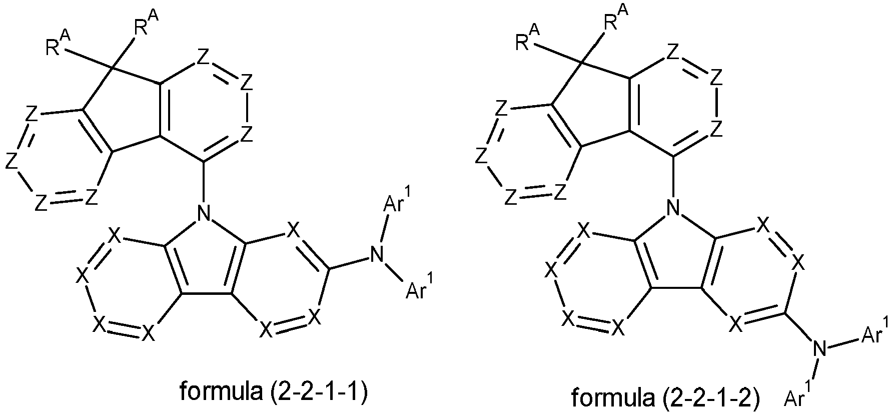 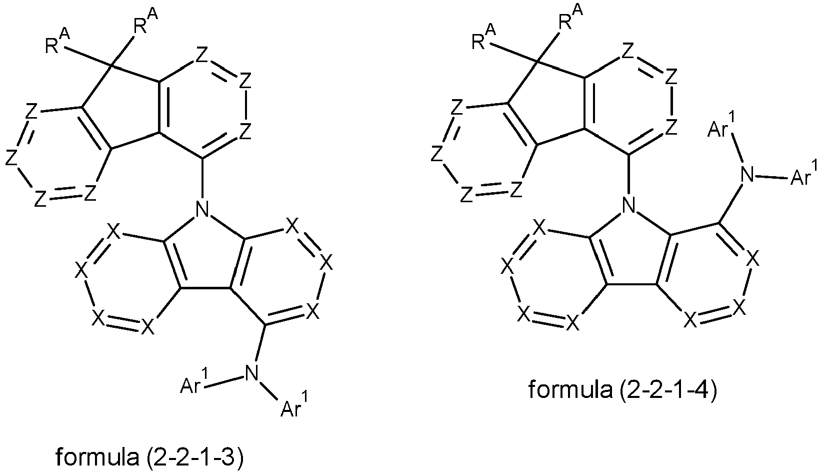 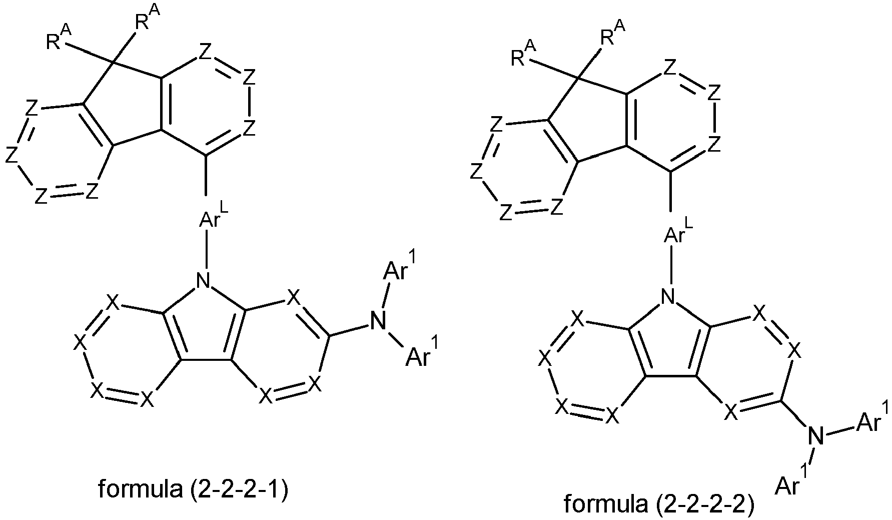 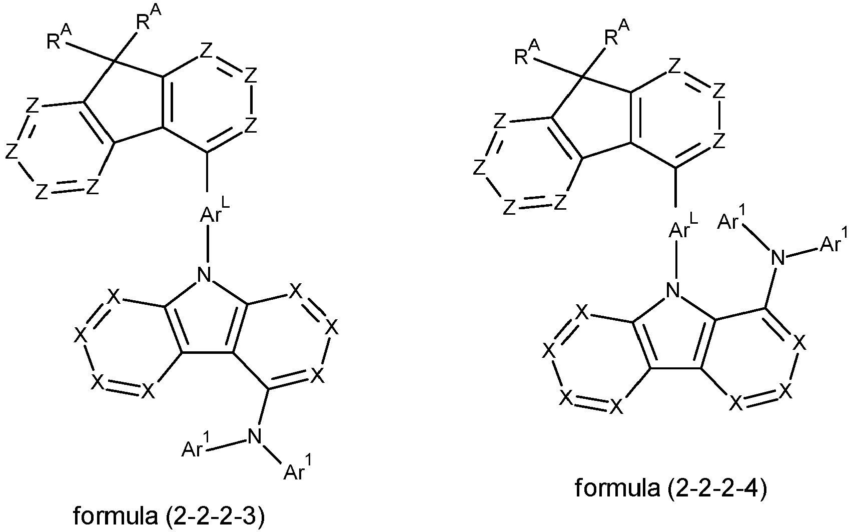 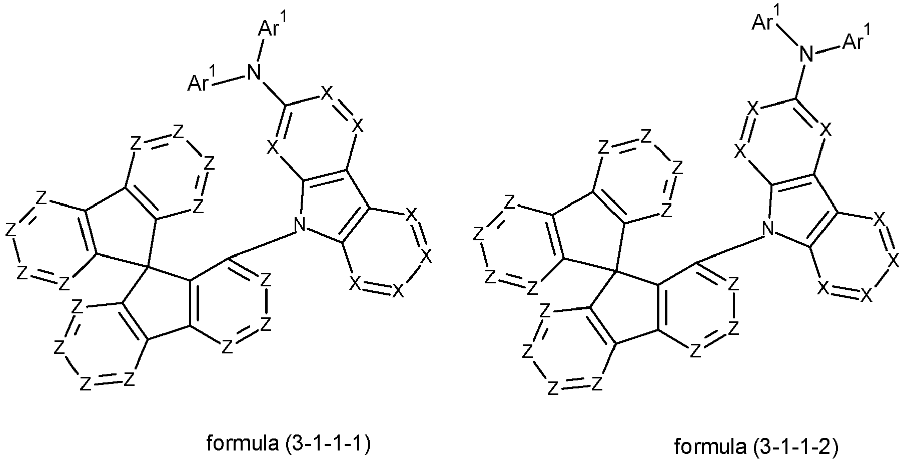 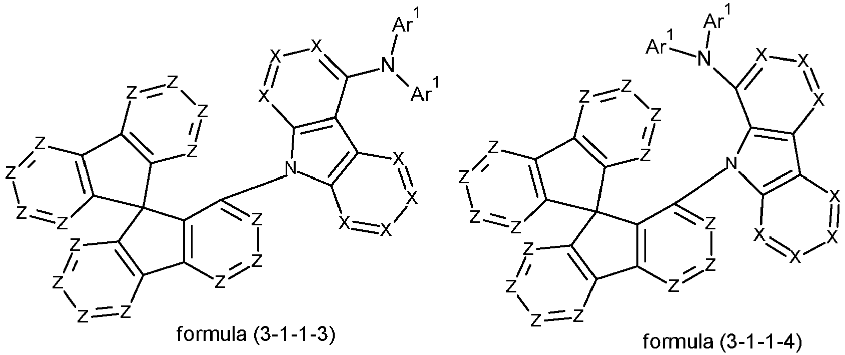 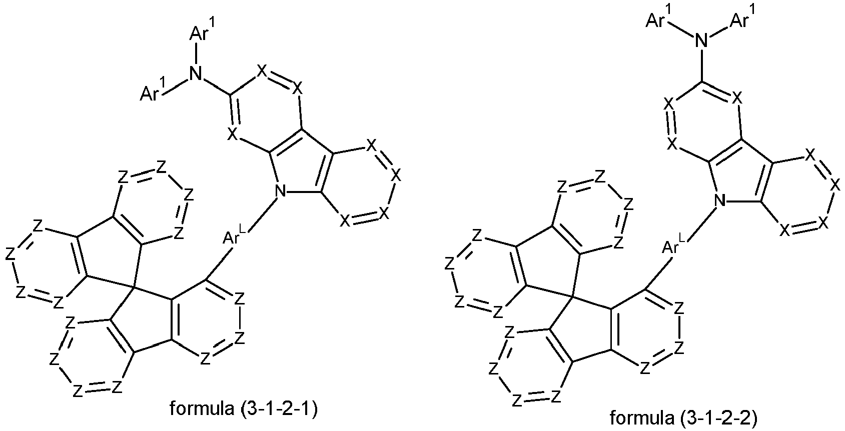 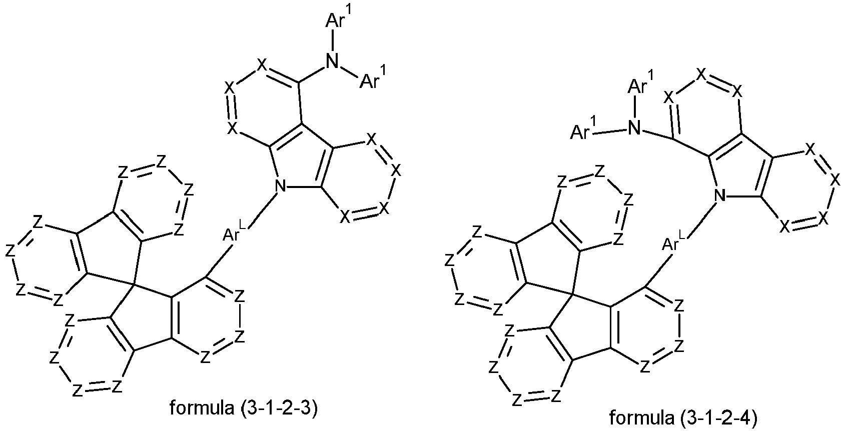 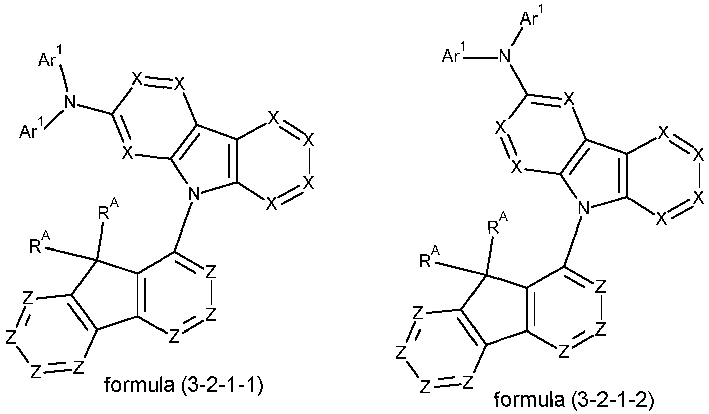 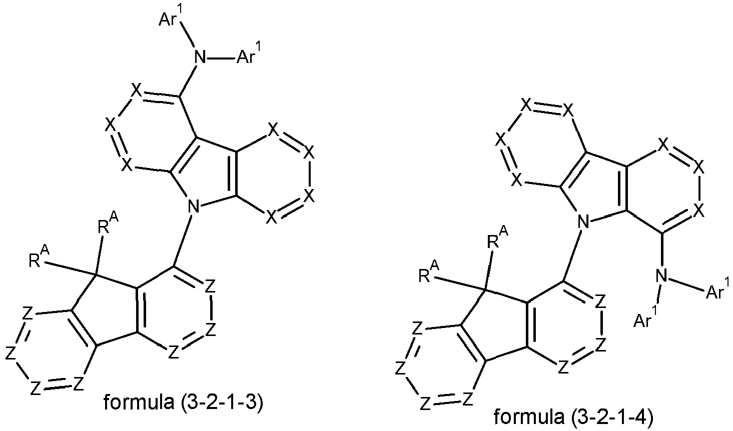 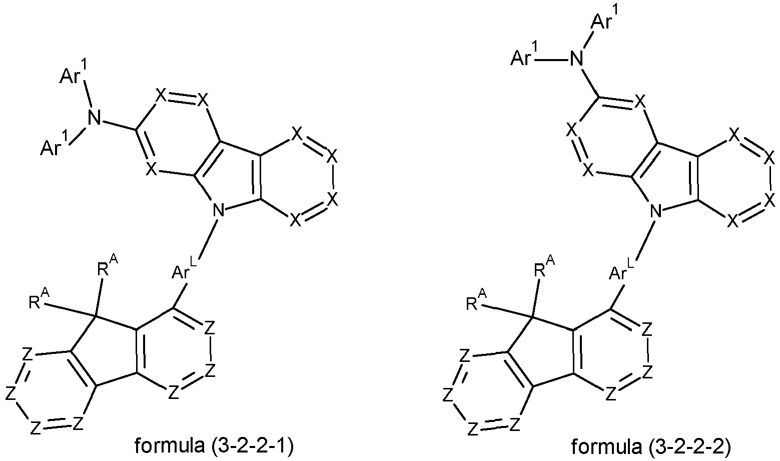 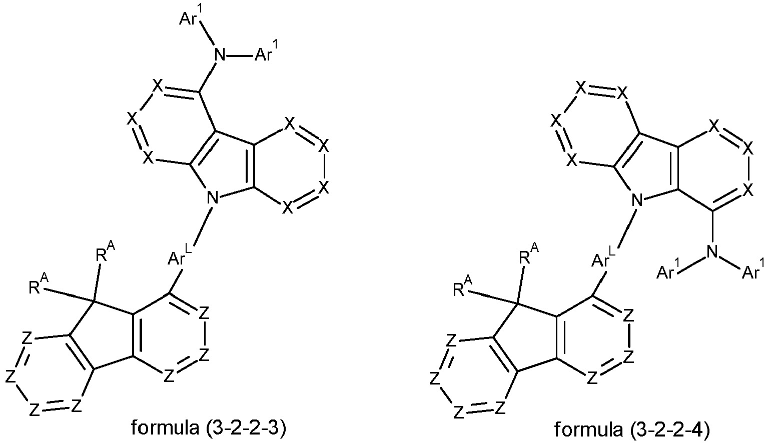 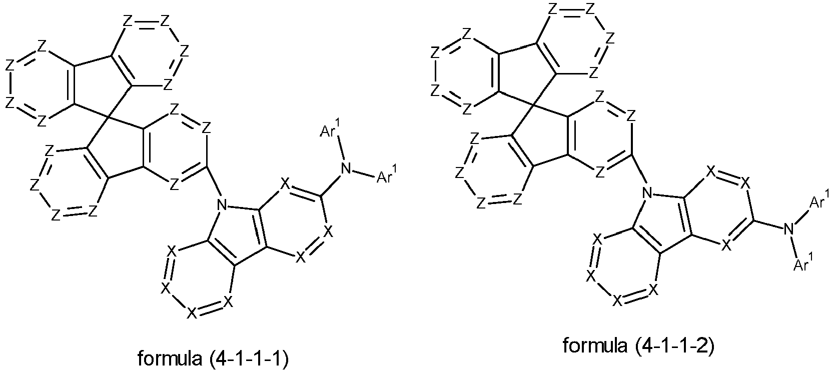 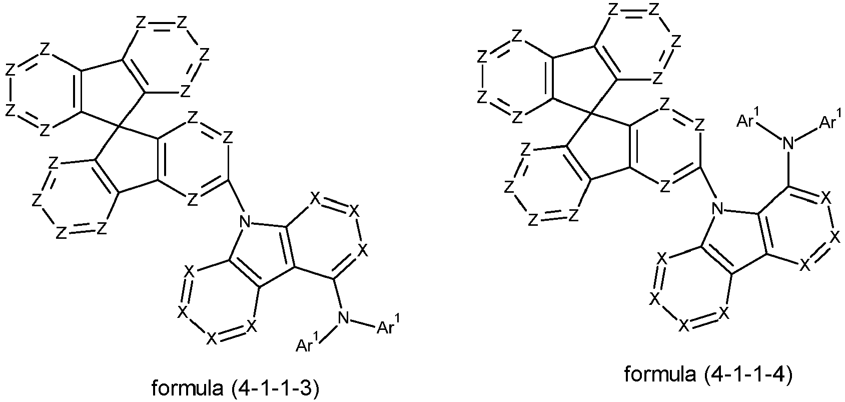 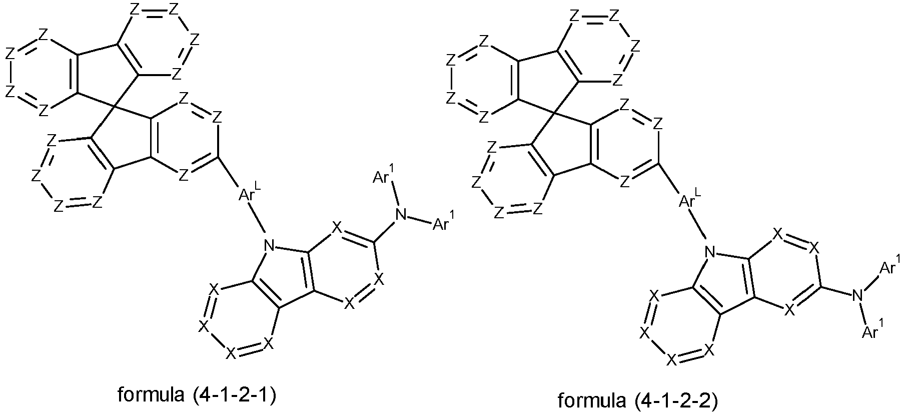 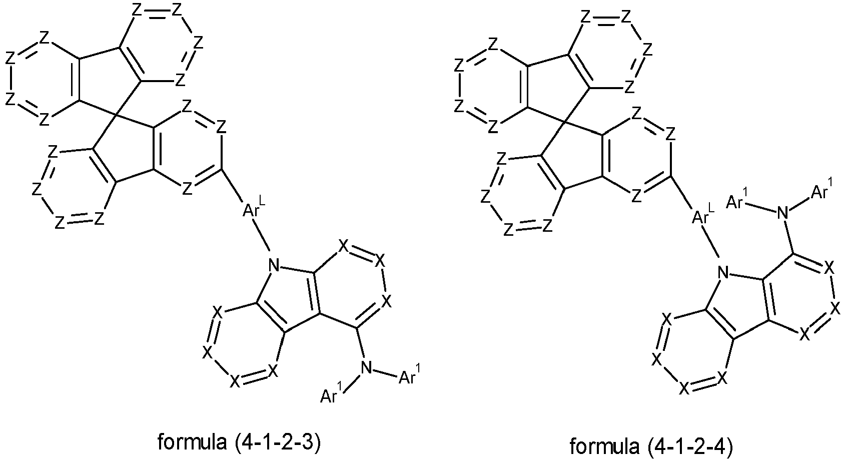 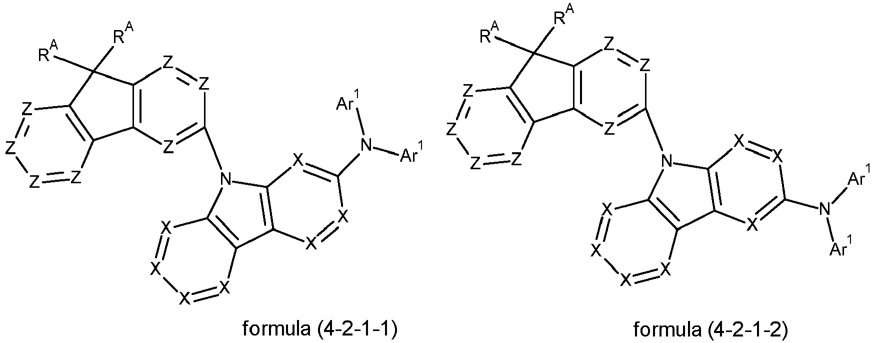 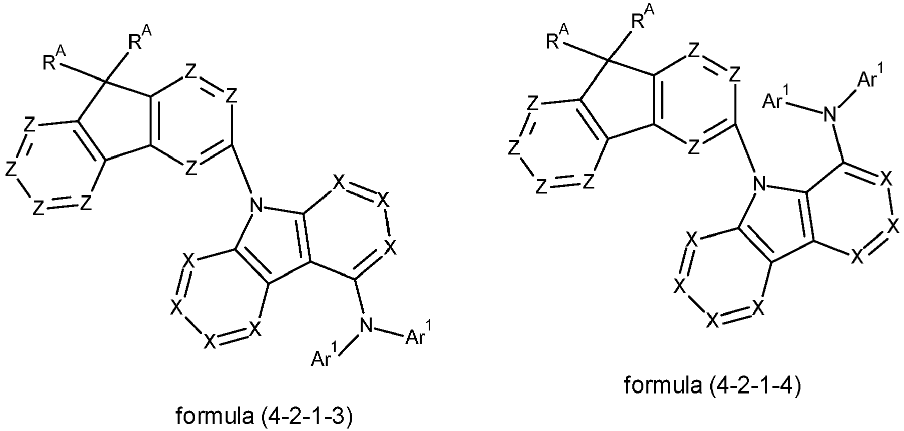 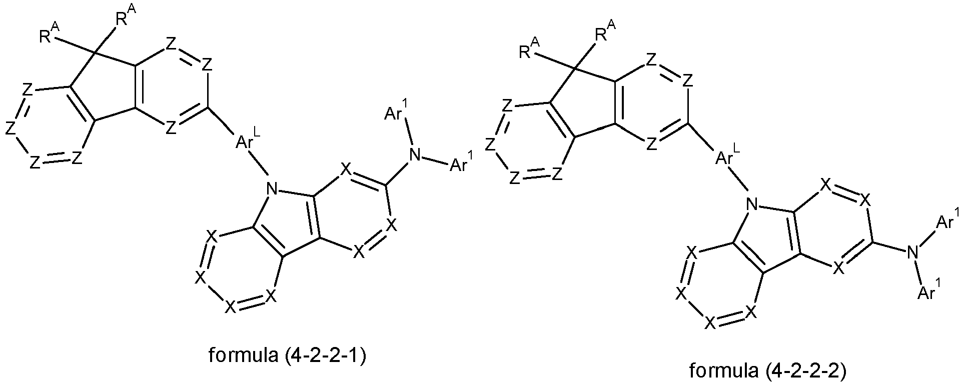 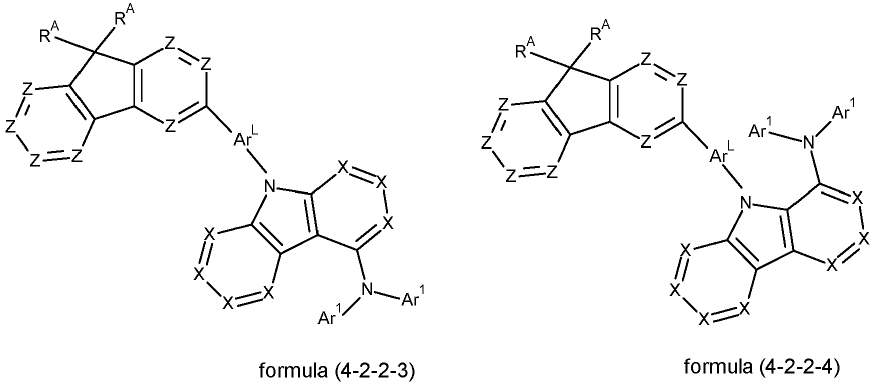 where the symbols have the same meaning as above.

Among the compounds of formulae (2-1-1-1) to (4-2-2-4), the compounds of formulae (2-1-1-1), (2-1-1-2), (2-2-1-1) and (2-2-1-2) are preferred, the compounds of formulae (2-1-1-1) and (2-2-1-1) are more preferred, the compounds of formulae (2-1-1-1) are particularly more preferred.

Preferably, the group Ar¹ is on each occurrence, identically or differently, selected from phenyl, biphenyl, terphenyl, quaterphenyl, naphthyl, fluorenyl, especially 9,9'-dimethylfluorenyl and 9,9'-diphenylfluorenyl, benzofluorenyl, spirobifluorenyl, indenofluorenyl, dibenzofuranyl, dibenzothiophenyl, carbazolyl, benzofuranyl, benzothiophenyl, benzofused dibenzofuranyl, benzofused dibenzothiophenyl, benzimidazobenzimidazolyl, naphthyl-substituted phenyl, fluorenyl-substituted phenyl, spirobifluorenyl-substituted phenyl, dibenzofuranyl-substituted phenyl, dibenzothiophenyl-substituted phenyl, benzimidazobenzimidazolyl-substituted phenyl, carbazolyl-substituted phenyl, indolyl, quinolinyl, pyridyl, pyrimidyl, pyrazinyl, pyridazinyl, and triazinyl, each of which may optionally be substituted by one or more radicals R.

In accordance with a preferred embodiment, at least one group Ar¹ is selected from biphenyl, terphenyl, quaterphenyl, fluorenyl, indenofluorenyl, spirobifluorenyl, benzimidazobenzimidazolyl, carbazolyl, dibenzofuranyl, dibenzothiophenyl, naphthyl-substituted phenyl, fluorenyl-substituted phenyl, spirobifluorenyl-substituted phenyl, carbazolyl-substituted phenyl, benzimidazobenzimidazolyl-substituted phenyl, dibenzofuranyl-substituted phenyl and dibenzothiophenyl-substituted phenyl, each of which may be substituted by one or more radicals R.

In accordance with a very preferred embodiments, both groups Ar¹ in the group -N(Ar¹)₂ of formula (1) are selected from biphenyl, terphenyl, quaterphenyl, fluorenyl, indenofluorenyl, spirobifluorenyl, benzimidazobenzimidazolyl, carbazolyl, dibenzofuranyl, dibenzothiophenyl, naphthyl-substituted phenyl, fluorenyl-substituted phenyl, spirobifluorenyl-substituted phenyl, carbazolyl-substituted phenyl, benzimidazobenzimidazolyl-substituted phenyl, dibenzofuranyl-substituted phenyl and dibenzothiophenyl-substituted phenyl, each of which may be substituted by one or more radicals R.

Examples of particularly suitable groups Ar¹ are the groups of formulae (Ar-1) to (Ar-256) as depicted below:

| | | |
|---|---|---|
| 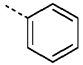 | 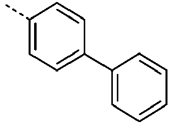 | 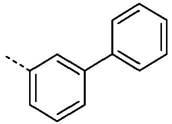 |
| Ar-1 | Ar-2 | Ar-3 |
| 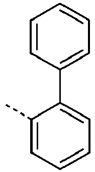 | 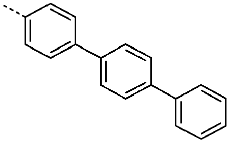 | 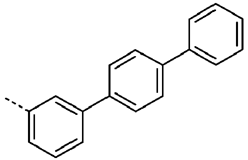 |
| Ar-4 | Ar-5 | Ar-6 |
| 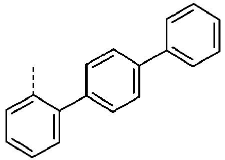 | 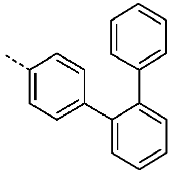 | 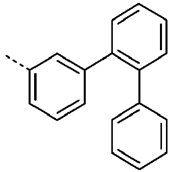 |
| Ar-7 | Ar-8 | Ar-9 |
| 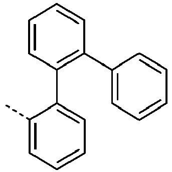 | 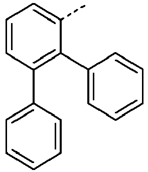 | 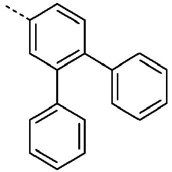 |
| Ar-10 | Ar-11 | Ar-12 |
| 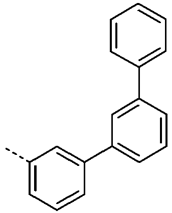 | 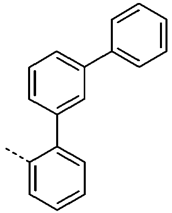 | 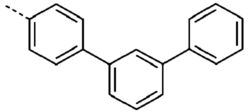 |
| Ar-13 | Ar-14 | Ar-15 |
| 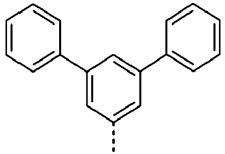 | 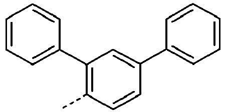 | 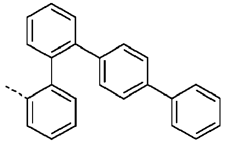 |
| Ar-16 | Ar-17 | Ar-18 |
| 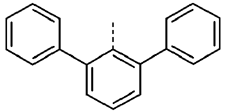 | | |
| Ar-19 | | |
| 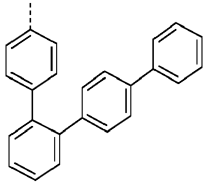 | 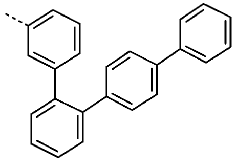 | 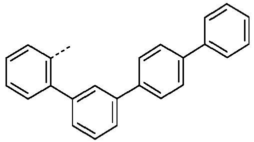 |
| Ar-20 | Ar-21 | Ar-22 |
| 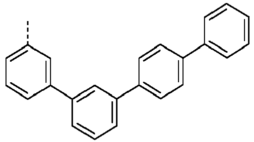 | 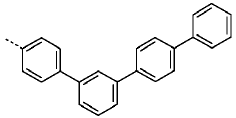 | 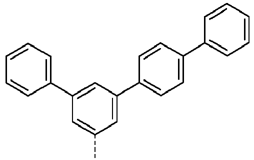 |
| Ar-23 | Ar-24 | Ar-25 |
| 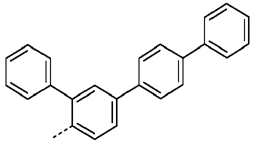 | 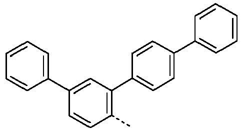 | 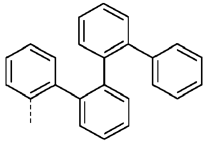 |
| Ar-26 | Ar-27 | Ar-28 |
| 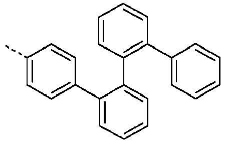 | 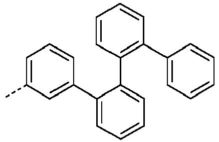 | 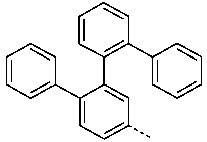 |
| Ar-29 | Ar-30 | Ar-31 |
| 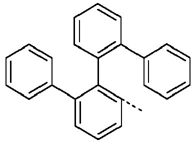 | 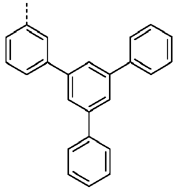 | 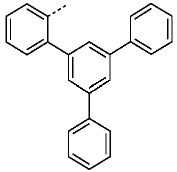 |
| Ar-32 | Ar-33 | Ar-34 |
| 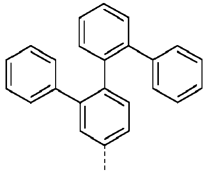 | 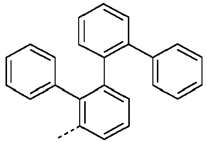 | 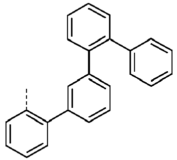 |
| Ar-35 | Ar-36 | Ar-37 |
| 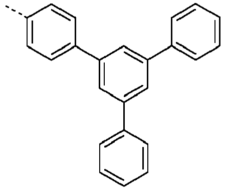 | 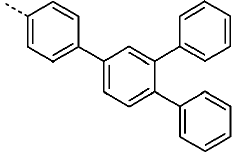 | 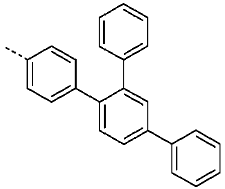 |
| Ar-38 | Ar-39 | Ar-40 |
| 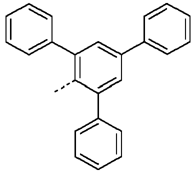 | 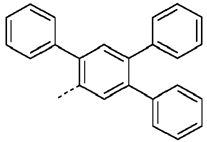 | 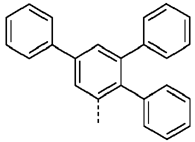 |
| Ar-41 | Ar-42 | Ar-43 |
| 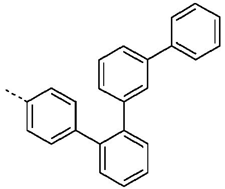 | 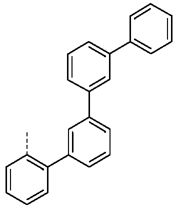 | 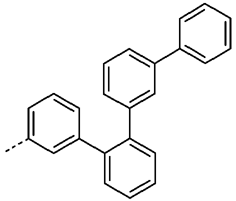 |
| Ar-44 | Ar-45 | Ar-46 |
| 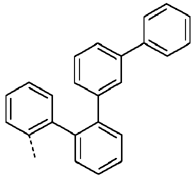 | | |
| Ar-47 | | |
| 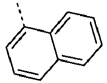 | 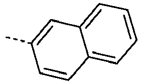 | 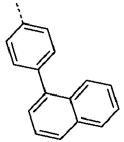 |
| Ar-48 | Ar-49 | Ar-50 |
| 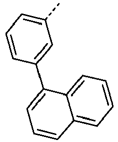 | 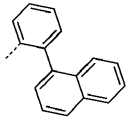 | 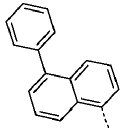 |
| Ar-51 | Ar-52 | Ar-53 |
| 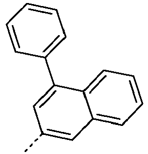 | 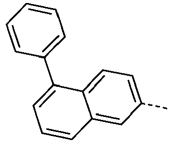 | 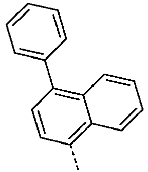 |
| Ar-54 | Ar-55 | Ar-56 |
| 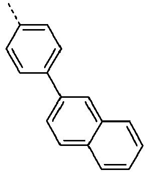 | 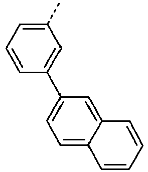 | 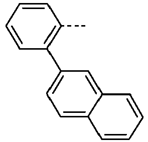 |
| Ar-57 | Ar-58 | Ar-59 |
| 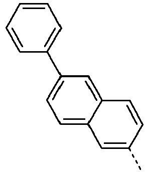 | 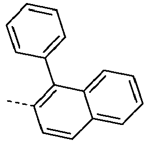 | 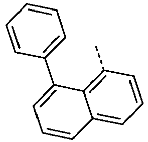 |
| Ar-60 | Ar-61 | Ar-62 |
| 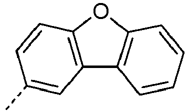 | 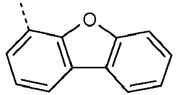 | 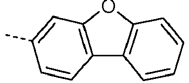 |
| Ar-63 | Ar-64 | Ar-65 |
| 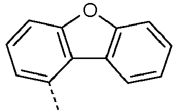 | 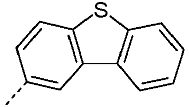 | 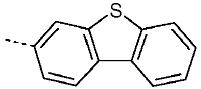 |
| Ar-66 | Ar-67 | Ar-68 |
| 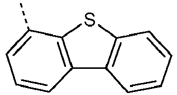 | 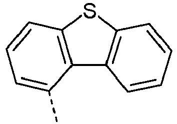 | 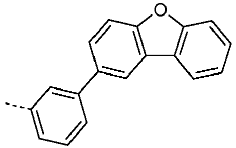 |
| Ar-69 | Ar-70 | Ar-71 |
| 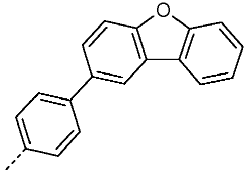 | 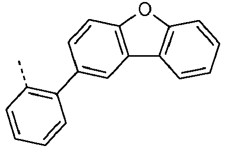 | 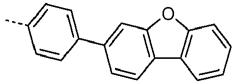 |
| Ar-72 | Ar-73 | Ar-74 |
| 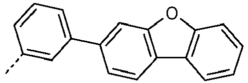 | 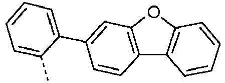 | 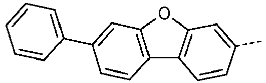 |
| Ar-75 | Ar-76 | Ar-77 |
| 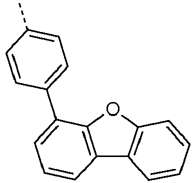 | 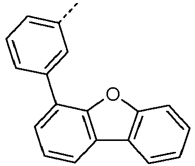 | 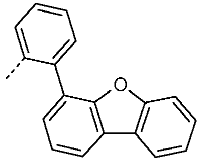 |
| Ar-78 | Ar-79 | Ar-80 |
| 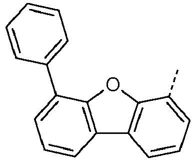 | 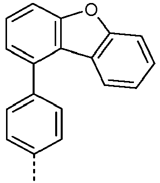 | 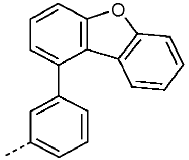 |
| Ar-81 | Ar-82 | Ar-83 |
| 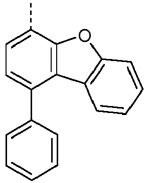 | 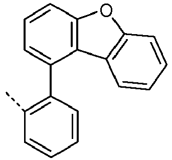 | 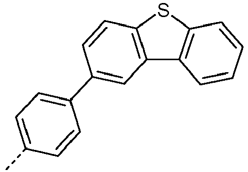 |
| Ar-84 | Ar-85 | Ar-86 |
| 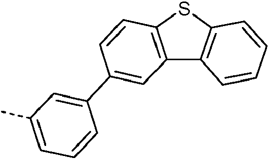 | 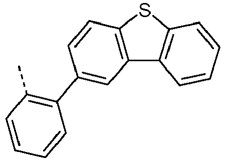 | 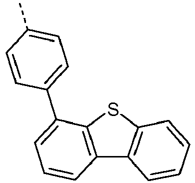 |
| Ar-87 | Ar-88 | Ar-89 |
| 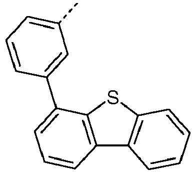 | 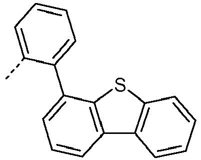 | 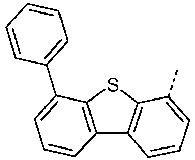 |
| Ar-90 | Ar-91 | Ar-92 |
| 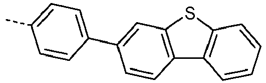 | 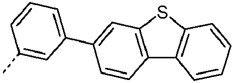 | 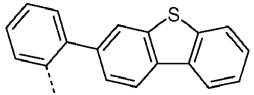 |
| Ar-93 | Ar-94 | Ar-95 |
| 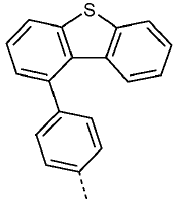 | 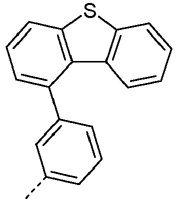 | 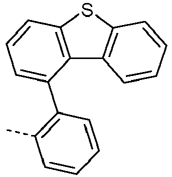 |
| Ar-96 | Ar-97 | Ar-98 |
| 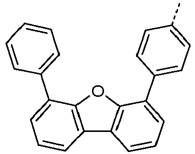 | 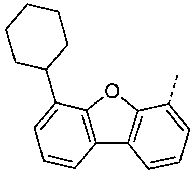 | 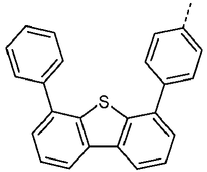 |
| Ar-99 | Ar-100 | Ar-101 |
| 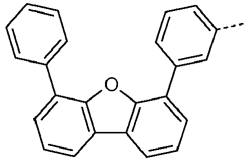 | 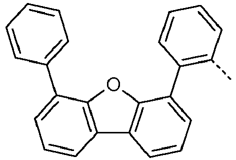 | 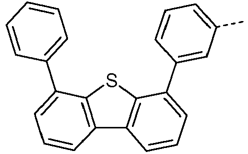 |
| Ar-102 | Ar-103 | Ar-104 |
| 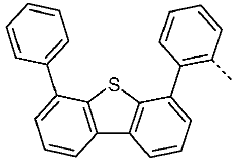 | | |
| Ar-105 | | |
| 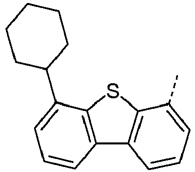 | 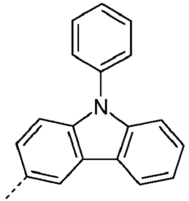 | 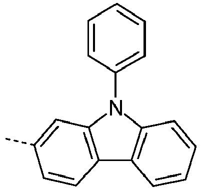 |
| Ar-106 | Ar-107 | Ar-108 |
| 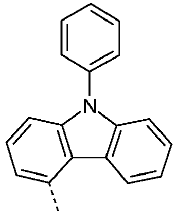 | 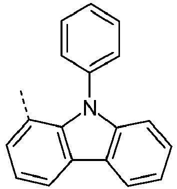 | 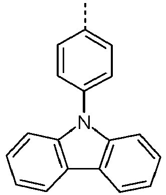 |
| Ar-109 | Ar-110 | Ar-111 |
| 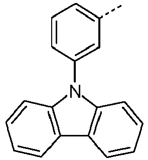 | 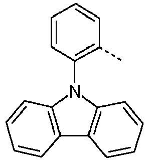 | |
| Ar-112 | Ar -113 | |
| 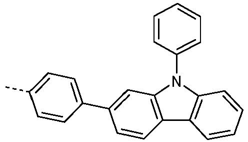 | 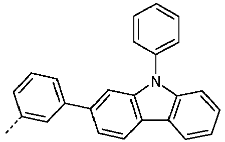 | 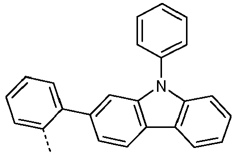 |
| Ar-114 | Ar-115 | Ar-116 |
| 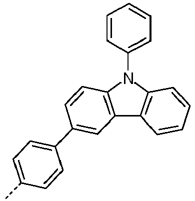 | 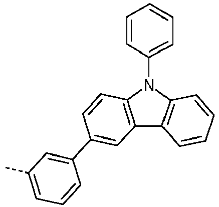 | 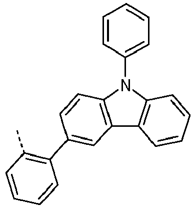 |
| Ar-117 | Ar-118 | Ar-119 |
| 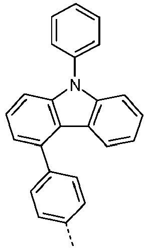 | 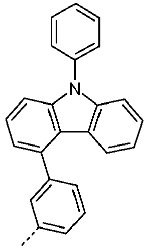 | 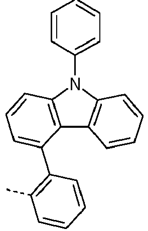 |
| Ar-120 | Ar-121 | Ar-122 |
| 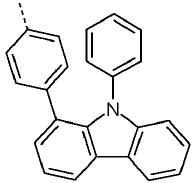 | 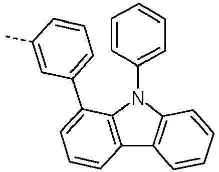 | 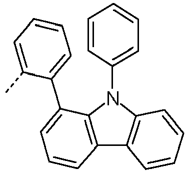 |
| Ar-123 | Ar-124 | Ar-125 |
| 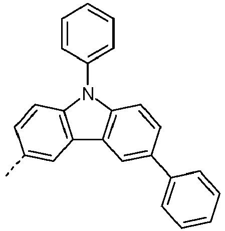 | 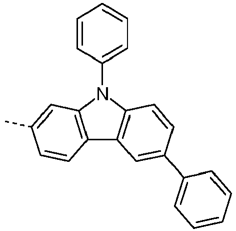 | 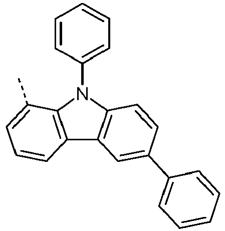 |
| Ar-126 | Ar-127 | Ar-128 |
| 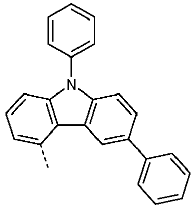 | 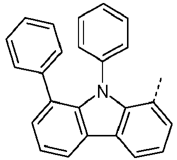 | 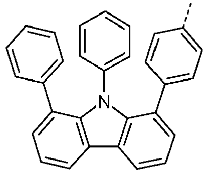 |
| Ar-129 | Ar-130 | Ar-131 |
| 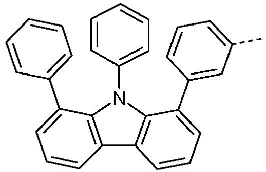 | 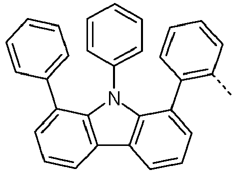 | |
| Ar-132 | Ar-133 | |
| 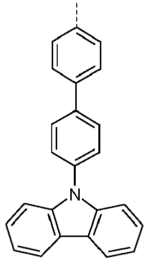 | 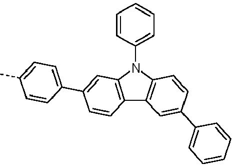 | 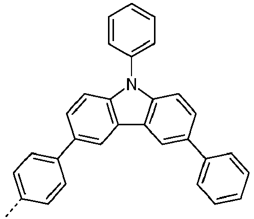 |
| Ar-134 | Ar-135 | Ar-136 |
| 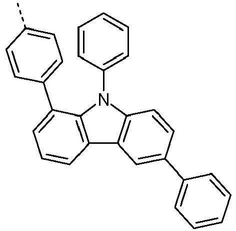 | 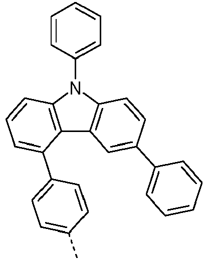 | 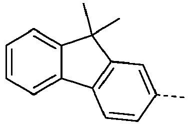 |
| Ar-137 | Ar-138 | Ar-139 |
| 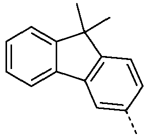 | 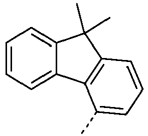 | 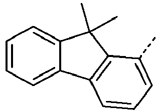 |
| Ar-140 | Ar-141 | Ar-142 |
| 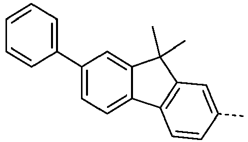 | 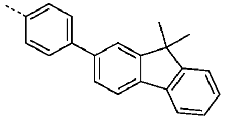 | 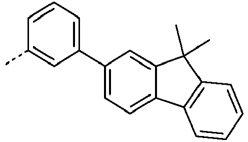 |
| Ar-143 | Ar-144 | Ar-145 |
| 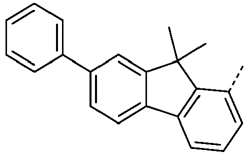 | 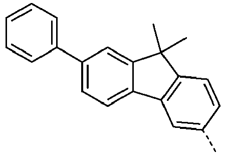 | 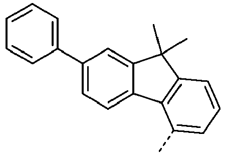 |
| Ar-146 | Ar-147 | Ar-148 |
| 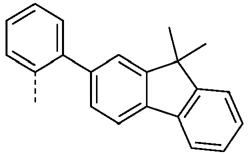 | 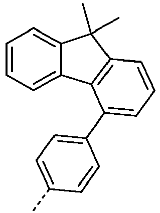 | 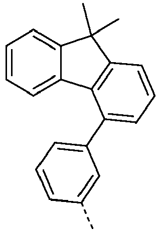 |
| Ar-149 | Ar-150 | Ar-151 |
| 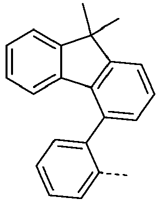 | 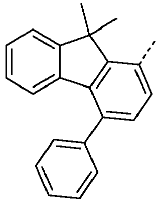 | 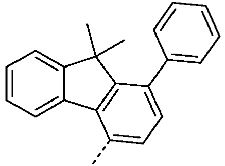 |
| Ar-152 | Ar-153 | Ar-154 |
| 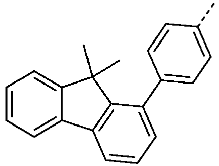 | 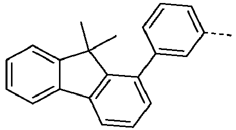 | 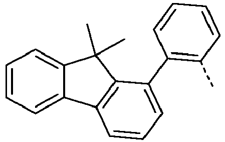 |
| Ar-155 | Ar-156 | Ar-157 |
| 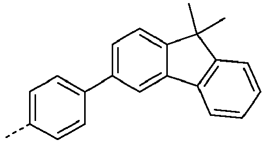 | 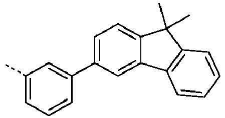 | 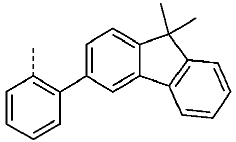 |
| Ar-158 | Ar-159 | Ar-160 |
| 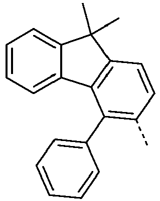 | 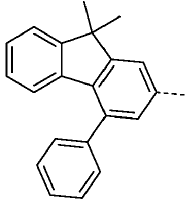 | 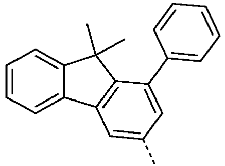 |
| Ar-161 | Ar-162 | Ar-163 |
| 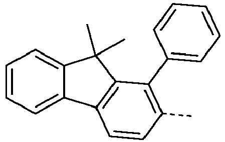 | 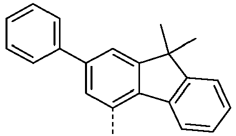 | 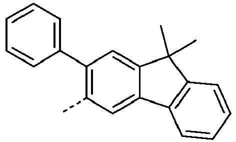 |
| Ar-164 | Ar-165 | Ar-166 |
| 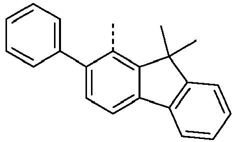 | 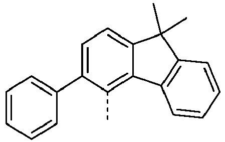 | 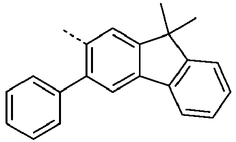 |
| Ar-167 | Ar-168 | Ar-169 |
| 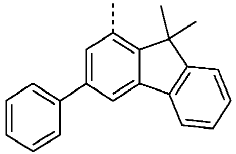 | 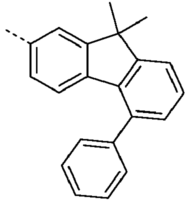 | 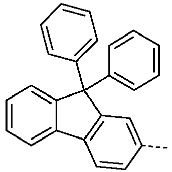 |
| Ar-170 | Ar-171 | Ar-172 |
| 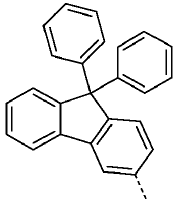 | 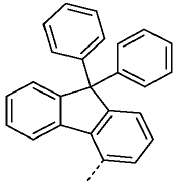 | 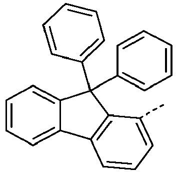 |
| Ar-173 | Ar-174 | Ar-175 |
| 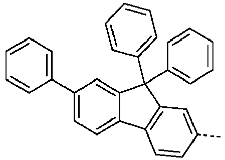 | 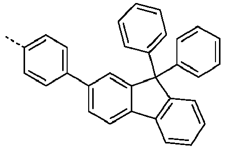 | 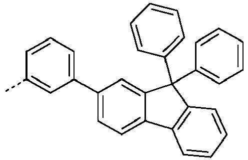 |
| Ar-176 | Ar-177 | Ar-178 |
| 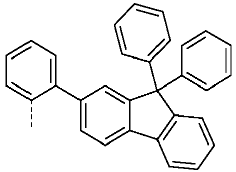 | 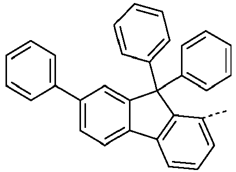 | 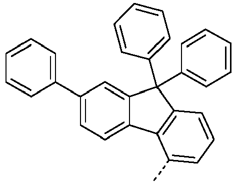 |
| Ar-179 | Ar-180 | Ar-181 |
| 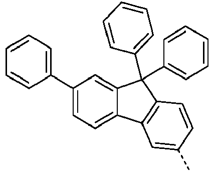 | 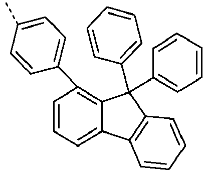 | 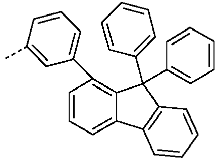 |
| Ar-182 | Ar-183 | Ar-184 |
| 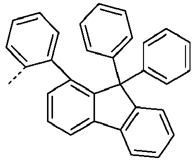 | 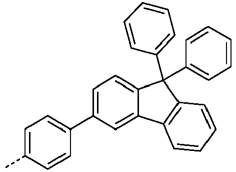 | 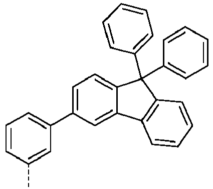 |
| Ar-185 | Ar-186 | Ar-187 |
| 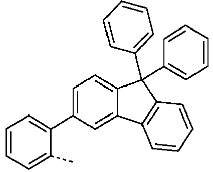 | 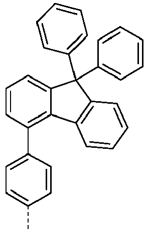 | 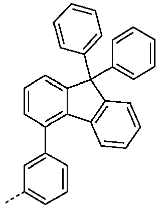 |
| Ar-188 | Ar-189 | Ar-190 |
| 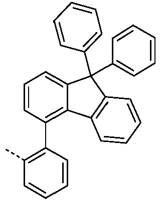 | | |
| | Ar-191 | |
| 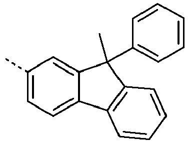 | 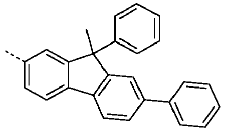 | 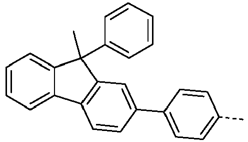 |
| Ar-192 | Ar-193 | Ar-194 |
| 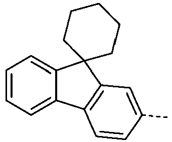 | 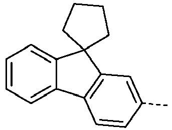 | 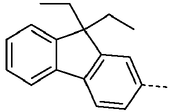 |
| Ar-195 | Ar-196 | Ar-197 |
| 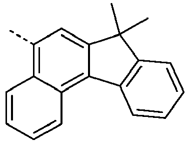 | 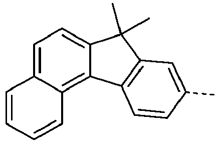 | 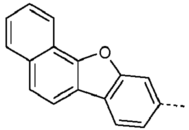 |
| Ar-198 | Ar-199 | Ar-200 |
| 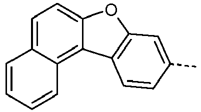 | 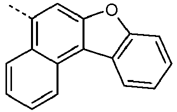 | 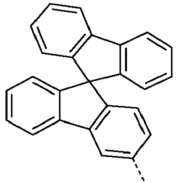 |
| Ar-201 | Ar-202 | Ar-203 |
| 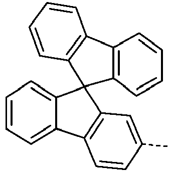 | 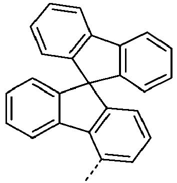 | 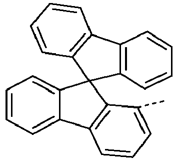 |
| Ar-204 | Ar-205 | Ar-206 |
| 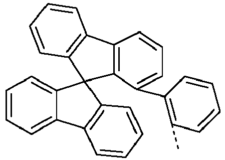 | 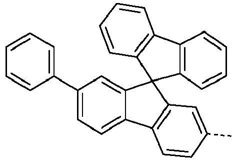 | 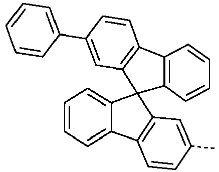 |
| Ar-207 | Ar-208 | Ar-209 |
| 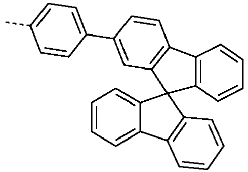 | 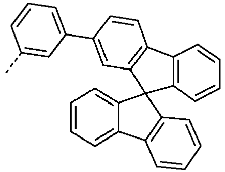 | 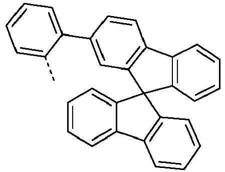 |
| Ar-210 | Ar-211 | Ar-212 |
| 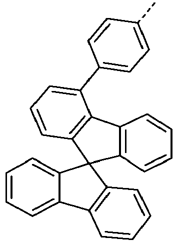 | 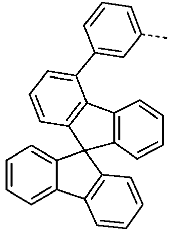 | 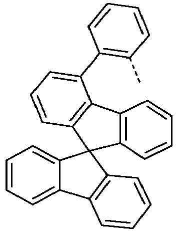 |
| Ar-213 | Ar-214 | Ar-215 |
| 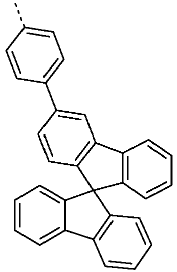 | 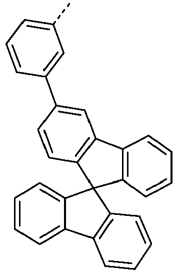 | 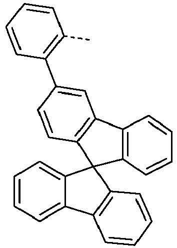 |
| Ar-216 | Ar-217 | Ar-218 |
| 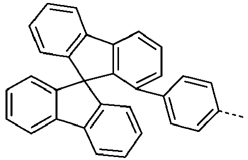 | 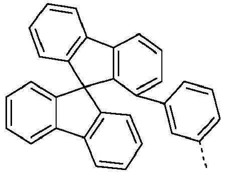 | 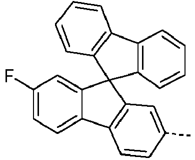 |
| Ar-219 | Ar-220 | Ar-221 |
| 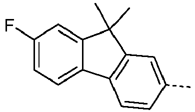 | 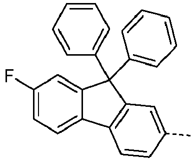 | 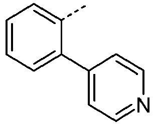 |
| Ar-222 | Ar-223 | Ar-224 |
| 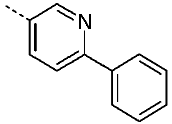 | 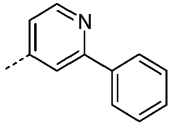 | 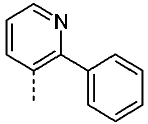 |
| Ar-225 | Ar-226 | Ar-227 |
| 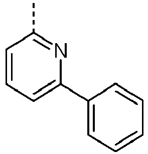 | 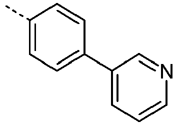 | 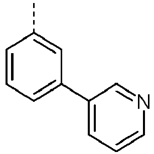 |
| Ar-228 | Ar-229 | Ar-230 |
| 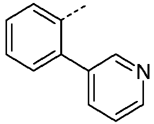 | 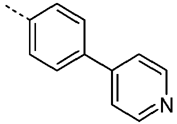 | 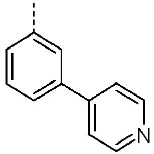 |
| Ar-231 | Ar-232 | Ar-233 |
| 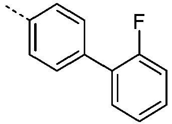 | 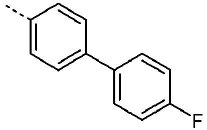 | 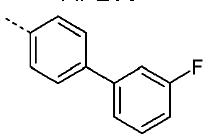 |
| Ar-234 | Ar-235 | Ar-236 |
| 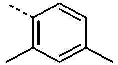 | 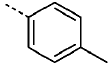 | 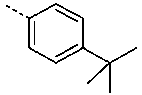 |
| Ar-237 | Ar-238 | Ar-239 |
| 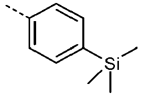 | 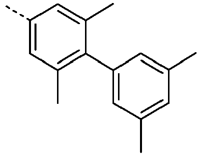 | 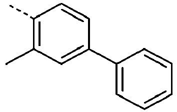 |
| Ar-240 | Ar-241 | Ar-242 |
| 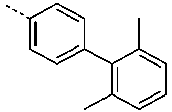 | 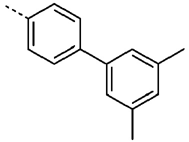 | 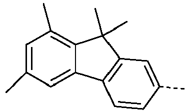 |
| Ar-243 | Ar-244 | Ar-245 |
| 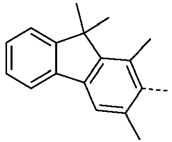 | 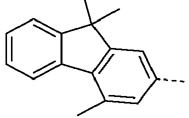 | |
| Ar-246 | Ar-247 | |
| 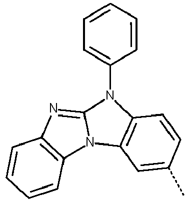 | 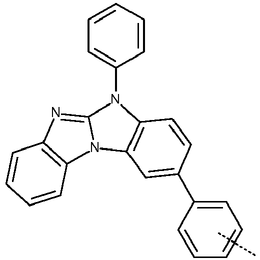 | |
| Ar-248 | Ar-249 | |
| 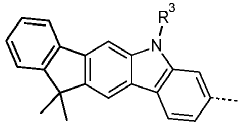 | 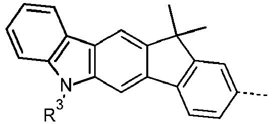 | |
| Ar-250 | Ar-251 | |
| 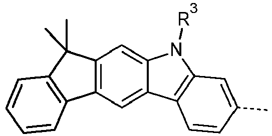 | 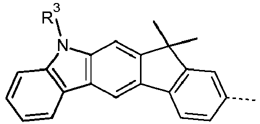 | |
| Ar-252 | Ar-253 | |
| 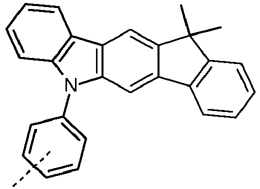 | 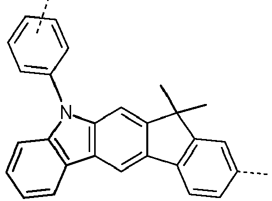 | |
| Ar-254 | Ar-255 | |
| 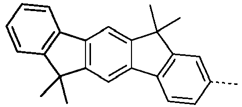 | | |
| Ar-256 | | |

where the groups (Ar-1) to (Ar-256) might be further substituted at any free position by a radical R.

Preferably, the radicals R^{A}, R^{X} and R^{Z} stand on each occurrence, identically or differently, for H, D, F, a straight-chain alkyl group having 1 to 20 C atoms or a branched or cyclic alkyl group having 3 to 20 C atoms, each of which may be substituted by one or more radicals R, where in each case one or more non-adjacent CH₂ groups may be replaced by RC=CR, C=C, O or S and where one or more H atoms may be replaced by D, F, Cl or CN, an aromatic or heteroaromatic ring system having 5 to 40 aromatic ring atoms, which may in each case be substituted by one or more radicals R; where two adjacent radicals R^{X}, two adjacent radicals R^{Z}, two adjacent radicals R^{A} may form a mono- or polycyclic, aliphatic ring system or aromatic ring system, which may be substituted by one or more radicals R.

When two adjacent radicals R^{X} or two adjacent radicals R^{Z} form a ring, they preferably formed an aromatic or heteroaromatic ring, which is condensed to the ring to which the two adjacent radicals R^{X} or R^{Z} are bonded. Preferably, the formed ring is a benzene ring or a pyridine ring, most preferably a benzene ring. In each case, the formed ring may be substituted by one or more radicals R.

When two adjacent radicals R^{A} form a ring, they preferably formed an aliphatic ring system, which may be substituted by one or more radicals R.

Preferably, the group R^{X} stands on each occurrence, identically or differently, for H, a straight-chain alkyl group having 1 to 20 C atoms or a branched alkyl group having 3 to 20 C atoms, each of which may be substituted by one or more radicals R, or an aromatic ring system having 6 to 40, preferably 6 to 30, more preferably 6 to 18 aromatic ring atoms, which may in each case be substituted by one or more radicals R, or an heteroaromatic ring system having 5 to 40, preferably 5 to 30, more preferably 5 to 18 aromatic ring atoms, which may in each case be substituted by one or more radicals R; where two adjacent radicals R^{X} may form a mono- or polycyclic, aliphatic ring system or aromatic ring system, which may be substituted by one or more radicals R. More preferably, the group R^{X} stands on each occurrence, identically or differently, for H, a straight-chain alkyl group having 1 to 10 C atoms or a branched alkyl group having 3 to 10 C atoms, each of which may be substituted by one or more radicals R, an aromatic ring system having 6 to 18 aromatic ring atoms or an heteroaromatic ring system having 5 to 18 aromatic ring atoms, each of which may in each case be substituted by one or more radicals R.

Preferably, the group R^{Z} stands on each occurrence, identically or differently, for H, a straight-chain alkyl group having 1 to 20 C atoms or a branched alkyl group having 3 to 20 C atoms, each of which may be substituted by one or more radicals R, or an or an aromatic ring system having 6 to 40, preferably 6 to 30, more preferably 6 to 18 aromatic ring atoms, which may in each case be substituted by one or more radicals R, or an heteroaromatic ring system having 5 to 40, preferably 5 to 30, more preferably 5 to 18 aromatic ring atoms, which may in each case be substituted by one or more radicals R; where two adjacent radicals R^{Z} may form a mono- or polycyclic, aliphatic ring system or aromatic ring system, which may be substituted by one or more radicals R. More preferably, the group R^{Z} stands on each occurrence, identically or differently, for H, a straight-chain alkyl group having 1 to 10 C atoms or a branched alkyl group having 3 to 10 C atoms, each of which may be substituted by one or more radicals R, an aromatic ring system having 6 to 18 aromatic ring atoms or an heteroaromatic ring system having 5 to 18 aromatic ring atoms, each of which may in each case be substituted by one or more radicals R.

Preferably, the group R^{A} stands on each occurrence, identically or differently, for H, a straight-chain alkyl group having 1 to 20 C atoms or a branched alkyl group having 3 to 20 C atoms, each of which may be substituted by one or more radicals R, or an or an aromatic ring system having 6 to 40, preferably 6 to 30, more preferably 6 to 18 aromatic ring atoms, which may in each case be substituted by one or more radicals R, or an heteroaromatic ring system having 5 to 40, preferably 5 to 30, more preferably 5 to 18 aromatic ring atoms, which may in each case be substituted by one or more radicals R; where two adjacent radicals R^{A} may form a mono- or polycyclic, aliphatic ring system or aromatic ring system, which may be substituted by one or more radicals R. More preferably, the group R^{A} stands on each occurrence, identically or differently, for H, a straight-chain alkyl group having 1 to 10 C atoms or a branched alkyl group having 3 to 10 C atoms, each of which may be substituted by one or more radicals R, an aromatic ring system having 6 to 18 aromatic ring atoms or an heteroaromatic ring system having 5 to 18 aromatic ring atoms, each of which may in each case be substituted by one or more radicals R. Particularly preferably, the group R^{A} stands on each occurrence, identically or differently, for H, a straight-chain alkyl group having 1 to 6 C atoms, each of which may be substituted by one or more radicals R or an aromatic ring system selected from phenyl, biphenyl, naphthyl, each of which may in each case be substituted by one or more radicals R.

Preferably, R stands on each occurrence, identically or differently, for H, D, F, CN, a straight-chain alkyl or alkoxy group having 1 to 20 C atoms or branched or cyclic alkyl or alkoxy group having 3 to 20 C atoms, each of which may be substituted by one or more radicals R', an aromatic or heteroaromatic ring systems having 5 to 40, preferably 5 to 30, more preferably 5 to 18 aromatic ring atoms, which may in each case be substituted by one or more radicals R', where two radicals R may form a ring system with one another, which may be substituted by one or more radicals R'. More preferably, R stands on each occurrence, identically or differently, for H, D, F, CN, a straight-chain alkyl group having 1 to 10 C atoms or branched or cyclic alkyl group having 3 to 10 C atoms, each of which may be substituted by one or more radicals R', an aromatic or heteroaromatic ring systems having 5 to 18 aromatic ring atoms, which may in each case be substituted by one or more radicals R'.

Preferably, Ar is an aromatic or heteroaromatic ring system having 5 to 18 aromatic ring atoms, which may in each case also be substituted by one or more radicals R'.

Preferably, R' stands on each occurrence, identically or differently, for H, D, F, Cl, Br, I, CN, a straight-chain alkyl or alkoxy group having 1 to 20, preferably 1 to 10, more preferably 1 to 5 C atoms or branched or cyclic alkyl or alkoxy group having 3 to 20, preferably 1 to 10, more preferably 1 to 5 C atoms, where one or more H atoms may be replaced by D, F, Cl, Br or I, or an aromatic or heteroaromatic ring system having 5 to 24 C, preferably 5 to 18 C atoms.

Examples of suitable compounds according to the invention are the structures shown in the table below: (the symbol * indicate the compounds that are not according to the invention)

| | |
|---|---|
| 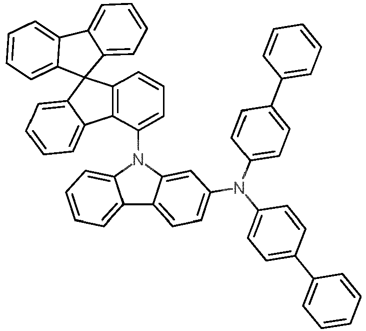 | 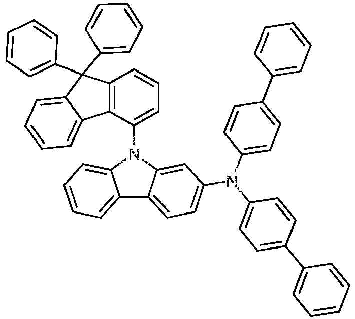 |
| 1 | 2 |
| 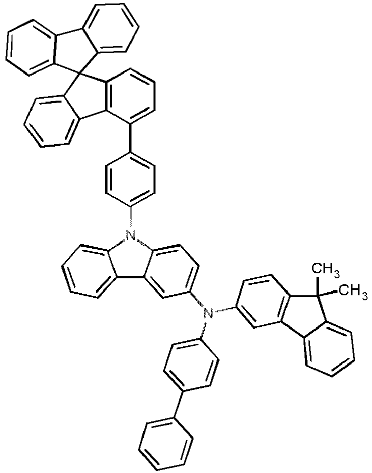 | 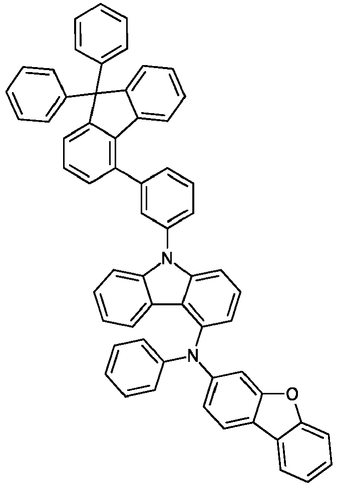 |
| 3 | 4 |
| 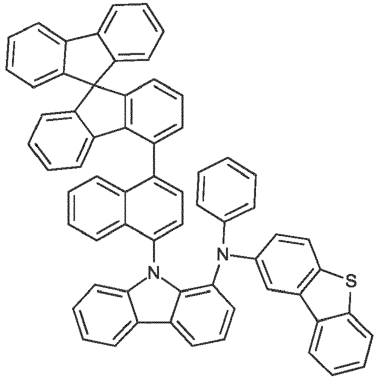 | 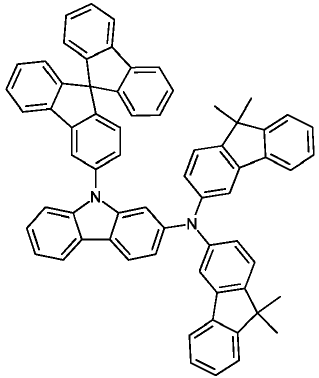 |
| 5 | 6 |
| 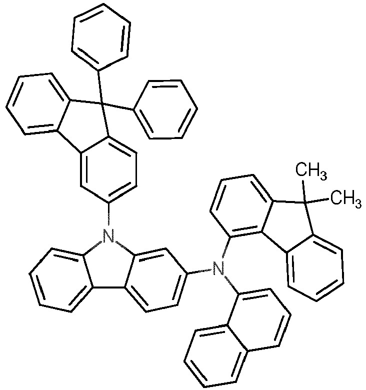 | 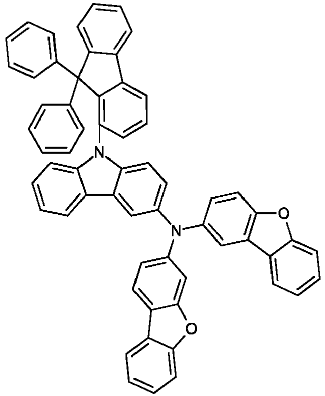 |
| 7 | 8 |
| 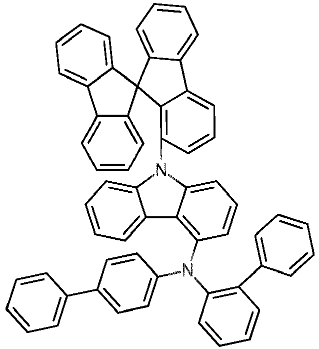 | 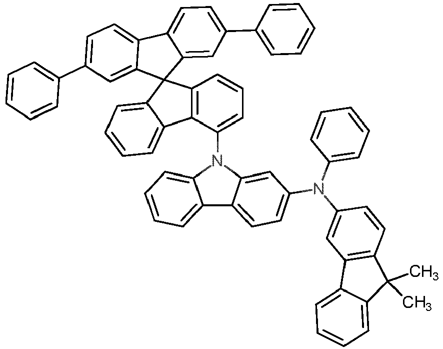 |
| 9 | 10 |
| 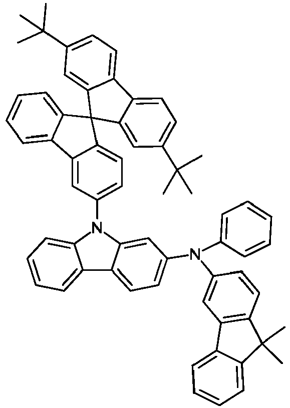 | 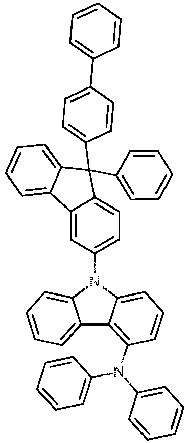 |
| 11 | 12 |
| 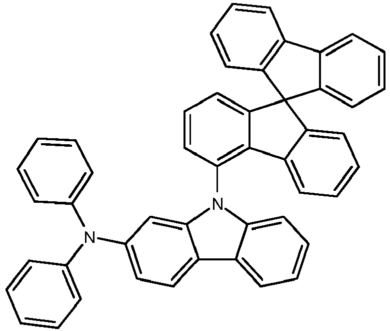 | 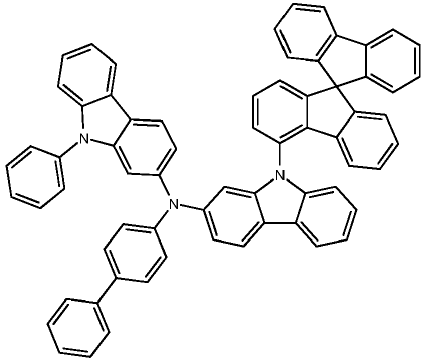 |
| 13 | 14 |
| 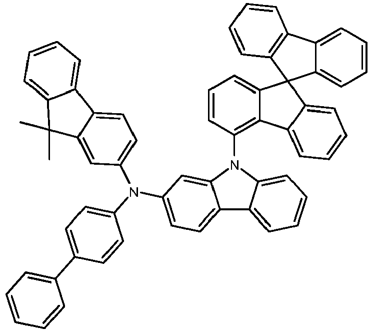 | 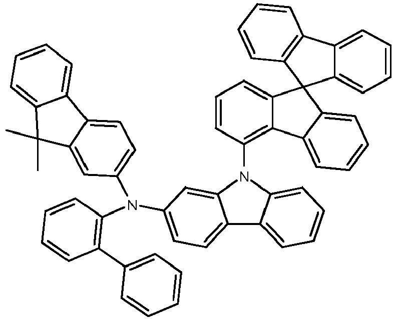 |
| 15 | 16 |
| 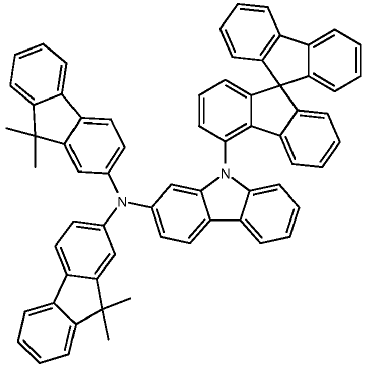 | 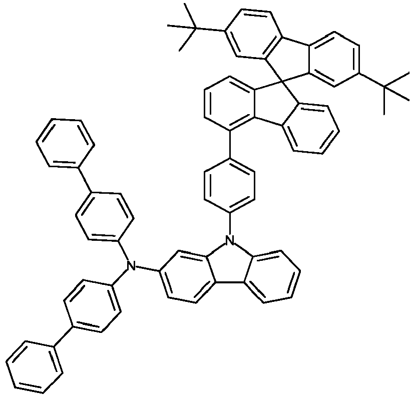 |
| 17 | 18 |
| 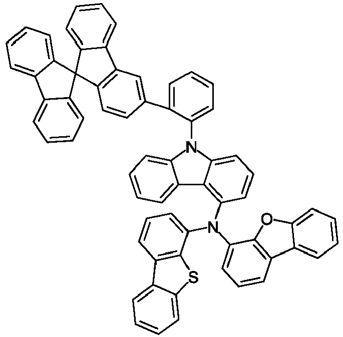 | 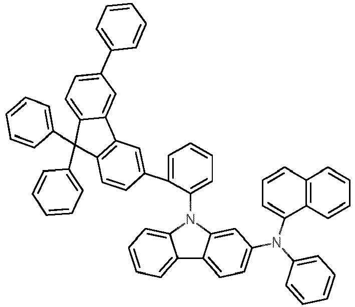 |
| 19 | 20 |
| 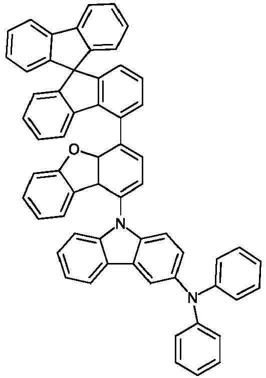 | 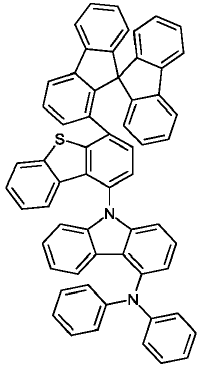 |
| 21 | 22 |
| 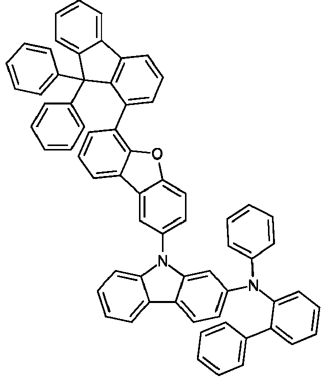 | 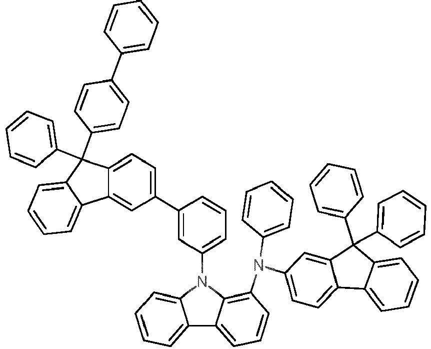 |
| 23 | 24 |
| 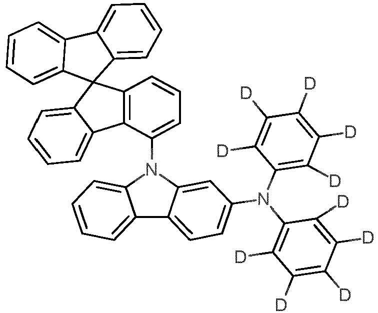 | 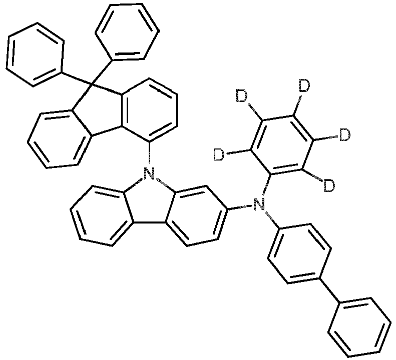 |
| 25 | 26 |
| 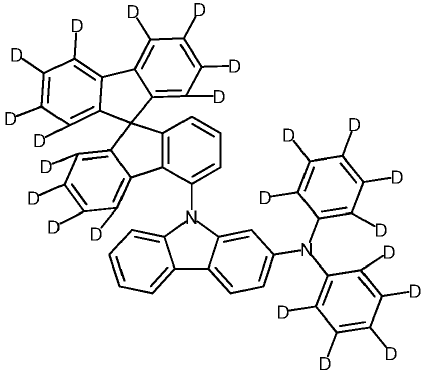 | 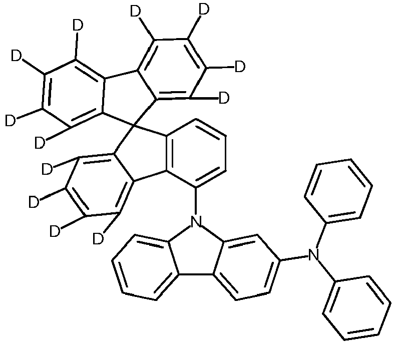 |
| 27 | 28 |
| 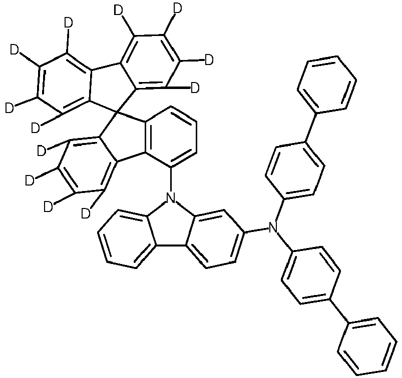 | 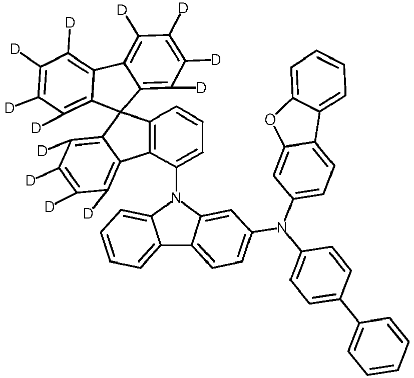 |
| 29 | 30 |
| 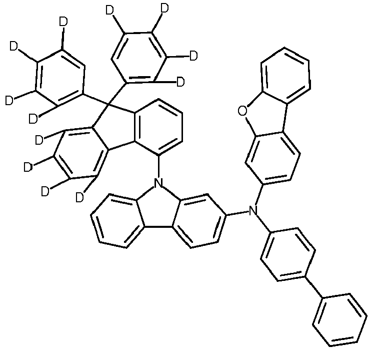 | 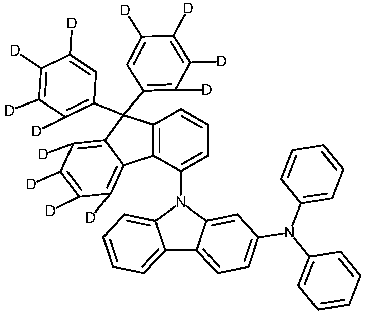 |
| 31 | 32 |
| 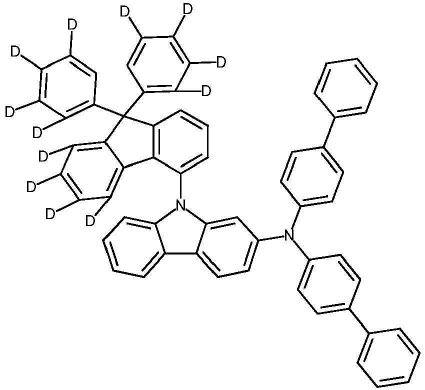 | 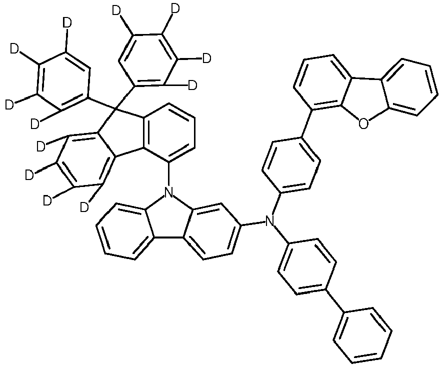 |
| 33 | 34 |
| 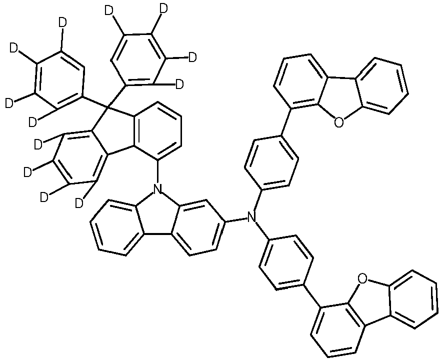 | 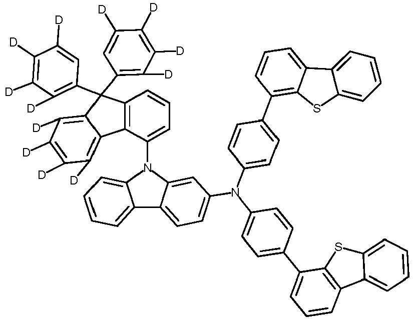 |
| 35 | 36 |
| 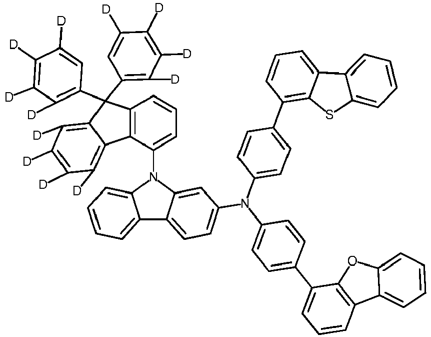 | 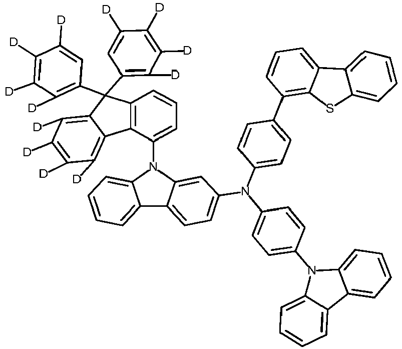 |
| 37 | 38 |
| 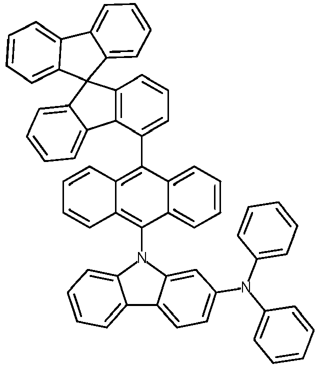 | 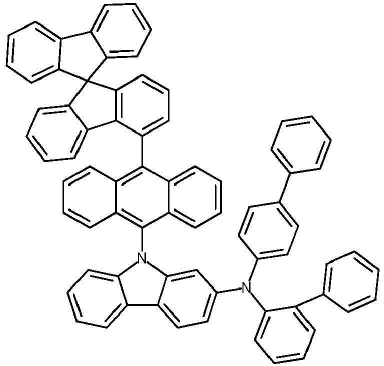 |
| 39 | 40 |
| 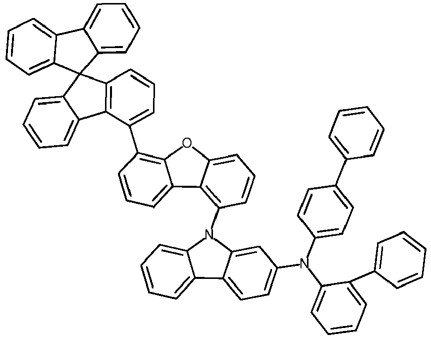 | 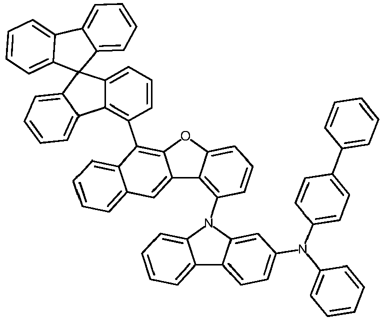 |
| 41 | 42 |
| 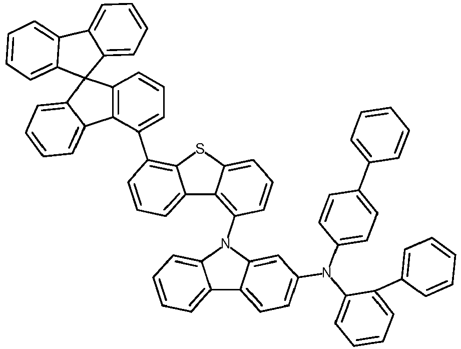 | 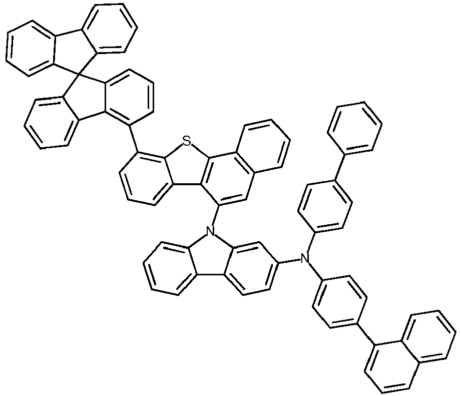 |
| 43 | 44 |
| 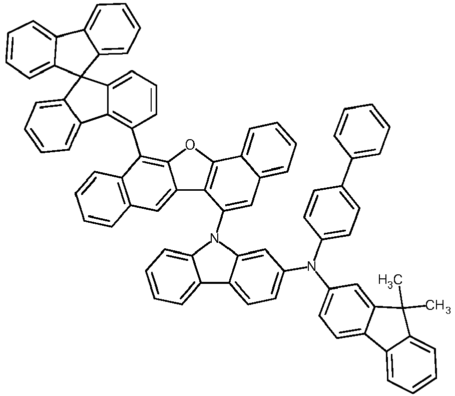 | 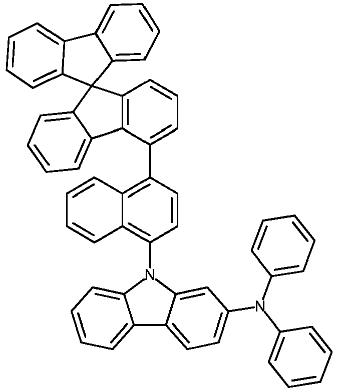 |
| 45 | 46 |
| 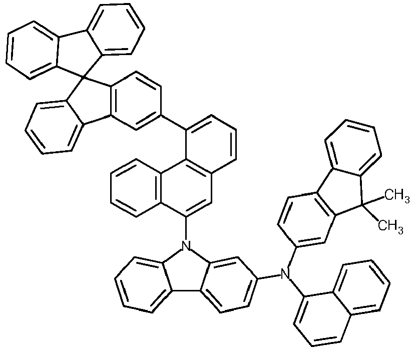 | 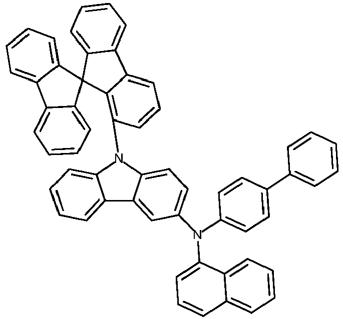 |
| 47 | 48 |
| 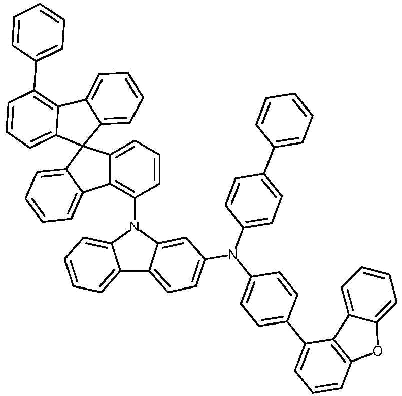 | 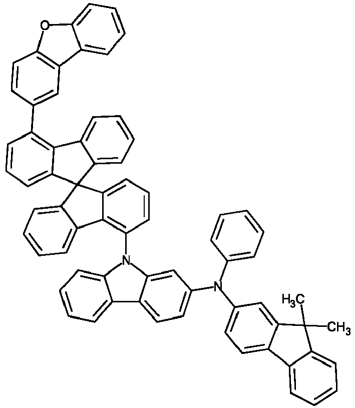 |
| 49 | 50 |
| 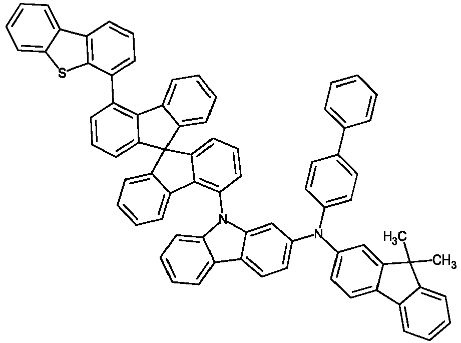 | 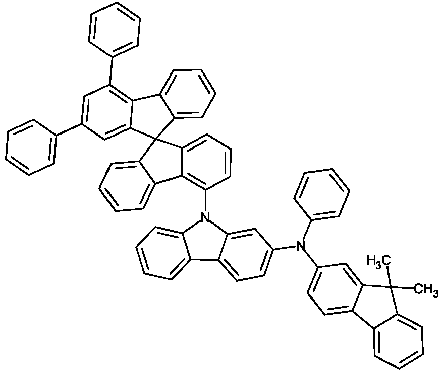 |
| 51 | 52 |
| 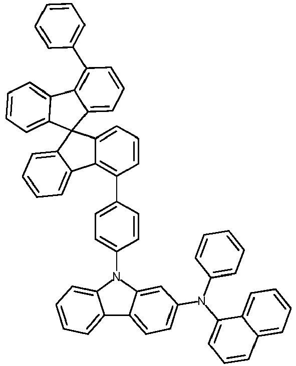 | 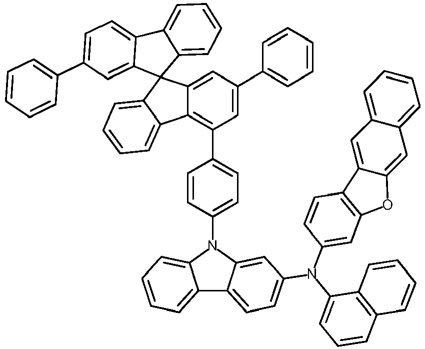 |
| 53 | 54 |
| 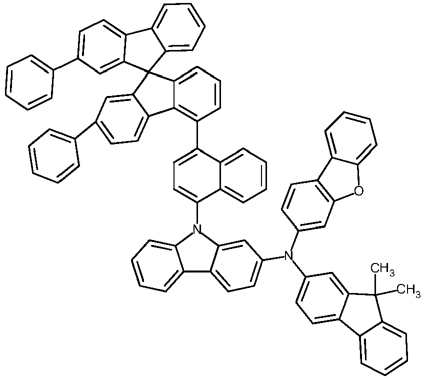 | 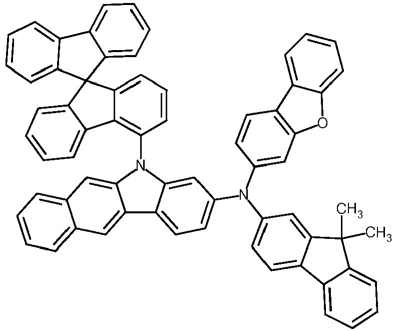 |
| 55 | 56 |
| 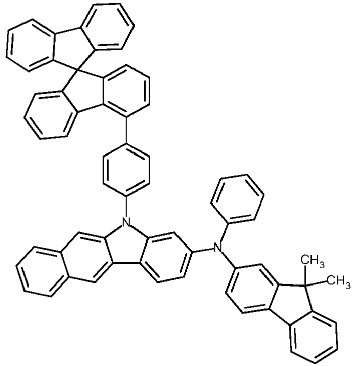 | 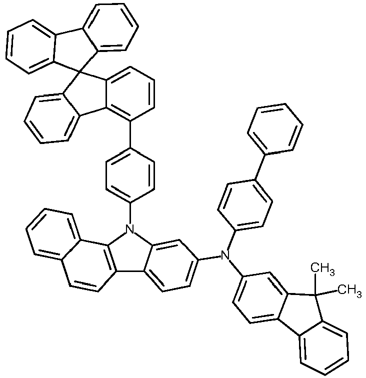 |
| 57 | 58 |
| 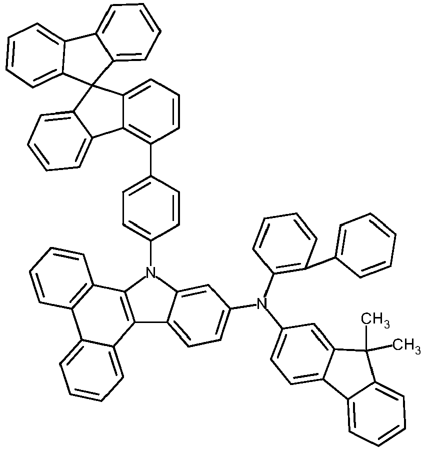 | 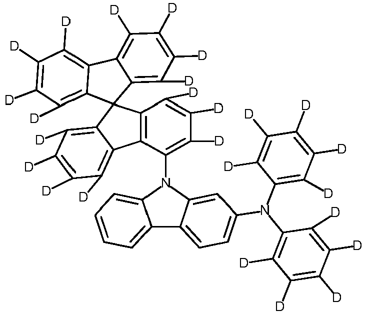 |
| 59 | 60 |
| 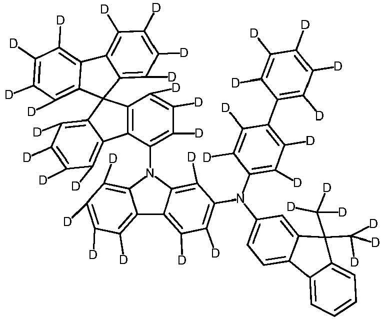 | 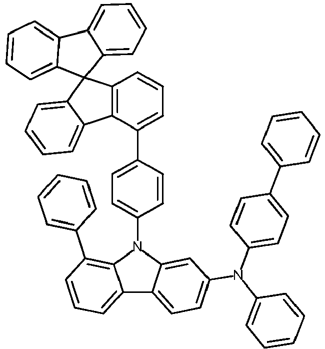 |
| 61 | 62 |
| 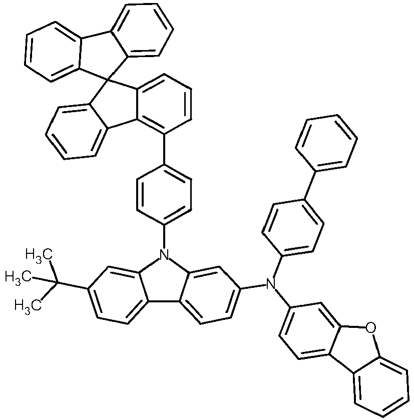 | 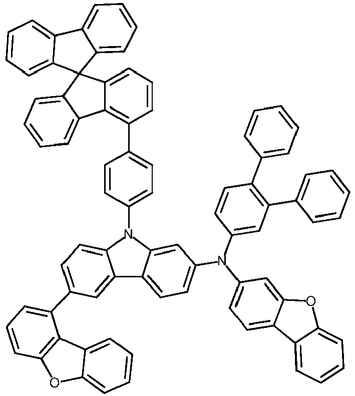 |
| 63 | 64 |
| 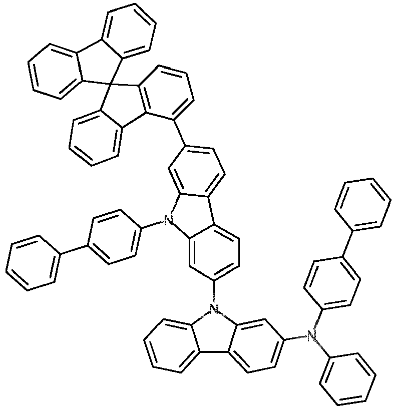 | 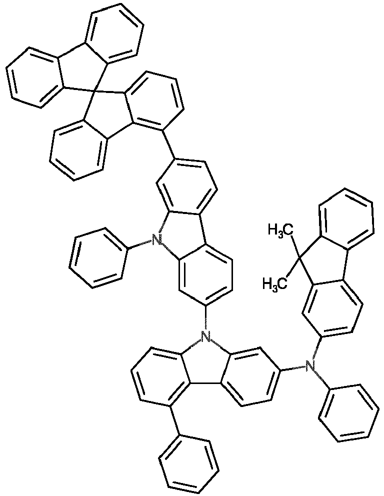 |
| 65 | 66 |
| 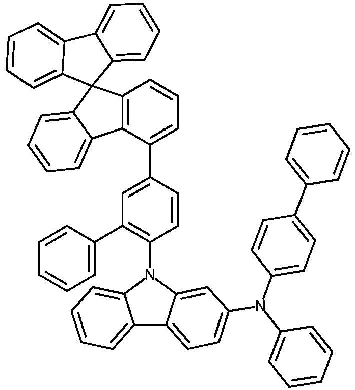 | 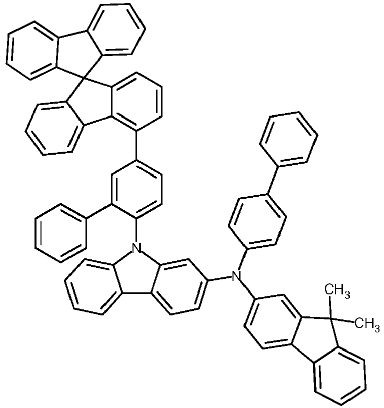 |
| 67 | 68 |
| 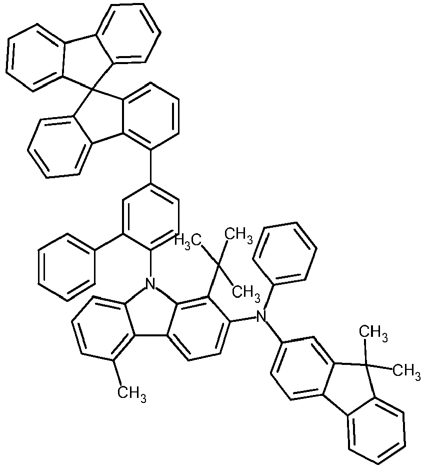 | 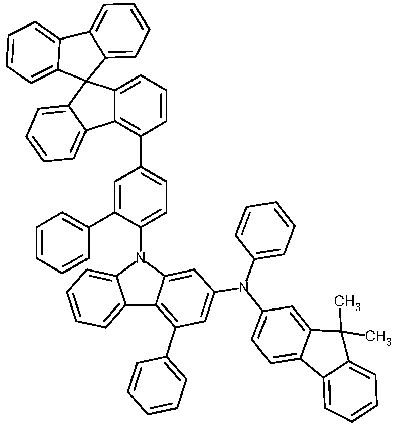 |
| 69 | 70 |
| 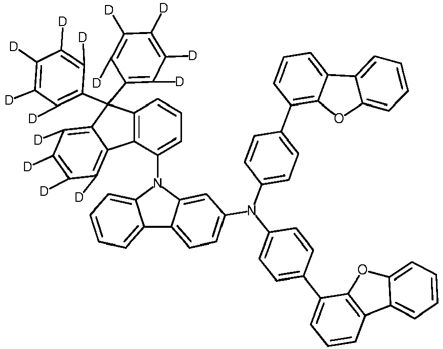 | 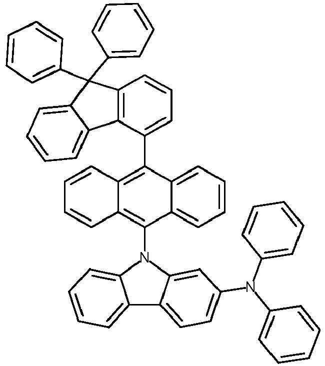 |
| 71 | 72 |
| 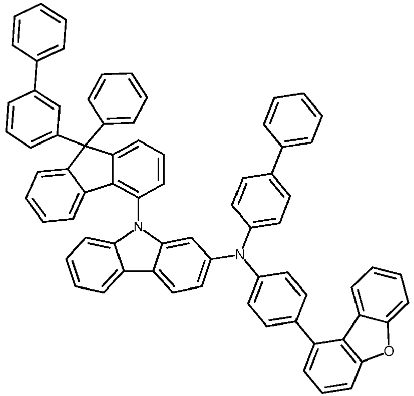 | 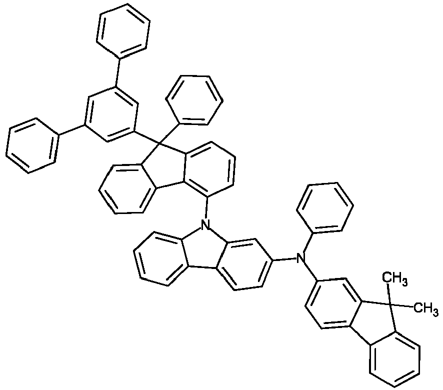 |
| 73 | 74 |
| 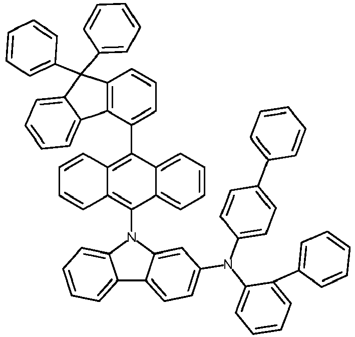 | 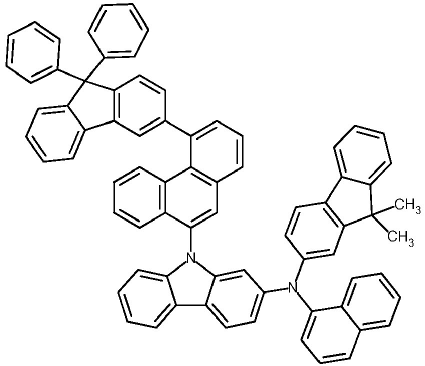 |
| 75 | 76 |
| 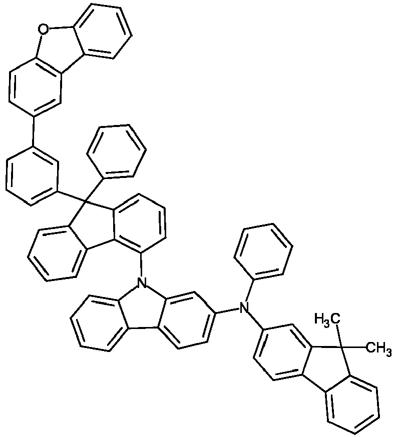 | 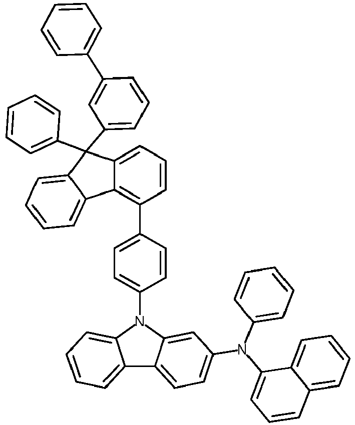 |
| 77 | 78 |
| 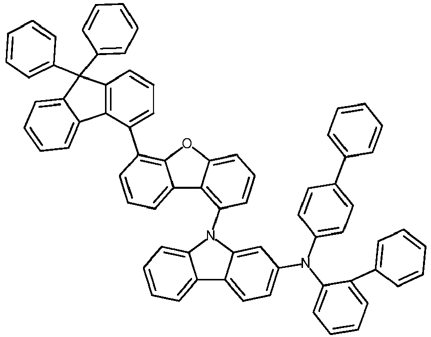 | 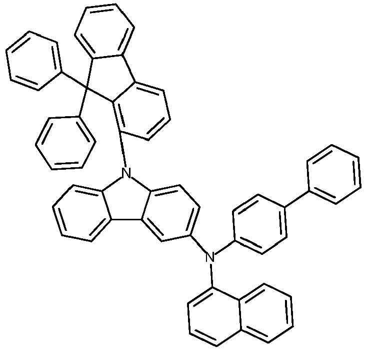 |
| 79 | 80 |
| 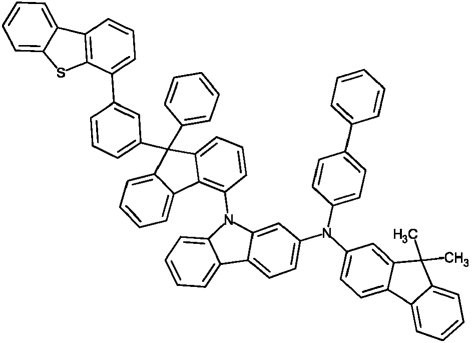 | 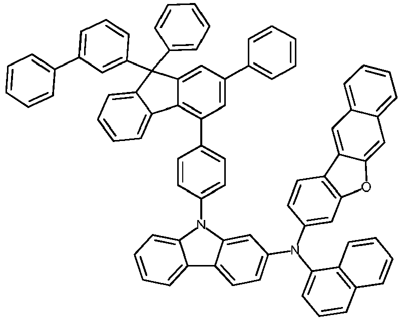 |
| 81 | 82 |
| 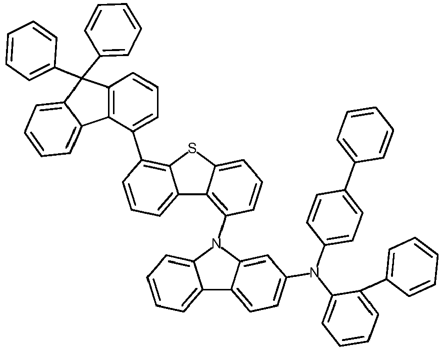 | 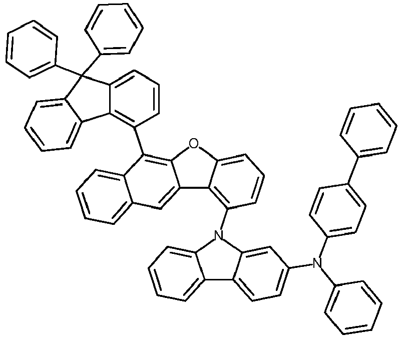 |
| 83 | 84 |
| 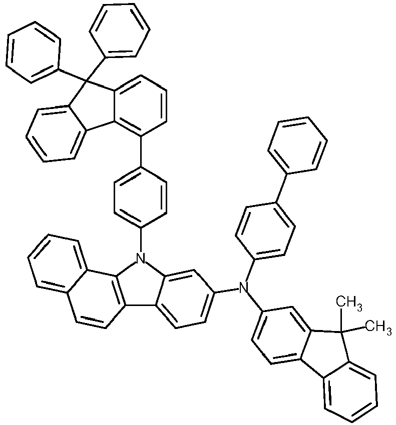 | 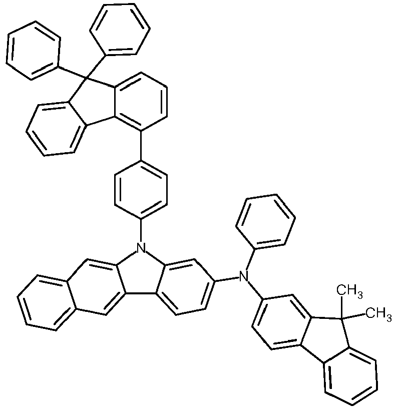 |
| 85 | 86 |
| 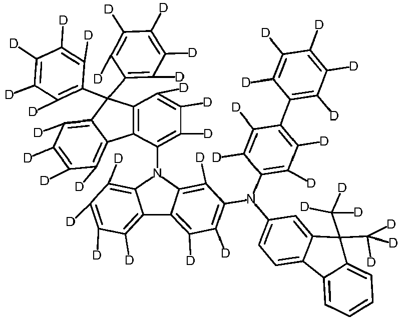 | 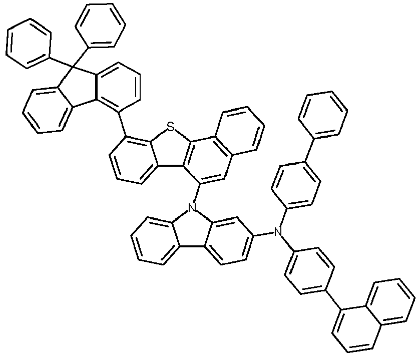 |
| 87 | 88 |
| 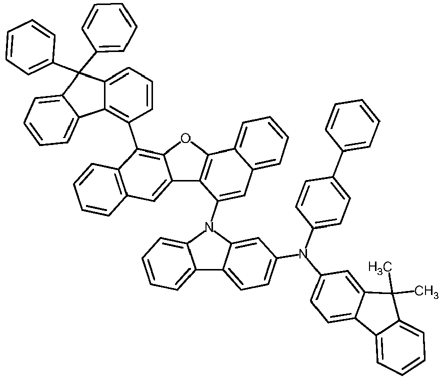 | 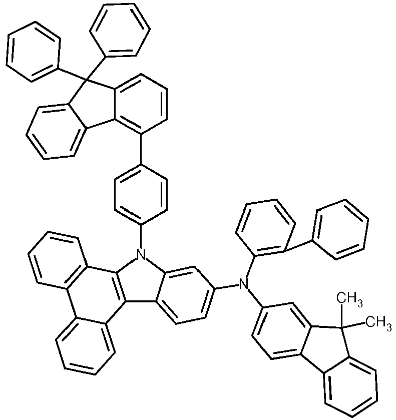 |
| 89 | 90 |
| 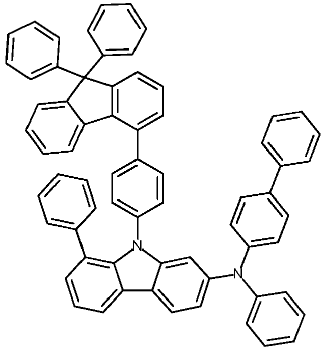 | 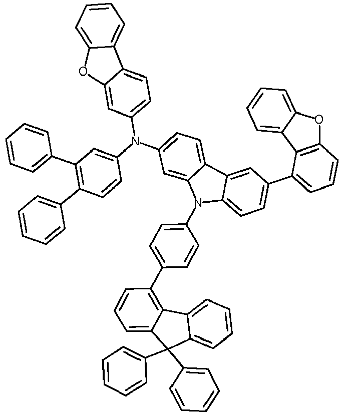 |
| 91 | 92 |
| 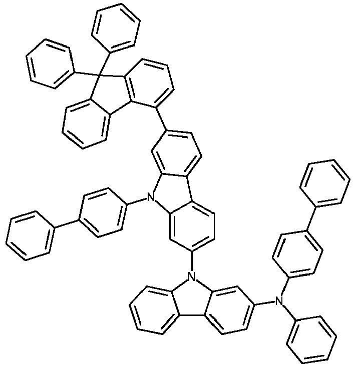 | 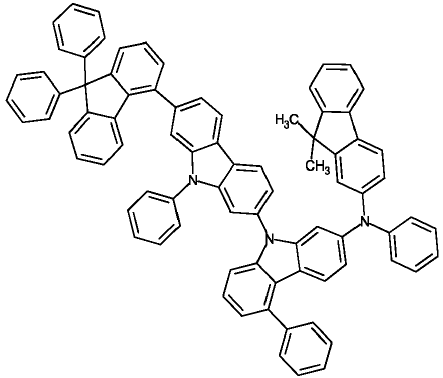 |
| 93 | 94 |
| 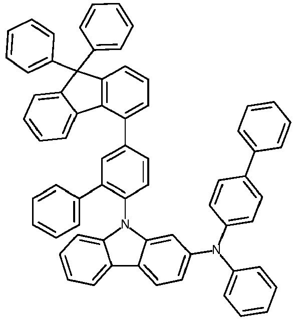 | 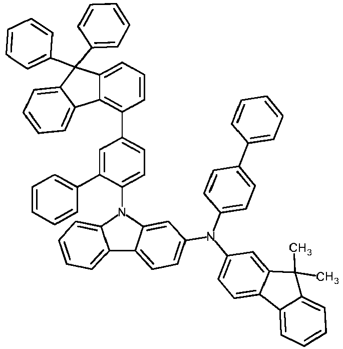 |
| 95 | 96 |
| 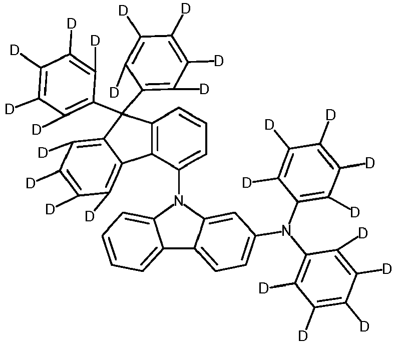 | 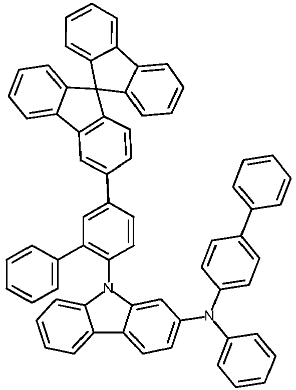 |
| 97 | 98 |
| 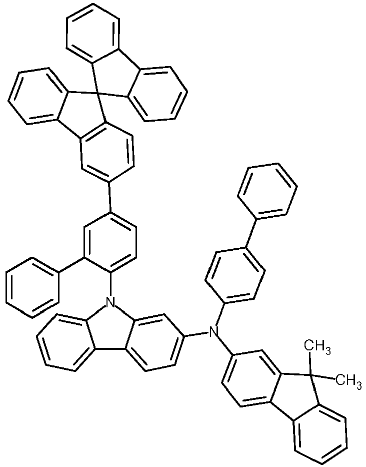 | 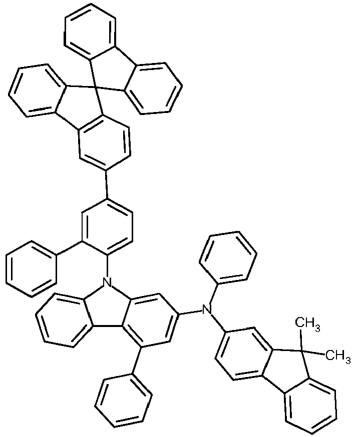 |
| 99 | 100 |
| 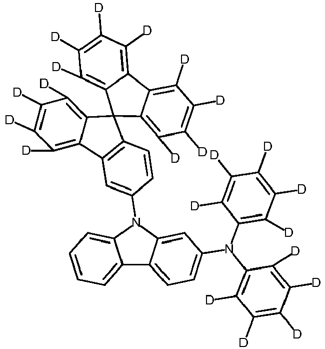 | 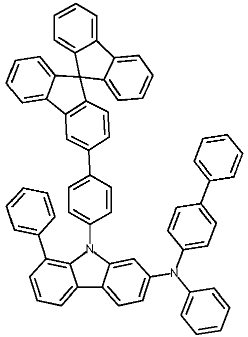 |
| 101 | 102 |
| 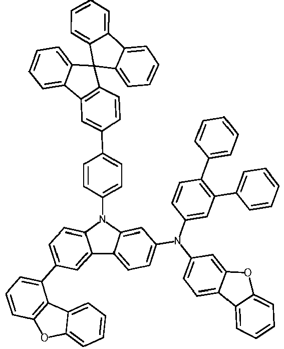 | 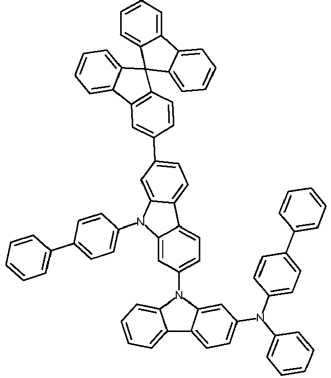 |
| 103 | 104 |
| 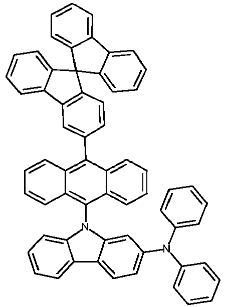 | 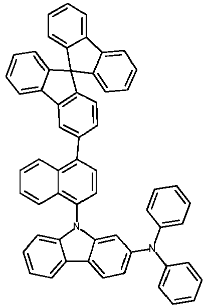 |
| 105 | 106 |
| 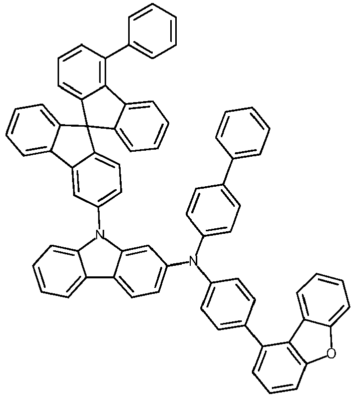 | 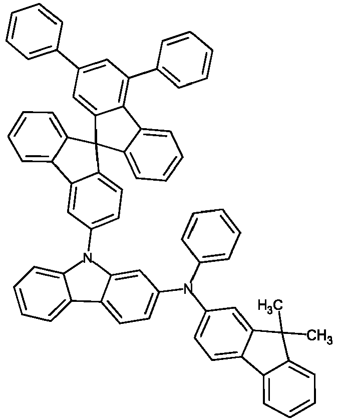 |
| 107 | 108 |
| 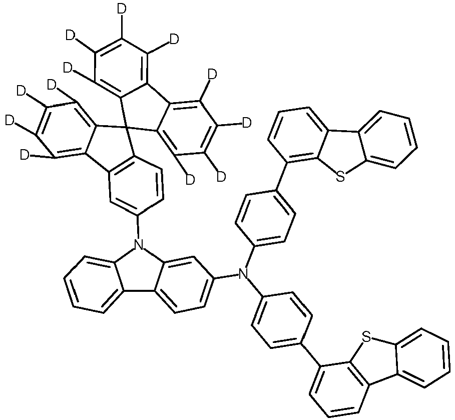 | 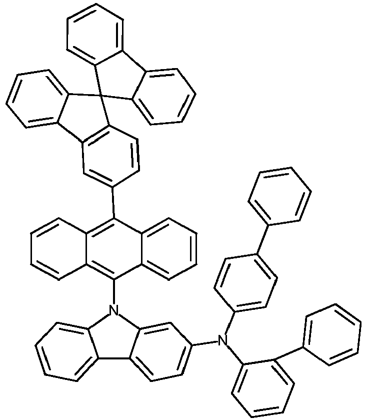 |
| 109 | 110 |
| 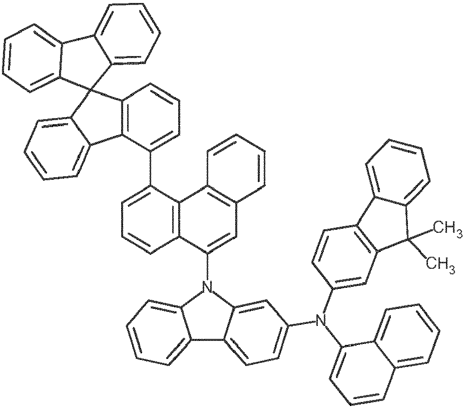 | 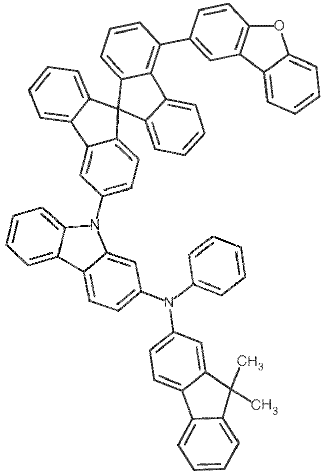 |
| 111 | 112 |
| 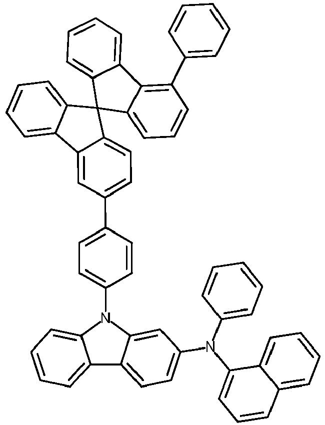 | 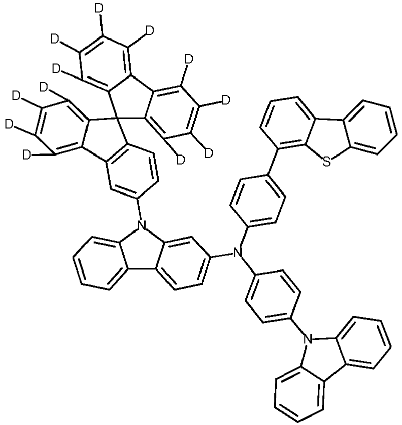 |
| 113 | 114 |
| 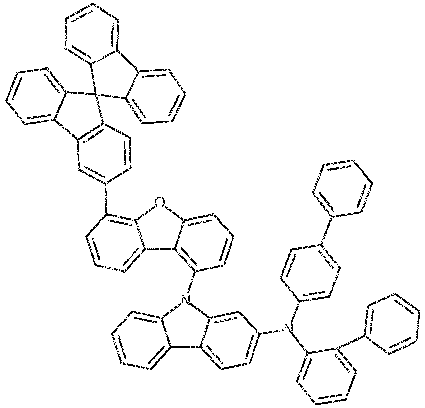 | 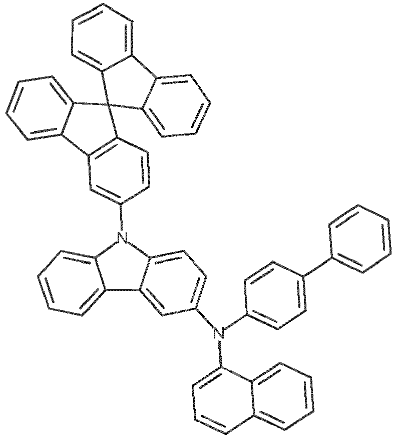 |
| 115 | 116 |
| 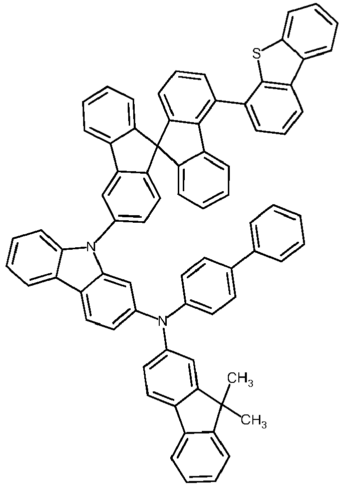 | 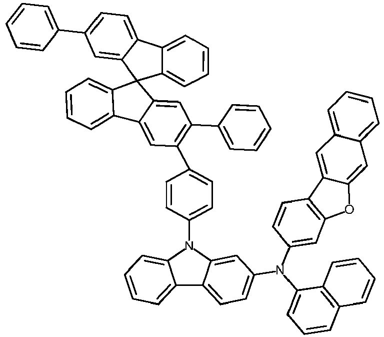 |
| 117 | 118 |
| 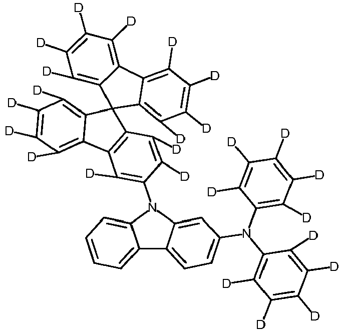 | 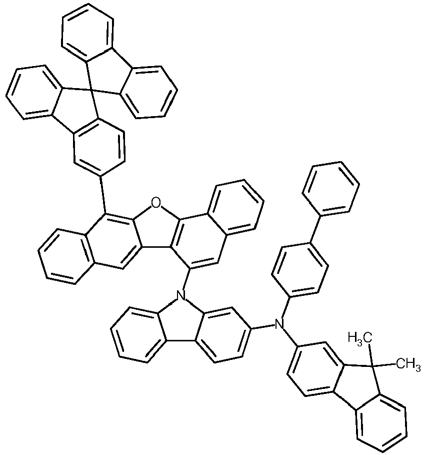 |
| 119 | 120 |
| 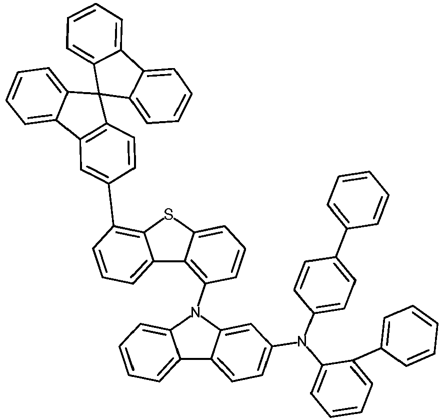 | 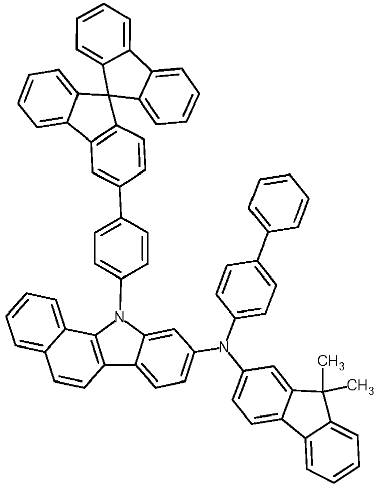 |
| 121 | 122 |
| 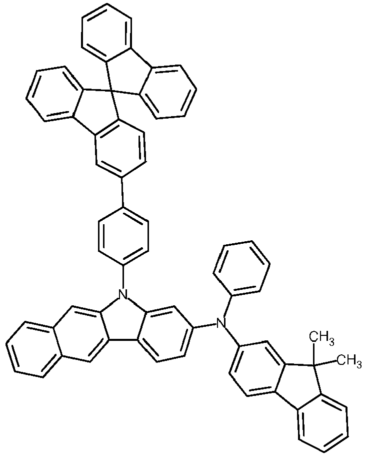 | 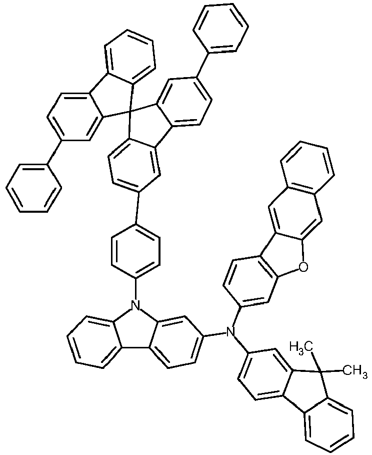 |
| 123 | 124 |
| 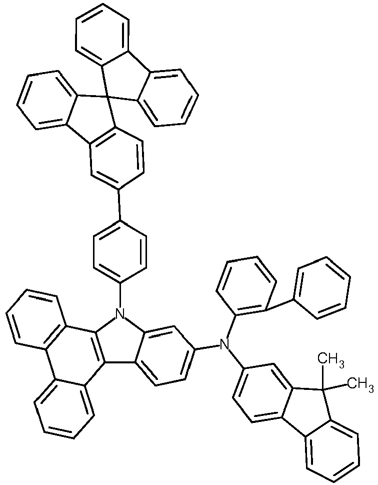 | 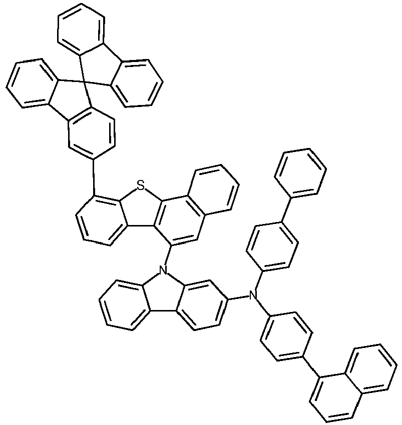 |
| 125 | 126 |
| 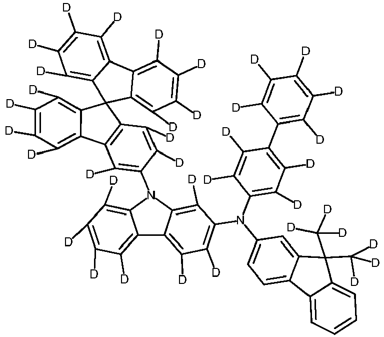 | 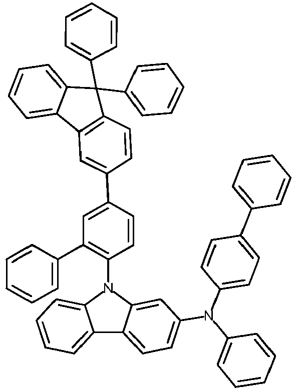 |
| 127 | 128 |
| 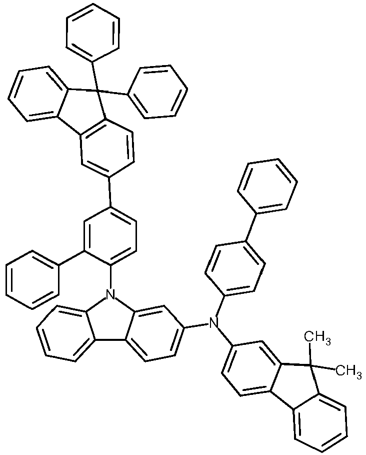 | 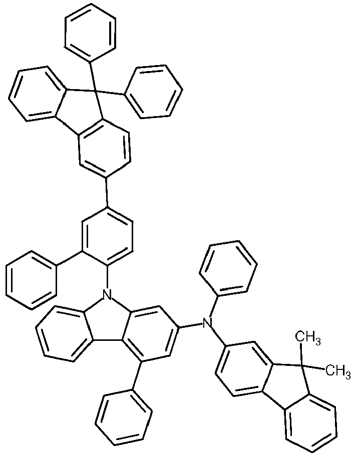 |
| 129 | 130 |
| 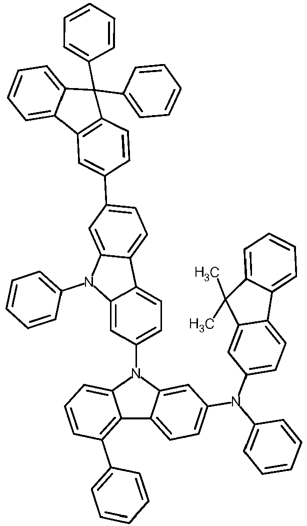 | 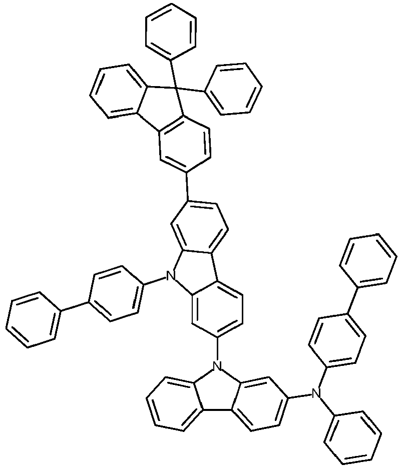 |
| 131 | 132 |
| 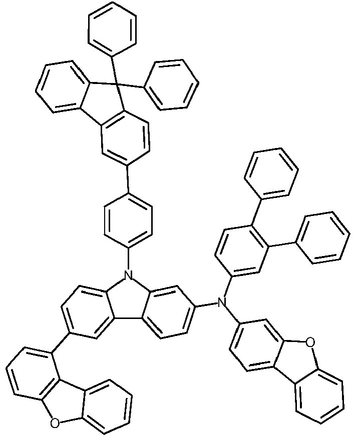 | 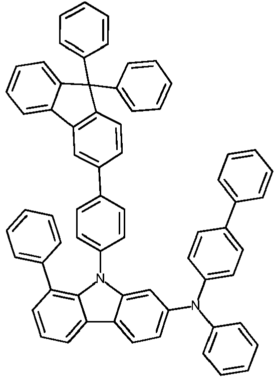 |
| 133 | 134 |
| 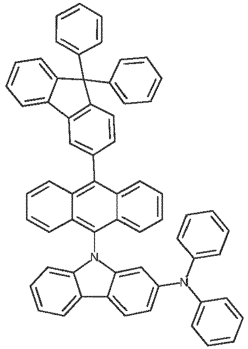 | 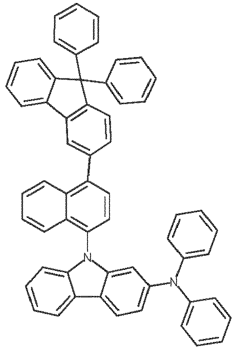 |
| 135 | 136 |
| 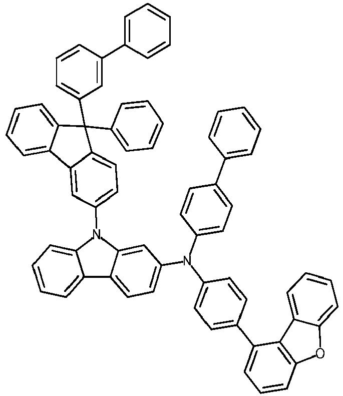 | 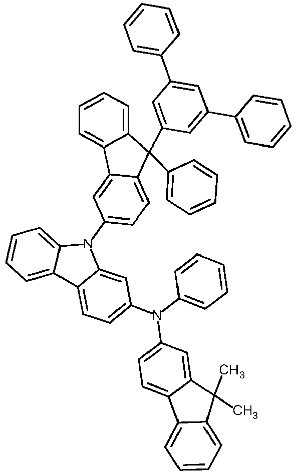 |
| 137 | 138 |
| 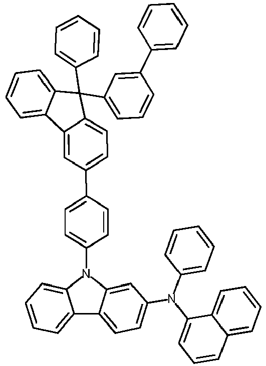 | 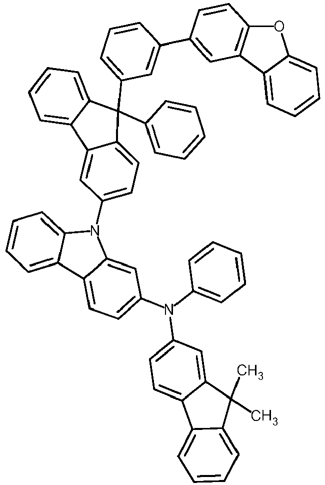 |
| 139 | 140 |
| 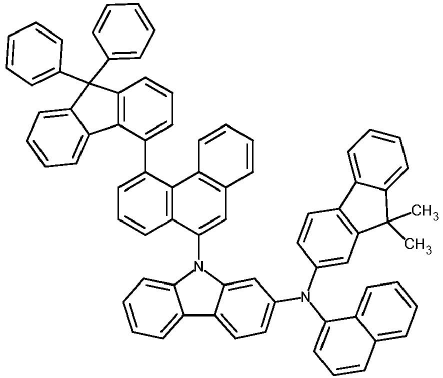 | 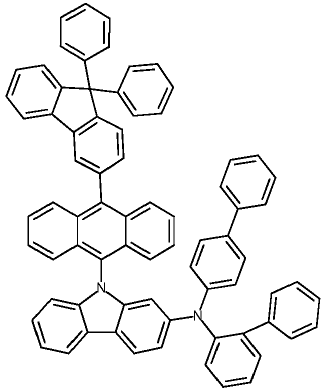 |
| 141 | 142 |
| 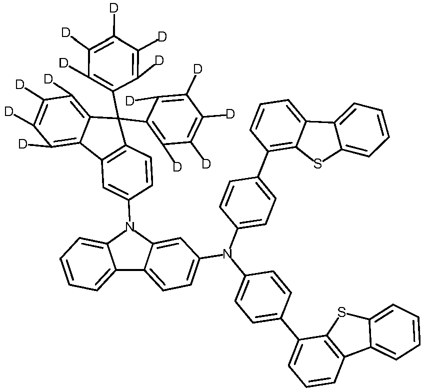 | 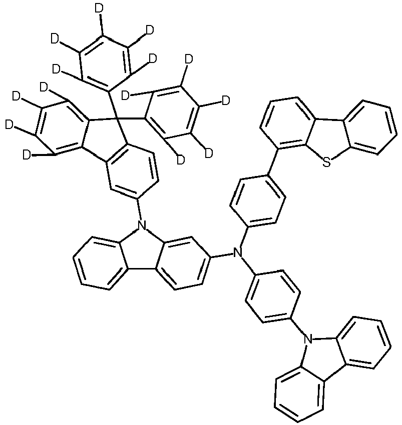 |
| 143 | 144 |
| 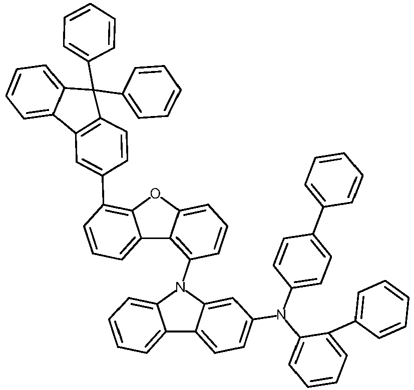 | 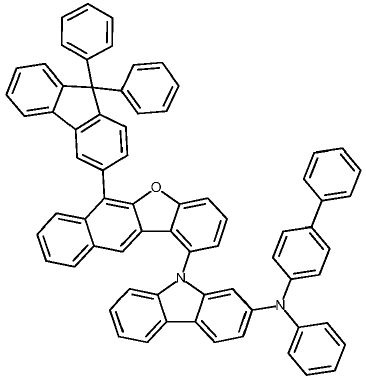 |
| 145 | 146 |
| 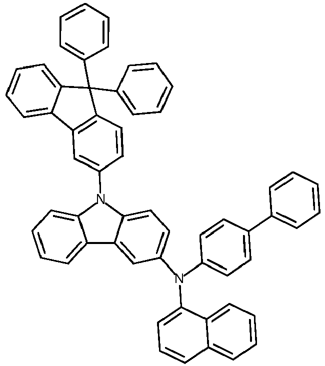 | 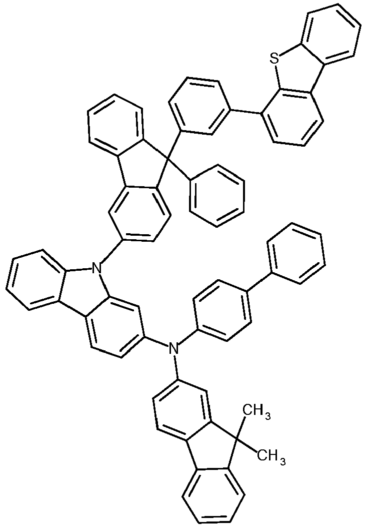 |
| 147 | 148 |
| 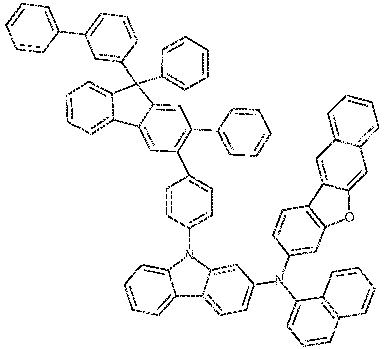 | 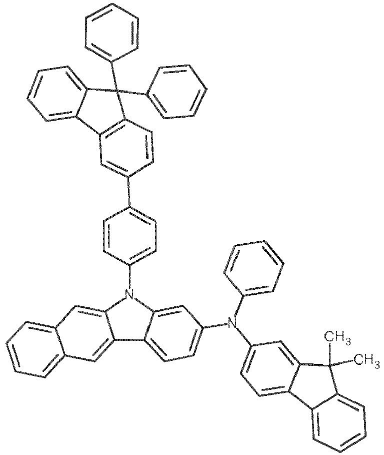 |
| 149 | 150 |
| 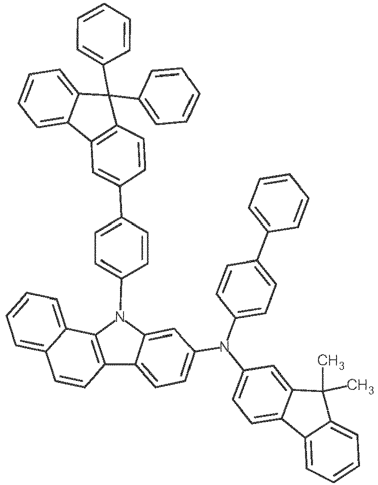 | 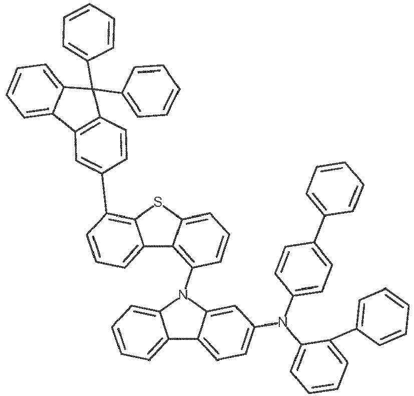 |
| 151 | 152 |
| 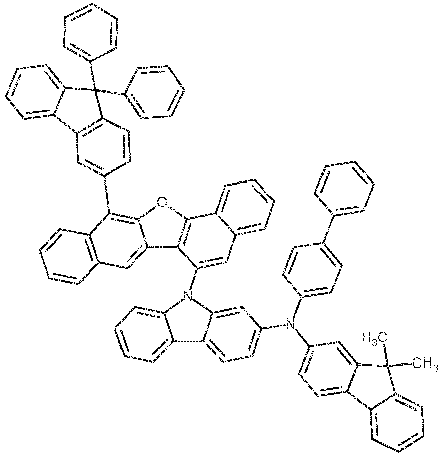 | 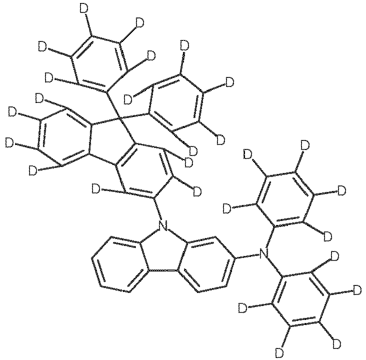 |
| 153 | 154 |
| 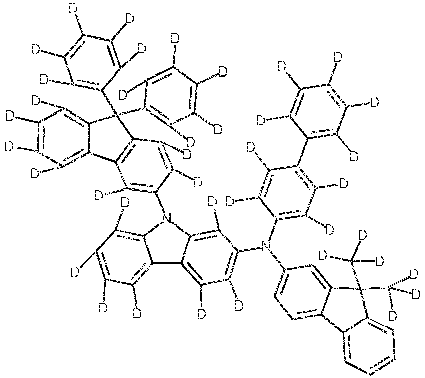 | 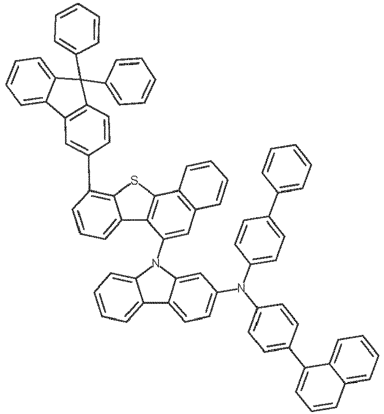 |
| 155 | 156 |
| 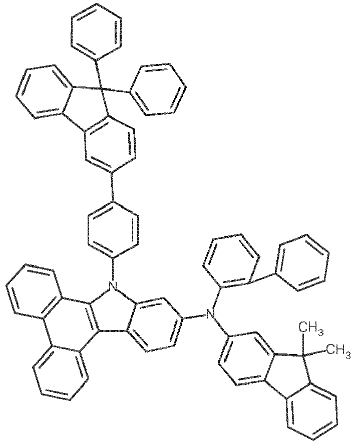 | 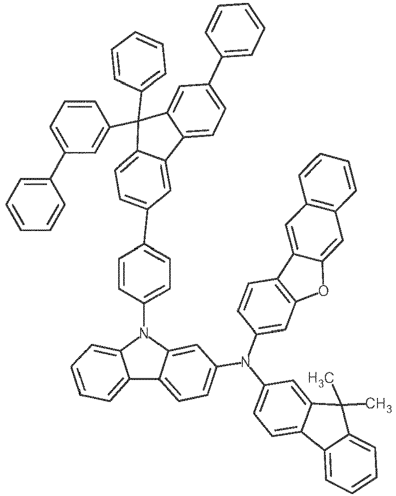 |
| 158 | 159 |
| 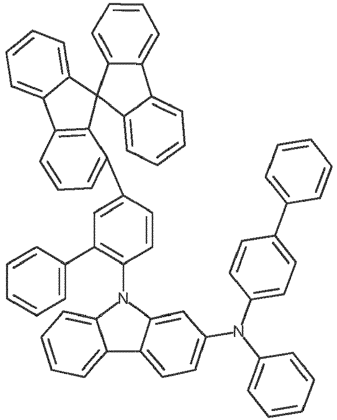 | 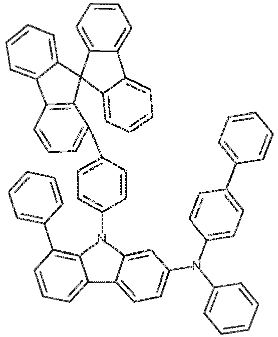 |
| 160 | 161 |
| 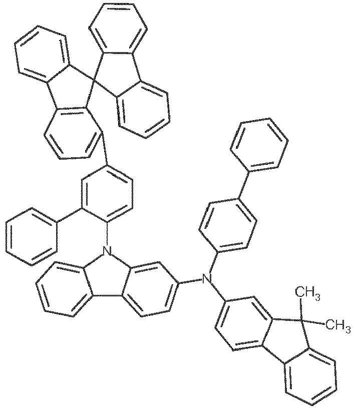 | 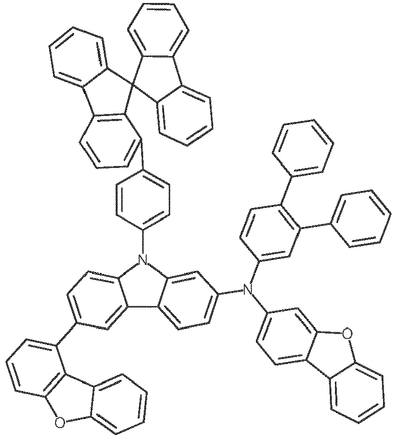 |
| 162 | 163 |
| 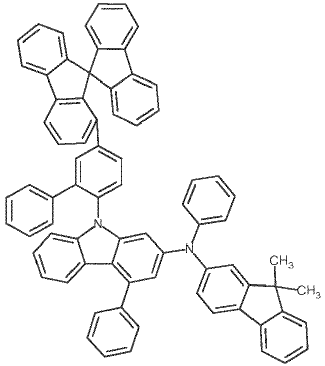 | 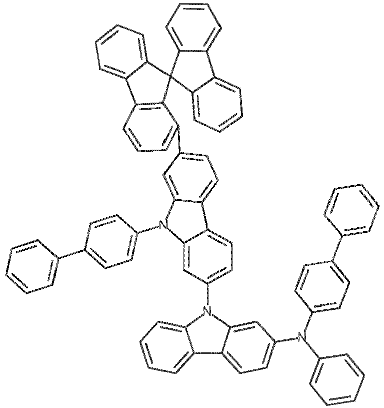 |
| 164 | 165 |
| 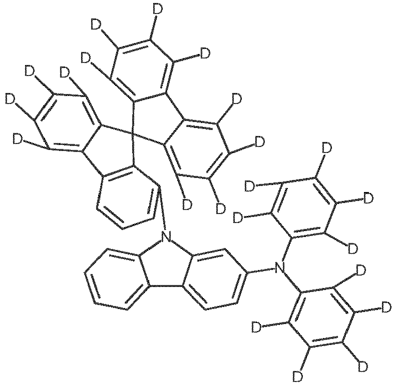 | 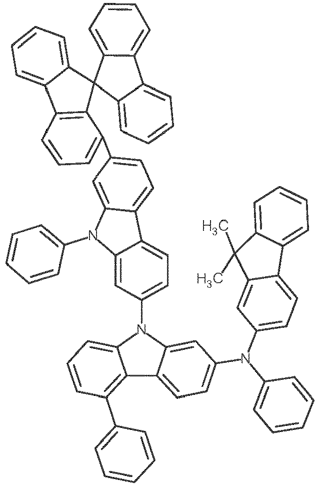 |
| 166 | 167 |
| 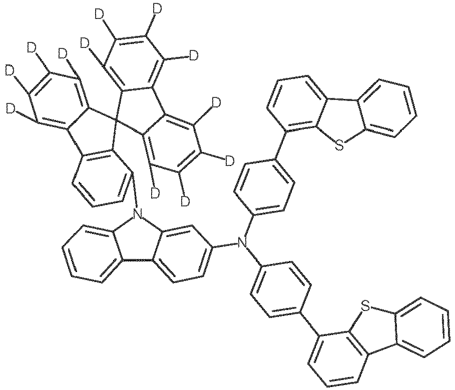 | 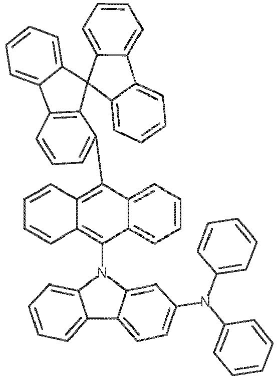 |
| 168 | 169 |
| 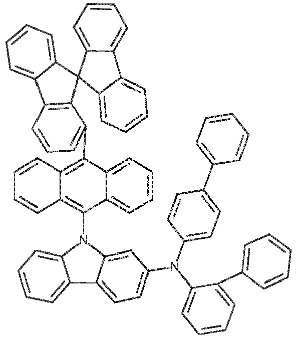 | 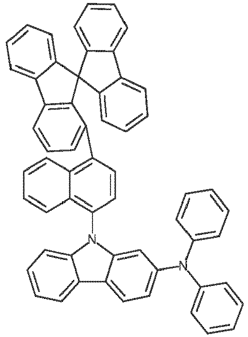 |
| 170 | 171 |
| 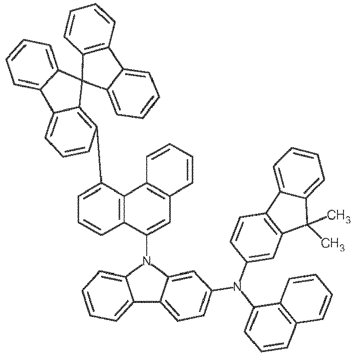 | 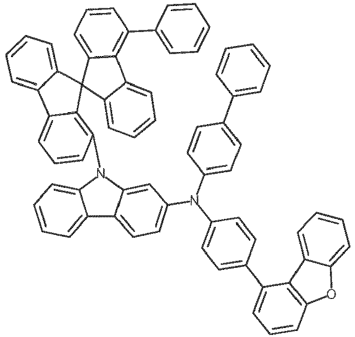 |
| 172 | 173 |
| 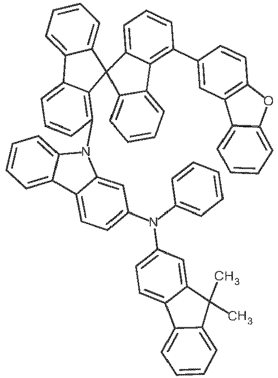 | 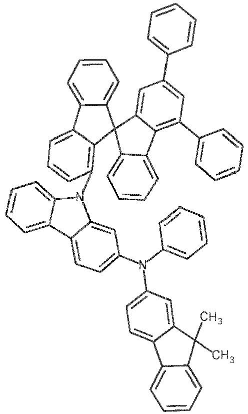 |
| 174 | 175 |
| 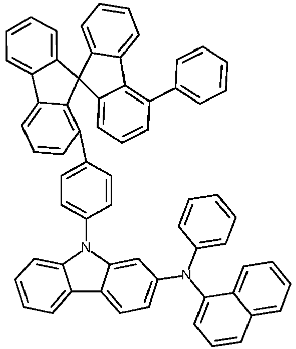 | 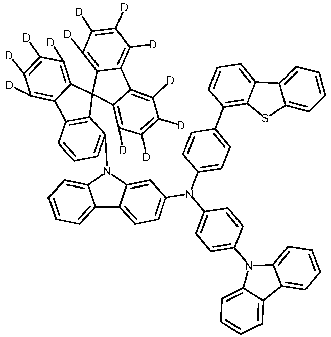 |
| 176 | 177 |
| 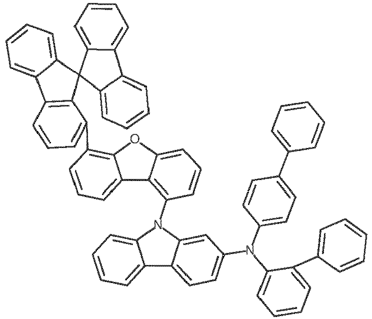 | 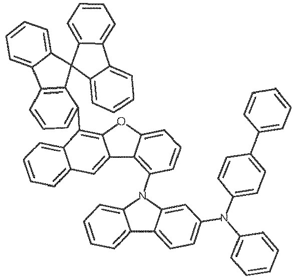 |
| 178 | 179 |
| 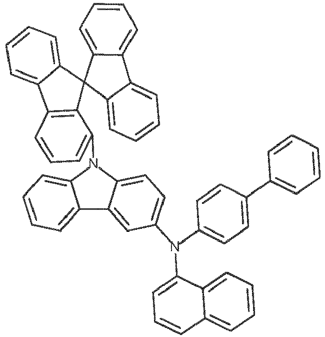 | 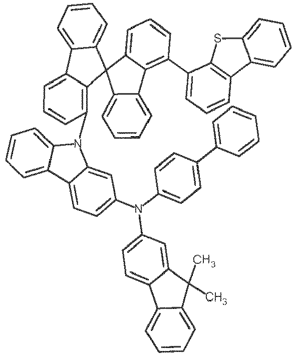 |
| 180 | 181 |
| 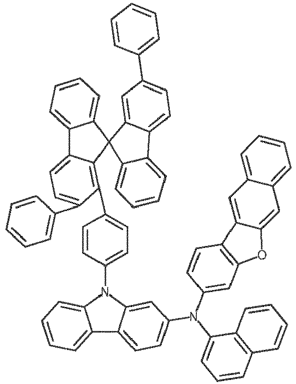 | 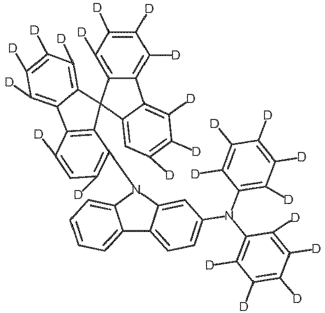 |
| 182 | 183 |
| 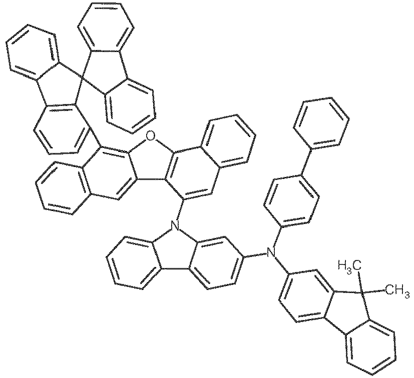 | 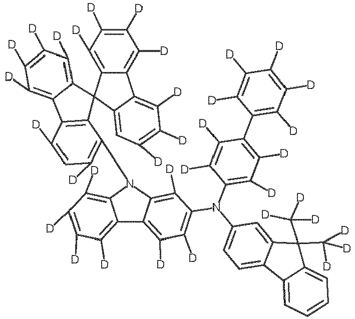 |
| 184 | 185 |
| 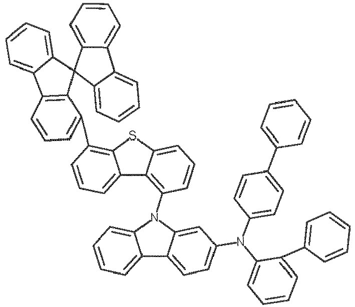 | 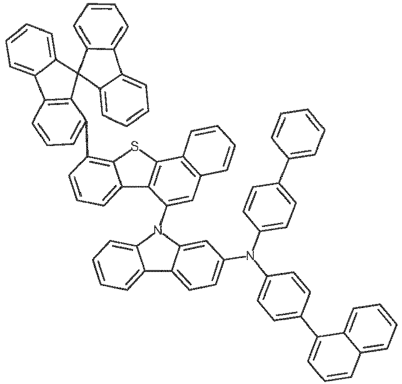 |
| 187 | 189 |
| 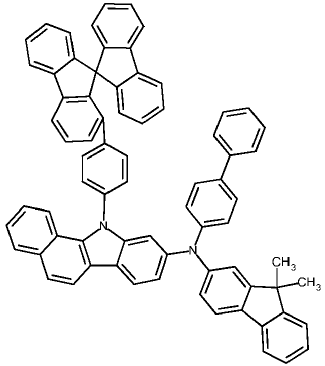 | 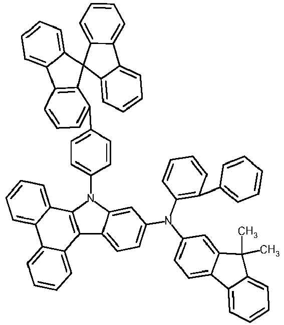 |
| 190 | 191 |
| 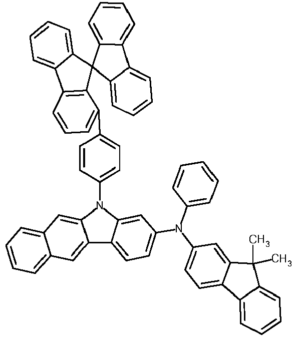 | 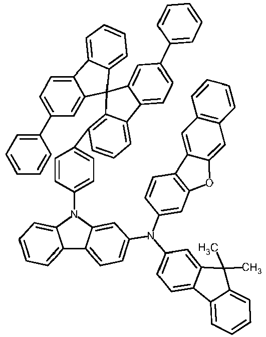 |
| 192 | 193 |
| 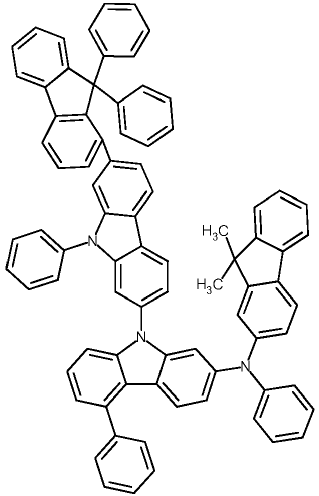 | 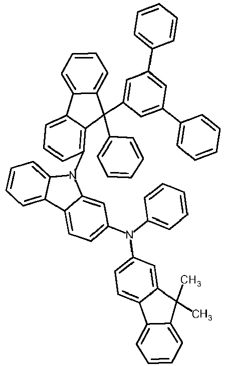 |
| 194 | 195 |
| 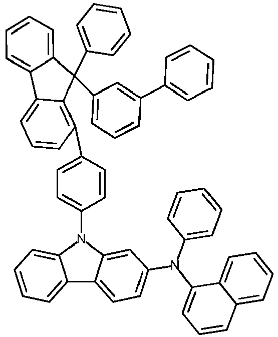 | 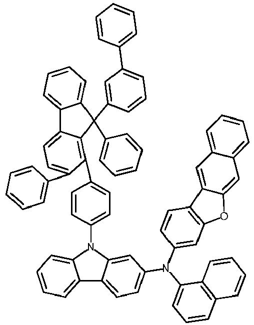 |
| 196 | 197 |
| 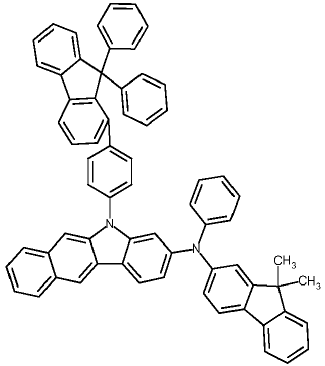 | 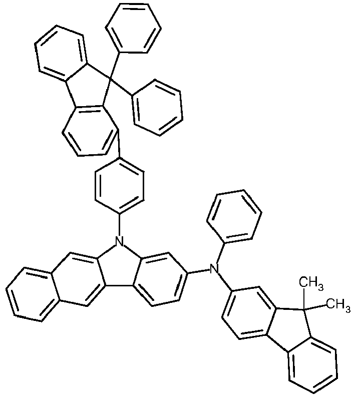 |
| 198 | 199 |
| 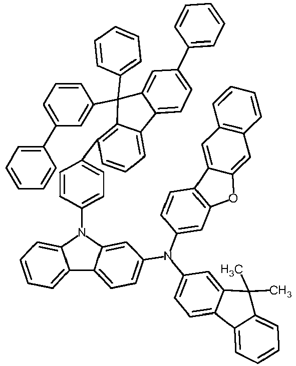 | 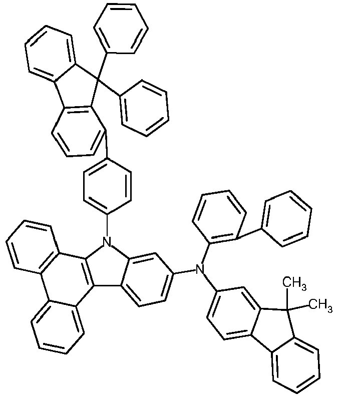 |
| 200 | 201 |
| 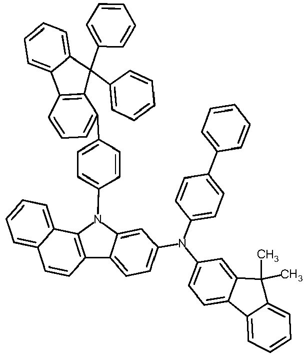 | 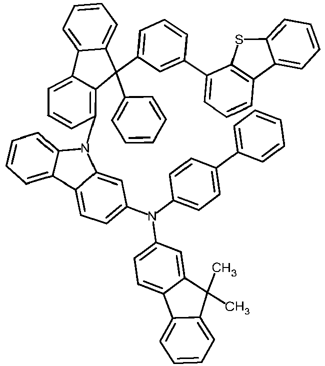 |
| 202 | 203 |
| 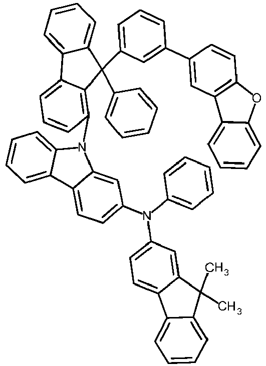 | 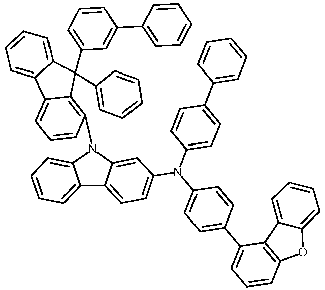 |
| 204 | 205 |
| 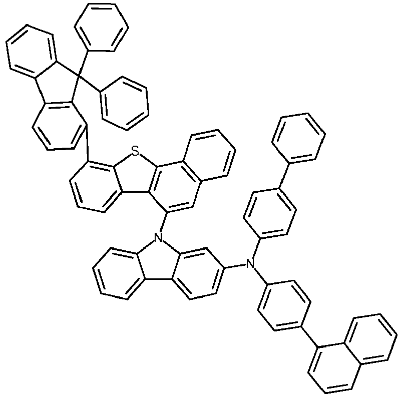 | 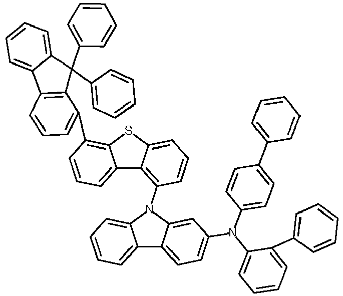 |
| 206 | 207 |
| 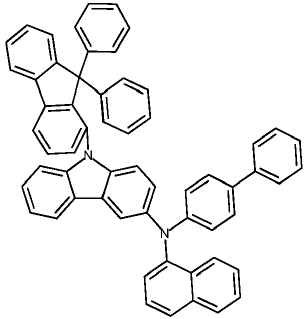 | 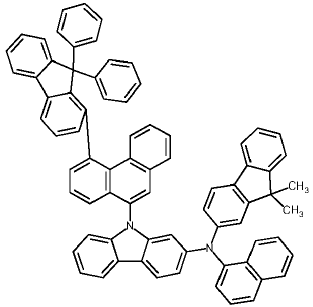 |
| 208 | 209 |
| 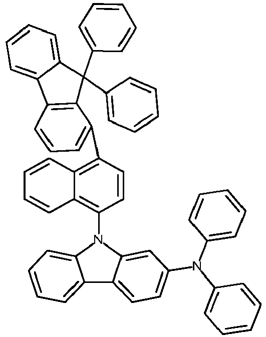 | 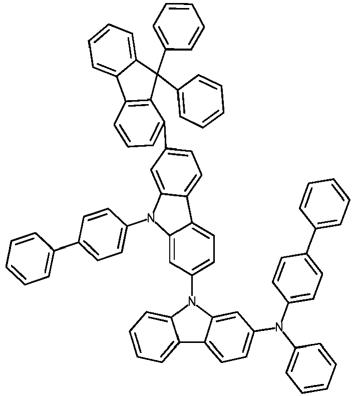 |
| 210 | 211 |
| 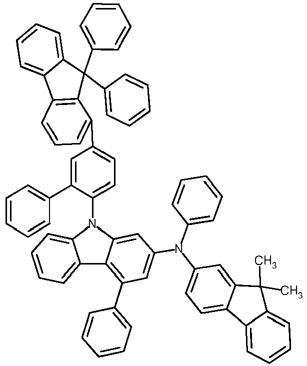 | 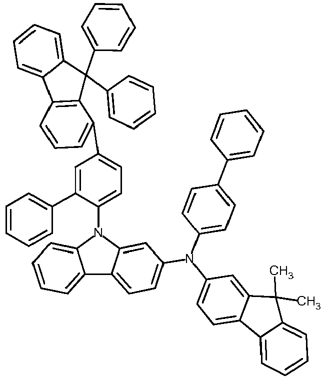 |
| 212 | 213 |
| 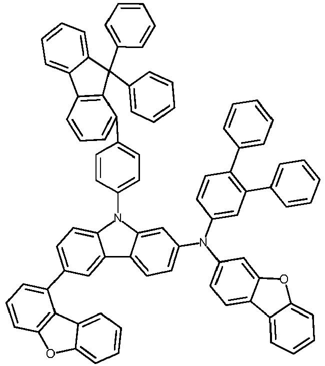 | 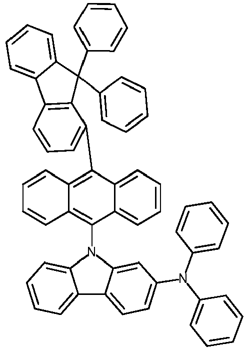 |
| 214 | 215 |
| 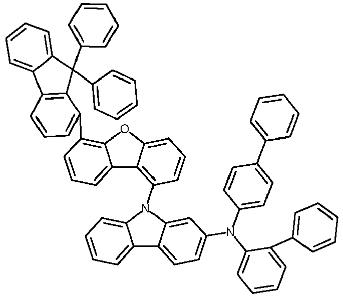 | 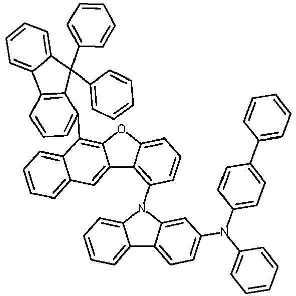 |
| 216 | 217 |
| 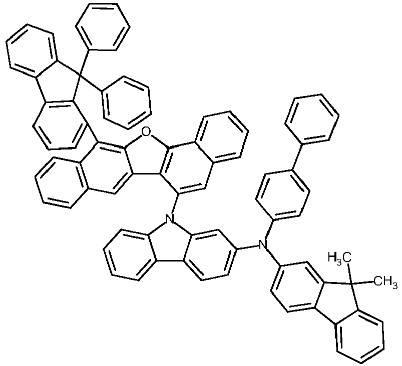 | 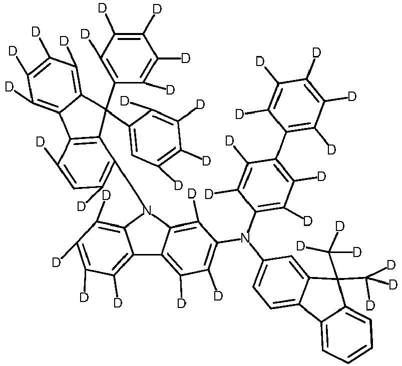 |
| 218 | 219 |
| 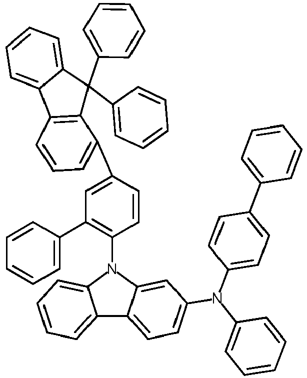 | 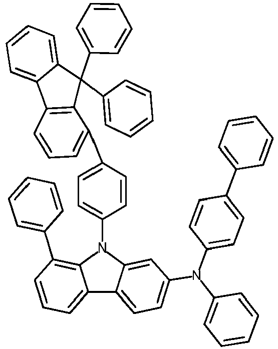 |
| 220 | 221 |
| 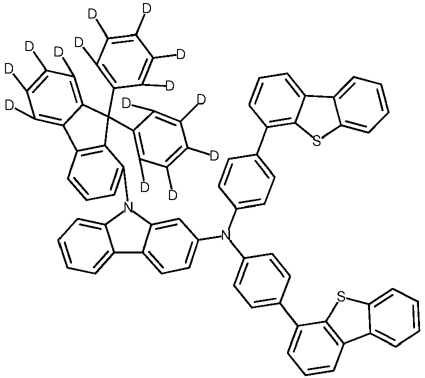 | 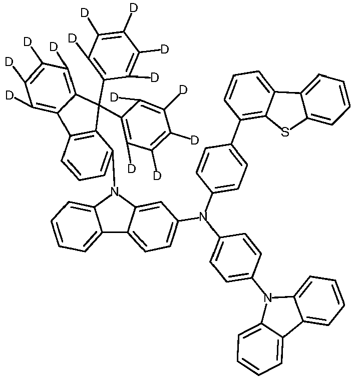 |
| 222 | 223 |
| 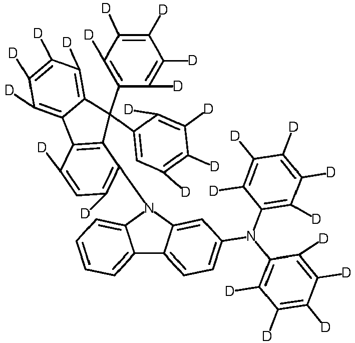 | 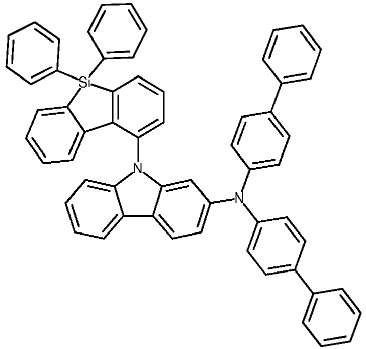 |
| 224 | 225* |
| 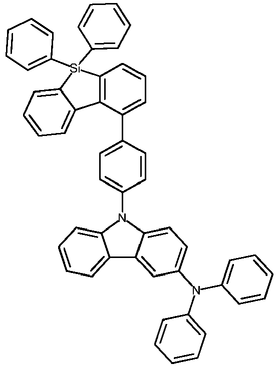 | 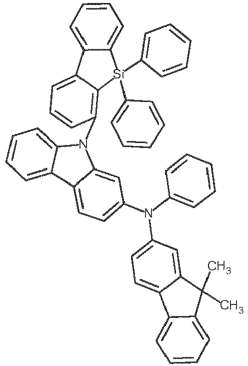 |
| 226* | 227* |
| 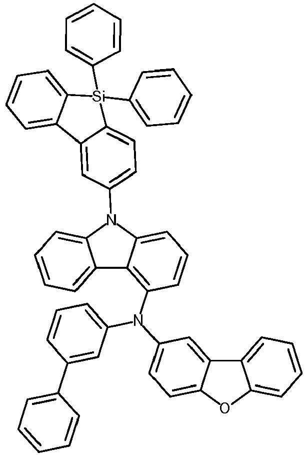 | 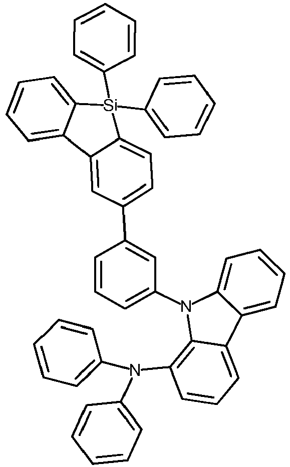 |
| 228* | 229* |

The compounds of the present application can be synthesized by making use of established methods of organic synthesis, such as transition metal catalyzed coupling reactions, preferably Buchwald coupling.

A preferred general synthetic process for preparation of the compounds of formula (1) is shown in Scheme 1 below: 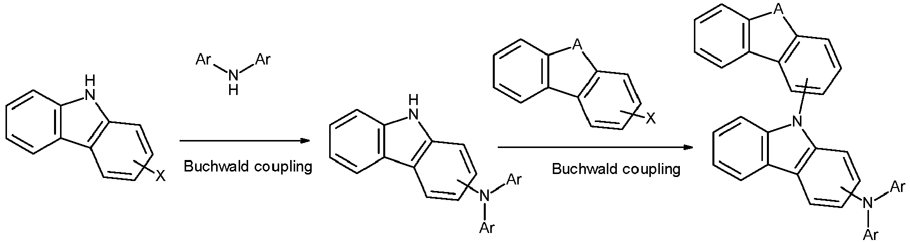
X = leaving group, preferably a halogen, more preferably Br, Cl, I
Ar = Aromatic or heteroaromatic ring system
A = group of formula (A-1) or (A-2) as defined above

The structures depicted in Scheme 1 can be further substituted at any free positions.

A further subject of the present application is thus a process for preparation of a compound according to formula (1), characterized in that it comprises the following steps:
i) coupling reaction, preferably Buchwald coupling reaction, of a diarylamine with a carbazole having a reactive group, to obtain a diarylamine-carbazole derivative;
ii) coupling reaction, preferably Buchwald coupling reaction, of the diarylamine-carbazole derivative obtained in step i) with a 9,9-spirobifluorene or 9,9-fluorene.

The following syntheses are, unless indicated otherwise, carried out under a protective-gas atmosphere in dried solvents. Compounds (I), (II), (IV), and (VI) are commercially available. Comparative compounds (A) and (B) can be prepared in accordance with KR20150051662.

Preferably, the reactive group mentioned in step i) is selected from halogen groups, most preferably Cl, Br and I, particularly preferably Br.

The above-described compounds, especially compounds substituted by reactive leaving groups, such as bromine, iodine, chlorine, may find use as monomers for production of corresponding oligomers, dendrimers or polymers. Suitable reactive leaving groups are, for example, bromine, iodine, chlorine but also boronic acids, boronic esters, amines, alkenyl or alkynyl groups having a terminal C-C double bond or C-C triple bond, oxiranes, oxetanes, groups which enter into a cycloaddition, for example a 1,3-dipolar cycloaddition, for example dienes or azides, carboxylic acid derivatives, alcohols and silanes.

The invention therefore further provides oligomers, polymers or dendrimers containing one or more compounds of formula (1), wherein the bond(s) to the polymer, oligomer or dendrimer may be localized at any desired positions substituted by R^{X}, R^{Z} or R in the formulae. According to the linkage of the compound, the compound is part of a side chain of the oligomer or polymer or part of the main chain. An oligomer in the context of this invention is understood to mean a compound formed from at least three monomer units. A polymer in the context of the invention is understood to mean a compound formed from at least ten monomer units. The polymers, oligomers or dendrimers of the invention may be conjugated, partly conjugated or nonconjugated. The oligomers or polymers of the invention may be linear, branched or dendritic. In the structures having linear linkage, the units of the above formulae may be joined directly to one another, or they may be joined to one another via a bivalent group, for example via a substituted or unsubstituted alkylene group, via a heteroatom or via a bivalent aromatic or heteroaromatic group. In branched and dendritic structures, it is possible, for example, for three or more units of the above formulae to be joined via a trivalent or higher-valency group, for example via a trivalent or higher-valency aromatic or heteroaromatic group, to give a branched or dendritic oligomer or polymer.

For the repeat units of the above formulae in oligomers, dendrimers and polymers, the same preferences apply as described above for the compounds of the above formulae.

For preparation of the oligomers or polymers, the monomers of the invention are homopolymerized or copolymerized with further monomers. Suitable and preferred comonomers are chosen from fluorenes, spirobifluorenes, paraphenylenes, carbazoles, thiophenes, dihydrophenanthrenes, cis- and trans-indenofluorenes, ketones, phenanthrenes or else a plurality of these units. The polymers, oligomers and dendrimers typically contain still further units, for example emitting (fluorescent or phosphorescent) units, for example vinyltriarylamines or phosphorescent metal complexes, and/or charge transport units, especially those based on triarylamines.

The polymers and oligomers of the invention are generally prepared by polymerization of one or more monomer types, of which at least one monomer leads to repeat units of the above formulae in the polymer. Suitable polymerization reactions are known to those skilled in the art and are described in the literature. Particularly suitable and preferred polymerization reactions which lead to formation of C-C or C-N bonds are the Suzuki polymerization, the Yamamoto polymerization, the Stille polymerization and the Hartwig-Buchwald polymerization.

For the processing of the compounds of the invention from a liquid phase, for example by spin-coating or by printing methods, formulations of the compounds of the invention are required. These formulations may, for example, be solutions, dispersions or emulsions. For this purpose, it may be preferable to use mixtures of two or more solvents. Suitable and preferred solvents are, for example, toluene, anisole, o-, m- or p-xylene, methyl benzoate, mesitylene, tetralin, veratrole, THF, methyl-THF, THP, chlorobenzene, dioxane, phenoxytoluene, especially 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, □-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol monobutyl ether, tripropylene glycol dimethyl ether, tetraethylene glycol dimethyl ether, 2-isopropyl naphthalene, pentylbenzene, hexylbenzene, heptylbenzene, octylbenzene, 1,1-bis(3,4-dimethylphenyl)ethane or mixtures of these solvents.

The invention therefore further provides a formulation, especially a solution, dispersion or emulsion, comprising at least one compound of formula (1) and at least one solvent, preferably an organic solvent. The way in which such solutions can be prepared is known to those skilled in the art and is described, for example, in WO 2002/072714, WO 2003/019694 and the literature cited therein.

The compounds of the invention are suitable for use in electronic devices, especially in organic electroluminescent devices (OLEDs). Depending on the substitution, the compounds are used in different functions and layers.

The invention therefore further provides for the use of the compound in an electronic device. This electronic device is preferably selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and more preferably organic electroluminescent devices (OLEDs).

The invention further provides, as already set out above, an electronic device comprising at least one compound of formula (1). This electronic device is preferably selected from the abovementioned devices.

It is more preferably an organic electroluminescent device (OLED) comprising anode, cathode and at least one emitting layer, characterized in that at least one organic layer, which may be an emitting layer, a hole transport layer or another layer, preferably an emitting layer or a hole transport layer, particularly preferably a hole transport layer, comprises at least one compound of formula (1).

Apart from the cathode, anode and emitting layer, the organic electroluminescent device may also comprise further layers. These are selected, for example, from in each case one or more hole injection layers, hole transport layers, hole blocking layers, electron transport layers, electron injection layers, electron blocking layers, exciton blocking layers, interlayers, charge generation layers and/or organic or inorganic p/n junctions.

The sequence of the layers of the organic electroluminescent device comprising the compound of the above formulae is preferably as follows:
anode-hole injection layer-hole transport layer-optionally further hole transport layer(s)-optionally electron blocking layer-emitting layer-optionally hole blocking layer-electron transport layer-electron injection layer-cathode. It is additionally possible for further layers to be present in the OLED.

The organic electroluminescent device of the invention may contain two or more emitting layers. More preferably, these emission layers in this case have several emission maxima between 380 nm and 750 nm overall, such that the overall result is white emission; in other words, various emitting compounds which may fluoresce or phosphoresce and which emit blue, green, yellow, orange or red light are used in the emitting layers. Especially preferred are three-layer systems, i.e. systems having three emitting layers, where the three layers show blue, green and orange or red emission. The compounds of the invention are preferably present in the hole transport layer, hole injection layer or electron blocking layer.

It is preferable in accordance with the invention when the compound of formula (1) is used in an electronic device comprising one or more phosphorescent emitting compounds. In this case, the compound may be present in different layers, preferably in a hole transport layer, an electron blocking layer, a hole injection layer or in an emitting layer.

The term "phosphorescent emitting compounds" typically encompasses compounds where the emission of light takes place through a spin-forbidden transition, for example a transition from an excited triplet state or a state having a higher spin quantum number, for example a quintet state.

Suitable phosphorescent emitting compounds (= triplet emitters) are especially compounds which, when suitably excited, emit light, preferably in the visible region, and also contain at least one atom of atomic number greater than 20, preferably greater than 38, and less than 84, more preferably greater than 56 and less than 80. Preference is given to using, as phosphorescent emitting compounds, compounds containing copper, molybdenum, tungsten, rhenium, ruthenium, osmium, rhodium, iridium, palladium, platinum, silver, gold or europium, especially compounds containing iridium, platinum or copper. In the context of the present invention, all luminescent iridium, platinum or copper complexes are considered to be phosphorescent emitting compounds. In general, all phosphorescent complexes as used for phosphorescent OLEDs according to the prior art and as known to those skilled in the art in the field of organic electroluminescent devices are suitable. It is also possible for the person skilled in the art, without exercising inventive skill, to use further phosphorescent complexes in combination with the compounds of formula (1) in organic electroluminescent devices. Further examples are listed in a table which follows.

It is also possible in accordance with the invention to use the compound of formula (1) in an electronic device comprising one or more fluorescent emitting compounds.

In a preferred embodiment of the invention, the compounds of formula (1) are used as hole-transporting material. In that case, the compounds are preferably present in a hole transport layer, an electron blocking layer or a hole injection layer. Particular preference is given to use in an electron blocking layer or in a hole transport layer.

A hole transport layer according to the present application is a layer having a hole-transporting function between the anode and emitting layer.

Hole injection layers and electron blocking layers are understood in the context of the present application to be specific embodiments of hole transport layers. A hole injection layer, in the case of a plurality of hole transport layers between the anode and emitting layer, is a hole transport layer which directly adjoins the anode or is separated therefrom only by a single coating of the anode. An electron blocking layer, in the case of a plurality of hole transport layers between the anode and emitting layer, is that hole transport layer which directly adjoins the emitting layer on the anode side. Preferably, the OLED of the invention comprises two, three or four hole-transporting layers between the anode and emitting layer, at least one of which preferably contains a compound of formula (1), and more preferably exactly one or two contain a such compound.

If the compound of formula (1) is used as hole transport material in a hole transport layer, a hole injection layer or an electron blocking layer, the compound can be used as pure material, i.e. in a proportion of 100%, in the hole transport layer, or it can be used in combination with one or more further compounds. In a preferred embodiment, the organic layer comprising the compound of one of the above-mentioned formulae then additionally contains one or more p-dopants. p-Dopants used according to the present invention are preferably those organic electron acceptor compounds capable of oxidizing one or more of the other compounds in the mixture.

Particularly preferred p-dopants are quinodimethane compounds, azaindenofluorenediones, azaphenalenes, azatriphenylenes, I₂, metal halides, preferably transition metal halides, metal oxides, preferably metal oxides containing at least one transition metal or a metal of main group 3, and transition metal complexes, preferably complexes of Cu, Co, Ni, Pd and Pt with ligands containing at least one oxygen atom as bonding site. Preference is further given to transition metal oxides as dopants, preferably oxides of rhenium, molybdenum and tungsten, more preferably Re₂O₇, MoO₃, WO₃ and ReOs. Further preferable p-dopants are selected from Bi(III)-containing metal complexes, in particular Bi(lll) complexes of benzoic acid or benzoic acid derivatives.

The p-dopants are preferably in substantially homogeneous distribution in the p-doped layers. This can be achieved, for example, by coevaporation of the p-dopant and the hole transport material matrix.

Preferred p-dopants are especially the following compounds:

| | | |
|---|---|---|
| 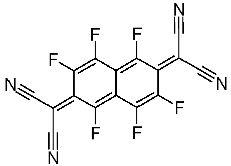 | 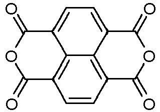 | 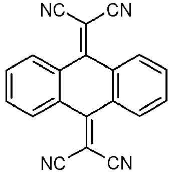 |
| (D-1) | (D-2) | (D-3) |
| 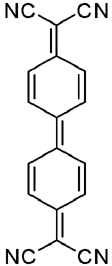 | 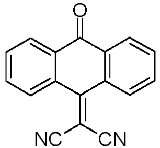 | 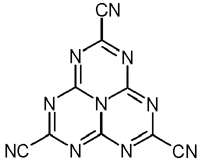 |
| (D-4) | (D-5) | (D-6) |
| 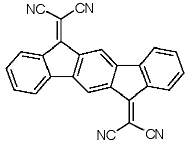 | 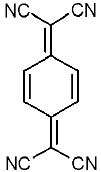 | 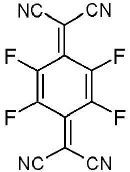 |
| (D-7) | (D-8) | (D-9) |
| 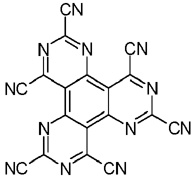 | 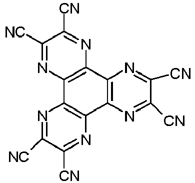 | 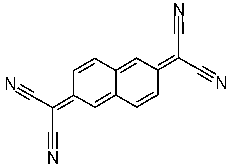 |
| (D-10) | (D-11) | (D-12) |
| 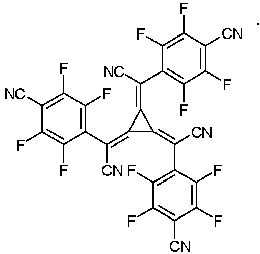 | 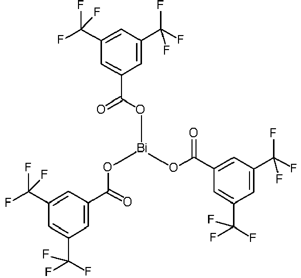 | |
| (D-13) | (D-14) | |

In a further preferred embodiment of the invention, the compound is used as hole transport material in a hole transporting layer, and there is a layer present between anode and this hole transporting layer (called hole injection layer), which comprises an electron accepting material. Preferably, this electron accepting material is selected from the compound classes mentioned above for use as p-dopants, particularly preferably from the compounds (D-1) to (D-14) mentioned above, most preferably from the compounds (D-6), (D-7) and (D-14). Preferably, the above-mentioned hole injection layer comprises one of the above-mentioned compounds in non-doped form, with no other compounds admixed. Most preferably, it consists of only one of the above-mentioned compounds and comprises no other compound.

In a further embodiment of the present invention, the compound is used in an emitting layer as matrix material in combination with one or more emitting compounds, preferably phosphorescent emitting compounds.

The proportion of the matrix material in the emitting layer in this case is between 50.0% and 99.9% by volume, preferably between 80.0% and 99.5% by volume, and more preferably between 92.0% and 99.5% by volume for fluorescent emitting layers and between 85.0% and 97.0% by volume for phosphorescent emitting layers.

Correspondingly, the proportion of the emitting compound is between 0.1% and 50.0% by volume, preferably between 0.5% and 20.0% by volume, and more preferably between 0.5% and 8.0% by volume for fluorescent emitting layers and between 3.0% and 15.0% by volume for phosphorescent emitting layers.

An emitting layer of an organic electroluminescent device may also comprise systems comprising a plurality of matrix materials (mixed matrix systems) and/or a plurality of emitting compounds. In this case too, the emitting compounds are generally those compounds having the smaller proportion in the system and the matrix materials are those compounds having the greater proportion in the system. In individual cases, however, the proportion of a single matrix material in the system may be less than the proportion of a single emitting compound.

It is preferable that the compound is used as a component of mixed matrix systems. The mixed matrix systems preferably comprise two or three different matrix materials, more preferably two different matrix materials. Preferably, in this case, one of the two materials is a material having hole-transporting properties and the other material is a material having electron-transporting properties. The compound is preferably the matrix material having hole-transporting properties. The desired electron-transporting and hole-transporting properties of the mixed matrix components may, however, also be combined mainly or entirely in a single mixed matrix component, in which case the further mixed matrix component(s) fulfill(s) other functions. The two different matrix materials may be present in a ratio of 1:50 to 1:1, preferably 1:20 to 1:1, more preferably 1:10 to 1:1 and most preferably 1:4 to 1:1. Preference is given to using mixed matrix systems in phosphorescent organic electroluminescent devices.

The mixed matrix systems may comprise one or more emitting compounds, preferably one or more phosphorescent emitting compounds. In general, mixed matrix systems are preferably used in phosphorescent organic electroluminescent devices.

Particularly suitable matrix materials which can be used in combination with the compounds of the invention as matrix components of a mixed matrix system are selected from the preferred matrix materials specified below for phosphorescent emitting compounds or the preferred matrix materials for fluorescent emitting compounds, according to what type of emitting compound is used in the mixed matrix system.

Preferred phosphorescent emitting compounds for use in mixed matrix systems are the same as detailed further up as generally preferred phosphorescent emitter materials.

Preferred embodiments of the different functional materials in the electronic device are listed hereinafter.

Preferred phosphorescent emitting compounds are the following ones:

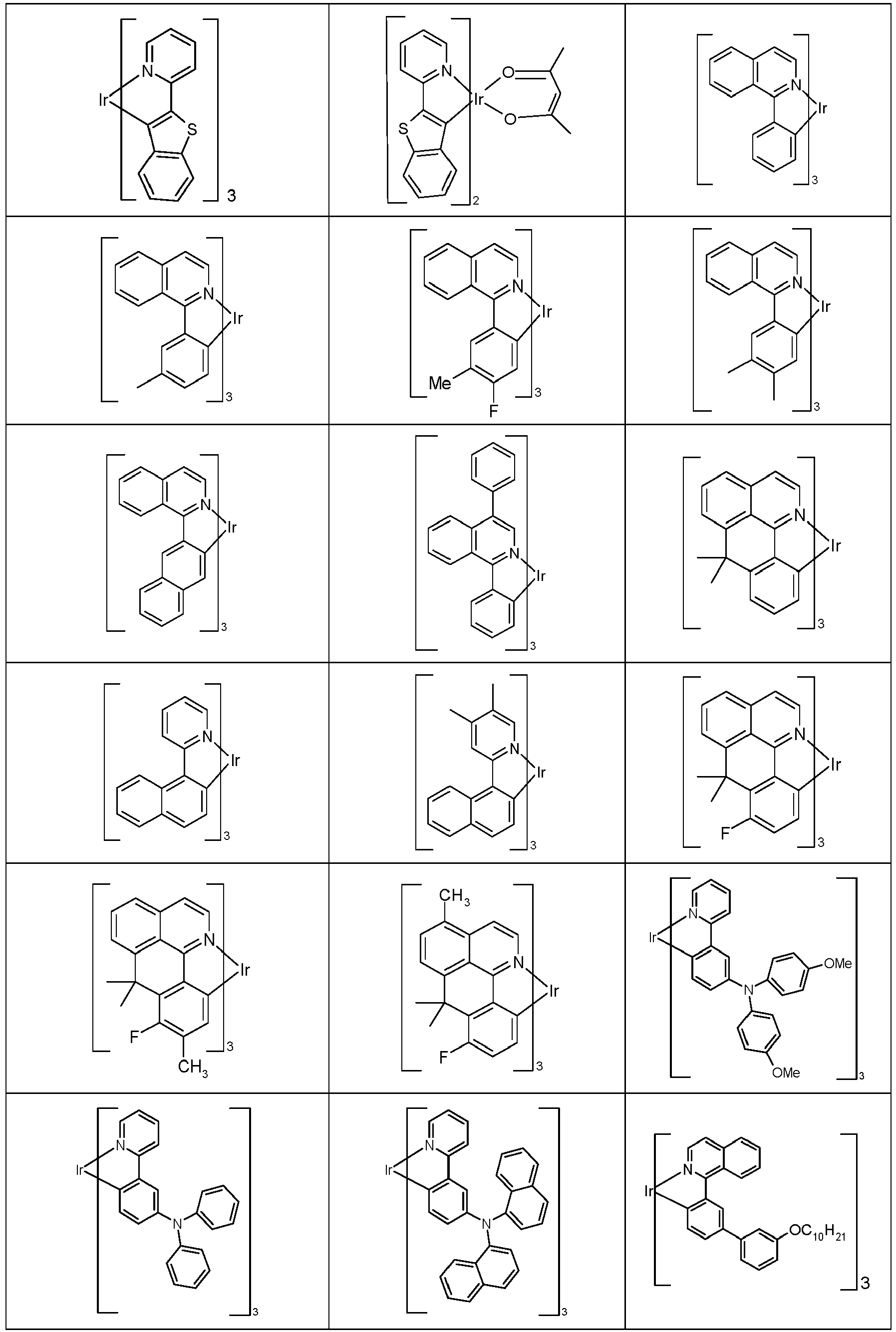

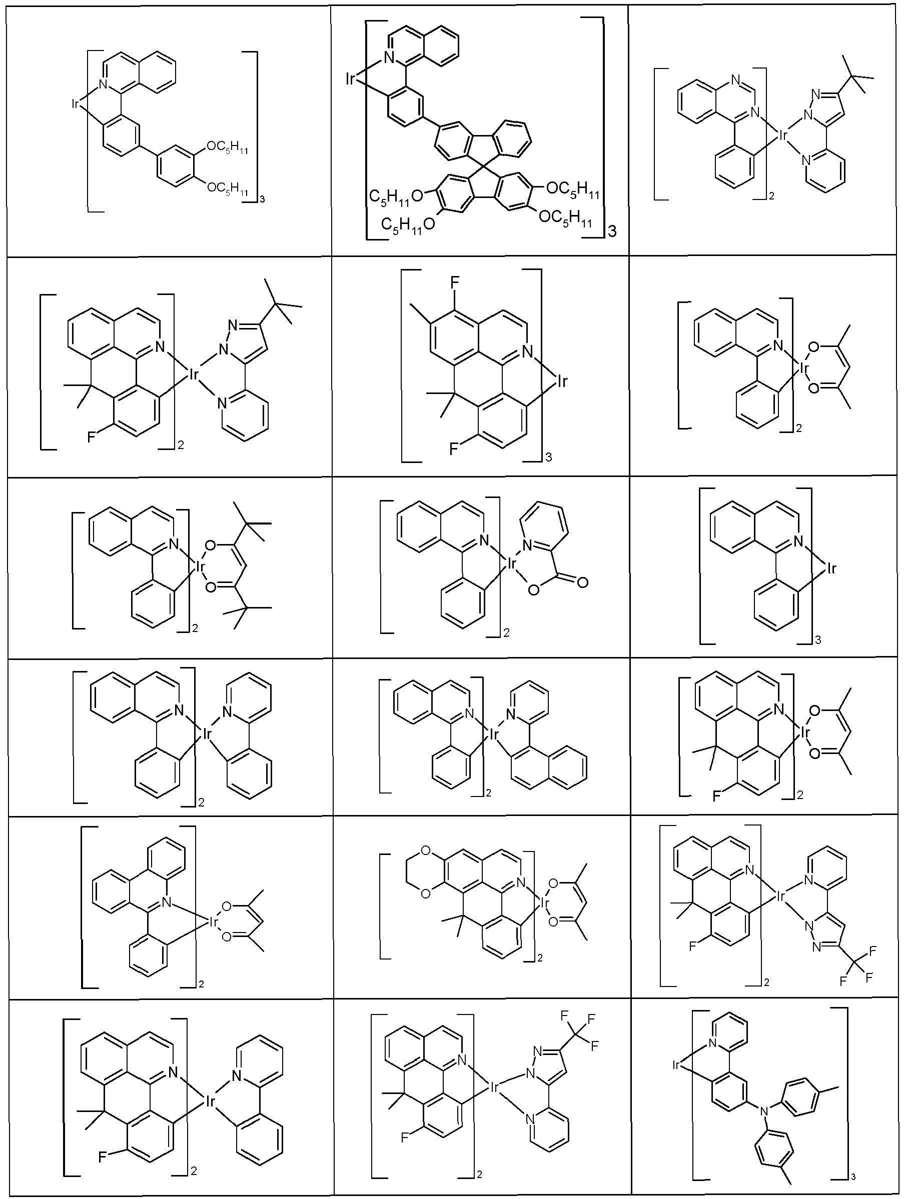

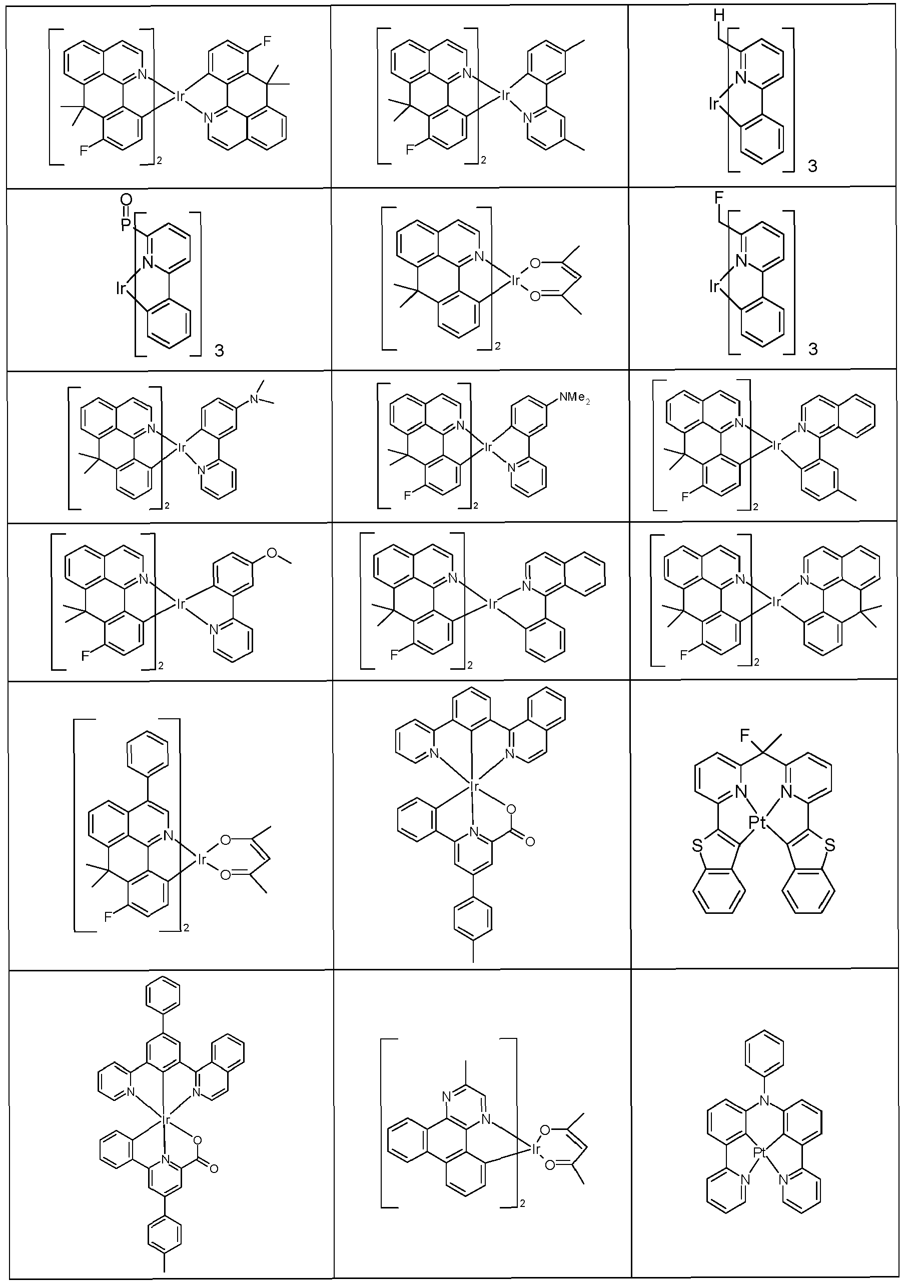

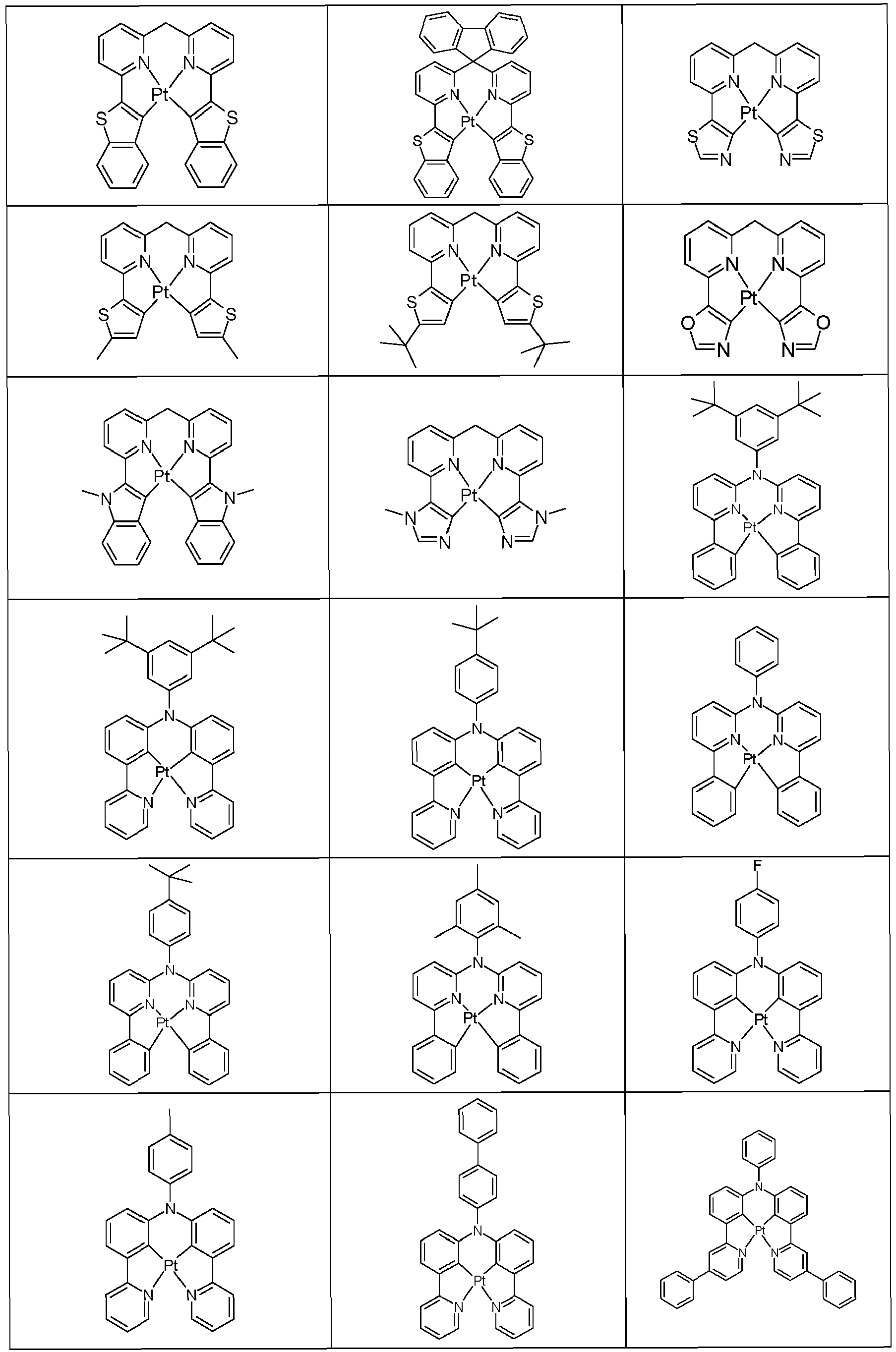

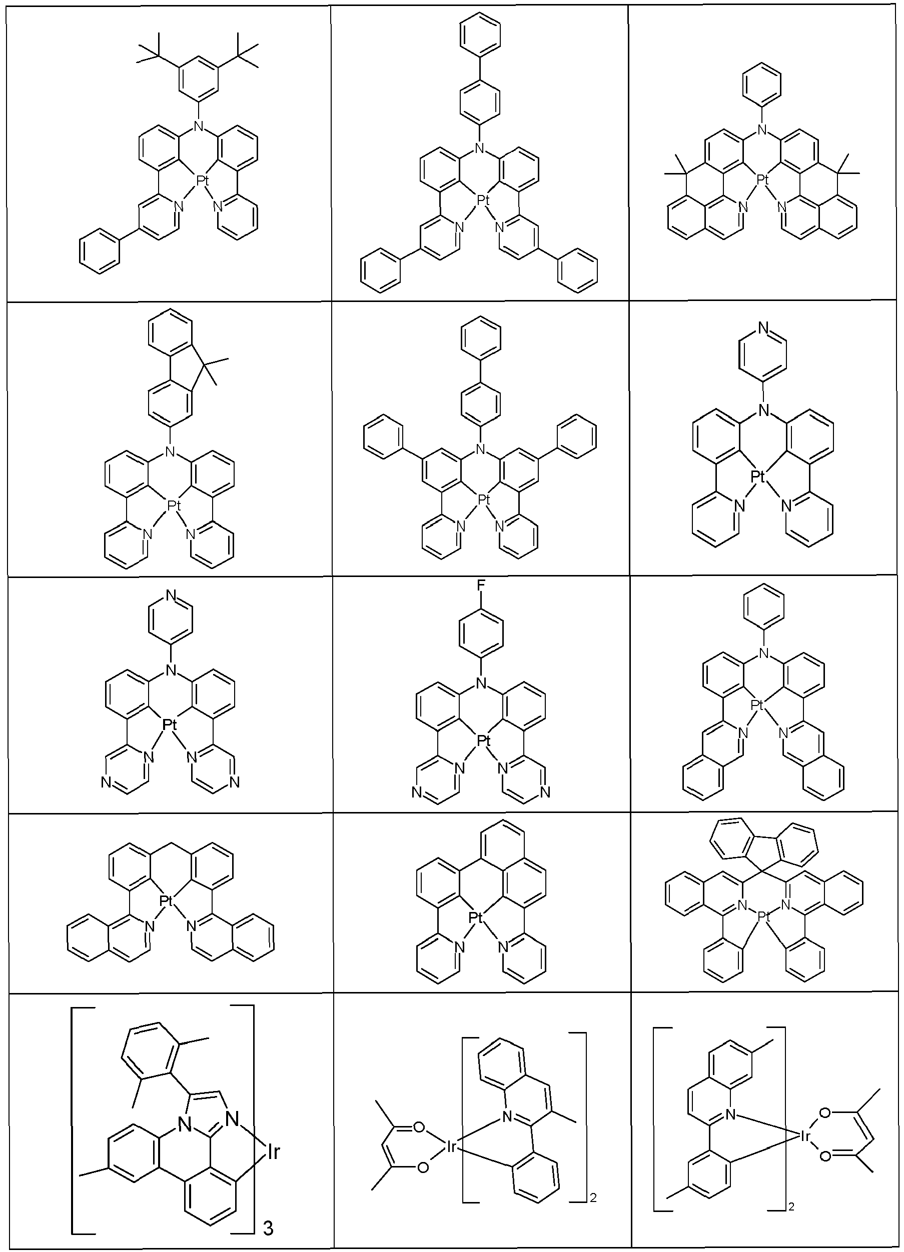

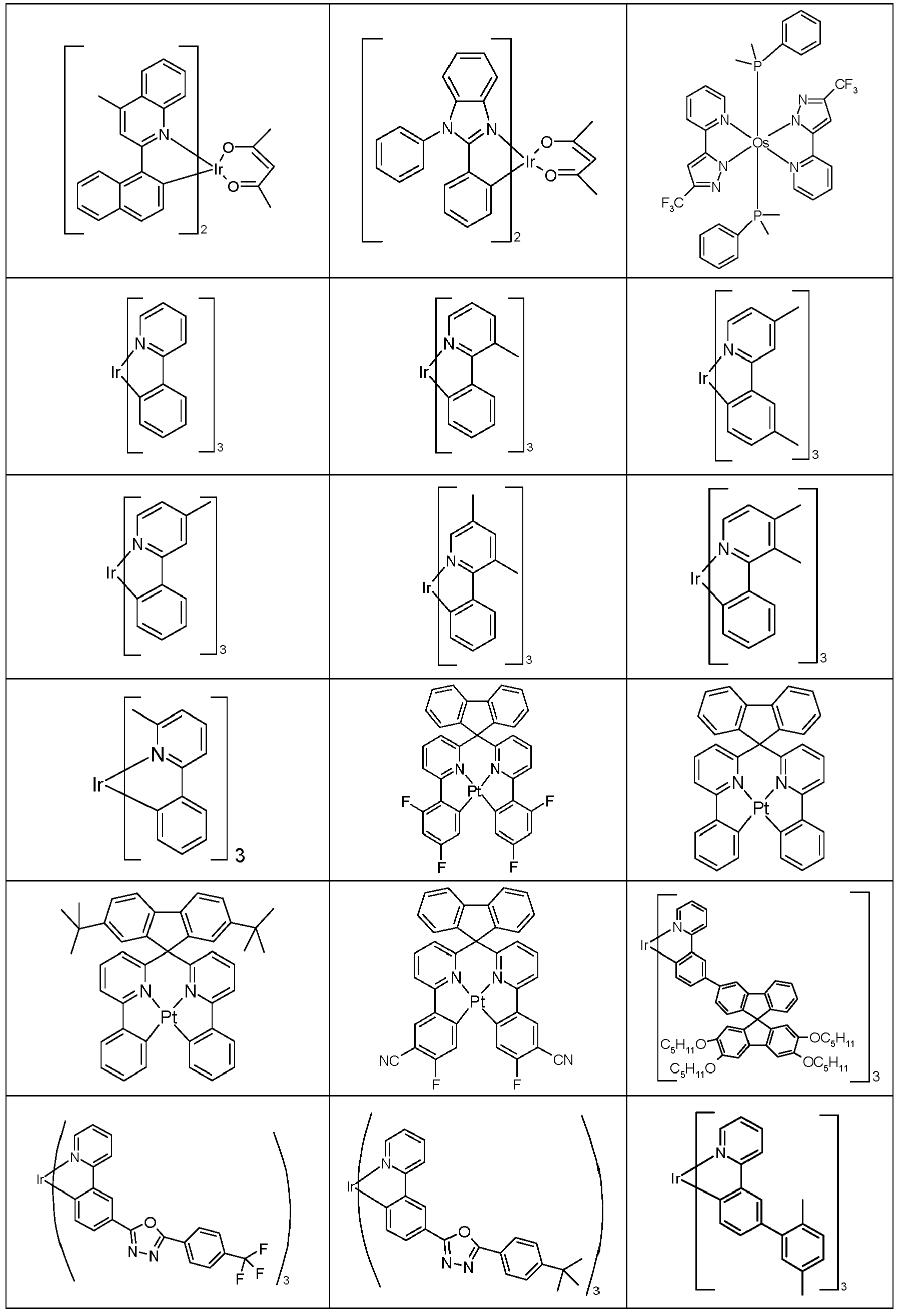

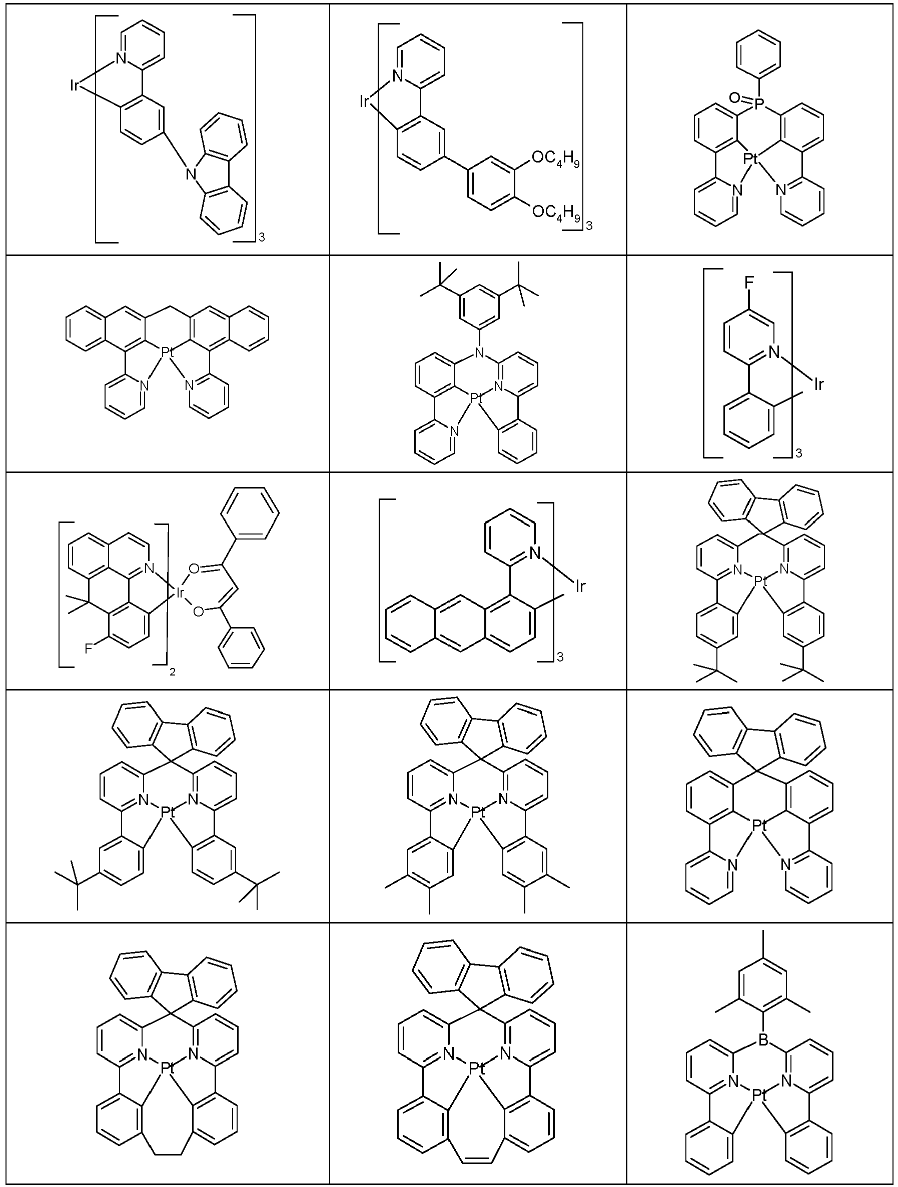

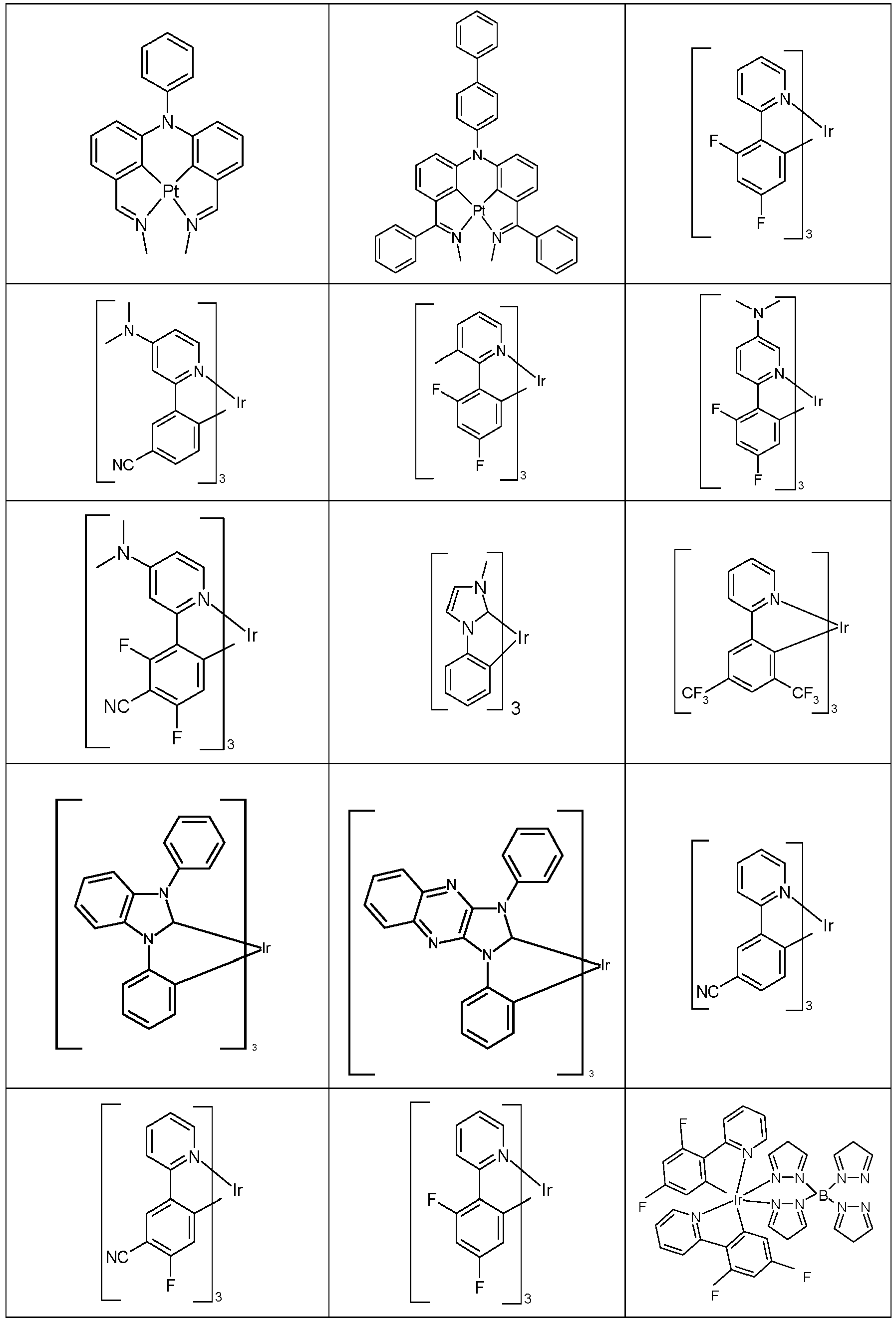

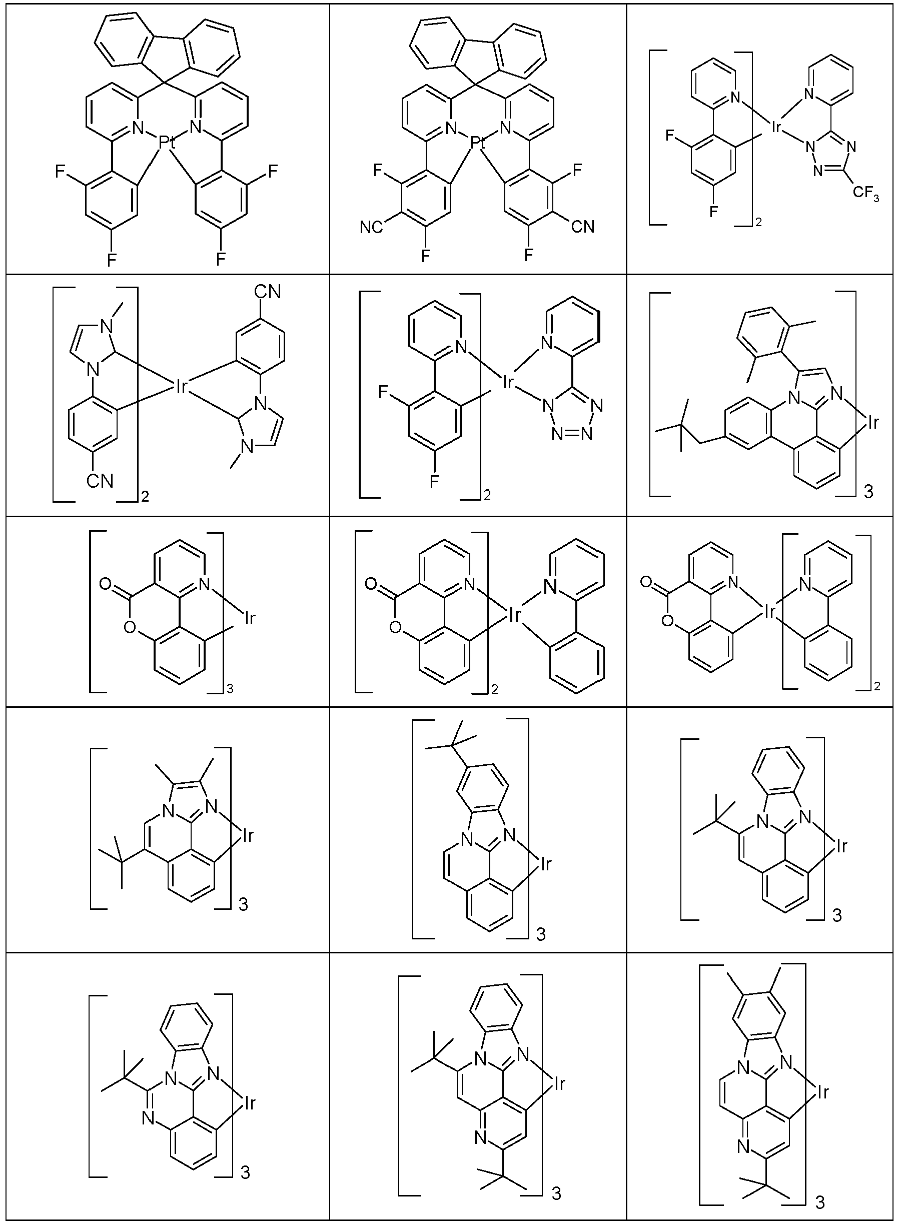

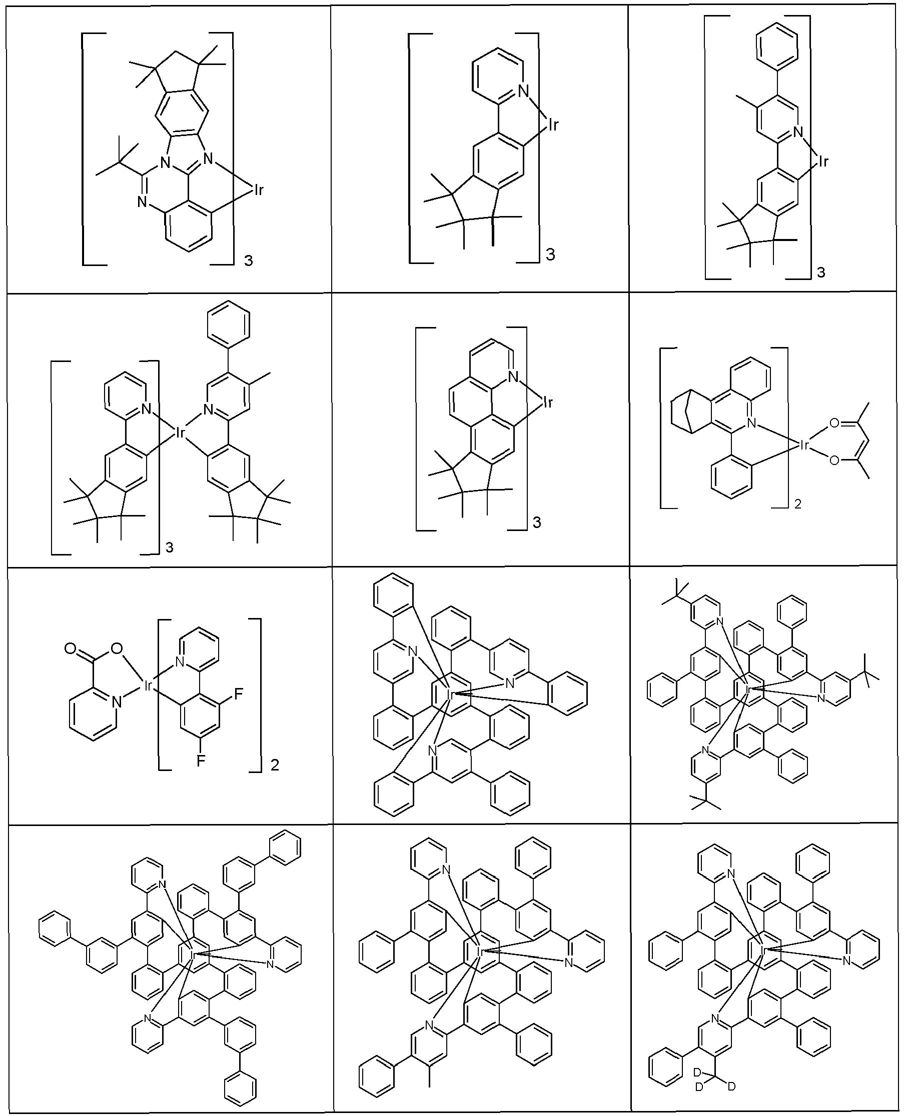

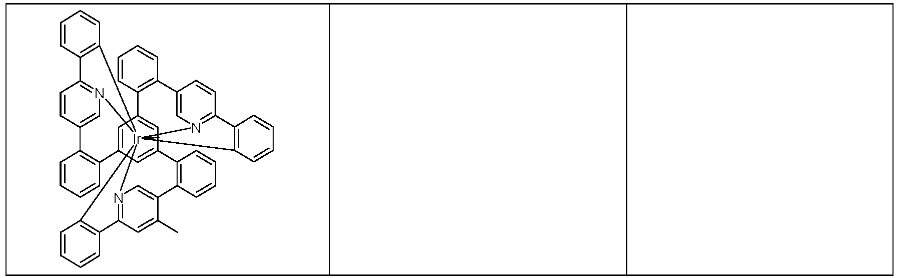

Preferred fluorescent emitting compounds are selected from the class of the arylamines. An arylamine or an aromatic amine in the context of this invention is understood to mean a compound containing three substituted or unsubstituted aromatic or heteroaromatic ring systems bonded directly to the nitrogen. Preferably, at least one of these aromatic or heteroaromatic ring systems is a fused ring system, more preferably having at least 14 aromatic ring atoms. Preferred examples of these are aromatic anthracenamines, aromatic anthracenediamines, aromatic pyrenamines, aromatic pyrenediamines, aromatic chrysenamines or aromatic chrysenediamines. An aromatic anthracenamine is understood to mean a compound in which a diarylamino group is bonded directly to an anthracene group, preferably in the 9 position. An aromatic anthracenediamine is understood to mean a compound in which two diarylamino groups are bonded directly to an anthracene group, preferably in the 9,10 positions. Aromatic pyrenamines, pyrenediamines, chrysenamines and chrysenediamines are defined analogously, where the diarylamino groups are bonded to the pyrene preferably in the 1-position or 1,6-positions. Further preferred emitting compounds are indenofluorenamines and -fluorenediamines, benzoindenofluorenamines and -fluorenediamines, dibenzoindenofluoreneamines and -diamines, and indenofluorene derivatives having fused aryl groups. Likewise preferred are pyrenearylamines. Likewise preferred are benzoindenofluorenamines, benzofluorenamines, extended benzoindenofluorenes, phenoxazines, and fluorene derivatives bonded to furan units or to thiophene units.

Useful matrix materials, preferably for fluorescent emitting compounds, include materials of various substance classes. Preferred matrix materials are selected from the classes of the oligoarylenes (e.g. 2,2',7,7'-tetraphenylspirobifluorene or dinaphthylanthracene), especially of oligoarylenes containing fused aromatic groups, oligoarylenevinylenes (e.g. DPVBi or spiro-DPVBi), polypodal metal complexes, hole-conducting compounds, electron-conducting compounds, especially ketones, phosphine oxides, and sulphoxides, and atropisomers, boronic acid derivatives or benzanthracenes. Particularly preferred matrix materials are selected from the classes of the oligoarylenes comprising naphthalene, anthracene, benzanthracene and/or pyrene or atropisomers of these compounds, the oligoarylenevinylenes, the ketones, the phosphine oxides and the sulphoxides. Very particularly preferred matrix materials are selected from the classes of the oligoarylenes comprising anthracene, benzanthracene, benzophenanthrene and/or pyrene or atropisomers of these compounds. An oligoarylene in the context of this invention shall be understood to mean a compound in which at least three aryl or arylene groups are bonded to one another.

Preferred matrix materials for phosphorescent emitting compounds are, as well as the compounds of the present application, aromatic ketones, aromatic phosphine oxides or aromatic sulphoxides or sulphones, triarylamines, carbazole derivatives, e.g. CBP (N,N-biscarbazolylbiphenyl) or carbazole derivatives, indolocarbazole derivatives, indenocarbazole derivatives, azacarbazole derivatives, bipolar matrix materials, silanes, azaboroles or boronic esters, triazine derivatives, zinc complexes, diazasilole or tetraazasilole derivatives, diazaphosphole derivatives, bridged carbazole derivatives, triphenylene derivatives, or lactams.

Suitable charge transport materials as usable in the hole injection or hole transport layer or electron blocking layer or in the electron transport layer of the electronic device of the invention are, other than the compounds of the present application, for example, the compounds disclosed in Y. Shirota et al., Chem. Rev. 2007, 107(4), 953-1010, or other materials as used in these layers according to the prior art.

Particularly preferable materials for use in a hole transporting layer in the OLED are shown below:

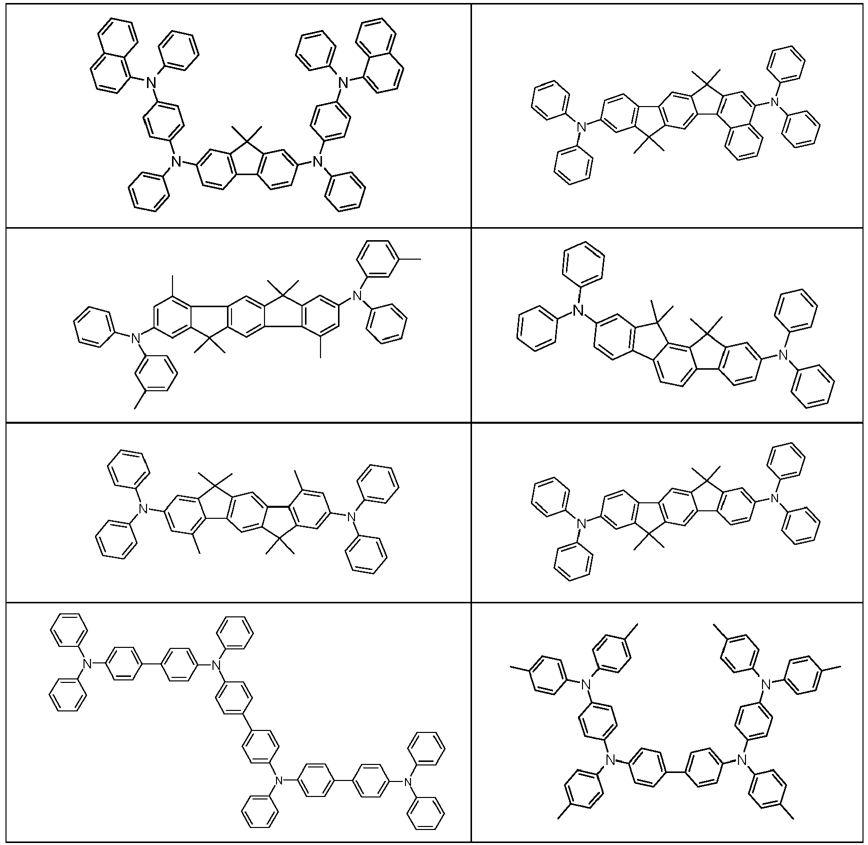

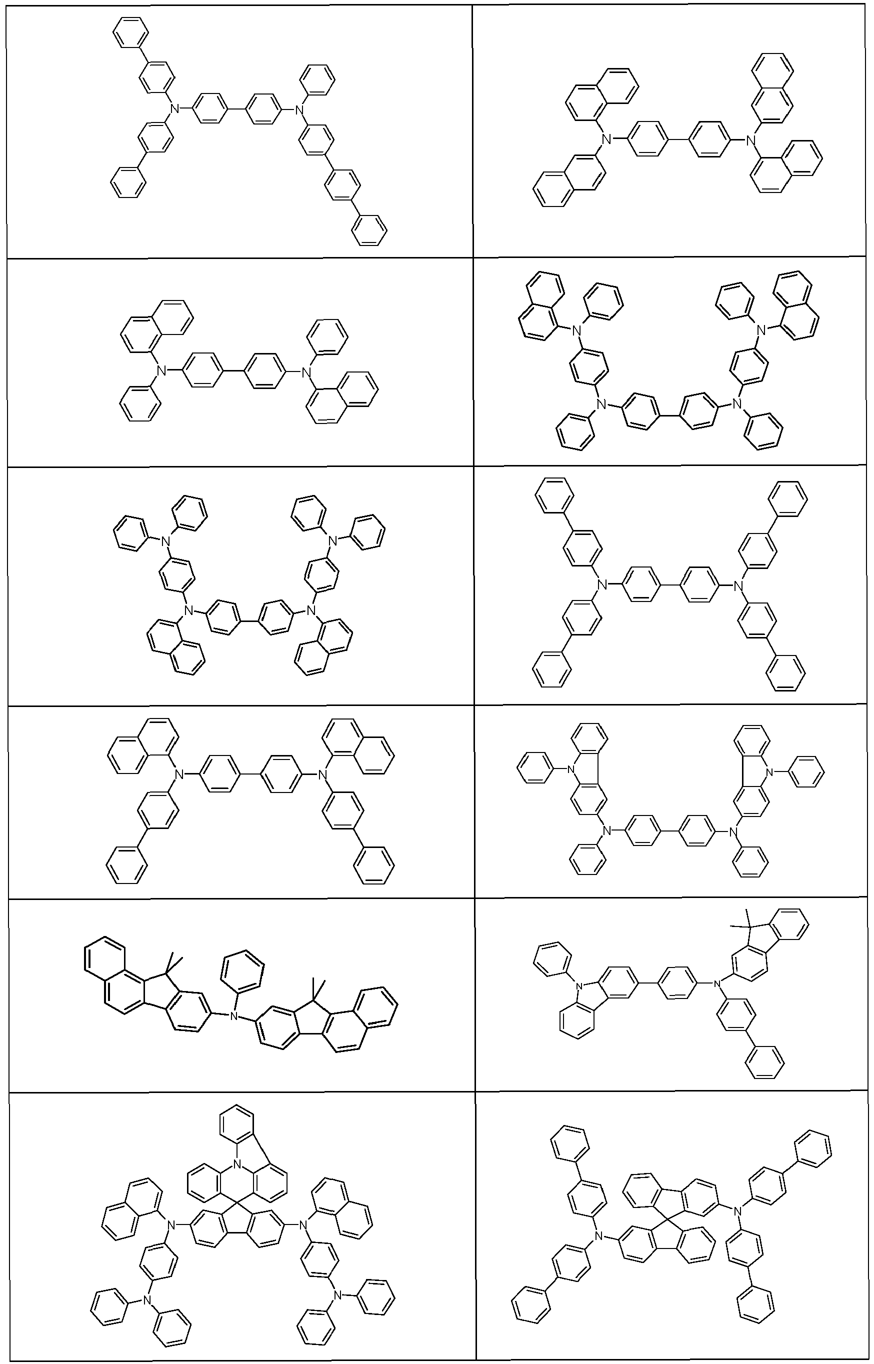

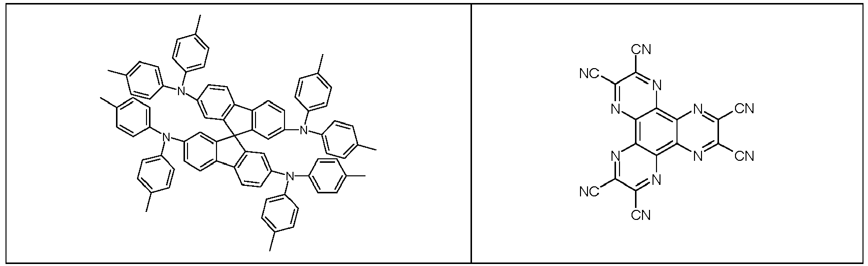

Preferably, the inventive OLED comprises two or more different hole-transporting layers. The compound of the formula (I) may be used here in one or more of or in all the hole-transporting layers. In a preferred embodiment, the compound is used in exactly one or exactly two hole-transporting layers, and other compounds, preferably aromatic amine compounds, are used in the further hole-transporting layers present. Further compounds which are used alongside the compounds of the formula (I), preferably in hole-transporting layers of the OLEDs of the invention, are especially indenofluorenamine derivatives, hexaazatriphenylene derivatives, amine derivatives with fused aromatics, monobenzoindenofluorenamines, dibenzoindenofluorenamines, spirobifluorenamines, fluorenamines, spirodibenzopyranamines, dihydroacridine derivatives, spirodibenzofurans and spirodibenzothiophenes, phenanthrenediarylamines, spirotribenzotropolones, spirobifluorenes with meta-phenyldiamine groups, spirobisacridines, xanthenediarylamines, and 9,10-dihydroanthracene spiro compounds with diarylamino groups.

Very particular preference is given to the use of spirobifluorenes substituted by diarylamino groups in the 4 position as hole-transporting compounds, and to the use of spirobifluorenes substituted by diarylamino groups in the 2 position as hole-transporting compounds.

Materials used for the electron transport layer may be any materials as used according to the prior art as electron transport materials in the electron transport layer. Especially suitable are aluminum complexes, for example Alqs, zirconium complexes, for example Zrq₄, lithium complexes, for example Liq, benzimidazole derivatives, triazine derivatives, pyrimidine derivatives, pyridine derivatives, pyrazine derivatives, quinoxaline derivatives, quinoline derivatives, oxadiazole derivatives, aromatic ketones, lactams, boranes, diazaphosphole derivatives and phosphine oxide derivatives.

Particularly preferably electron transporting material for use in the OLEDs are shown below:

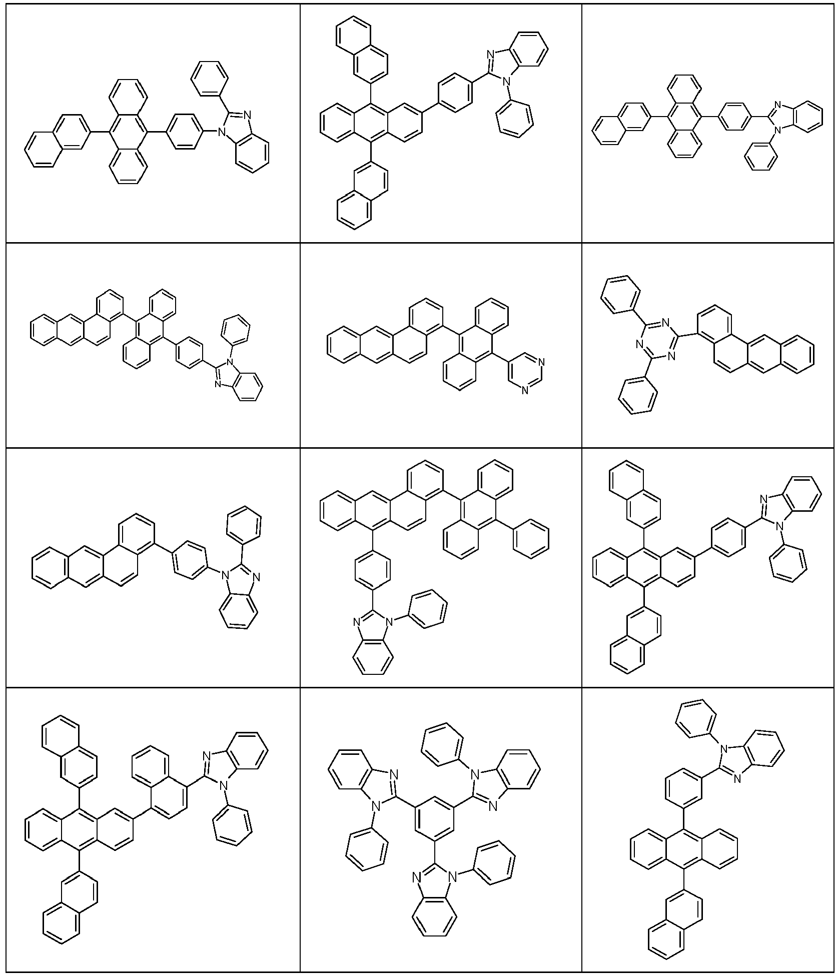

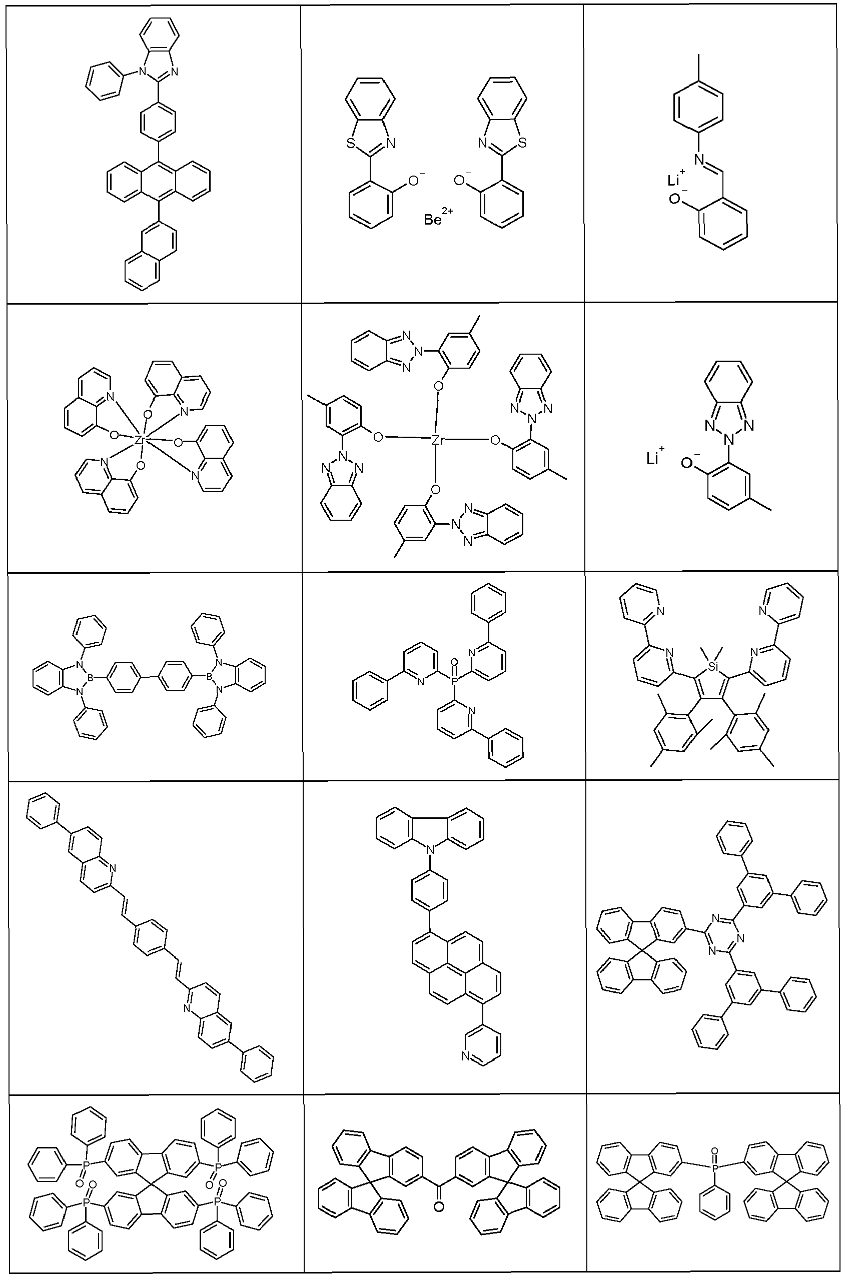

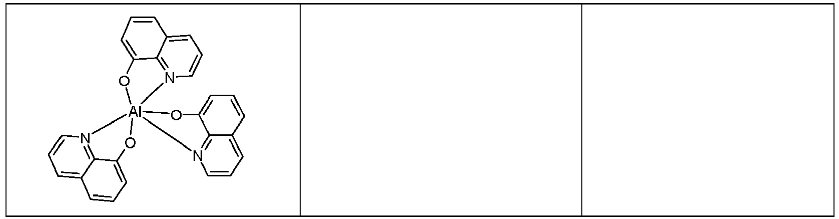

Preferred cathodes of the electronic device are metals having a low work function, metal alloys or multilayer structures composed of various metals, for example alkaline earth metals, alkali metals, main group metals or lanthanoids (e.g. Ca, Ba, Mg, Al, In, Mg, Yb, Sm, etc.). Additionally suitable are alloys composed of an alkali metal or alkaline earth metal and silver, for example an alloy composed of magnesium and silver. In the case of multilayer structures, in addition to the metals mentioned, it is also possible to use further metals having a relatively high work function, for example Ag or Al, in which case combinations of the metals such as Ca/Ag, Mg/Ag or Ba/Ag, for example, are generally used. It may also be preferable to introduce a thin interlayer of a material having a high dielectric constant between a metallic cathode and the organic semiconductor. Examples of useful materials for this purpose are alkali metal or alkaline earth metal fluorides, but also the corresponding oxides or carbonates (e.g. LiF, Li₂O, BaF₂, MgO, NaF, CsF, Cs₂CO₃, etc.). It is also possible to use lithium quinolinate (LiQ) for this purpose. The layer thickness of this layer is preferably between 0.5 and 5 nm.

Preferred anodes are materials having a high work function. Preferably, the anode has a work function of greater than 4.5 eV versus vacuum. Firstly, metals having a high redox potential are suitable for this purpose, for example Ag, Pt or Au. Secondly, metal/metal oxide electrodes (e.g. Al/Ni/NiOₓ, Al/PtOₓ) may also be preferred. For some applications, at least one of the electrodes has to be transparent or partly transparent in order to enable the irradiation of the organic material (organic solar cell) or the emission of light (OLED, O-laser). Preferred anode materials here are conductive mixed metal oxides. Particular preference is given to indium tin oxide (ITO) or indium zinc oxide (IZO). Preference is further given to conductive doped organic materials, especially conductive doped polymers. In addition, the anode may also consist of two or more layers, for example of an inner layer of ITO and an outer layer of a metal oxide, preferably tungsten oxide, molybdenum oxide or vanadium oxide.

The device is structured appropriately (according to the application), contact-connected and finally sealed, in order to rule out damaging effects by water and air.

In a preferred embodiment, the electronic device is characterized in that one or more layers are coated by a sublimation process. In this case, the materials are applied by vapour deposition in vacuum sublimation systems at an initial pressure of less than 10⁻⁵ mbar, preferably less than 10⁻⁶ mbar. In this case, however, it is also possible that the initial pressure is even lower, for example less than 10⁻⁷ mbar.

Preference is likewise given to an electronic device, characterized in that one or more layers are coated by the OVPD (organic vapour phase deposition) method or with the aid of a carrier gas sublimation. In this case, the materials are applied at a pressure between 10⁻⁵ mbar and 1 bar. A special case of this method is the OVJP (organic vapour jet printing) method, in which the materials are applied directly by a nozzle and thus structured (for example M. S. Arnold et al., Appl. Phys. Lett. 2008, 92, 053301).

Preference is additionally given to an electronic device, characterized in that one or more layers are produced from solution, for example by spin-coating, or by any printing method, for example screen printing, flexographic printing, nozzle printing or offset printing, but more preferably LITI (light-induced thermal imaging, thermal transfer printing) or inkjet printing. For this purpose, soluble compounds are needed. High solubility can be achieved by suitable substitution of the compounds.

It is further preferable that an electronic device of the invention is produced by applying one or more layers from solution and one or more layers by a sublimation method.

According to the invention, the electronic devices comprising one or more compounds of formula (I) can be used in displays, as light sources in lighting applications and as light sources in medical and/or cosmetic applications.

### Examples

### A) Synthesis examples

All reactions are carried out under nitrogen and by using dried solvents unless stated otherwise.

### Example 1: Preparation of compound (V)

Synthetic procedure for the preparation of compound (V):

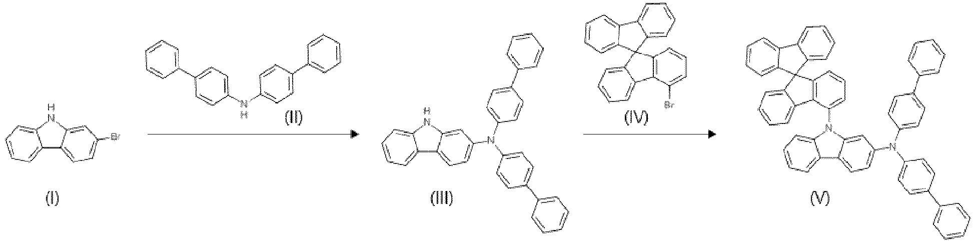

### Preparation of compound (III)

25.0 g (0.1 mol) of compound (I) and 32 g (0.1 mol) of compound (II) are suspended in 500 mL of toluene under Ar atmosphere. 1.1 g (0.05 mol) of Pd(OAc)₂ are added to the flask and stirred under Ar atmosphere then 2.0 mL of a 1 M tri-tert-butylphosphine solution and 57.6 g (0.6 mol) of sodium t-butoxide are added to the flask. The reaction mixture is stirred under reflux for 24 h. After cooling, the organic phase is separated off, washed three times with 200 mL of water, dried over magnesium sulfate, filtered and subsequently evaporated to dryness. The residue is purified by column chromatography on silica gel using a mixture of ethylacetate/heptane (1:3). The yield is 39.0 g (0.08 mol), corresponding to 80% of theory.

### Preparation of compound (V)

39.0 g (0.08 mol) of compound (III) and 31.6 g (0.08 mol) of compound (IV) are suspended in 500 mL of toluene under Ar atmosphere. 1.1 g (0.05 mol) of Pd(OAc)₂ are added to the flask and stirred under Ar atmosphere then 2.0 mL of a 1 M tri-tert-butylphosphine solution and 57.6 g (0.6 mol) of sodium t-butoxide are added to the flask. The reaction mixture is stirred under reflux for 24 h. After cooling, the organic phase is separated off, washed three times with 200 mL of water, dried over magnesium sulfate, filtered and subsequently evaporated to dryness. The residue is purified by column chromatography on silica gel using a mixture of ethylacetate/heptane (1:3). The yield is 60.0 g (0.075 mol), corresponding to 75% of theory.

The following compounds can be obtained analogously:

| Bromo-carbazole | Amine | Bromide | Product |
|---|---|---|---|
| 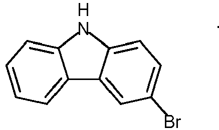 | 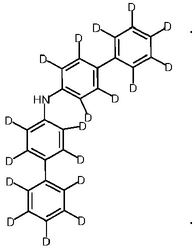 | 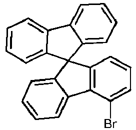 | 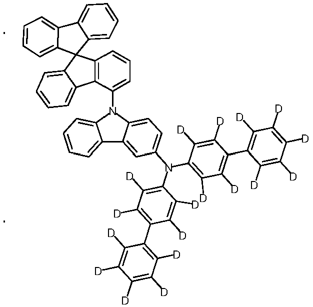 |
| 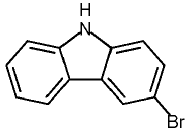 | 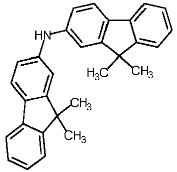 | 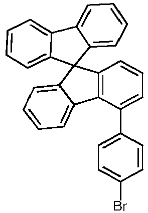 | 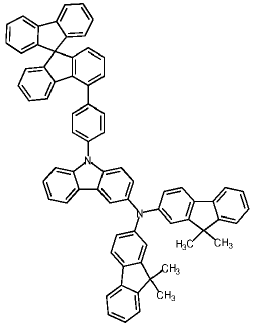 |
| 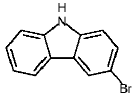 | 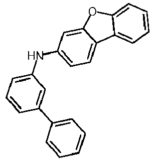 | 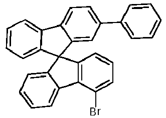 | 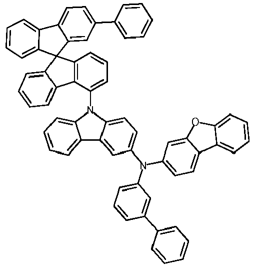 |
| 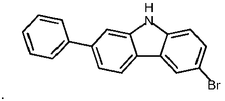 | 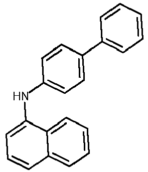 | 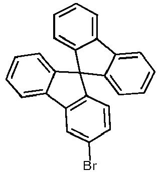 | 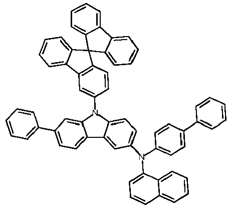 |
| 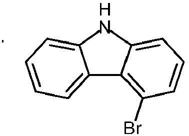 | 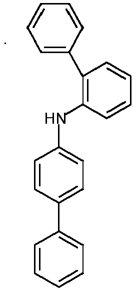 | 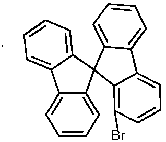 | 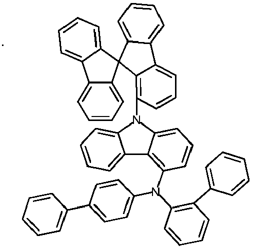 |
| 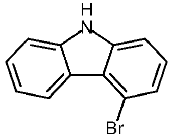 | 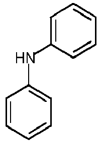 | 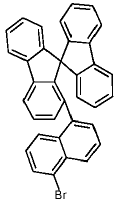 | 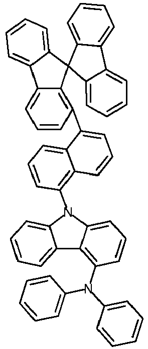 |
| 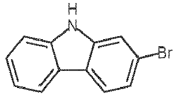 | 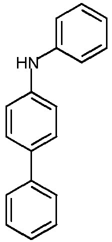 | 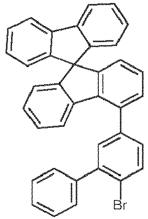 | 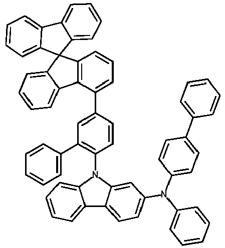 |
| 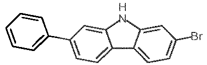 | 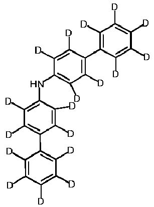 | 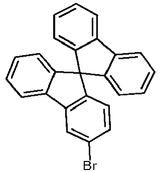 | 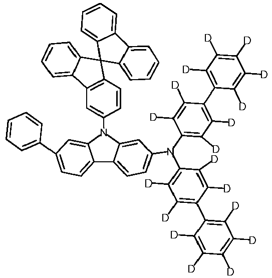 |
| 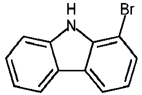 | 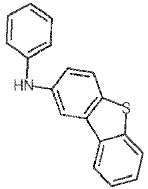 | 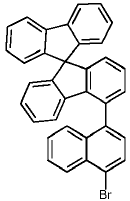 | 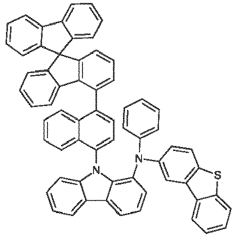 |

### Example 2: Preparation of compound (VII)

### Synthetic procedure for the preparation of compound (VII):

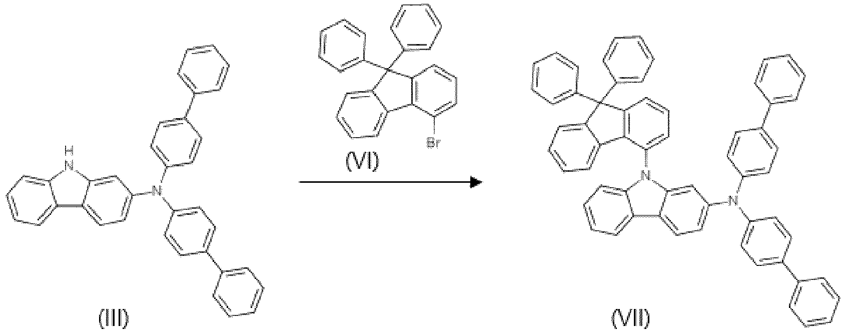

### Preparation of compound (VII)

48.6 g (0.1 mol) of compound (III) and 39.7 g (0.1 mol) of compound (VI) are suspended in 500 mL of toluene under Ar atmosphere. 1.1 g (0.05 mol) of Pd(OAc)₂ are added to the flask and stirred under Ar atmosphere then 2.0 mL of a 1 M tri-tert-butylphosphine solution and 57.6 g (0.6 mol) of sodium t-butoxide are added to the flask. The reaction mixture is stirred under reflux for 24 h. After cooling, the organic phase is separated off, washed three times with 200 mL of water, dried over magnesium sulfate, filtered and subsequently evaporated to dryness. The residue is purified by column chromatography on silica gel using a mixture of ethylacetate/heptane (1:3). The yield is 62.0 g (0.077 mol), corresponding to 77% of theory.

The following compounds can be obtained analogously:

| Bromo-carbazole | Amine | Bromide | Product |
|---|---|---|---|
| 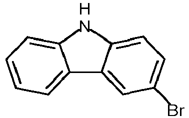 | 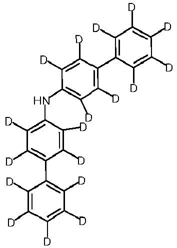 | 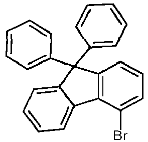 | 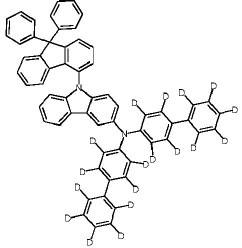 |
| 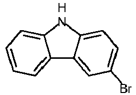 | 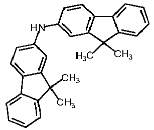 | 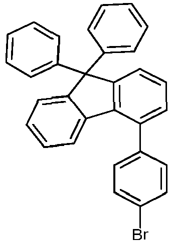 | 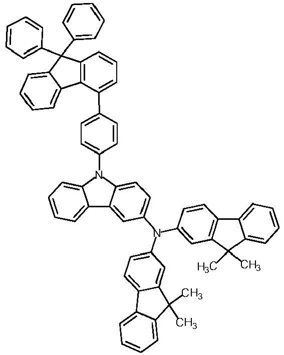 |
| 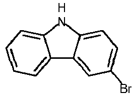 | 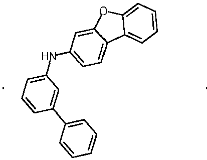 | 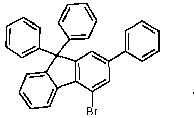 | 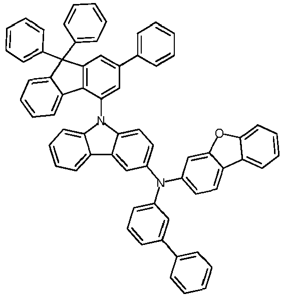 |
| 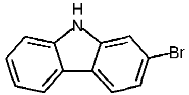 | 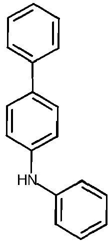 | 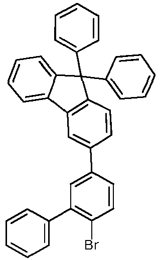 | 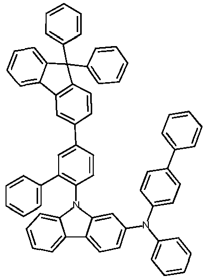 |
| 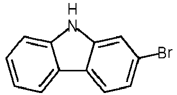 | 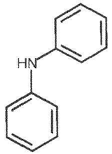 | 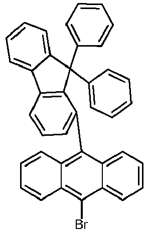 | 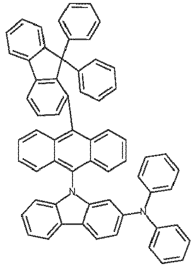 |
| 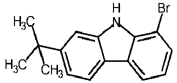 | 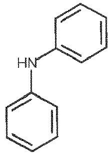 | 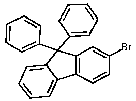 | 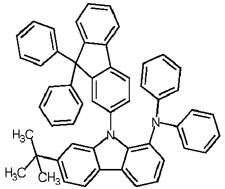 |
| 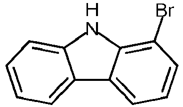 | 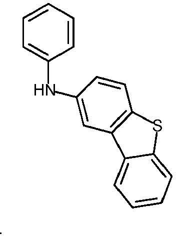 | 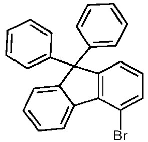 | 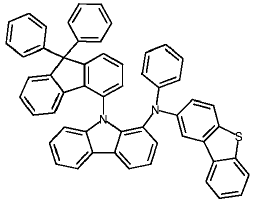 |
| 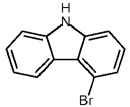 | 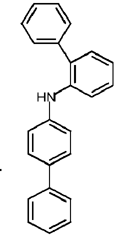 | 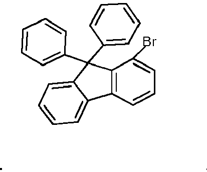 | 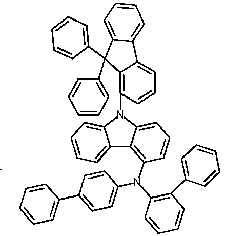 |
| 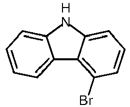 | 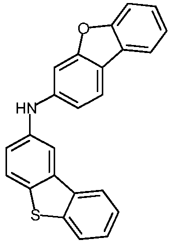 | 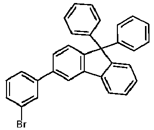 | 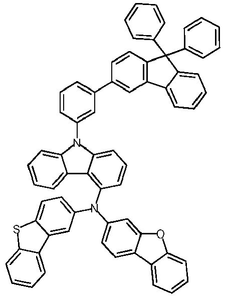 |

### B) Device Examples

OLED devices are prepared according to the following process:
The substrates used are glass plates coated with structured ITO (indium tin oxide) in a thickness of 50 nm. The OLEDs have the following layer structure: substrate / hole-injection layer (HIL) / hole-transport layer (HTL) / hole-injection layer (HTL2) / electron-blocking layer (EBL) / emission layer (EML) / electron-transport layer (ETL) / electron-injection layer (EIL) and finally a cathode. The cathode is formed by an aluminium layer with a thickness of 100nm. The precise structure of the prepared OLEDs is shown in Table 1. The materials required for the production of the OLEDs are shown in Table 3.

All materials are evaporated by thermal vapour deposition in a vacuum chamber. The emission layer always consists of minimum one matrix material (host material) and an emitting dopant (emitter), which is admixed with the matrix material or matrix materials in a certain proportion by volume by coevaporation. An expression such as H1:SEB (5%) denotes that material H1 is present in the layer in a proportion by volume of 95% and SEB is present in the layer in a proportion of 5%. Analogously, other layers may also consist of a mixture of two or more materials.

The OLEDs are characterized by standard methods. For this purpose, the electroluminescence spectra and the external quantum efficiency (EQE, measured in per cent) as a function of the luminous density, calculated from current/voltage/luminous density characteristic lines (IUL characteristic lines) assuming Lambertian emission characteristics, and the lifetime are determined. The expression EQE @ 10mA/cm² denotes the external quantum efficiency at an operating current density of 10mA/cm². LT80 @ 60mA/cm² is the lifetime until the OLED has dropped from its initial luminance of i.e. 5000cd/m² to 80% of the initial intensity, i.e. to 4000cd/m² without using any acceleration factor. The data for the various OLEDs containing inventive and comparative materials are summarised in Table 2.

Compounds according to the invention are suitable as HIL, HTL, EBL or matrix material in the EML in OLEDs. They are suitable as a single layer, but also as mixed component as HIL, HTL, EBL or within the EML.

Compared with compounds from prior art (V1 and V2), the samples comprising the compounds according to the invention (E1 and E2) exhibit both higher efficiencies and also improved lifetimes in singlet blue emitting devices.

| Table 1: Structure of the OLEDs | | | | | | | |
|---|---|---|---|---|---|---|---|
| Ex | HIL | HTL | HTL2 | EBL | EML | ETL | EIL |
| | Thickness / nm | Thickness / nm | Thickness / nm | Thickness / nm | Thickness / nm | Thickness / nm | Thickness / nm |
| E1 | HIM:F4TCNQ (5%) 20nm | HIM 160nm | (V):F4TCNQ( 5%) 20nm | (V) 10nm | H1:SEB(5%) 20 nm | ETM:LiQ (50%) 30 nm | LiQ 1nm |
| E2 | HIM:F4TCNQ (5%) 20nm | HIM 160nm | (VII):F4TCN Q(5%) 20nm | (VII) 10nm | H1:SEB(5%) 20 nm | ETM:LiQ (50%) 30 nm | LiQ 1nm |
| V1 | HIM:F4TCNQ (5%) 20nm | HIM 160nm | (A):F4TCNQ( 5%) 20nm | (A) 10nm | H1:SEB(5%) 20 nm | ETM:LiQ (50%) 30 nm | LiQ 1nm |
| V2 | HIM:F4TCNQ (5%) 20nm | HIM 160nm | (B):F4TCNQ( 5%) 20nm | ((B) 10nm | H1:SEB(5%) 20 nm | ETM:LiQ (50%) 30 nm | LiQ 1nm |

| Table 2: Data for the OLEDs | | |
|---|---|---|
| Ex. | EQE @ 10mA/cm² | LT80 @ 60mA/cm² |
| E1 | 8.0 | 380 |
| E2 | 8.3 | 370 |
| V1 | 7.1 | 290 |
| V2 | 7.1 | 290 |

| Table 3 - Structures of the materials used | | | |
|---|---|---|---|
| 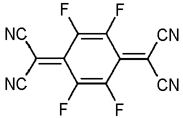 | 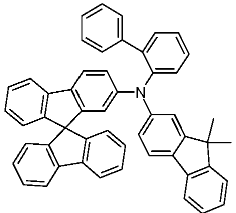 | | 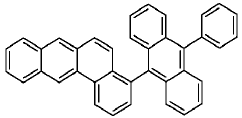 |
| F4TCNQ | HIM | | H1 |
| 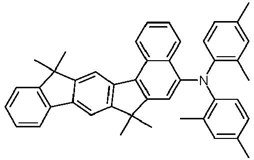 | 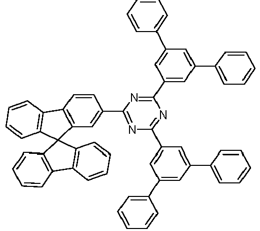 | | 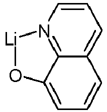 |
| SEB | ETM | | LiQ |
| 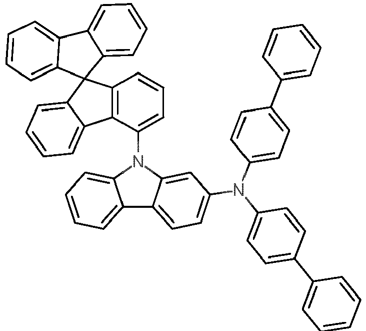 | | 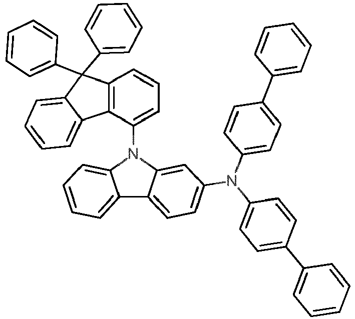 | |
| (V) | | (VII) | |
| 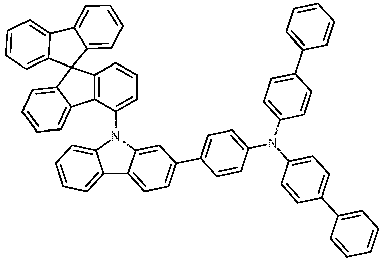 | | 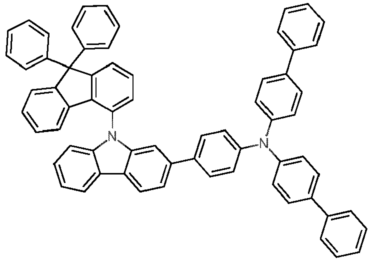 | |
| (A) | | (B) | |

## Claims

1. Organic electroluminescent device comprising two, three or four hole-transporting layers between the anode and the emitting layer, where two of the hole-transporting layers comprise a compound of the formula (1), 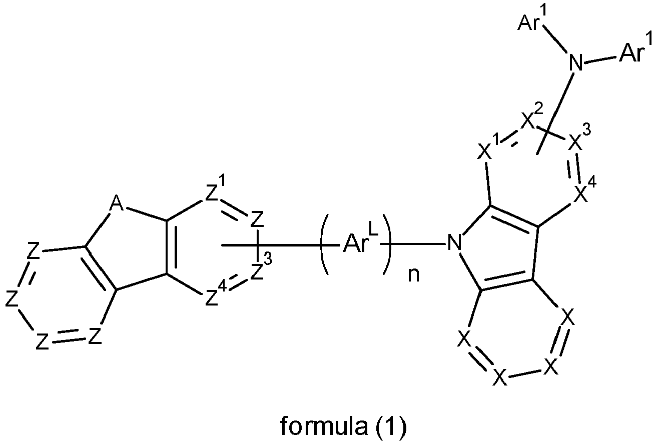 where the following applies to the symbols and indices used:
A stands for a group of formula (A-1) or (A-2), 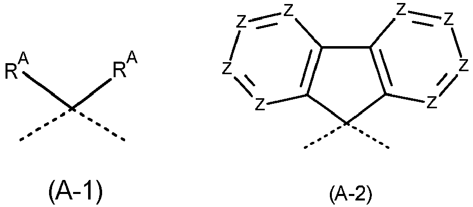 where the dashed bonds represent the bonds to the rest of the formula (1);
Z stands, on each occurrence, identically or differently, for CR^{Z};
Z¹, Z³, Z⁴ correspond to Z; with the proviso that, when n ≥ 1, then the group Ar^{L} is bonded to one of the group Z¹, Z³, Z⁴, which stands for C in this case and when n = 0, then the group Ar^{L} is absent and the carbazole moiety represented in formula (1) is directly bonded via its nitrogen atom to one of the groups Z¹, Z³, Z⁴, which stands for C in this case;
X stands, on each occurrence, identically or differently, for CR^{X};
X¹, X², X³, X⁴ correspond to X; with the proviso that the group -NAr¹Ar¹ as depicted in formula (1) is bonded to one of the groups X¹, X², X³, X⁴, which stands for C in this case;
Ar^{L} stands on each occurrence, identically or differently, for an aromatic ring system having 6 to 40 aromatic ring atoms and which may be substituted by one or more R radicals, or for an heteroaromatic ring systems having 5 to 40 aromatic ring atoms, which may be substituted by one or more R radicals;
Ar¹ stands on each occurrence, identically or differently, for an aromatic ring system having 6 to 60 aromatic ring atoms and which may be substituted by one or more R radicals, or for an heteroaromatic ring systems having 5 to 60 aromatic ring atoms, which may be substituted by one or more R radicals;
R^{A}, R^{X}, R^{Z} stand on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, a straight-chain alkyl, alkoxy or thioalkyl group having 1 to 40 C atoms or branched or cyclic alkyl, alkoxy or thioalkyl groups having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals; where two adjacent radicals R^{A}, two adjacent radicals R^{X}, two adjacent radicals R^{Z} may form a mono- or polycyclic, aliphatic ring system or aromatic ring system, which may be substituted by one or more radicals R;
R stands on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R')₃, B(OR')₂, OSO₂R', a straight-chain alkyl, alkoxy or thioalkyl group having 1 to 40 C atoms or branched or cyclic alkyl, alkoxy or thioalkyl groups having 3 to 40 C atoms, each of which may be substituted by one or more radicals R', where in each case one or more non-adjacent CH₂ groups may be replaced by R'C=CR', C=C, Si(R')₂, Ge(R')₂, Sn(R')₂, C=O, C=S, C=Se, P(=O)(R'), SO, SO₂, O, S or CONR' and where one or more H atoms may be replaced by D, F, Cl, Br, I, CN or NO₂, an aromatic or heteroaromatic ring system having 5 to 60 aromatic ring atoms, which may in each case be substituted by one or more radicals R', or an aryloxy group having 5 to 60 aromatic ring atoms, which may be substituted by one or more radicals R', where two adjacent radicals R may form a mono- or polycyclic, aliphatic ring system or aromatic ring system, which may be substituted by one or more radicals R';
Ar is on each occurrence, identically or differently, an aromatic or heteroaromatic ring system having 5 to 24 aromatic ring atoms, which may in each case also be substituted by one or more radicals R';
R stands on each occurrence, identically or differently, for H, D, F, Cl, Br, I, CN, a straight-chain alkyl, alkoxy or thioalkyl group having 1 to 20 C atoms or branched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, Cl, Br or I, or an aromatic or heteroaromatic ring system having 5 to 24 C atoms; and
n is 0, 1, 2 or 3;
and where a compound of formula (1) is used in combination with a p-dopant in a hole-transport layer.

2. Organic electroluminescent device according to claim 1, **characterized in that** the compound of formula (1) is selected from compounds of the formula (2), (3) or (4), 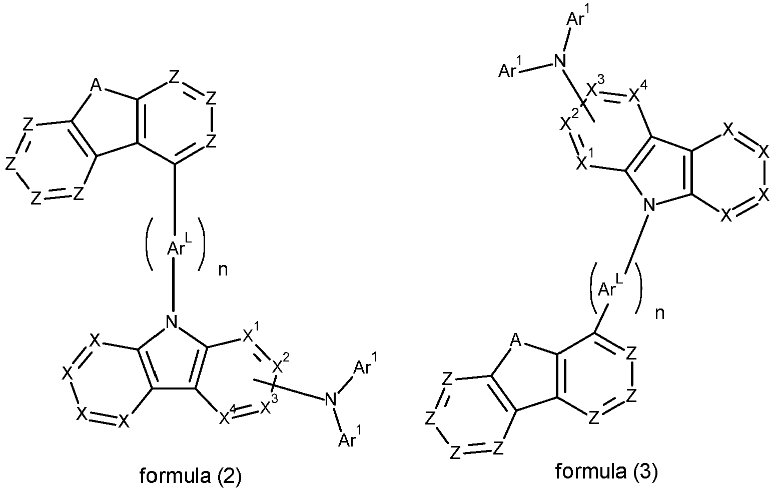 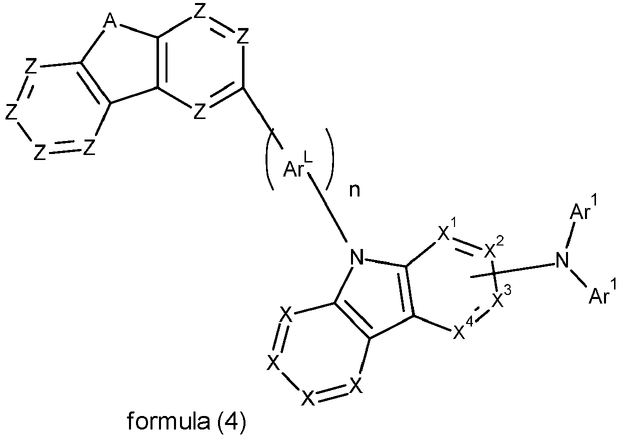 where the symbols have the same meaning as in claim 1.

3. Organic electroluminescent device according to claim 1 or 2, **characterized in that** the compound of formula (1) is selected from compounds of one of the formulae (2-1) to (4-2), 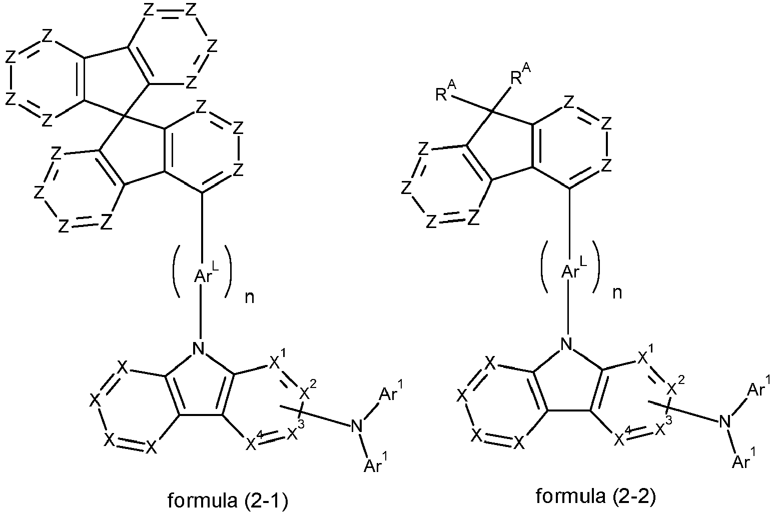 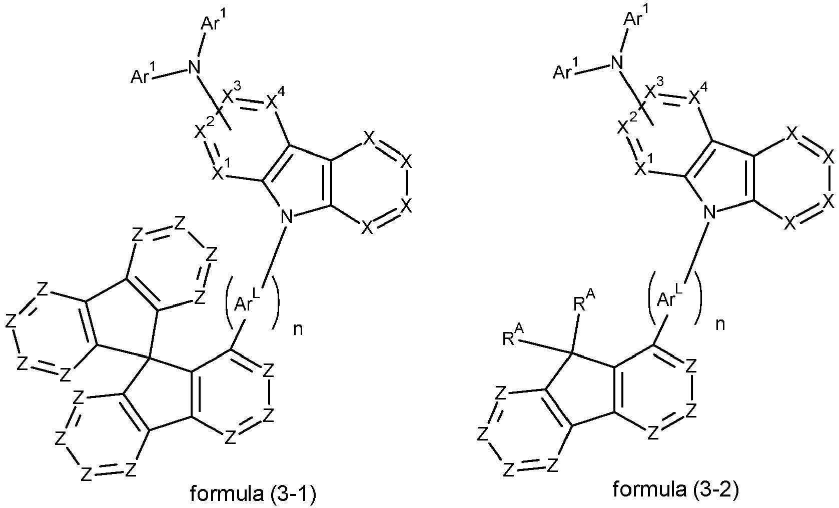 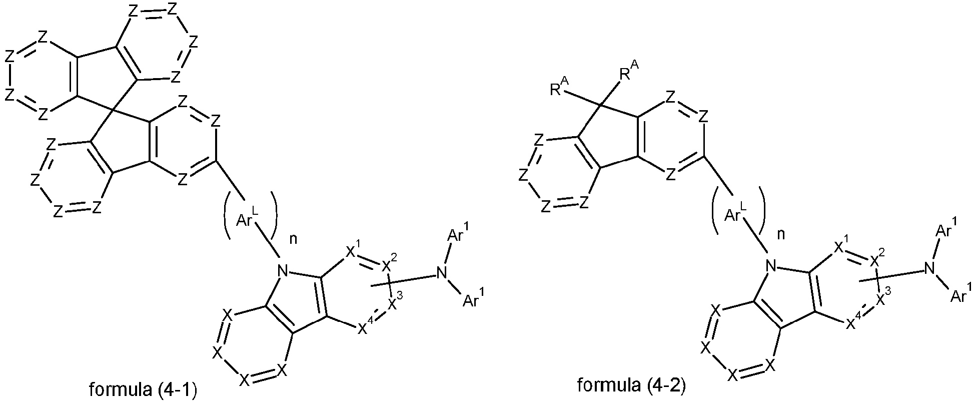 where the symbols have the same meaning as in claim 1.

4. Organic electroluminescent device according to one or more of the preceding claims, **characterized in that** n is 0 or 1.

5. Organic electroluminescent device according to one or more of the preceding claims, **characterized in that** the compound of formula (1) is selected from the compounds of one of the formulae (2-1-1) to (4-2-2), 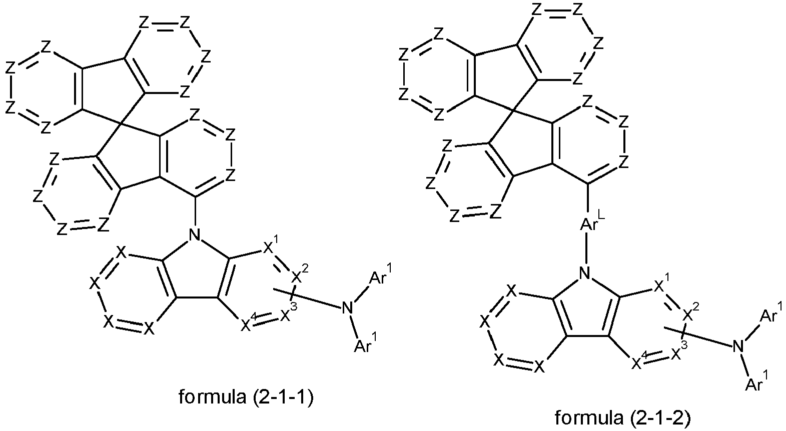 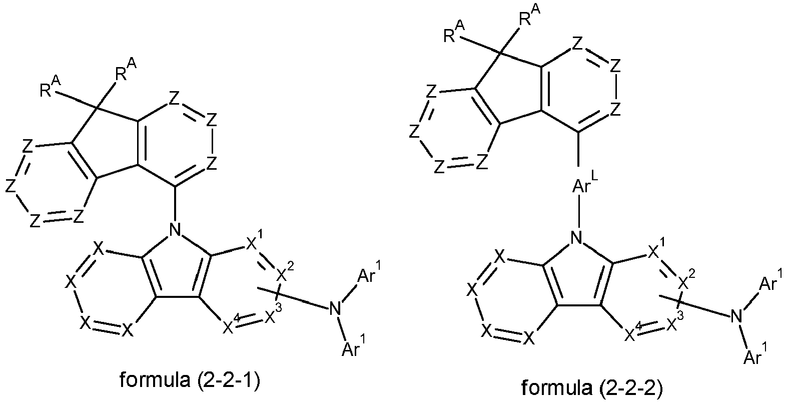 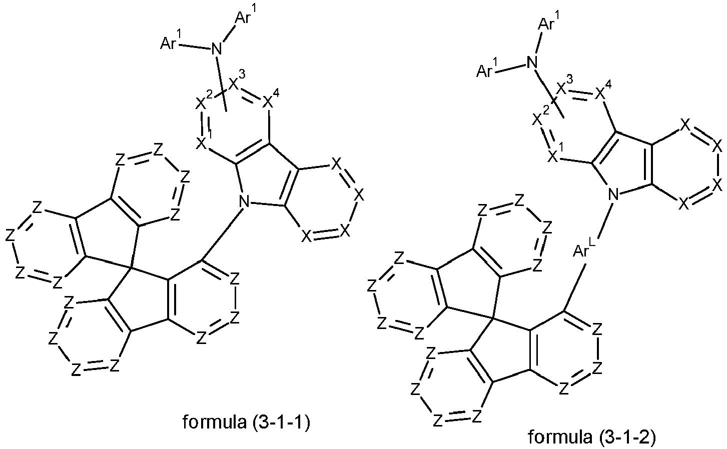 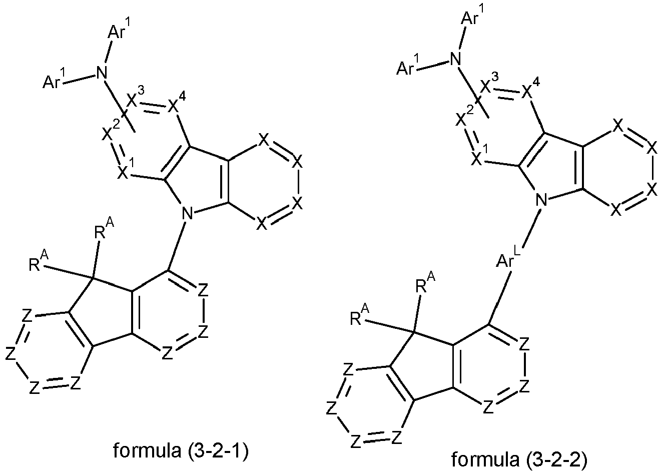 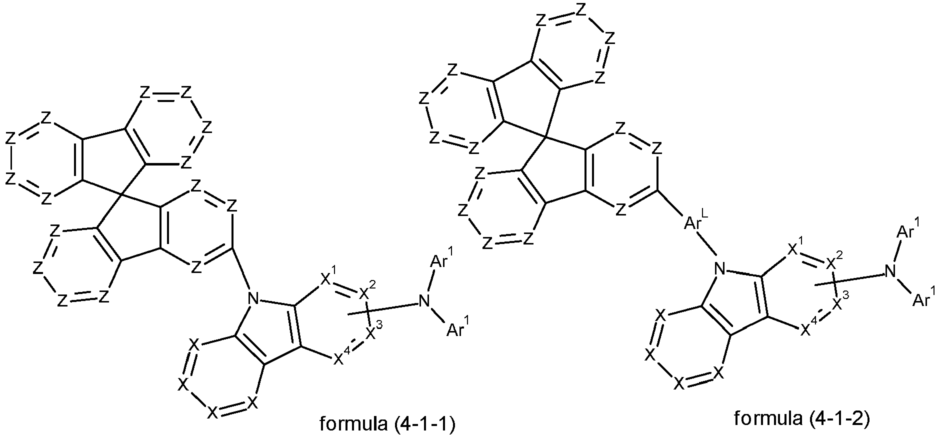 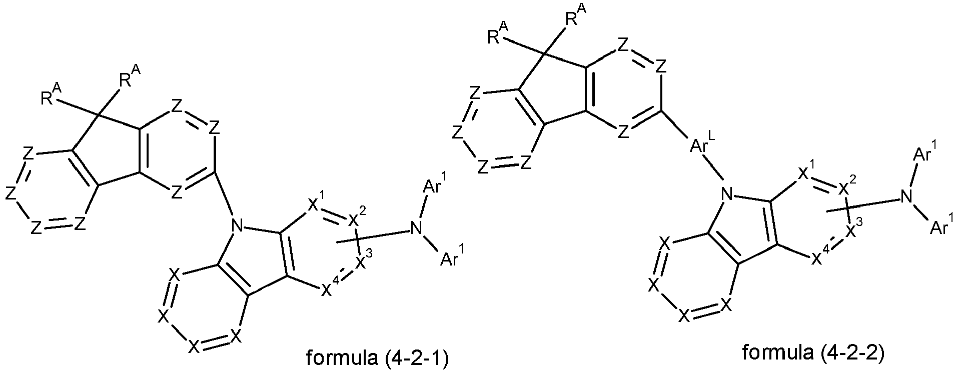 where the symbols have the same meaning as in claim 1.

6. Organic electroluminescent device according to one or more of the preceding claims, **characterized in that** the compound of formula (1) is selected from the compounds of one of the formulae (2-1-1-1) to (4-2-2-4), 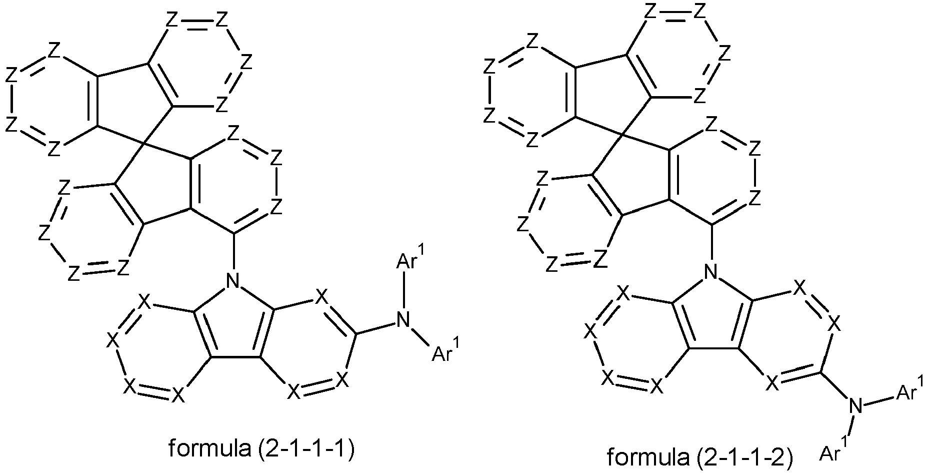 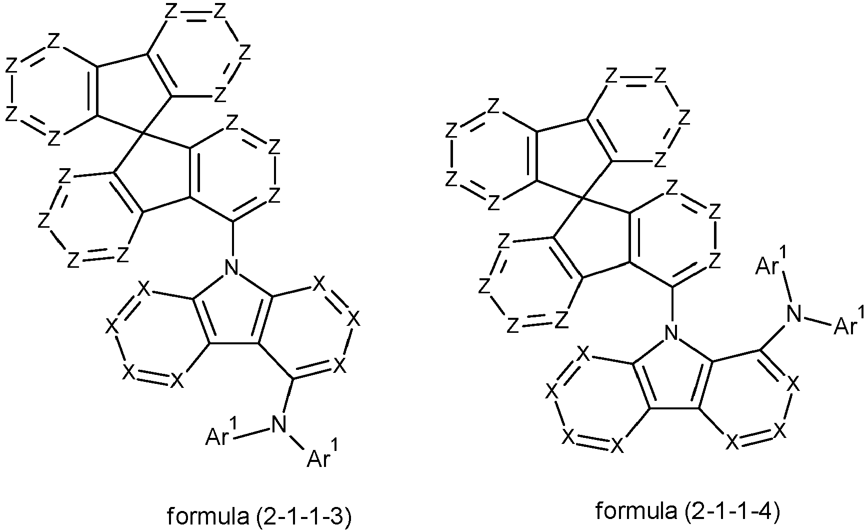 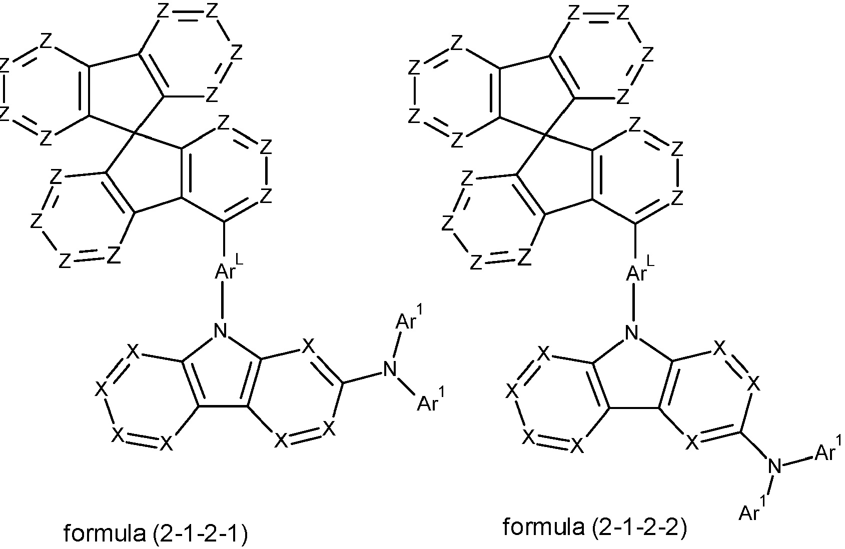 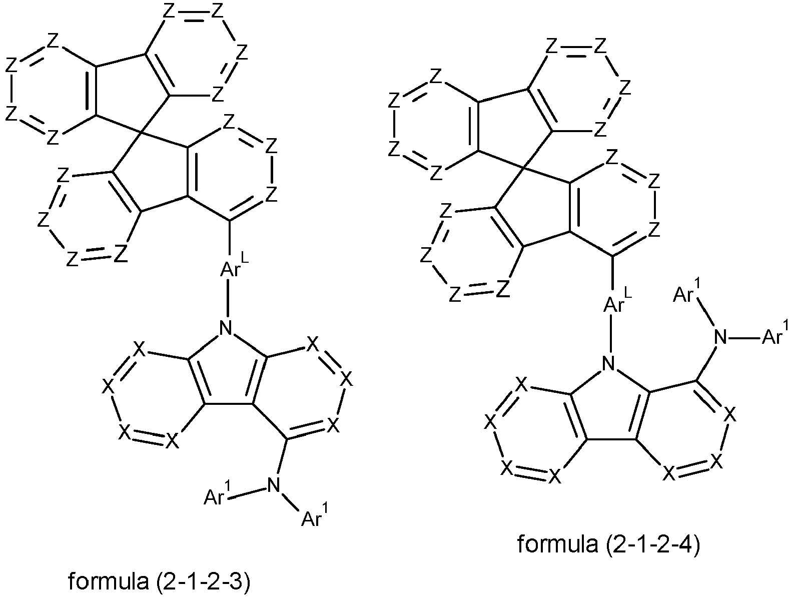 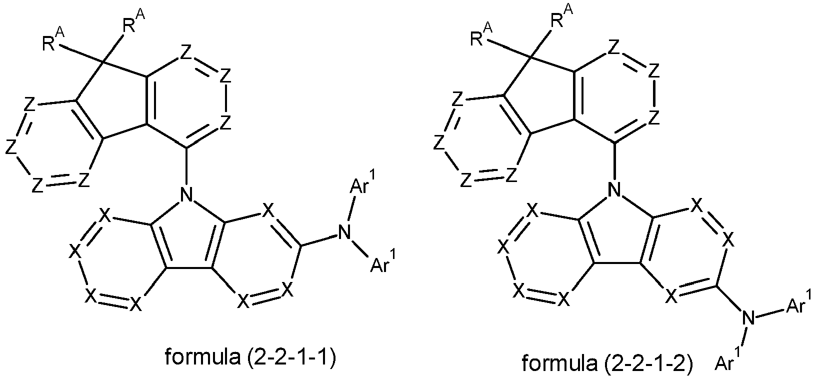 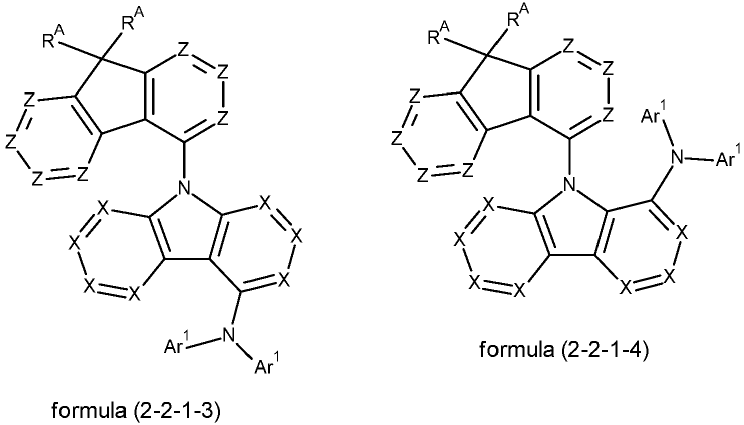 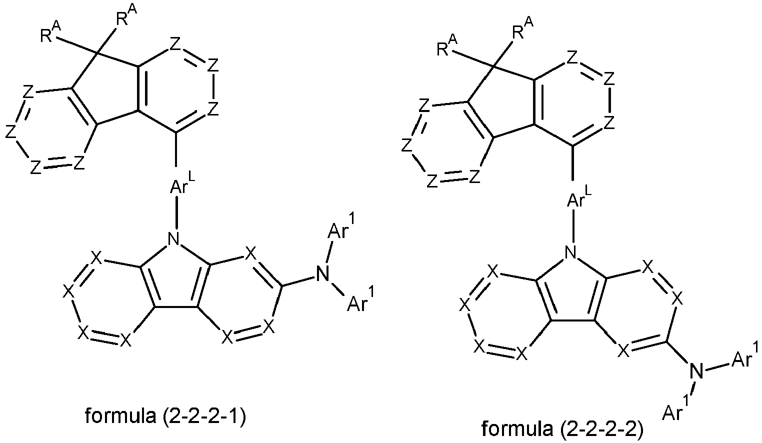 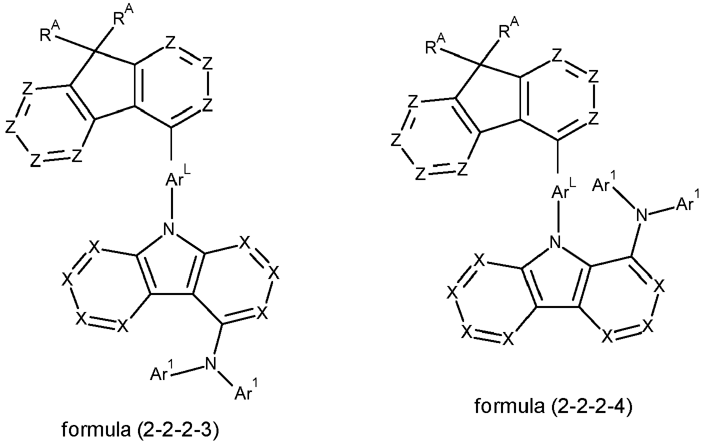 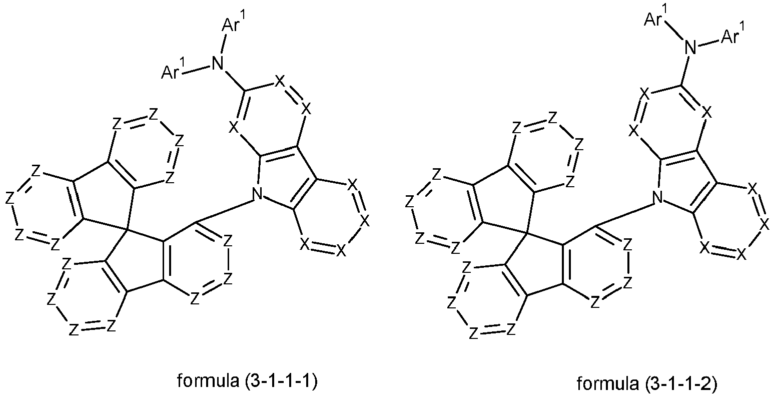 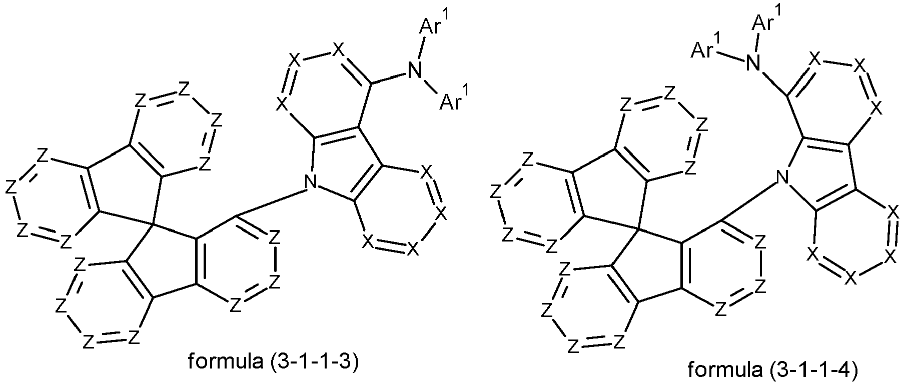 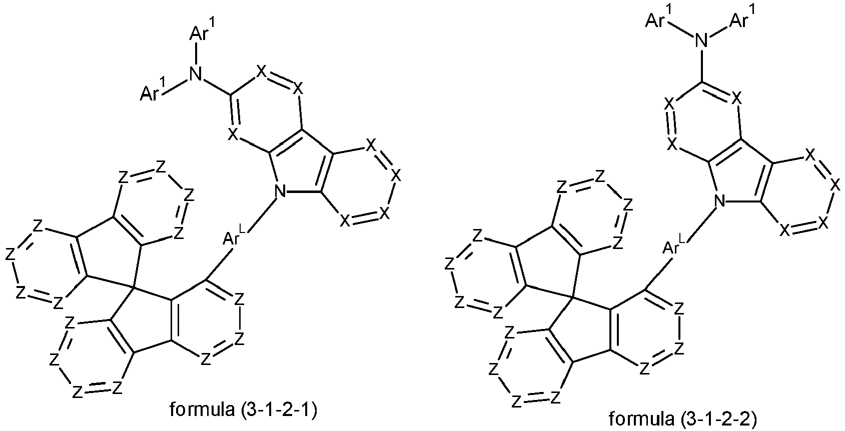 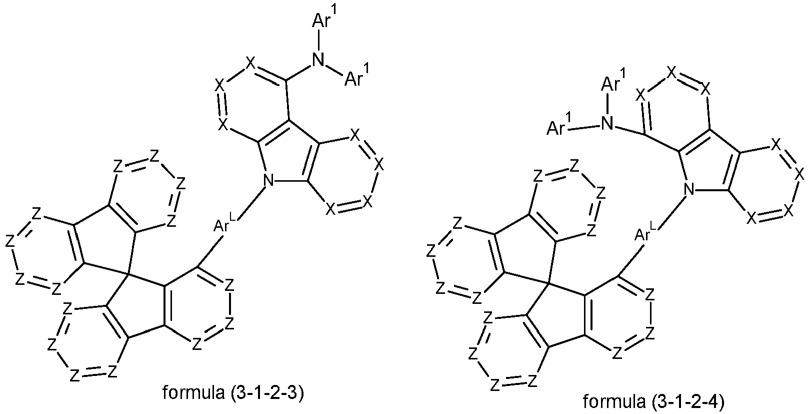 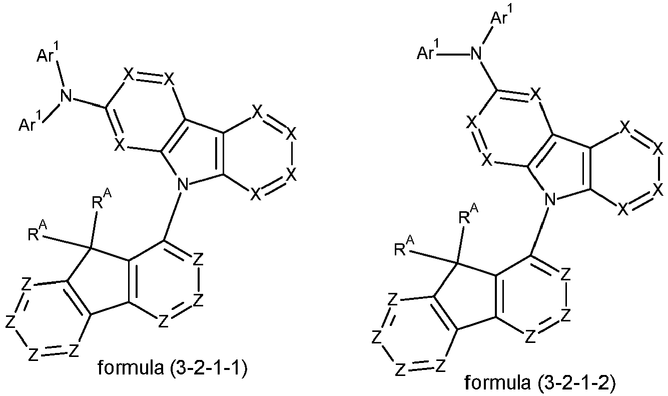 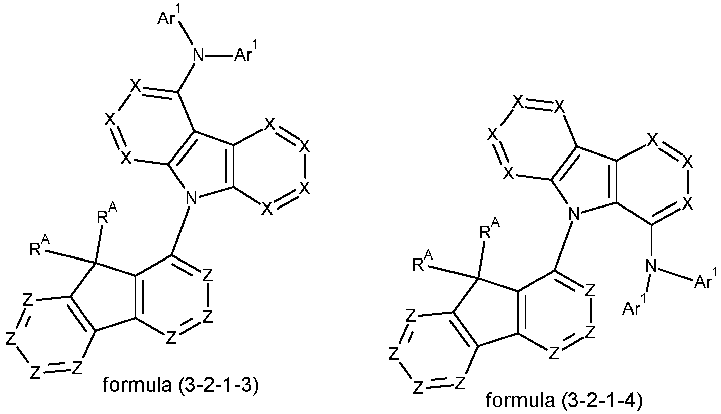 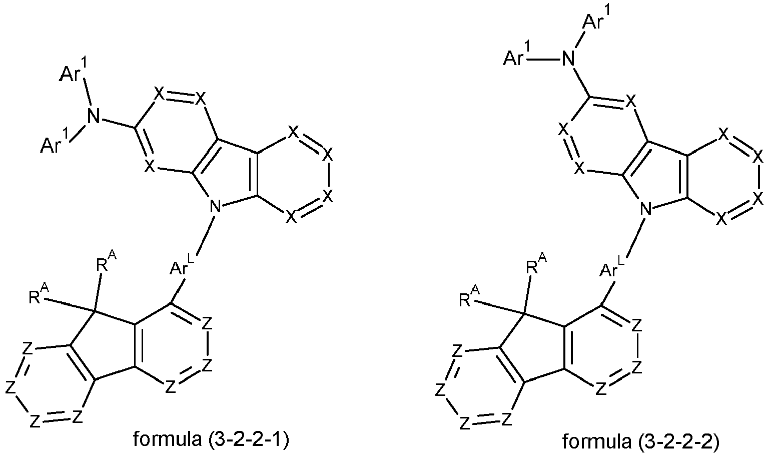 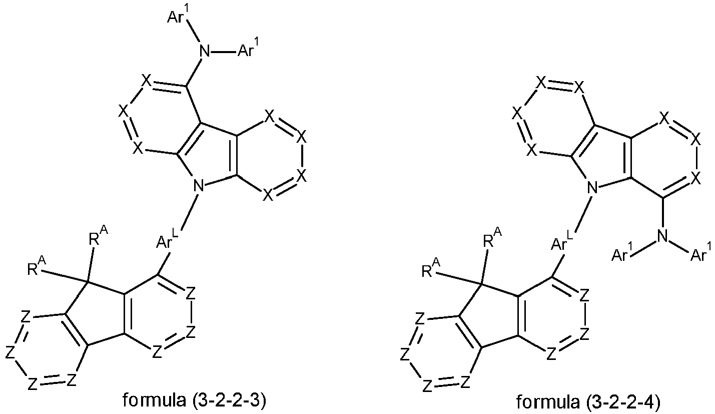 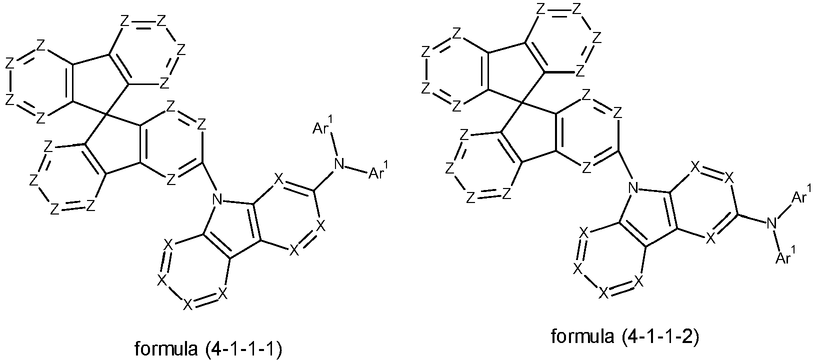 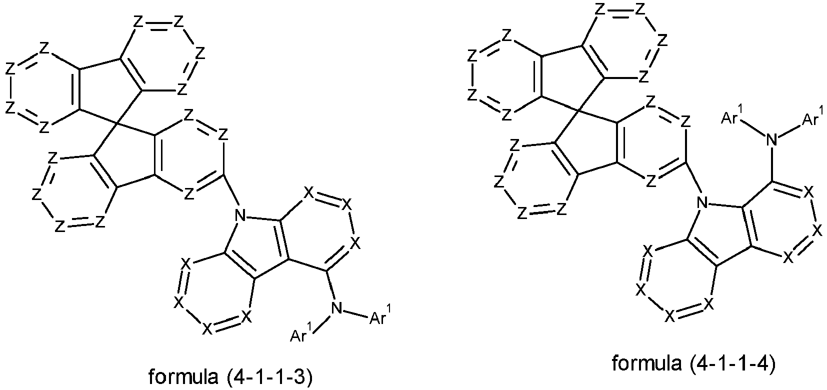 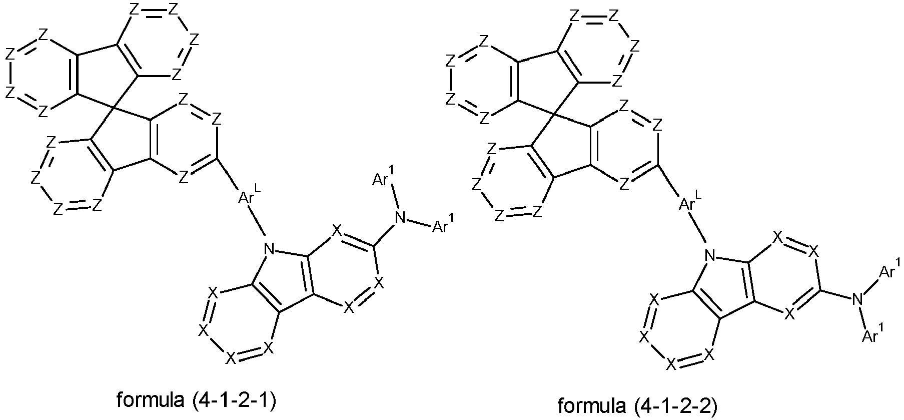 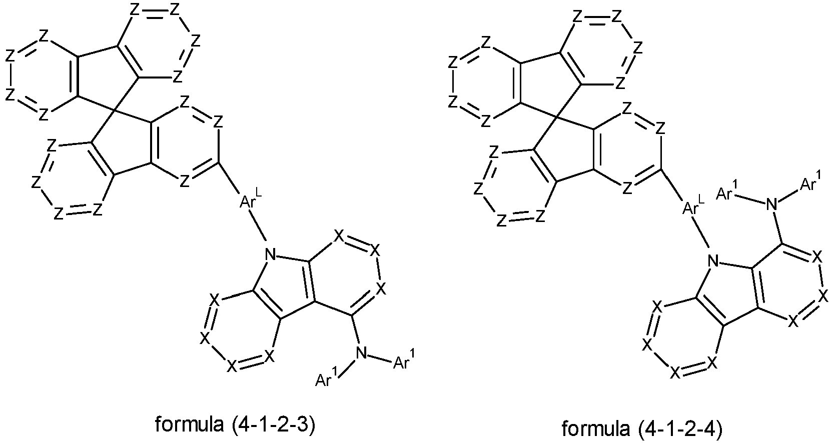 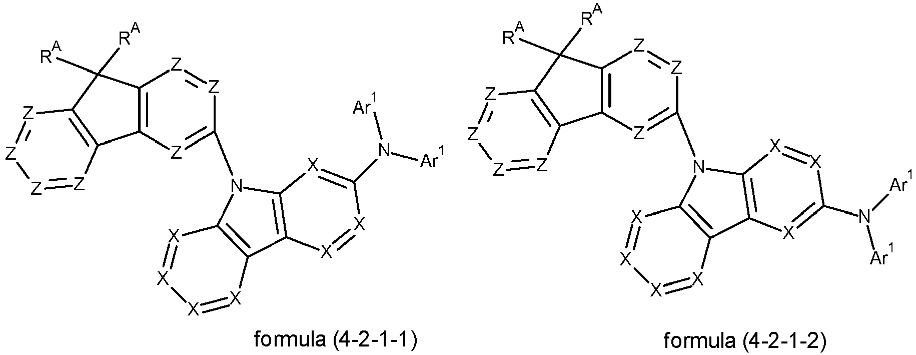 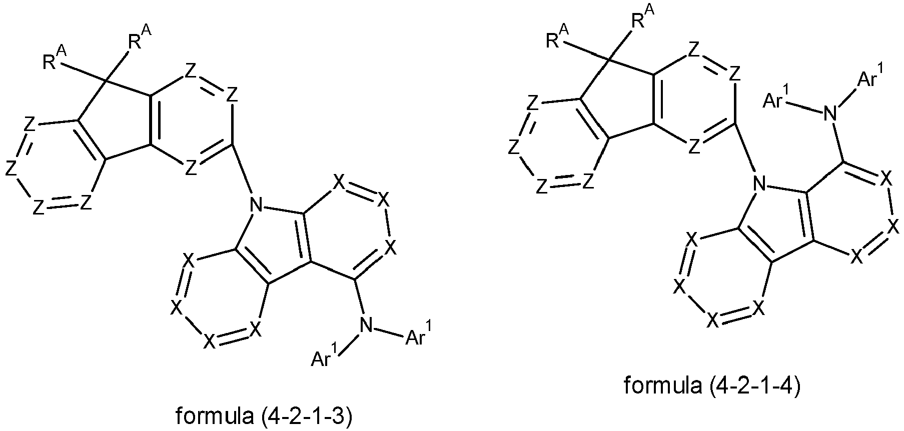 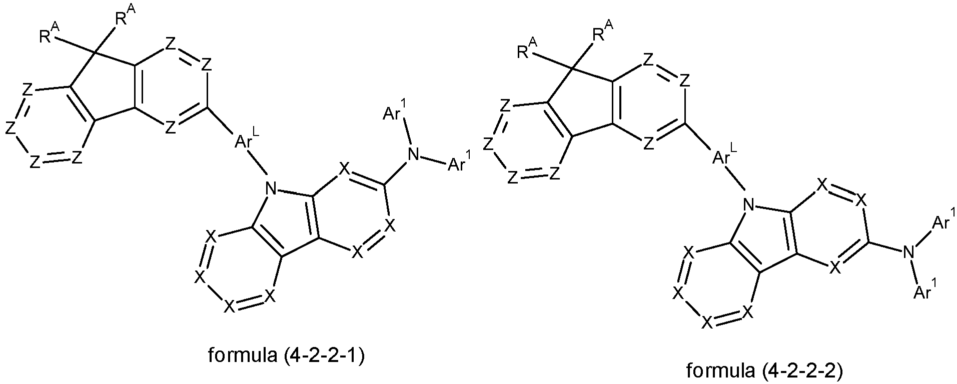 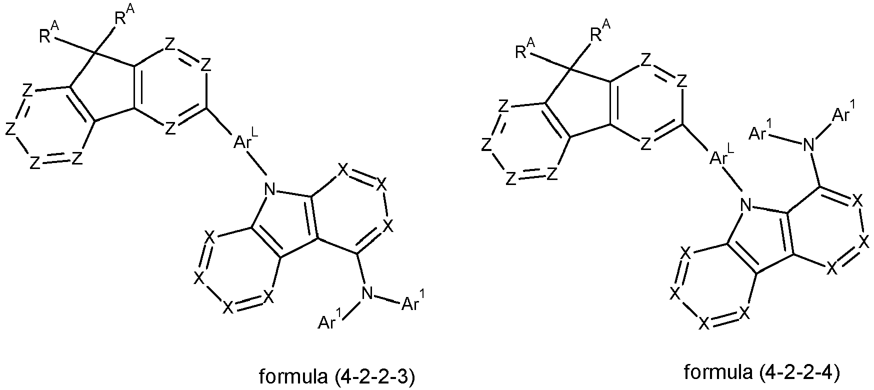 where the symbols have the same meaning as in claim 1.

7. Organic electroluminescent device according to one or more of the preceding claims, **characterized in that** R^{A}, R^{X} and R^{Z} stand on each occurrence, identically or differently, for H, D, F, a straight-chain alkyl group having 1 to 20 C atoms or a branched or cyclic alkyl group having 3 to 20 C atoms, each of which may be substituted by one or more radicals R, where in each case one or more non-adjacent CH₂ groups may be replaced by RC=CR, C=C, O or S and where one or more H atoms may be replaced by D, F, Cl or CN, an aromatic or heteroaromatic ring system having 5 to 40 aromatic ring atoms, which may in each case be substituted by one or more radicals R; where two adjacent radicals R^{X}, two adjacent radicals R^{Z}, two adjacent radicals R^{A} may form a mono- or polycyclic, aliphatic ring system or aromatic ring system, which may be substituted by one or more radicals R.

8. Organic electroluminescent device according to one or more of the preceding claims, **characterized in that** R^{X} stands on each occurrence, identically or differently, for H, a straight-chain alkyl group having 1 to 20 C atoms, each of which may be substituted by one or more radicals R, or an aromatic ring system having 5 to 40 aromatic ring atoms or an heteroaromatic ring system having 5 to 40, each of which may be substituted by one or more radicals R; where two adjacent radicals R^{X} may form a mono- or polycyclic, aliphatic ring system or aromatic ring system, which may be substituted by one or more radicals R.

9. Organic electroluminescent device according to one or more of the preceding claims, **characterized in that** R^{Z} stands on each occurrence, identically or differently, for H, a straight-chain alkyl group having 1 to 20 C atoms or a branched alkyl group having 3 to 20 C atoms, each of which may be substituted by one or more radicals R, or an or an aromatic ring system having 6 to 40 aromatic ring atoms or an heteroaromatic ring system having 5 to 40, each of which may be substituted by one or more radicals R; where two adjacent radicals R^{Z} may form a mono- or polycyclic, aliphatic ring system or aromatic ring system, which may be substituted by one or more radicals R.

10. Organic electroluminescent device according to one or more of the preceding claims, **characterized in that** Ar^{L} is selected, identically or differently, from benzene, biphenyl, terphenyl, naphthalene, fluorene, indenofluorene, spirobifluorene, triazine, benzoquinoline, benzoquinazoline, dibenzofuran, dibenzothiophene, and carbazole, where each of the above-mentioned groups may be substituted by one or more radicals R.

11. Organic electroluminescent device according to one or more of the preceding claims, **characterized in that** Ar¹ is on each occurrence, identically or differently, selected from phenyl, biphenyl, terphenyl, quaterphenyl, naphthyl, fluorenyl, benzofluorenyl, spirobifluorenyl, indenofluorenyl, dibenzofuranyl, dibenzothiophenyl, carbazolyl, benzofuranyl, benzothiophenyl, benzofused dibenzofuranyl, benzofused dibenzothiophenyl, benzimidazo-benzimidazolyl, naphthyl-substituted phenyl, fluorenyl-substituted phenyl, spirobifluorenyl-substituted phenyl, dibenzofuranyl-substituted phenyl, dibenzothiophenyl-substituted phenyl, benzimidazobenzimidazolyl-substituted phenyl and carbazolyl-substituted phenyl, each of which may optionally be substituted by one or more radicals R.

## Patentansprüche

1. Organische Elektrolumineszenzvorrichtung, enthaltend zwei, drei oder vier lochtransportierende Schichten zwischen der Anode und der emittierenden Schicht, wobei zwei der lochtransportierenden Schichten eine Verbindung der Formel (1) enthalten, 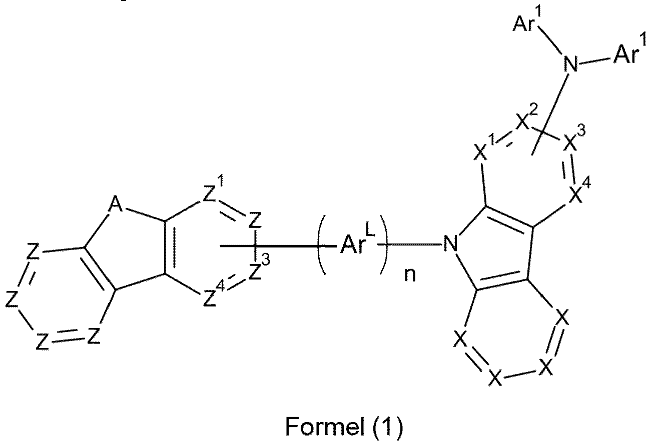 wobei für die verwendeten Symbole und Indices Folgendes gilt:
A steht für eine Gruppe der Formel (A-1) oder (A-2, 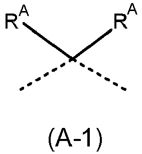 wobei die gestrichelten Bindungen die Bindungen an den Rest der Formel (1) darstellen;
Z steht bei jedem Auftreten gleich oder verschieden für CR^{Z};
Z¹, Z³, Z⁴ entsprechen Z; mit der Maßgabe, dass im Fall von n ≥ 1 die Gruppe Ar^{L} an eine der Gruppen Z¹, Z³, Z⁴, die in diesem Fall für C steht, gebunden ist, und im Fall von n = 0 die Gruppe Ar^{L} fehlt und die in Formel (1) dargestellte Carbazoleinheit direkt über ihr Stickstoffatom an eine der Gruppen Z¹, Z³, Z⁴, die in diesem Fall für C steht, gebunden ist;
X steht bei jedem Auftreten gleich oder verschieden für CR^{x};
X¹, X², X³, X⁴ entsprechen X; mit der Maßgabe, dass die Gruppe -NAr¹Ar¹ gemäß der Darstellung in Formel (1) an eine der Gruppen X¹, X², X³, X⁴, die in diesem Fall für C steht, gebunden ist;
Ar^{L} steht bei jedem Auftreten gleich oder verschieden für ein aromatisches Ringsystem mit 6 bis 40 aromatischen Ringatomen, das durch einen oder mehrere Reste R substituiert sein kann, oder für ein heteroaromatisches Ringsystem mit 5 bis 40 aromatischen Ringatomen, das durch einen oder mehrere Reste R substituiert sein kann;
Ar¹ steht bei jedem Auftreten gleich oder verschieden für ein aromatisches Ringsystem mit 6 bis 60 aromatischen Ringatomen, das durch einen oder mehrere Reste R substituiert sein kann, oder für ein heteroaromatisches Ringsystem mit 5 bis 60 aromatischen Ringatomen, das durch einen oder mehrere Reste R substituiert sein kann;
R^{A}, R^{x}, R^{Z} stehen bei jedem Auftreten gleich oder verschieden für H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, eine geradkettige Alkyl-, Alkoxy- oder Thioalkylgruppe mit 1 bis 40 C-Atomen oder verzweigte oder cyclische Alkyl-, Alkoxy- oder Thioalkylgruppen mit 3 bis 40 C-Atomen, die jeweils durch einen oder mehrere Reste R substituiert sein können, wobei jeweils eine oder mehrere nicht benachbarte CH₂-Gruppen durch RC=CR, C=C, Si(R)₂, Ge(R)₂, Sn(R)₂, C=O, C=S, C=Se, P(=O)(R), SO, SO₂, O, S oder CONR ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, das jeweils durch einen oder mehrere Reste R substituiert sein kann, eine Aryloxygruppe mit 5 bis 60 aromatischen Ringatomen, die durch einen oder mehrere Reste R substituiert sein kann, oder eine Aralkyl- oder Heteroaralkylgruppe mit 5 bis 60 aromatischen Ringatomen, die durch einen oder mehrere Reste R substituiert sein kann; wobei zwei benachbarte Reste R^{A}, zwei benachbarte Reste R^{X}, zwei benachbarte Reste R^{Z} ein mono- oder polycyclisches, aliphatisches Ringsystem oder aromatisches Ringsystem, das durch einen oder mehrere Reste R substituiert sein kann, bilden können;
R steht bei jedem Auftreten gleich oder verschieden für H, D, F, Cl, Br, I, CHO, CN, N(Ar)₂, C(=O)Ar, P(=O)(Ar)₂, S(=O)Ar, S(=O)₂Ar, NO₂, Si(R')₃, B(OR')₂, OSO₂R', eine geradkettige Alkyl-, Alkoxy- oder Thioalkylgruppe mit 1 bis 40 C-Atomen oder verzweigte oder cyclische Alkyl-, Alkoxy- oder Thioalkylgruppen mit 3 bis 40 C-Atomen, die jeweils durch einen oder mehrere Reste R' substituiert sein können, wobei jeweils eine oder mehrere nicht benachbarte CH₂-Gruppen durch R'C=CR', C=C, Si(R')₂, Ge(R')₂, Sn(R')₂, C=O, C=S, C=Se, P(=O)(R'), SO, SO₂, O, S oder CONR' ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, die jeweils durch einen oder mehrere Reste R' substituiert sein können, oder eine Aryloxygruppe mit 5 bis 60 aromatischen Ringatomen, die durch einen oder mehrere Reste R' substituiert sein kann, wobei zwei benachbarte Reste R ein mono- oder polycyclisches, aliphatisches Ringsystem oder aromatisches Ringsystem, das durch einen oder mehrere Reste R' substituiert sein kann, bilden können;
Ar ist bei jedem Auftreten gleich oder verschieden ein aromatisches oder heteroaromatisches Ringsystem mit 5 bis 24 aromatischen Ringatomen, das jeweils auch durch einen oder mehrere Reste R' substituiert sein kann;
R' steht bei jedem Auftreten gleich oder verschieden für H, D, F, Cl, Br, I, CN, eine geradkettige Alkyl-, Alkoxy- oder Thioalkylgruppe mit 1 bis 20 C-Atomen oder verzweigte oder cyclische Alkyl-, Alkoxy- oder Thioalkylgruppe mit 3 bis 20 C-Atomen, wobei jeweils ein oder mehrere nicht benachbarte CH₂-Gruppen durch SO, SO₂, O, S ersetzt sein können und wobei ein oder mehrere H-Atome durch D, F, Cl, Br oder I ersetzt sein können, oder ein aromatisches oder heteroaromatisches Ringsystem mit 5 bis 24 C-Atomen; und
n ist 0, 1, 2 oder 3;
und wobei eine Verbindung der Formel (1) in Kombination mit einem p-Dotanden in einer Lochtransportschicht verwendet wird.

2. Organische Elektrolumineszenzvorrichtung nach Anspruch 1, **dadurch gekennzeichnet, dass** die Verbindung der Formel (1) aus Verbindungen der Formel (2), (3) oder (4) ausgewählt ist, 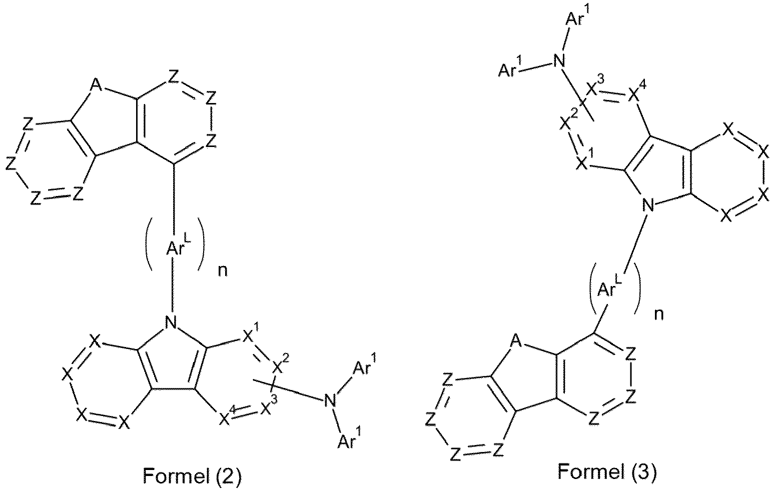 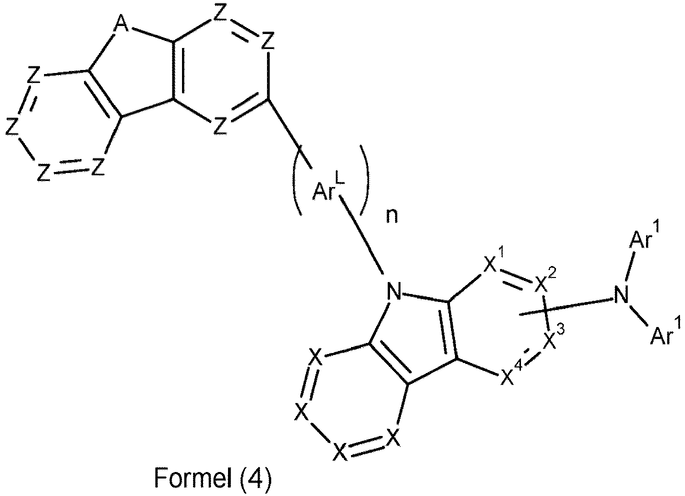 wobei die Symbole die gleiche Bedeutung wie in Anspruch 1 haben.

3. Organische Elektrolumineszenzvorrichtung nach Anspruch 1 oder 2, **dadurch gekennzeichnet, dass** die Verbindung der Formel (1) aus Verbindungen einer der Formeln (2-1) bis (4-2) ausgewählt ist, 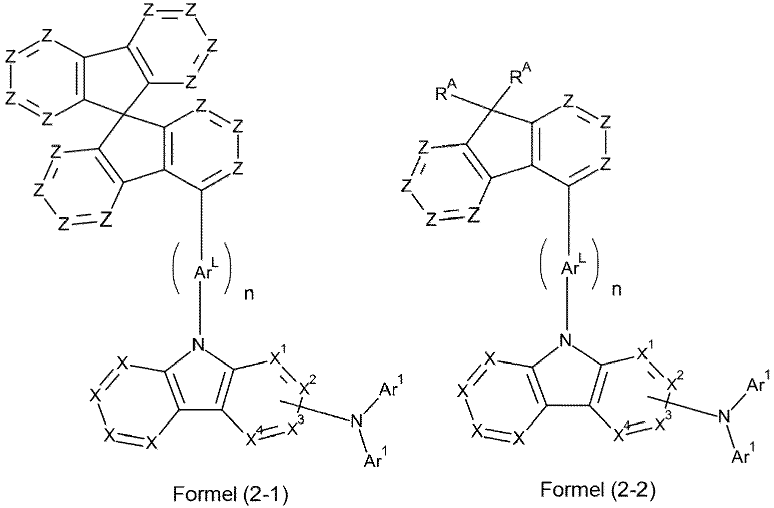 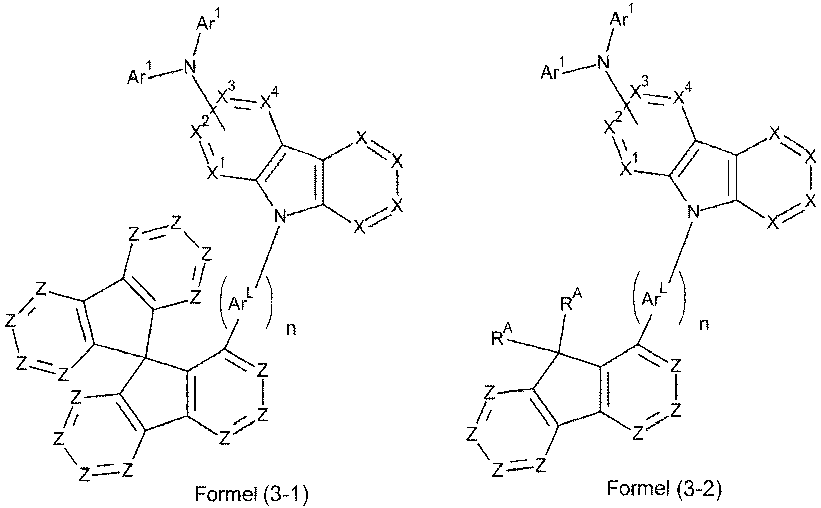 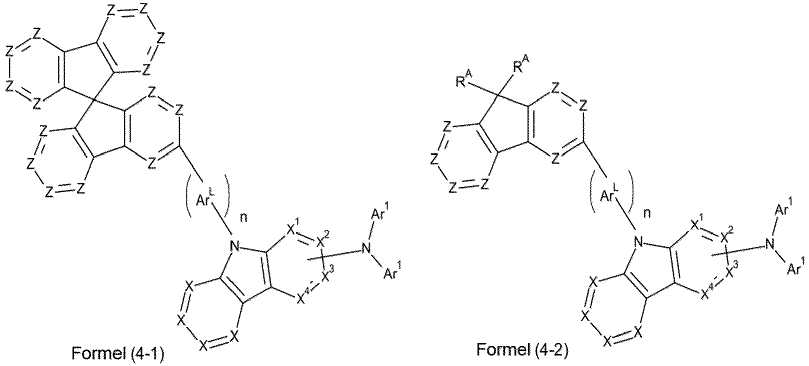 wobei die Symbole die gleiche Bedeutung wie in Anspruch 1 haben.

4. Organische Elektrolumineszenzvorrichtung nach einem oder mehreren der vorhergehenden Ansprüche, **dadurch gekennzeichnet, dass** n 0 oder 1 ist.

5. Organische Elektrolumineszenzvorrichtung nach einem oder mehreren der vorhergehenden Ansprüche, **dadurch gekennzeichnet, dass** die Verbindung der Formel (1) aus den Verbindungen einer der Formeln (2-1-1) bis (4-2-2) ausgewählt ist, 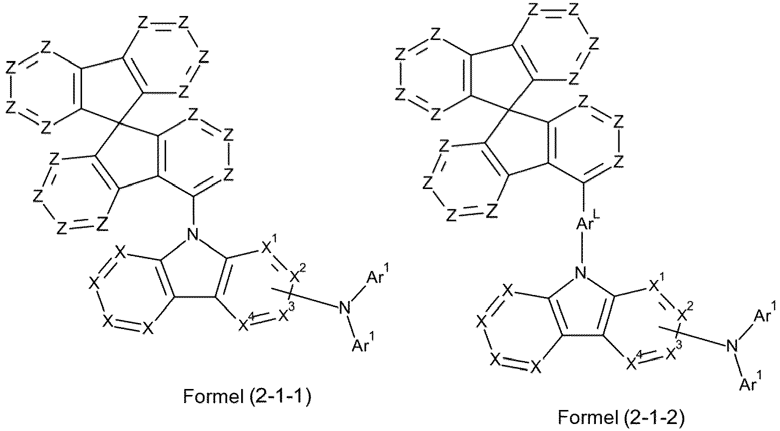 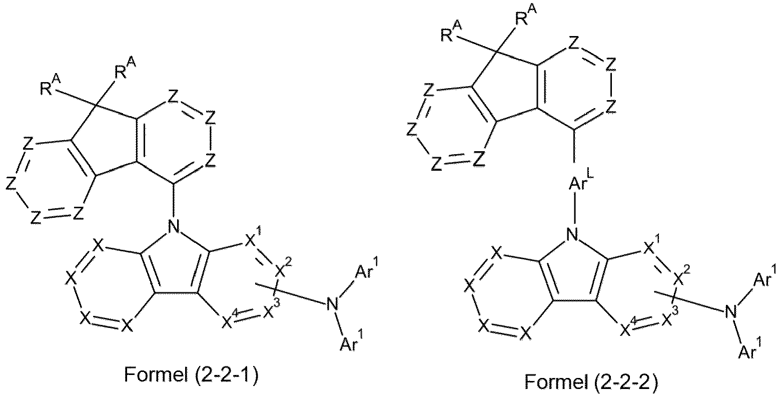 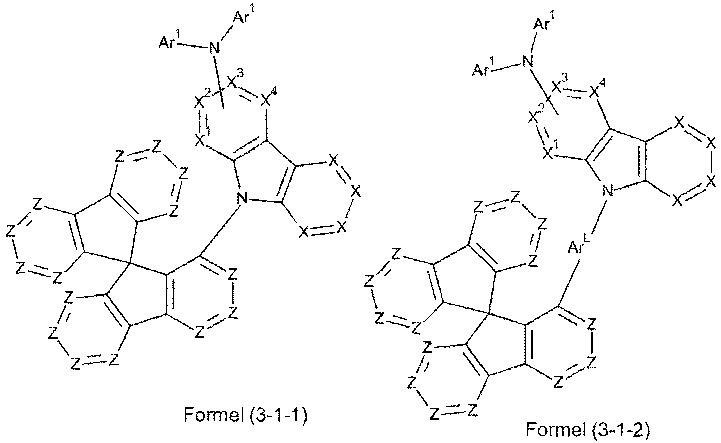 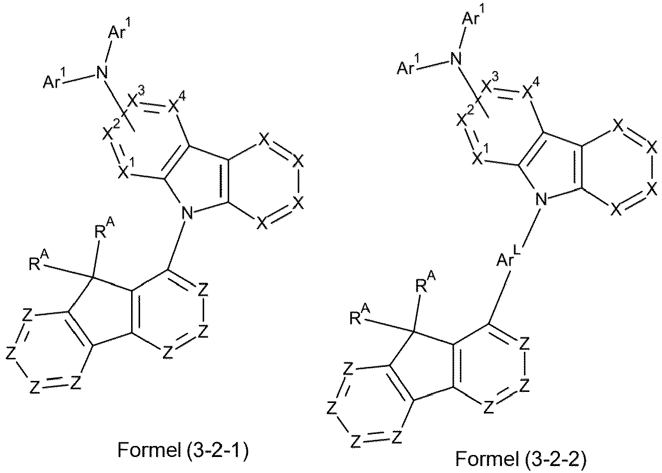 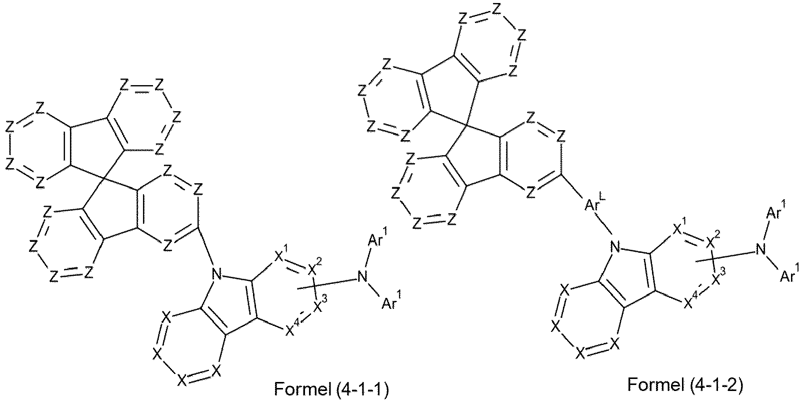 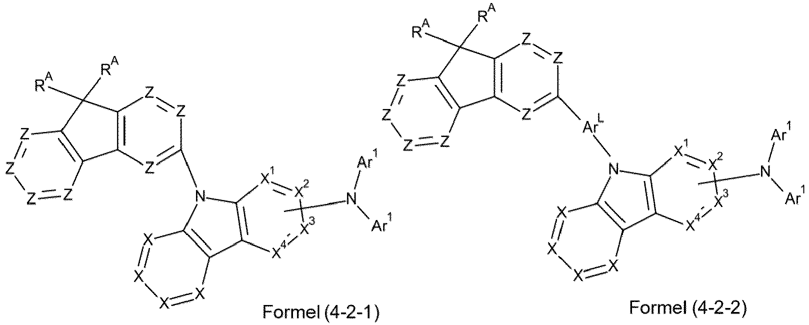 wobei die Symbole die gleiche Bedeutung wie in Anspruch 1 haben.

6. Organische Elektrolumineszenzvorrichtung nach einem oder mehreren der vorhergehenden Ansprüche, **dadurch gekennzeichnet, dass** die Verbindung der Formel (1) aus den Verbindungen einer der Formeln (2-1-1-1) bis (4-2-2-4) ausgewählt ist, 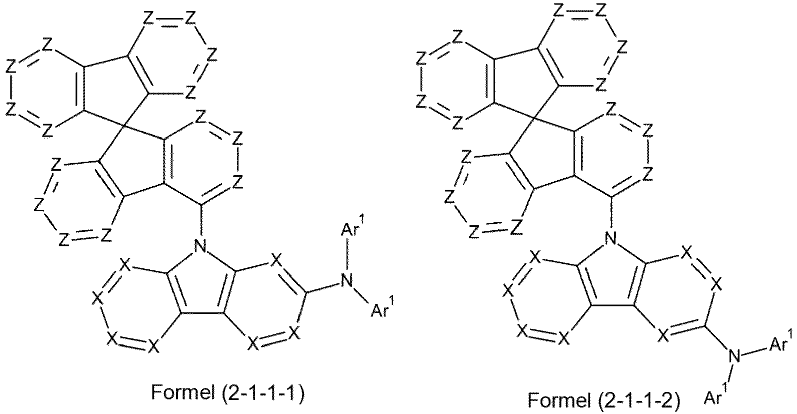 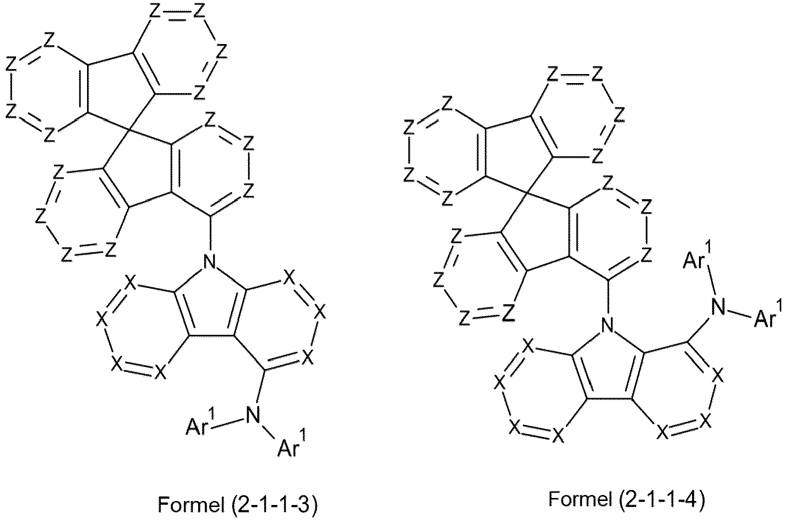 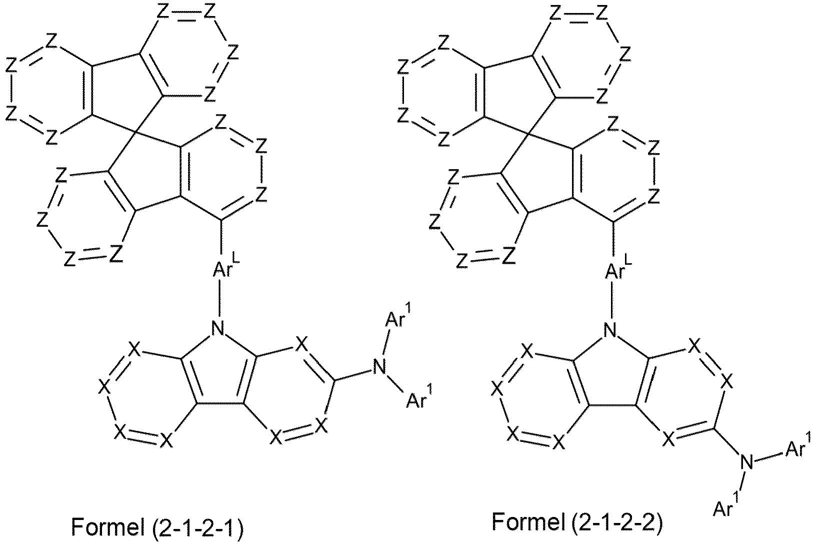 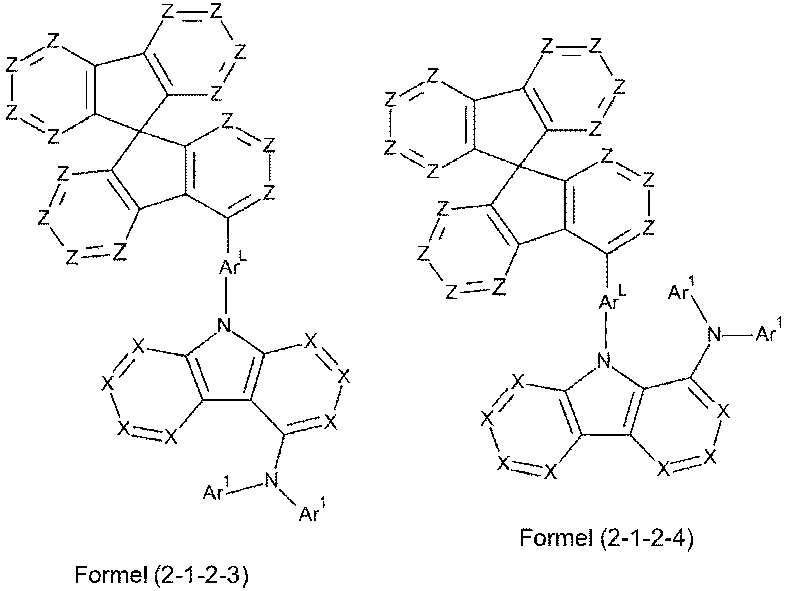 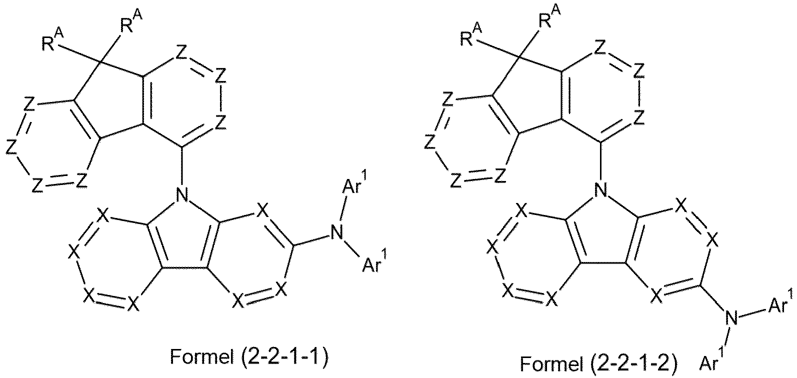 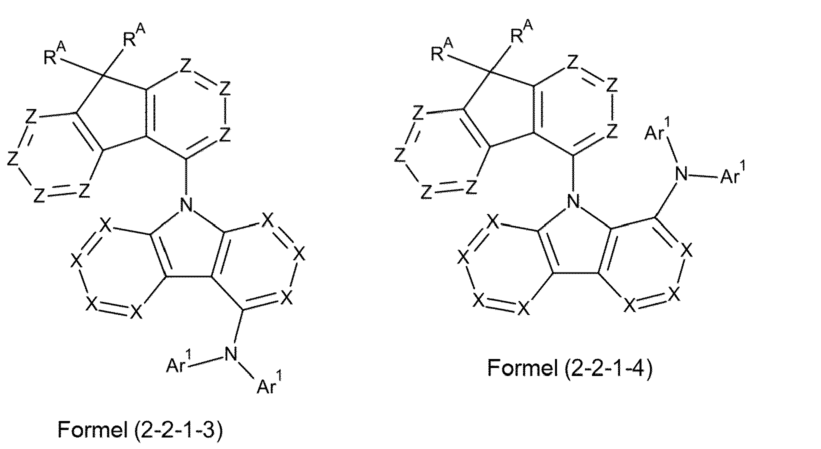 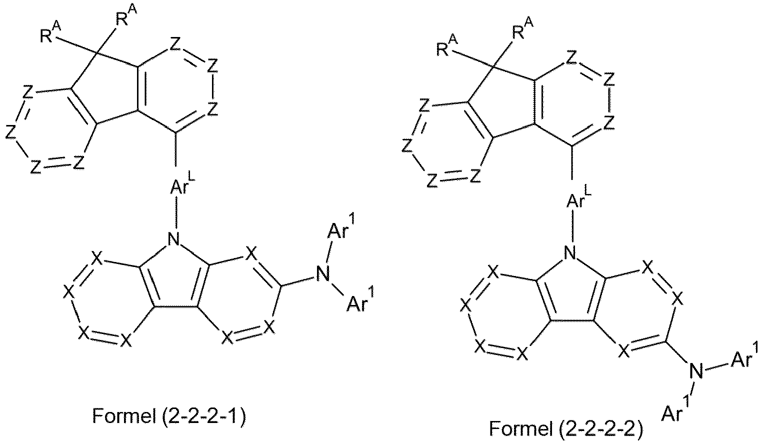 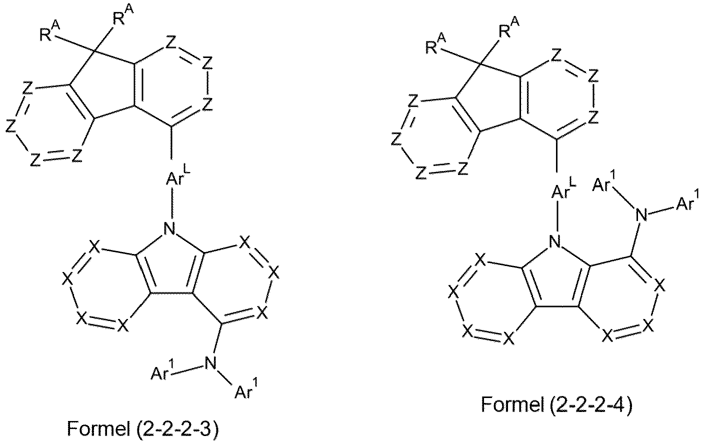 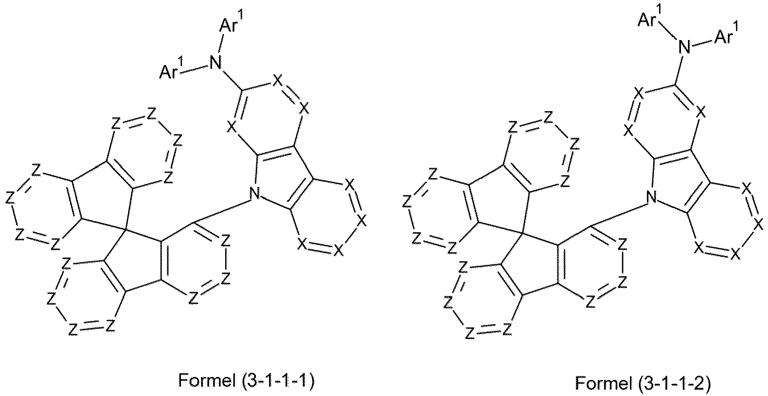 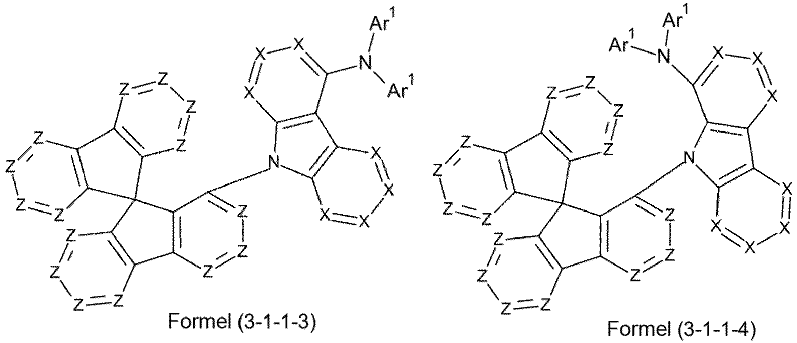 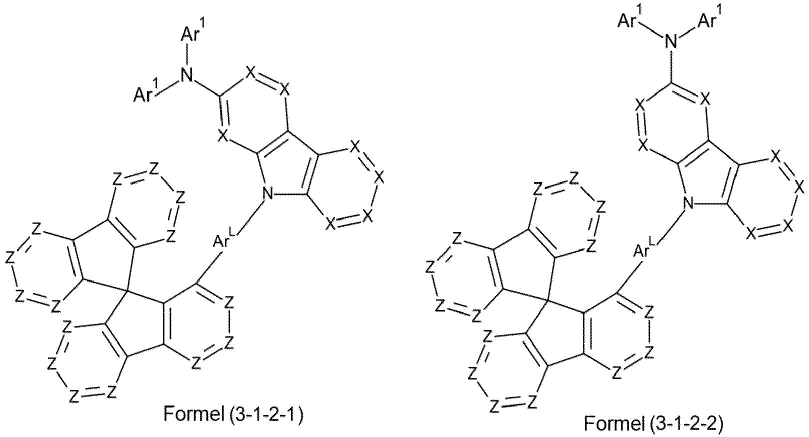 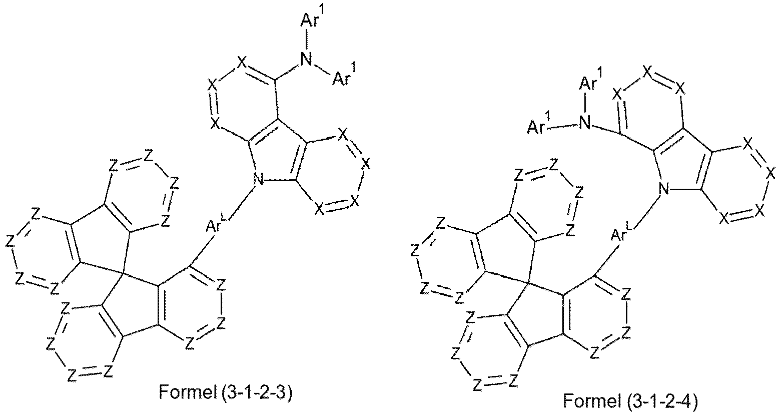 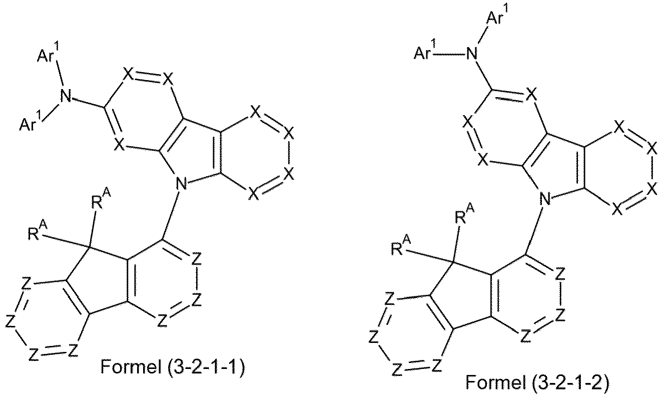 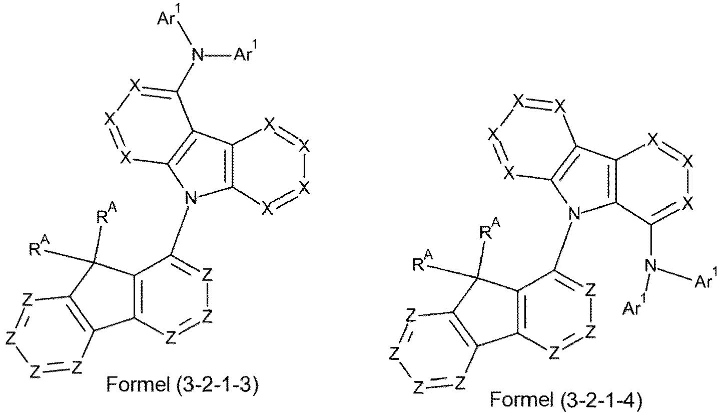 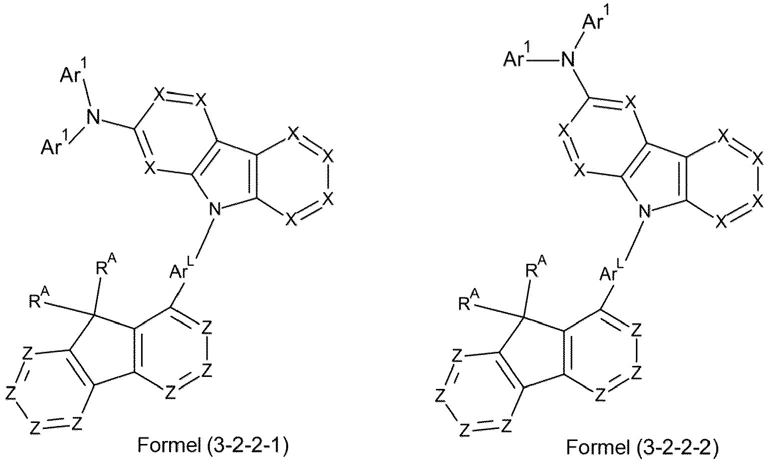 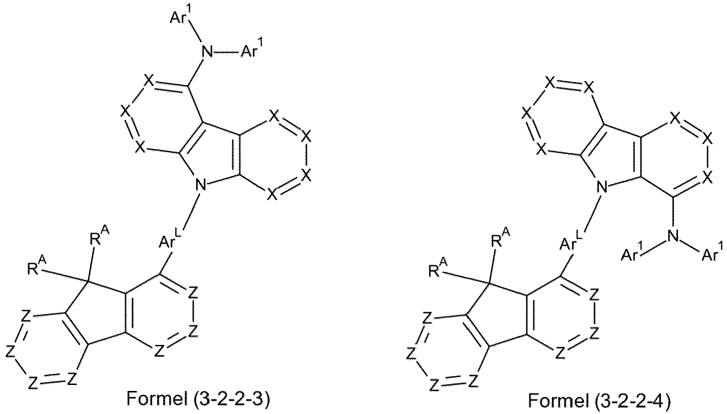 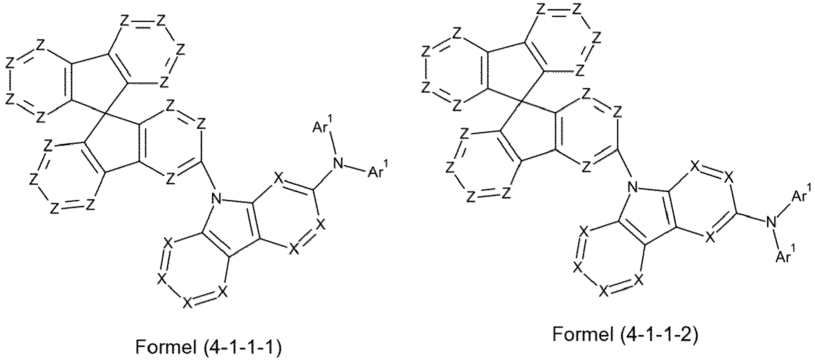 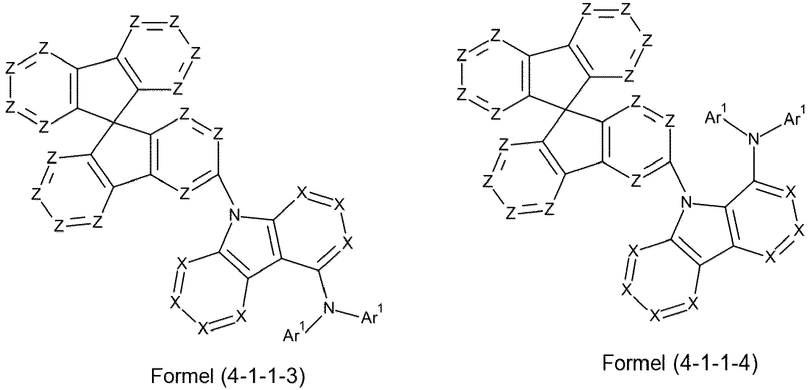 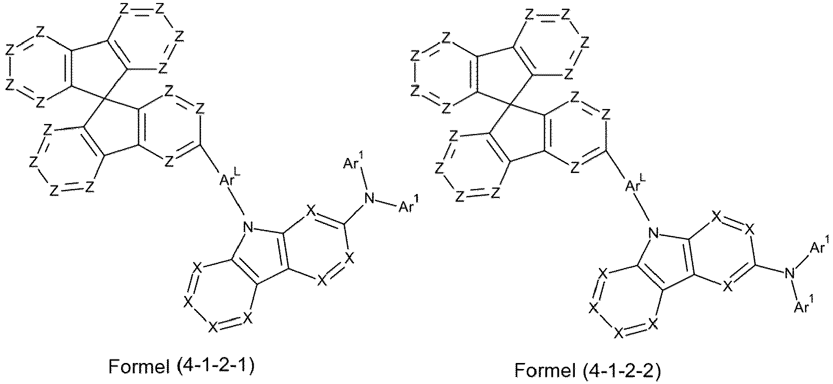 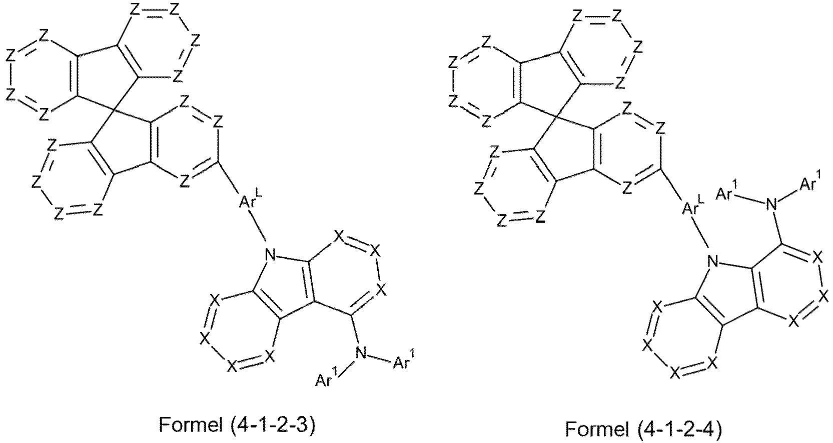 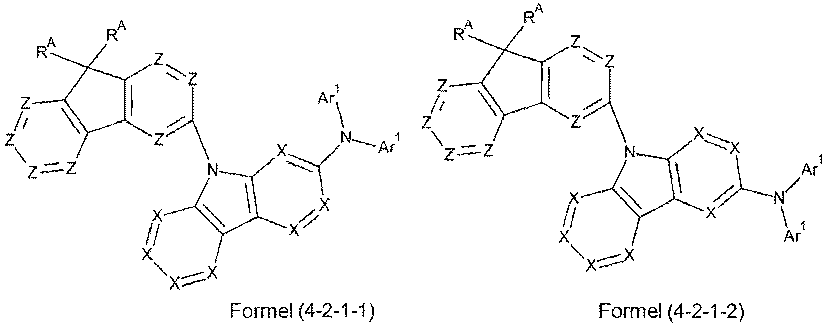 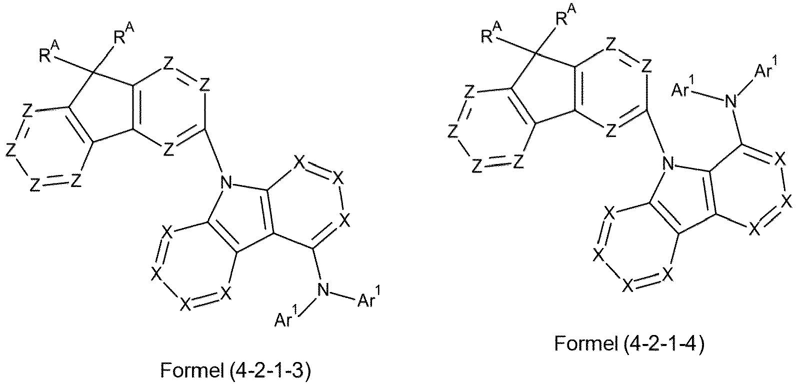 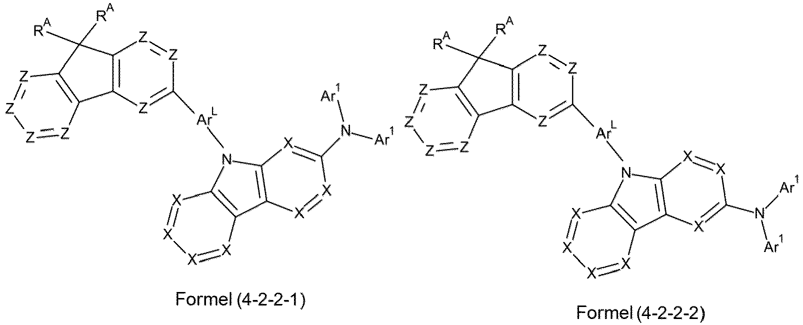 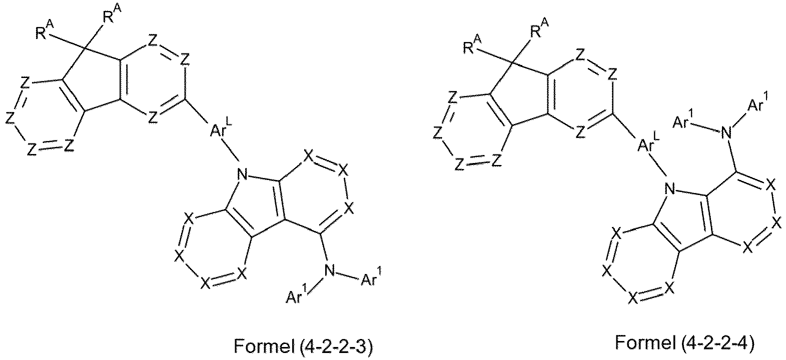 wobei die Symbole die gleiche Bedeutung wie in Anspruch 1 haben.

7. Organische Elektrolumineszenzvorrichtung nach einem oder mehreren der vorhergehenden Ansprüche, **dadurch gekennzeichnet, dass** R^{A}, R^{x} und R^{Z} bei jedem Auftreten gleich oder verschieden für H, D, F, eine geradkettige Alkylgruppe mit 1 bis 20 C-Atomen oder eine verzweigte oder cyclische Alkylgruppe mit 3 bis 20 C-Atomen, die jeweils durch einen oder mehrere Reste R substituiert sein können, wobei jeweils eine oder mehrere nicht benachbarte CH₂-Gruppen durch RC=CR, C≡C, O oder S ersetzt sein können und wobei ein oder mehrere H-Atome durch D, F, Cl oder CN ersetzt sein können, ein aromatisches oder heteroaromatisches Ringsystem mit 5 bis 40 aromatischen Ringatomen, das jeweils durch einen oder mehrere Reste R substituiert sein kann, stehen; wobei zwei benachbarte Reste R^{X}, zwei benachbarte Reste R^{Z}, zwei benachbarte Reste R^{A} ein mono- oder polycyclisches, aliphatisches Ringsystem oder aromatisches Ringsystem bilden können, das durch einen oder mehrere Reste R substituiert sein kann.

8. Organische Elektrolumineszenzvorrichtung nach einem oder mehreren der vorhergehenden Ansprüche, **dadurch gekennzeichnet, dass** R^{X} bei jedem Auftreten gleich oder verschieden für H, eine geradkettige Alkylgruppe mit 1 bis 20 C-Atomen, die jeweils durch einen oder mehrere Reste R substituiert sein kann, oder ein aromatisches Ringsystem mit 5 bis 40 aromatischen Ringatomen oder ein heteroaromatisches Ringsystem mit 5 bis 40, die jeweils durch einen oder mehrere Reste R substituiert sein können; wobei zwei benachbarte Reste R^{x} ein mono- oder polycyclisches, aliphatisches Ringsystem oder aromatisches Ringsystem bilden können, das mit einem oder mehreren Resten R substituiert sein kann.

9. Organische Elektrolumineszenzvorrichtung nach einem oder mehreren der vorhergehenden Ansprüche, **dadurch gekennzeichnet, dass** R^{Z} bei jedem Auftreten gleich oder verschieden für H, eine geradkettige Alkylgruppe mit 1 bis 20 C-Atomen oder eine verzweigte Alkylgruppe mit 3 bis 20 C-Atomen, die jeweils durch einen oder mehrere Reste R substituiert sein können, oder ein oder ein aromatisches Ringsystem mit 6 bis 40 aromatischen Ringatomen oder ein heteroaromatisches Ringsystem mit 5 bis 40, die jeweils durch einen oder mehrere Reste R substituiert sein können, steht; wobei zwei benachbarte Reste R^{Z} ein mono- oder polycyclisches, aliphatisches Ringsystem oder aromatisches Ringsystem, das durch einen oder mehrere Reste R substituiert sein kann, bilden können.

10. Organische Elektrolumineszenzvorrichtung nach einem oder mehreren der vorhergehenden Ansprüche, **dadurch gekennzeichnet, dass** Ar^{L} gleich oder verschieden aus Benzol, Biphenyl, Terphenyl, Naphthalin, Fluoren, Indenofluoren, Spirobifluoren, Triazin, Benzochinolin, Benzochinazolin, Dibenzofuran, Dibenzothiophen und Carbazol ausgewählt ist, wobei jede der oben genannten Gruppen durch einen oder mehrere Reste R substituiert sein kann.

11. Organische Elektrolumineszenzvorrichtung nach einem oder mehreren der vorhergehenden Ansprüche, **dadurch gekennzeichnet, dass** Ar¹ bei jedem Auftreten gleich oder verschieden aus Phenyl, Biphenyl, Terphenyl, Quaterphenyl, Naphthyl, Fluorenyl, Benzofluorenyl, Spirobifluorenyl, Indenofluorenyl, Dibenzofuranyl, Dibenzothiophenyl, Carbazolyl, Benzofuranyl, Benzothiophenyl, benzokondensiertem Dibenzofuranyl, benzokondensiertem Dibenzothiophenyl, Benzimidazobenzimidazolyl, naphthylsubstituiertem Phenyl, fluorenylsubstituiertem Phenyl, spirobifluorenylsubstituiertem Phenyl, dibenzofuranylsubstituiertem Phenyl, dibenzothiophenylsubstituiertem Phenyl, benzimidazobenzimidazolylsubstituiertem Phenyl und carbazolylsubstituiertem Phenyl, die jeweils gegebenenfalls durch einen oder mehrere Reste R substituiert sein können, ausgewählt ist.

## Revendications

1. Dispositif électroluminescent organique comprenant deux, trois ou quatre couches de transport de trous entre l'anode et la couche émettrice, dans lequel deux des couches de transport de trous comprennent un composé de formule (1), 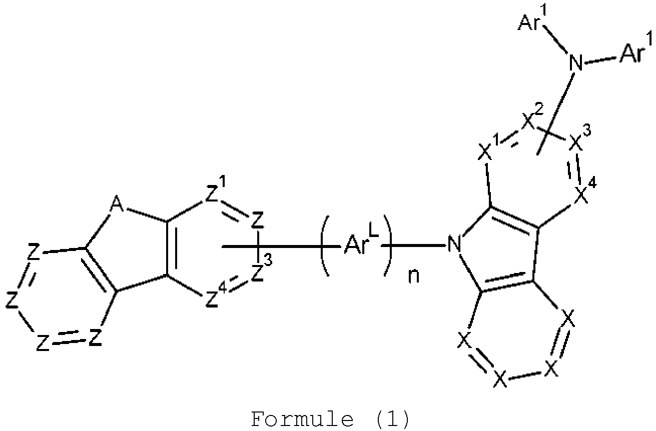 où ce qui suit s'applique aux symboles et indices utilisés :
A représente un groupe de formule (A-1) ou (A-2), 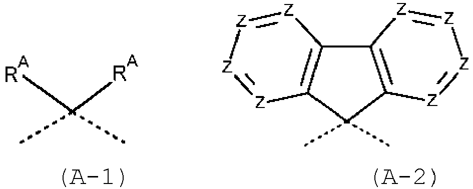
où les traits pointillés représentent les liaisons au reste de la formule (1) ;
Z représente, à chaque occurrence, de manière identique ou différente, CR^{Z} ;
Z¹, Z³, Z⁴ correspondent à Z ; à condition que, lorsque n ≥ 1, alors le groupe Ar^{L} est lié à l'un des groupes Z¹, Z³, Z⁴, qui représente C dans ce cas et lorsque n = 0, alors le groupe Ar^{L} est absent et le fragment carbazole représenté dans la formule (1) est directement lié via son atome d'azote à l'un des groupes Z¹, Z³, Z⁴, qui représente C dans ce cas ;
X représente, à chaque occurrence, de manière identique ou différente, CR^{X} ;
X¹, X², X³, X⁴ correspondent à X ; à condition que le groupe -NAr¹Ar¹ décrit dans la formule (1) soit lié à l'un des groupes X¹, X², X³, X⁴, qui représente C dans ce cas ;
Ar^{L} représente à chaque occurrence, de manière identique ou différente, un système cyclique aromatique ayant 6 à 40 atomes de cycle aromatique et qui peut être substitué par un ou plusieurs radicaux R, ou un système cyclique hétéroaromatique ayant 5 à 40 atomes de noyaux aromatiques, qui peut être substitué par un ou plusieurs radicaux R ;
Ar ¹ représente à chaque occurrence, de manière identique ou différente, un système cyclique aromatique ayant 6 à 60 atomes de cycle aromatique et qui peut être substitué par un ou plusieurs radicaux R, ou un système cyclique hétéroaromatique ayant 5 à 60 atomes de noyaux aromatiques, qui peut être substitué par un ou plusieurs radicaux R ;
R^{A}, R^{X}, R^{Z} représentent à chaque occurrence, de manière identique ou différente, H, D, F, Cl, Br, I, CHO, CN, N(Ar)₂, C(=O)Ar, P (=O)(Ar)₂, S(=O)Ar, S(=O)₂Ar, NO₂, Si(R)₃, B(OR)₂, OSO₂R, un groupe alkyle, alcoxy ou thioalkyle à chaîne linéaire ayant 1 à 40 atomes C ou des groupes alkyle, alcoxy ou thioalkyle ramifiés ou cycliques ayant 3 à 40 atomes C, chacun pouvant être substitué par un ou plusieurs radicaux R, où dans chaque cas un ou plusieurs groupes CH₂ non adjacents peuvent être remplacés par RC=CR, C≡C, Si(R)₂, Ge(R)₂, Sn(R)₂, C=O, C=S, C=Se, P(=O)(R), SO, SO₂, O, S ou CONR et où un ou plusieurs atomes H peuvent être remplacés par D, F, Cl, Br, I, CN ou NO₂, un système cyclique aromatique ou hétéroaromatique ayant 5 à 60 atomes de cycle aromatique, qui peut dans chaque cas être substitué par un ou plusieurs radicaux R, un groupe aryloxy ayant 5 à 60 atomes de cycle aromatique, qui peut être substitué par un ou plusieurs radicaux R, ou un groupe aralkyle ou hétéroaralkyle qui a 5 à 60 atomes de cycle aromatique, qui peut être substitué par un ou plusieurs radicaux R ; où deux radicaux R^{A} adjacents, deux radicaux R^{X} adjacents, deux radicaux R^{Z} adjacents peuvent former un système cyclique aliphatique ou un système cyclique aromatique mono- ou polycyclique, qui peut être substitué par un ou plusieurs radicaux R ;
R représente à chaque occurrence, de manière identique ou différente, H, D, F, Cl, Br, I, CHO, CN, N(Ar)₂, C(=O)Ar, P(=O)(Ar)₂, S (=O)Ar, S(=O)₂Ar, NO₂, Si(R')₃, B(OR')₂, OSO₂R', un groupe alkyle, alcoxy ou thioalkyle à chaîne linéaire ayant 1 à 40 atomes C ou des groupes alkyle, alcoxy ou thioalkyle ramifiés ou cycliques ayant 3 à 40 atomes C, chacun pouvant être substitué par un ou plusieurs radicaux R', où dans chaque cas un ou plusieurs groupes CH₂ non adjacents peuvent être remplacés par R'C=CR', C≡C, Si(R')₂, Ge(R')₂, Sn(R')₂, C=O, C=S, C=Se, P(=O)(R'), SO, SO₂, O, S ou CONR' et où un ou plusieurs atomes H peuvent être remplacés par D, F, Cl, Br, I, CN ou NO₂, un système cyclique aromatique ou hétéroaromatique ayant 5 à 60 atomes de cycle aromatique, qui peut dans chaque cas être substitué par un ou plusieurs radicaux R', ou un groupe aryloxy ayant 5 à 60 atomes de cycle aromatique, qui peut être substitué par un ou plusieurs radicaux R', où deux radicaux R adjacents peuvent former un système cyclique aliphatique ou un système cyclique aromatique mono- ou polycyclique, qui peut être substitué par un ou plusieurs radicaux R' ;
Ar représente à chaque occurrence, de manière identique ou différente, un système cyclique aromatique ou hétéroaromatique ayant 5 à 24 atomes de cycle aromatique, qui peuvent dans chaque cas également être substitués par un ou plusieurs radicaux R' ;
R' représente à chaque occurrence, de manière identique ou différente, H, D, F, Cl, Br, I, CN, un groupe alkyle, alcoxy ou thioalkyle à chaîne linéaire ayant de 1 à 20 atomes C ou un alkyle, alcoxy ou cyclique ramifié ou cyclique, un groupe thioalkyle ayant 3 à 20 atomes de carbone, où dans chaque cas un ou plusieurs groupes CH₂ non adjacents peuvent être remplacés par SO, SO₂, O, S et où un ou plusieurs atomes H peuvent être remplacés par D, F, Cl, Br ou I, ou un système cyclique aromatique ou hétéroaromatique ayant 5 à 24 atomes C ; et
n est 0, 1, 2 ou 3 ;
et où un composé de formule (1) est utilisé en combinaison avec un dopant p dans une couche de transport de trous.

2. Dispositif électroluminescent organique selon la revendication 1, **caractérisé en ce que** le composé de formule (1) est choisi parmi les composés de formule (2), (3) ou (4), 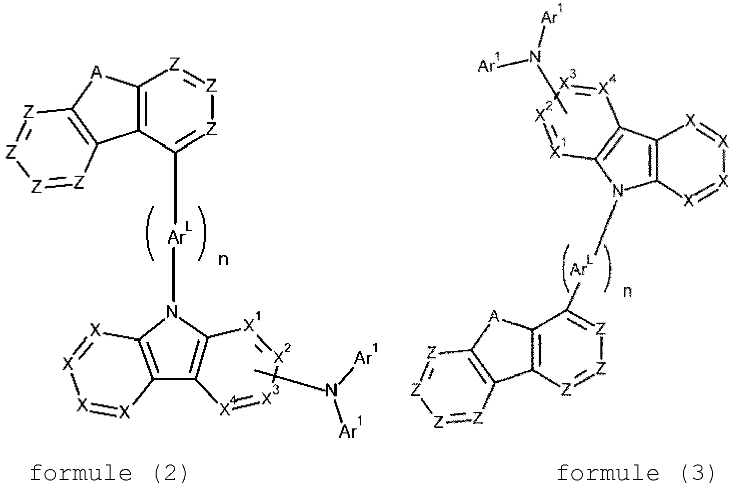 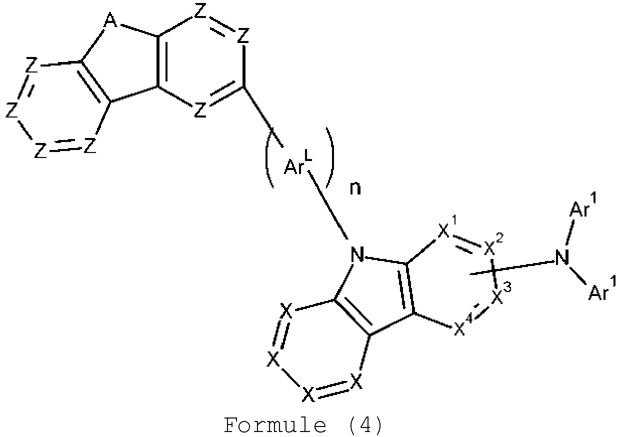 où les symboles ont la même signification que dans la revendication 1.

3. Dispositif électroluminescent organique selon la revendication 1 ou 2, **caractérisé en ce que** le composé de formule (1) est choisi parmi les composés de l'une des formules (2-1) à (4-2), 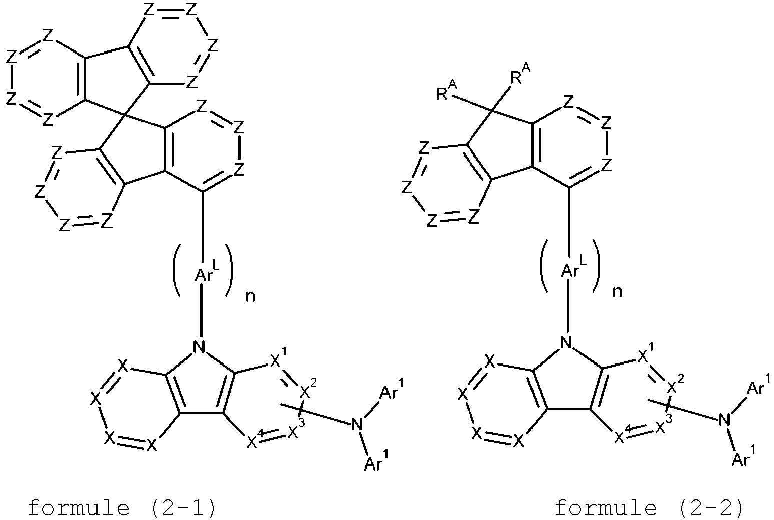 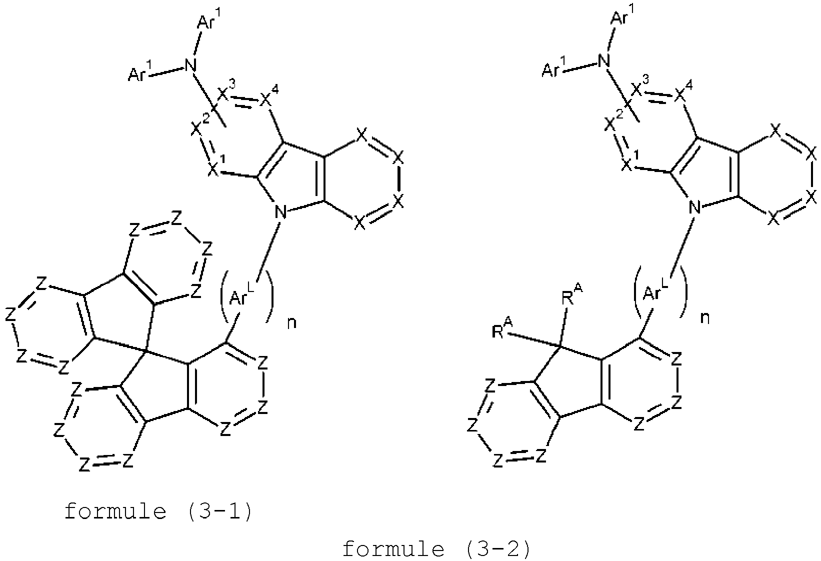 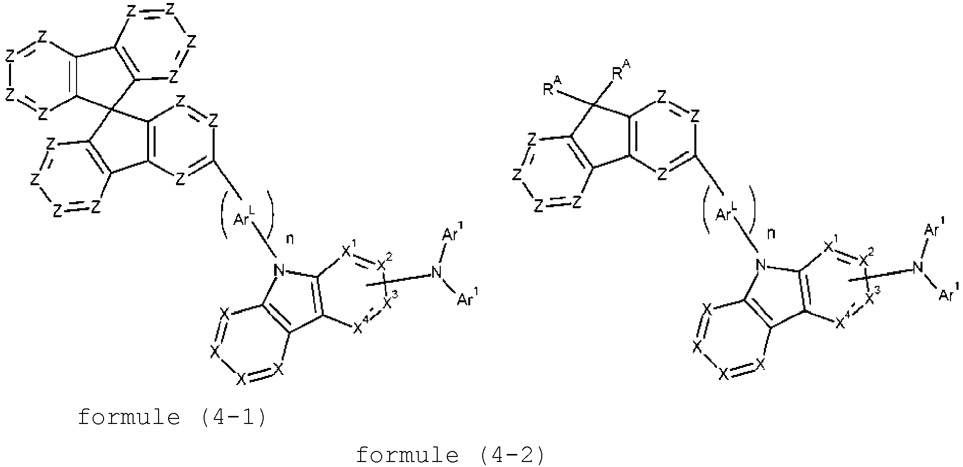 où les symboles ont la même signification que dans la revendication 1.

4. Dispositif électroluminescent organique selon une ou plusieurs des revendications précédentes, **caractérisé en ce que** n est 0 ou 1.

5. Dispositif électroluminescent organique selon une ou plusieurs des revendications précédentes, **caractérisé en ce que** le composé de formule (1) est choisi parmi les composés de l'une des formules (2-1-1) à (4-2-2), 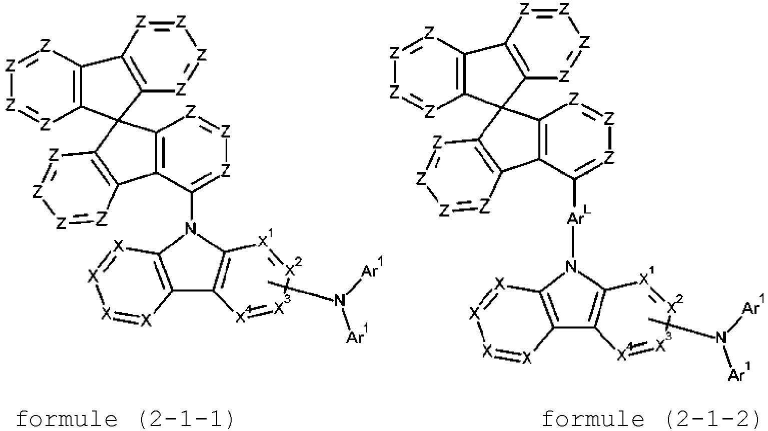 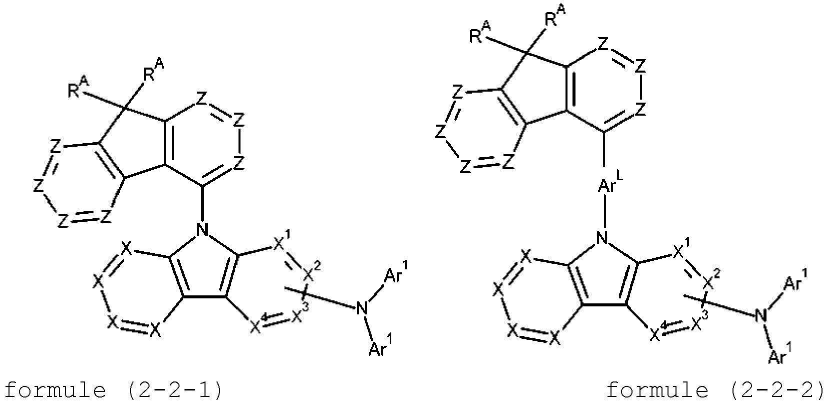 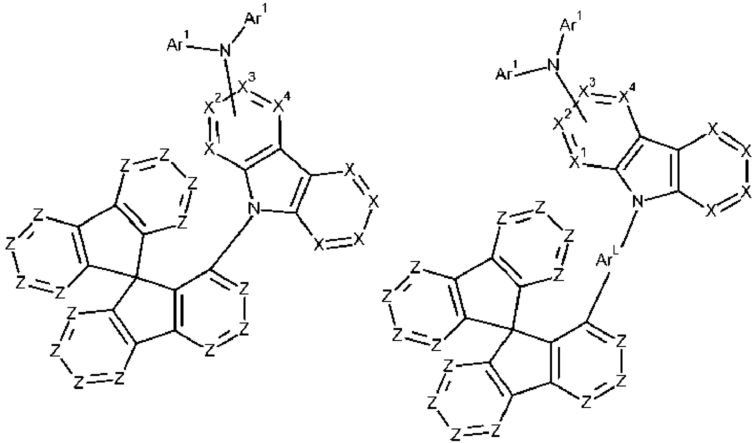 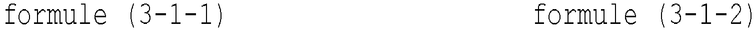 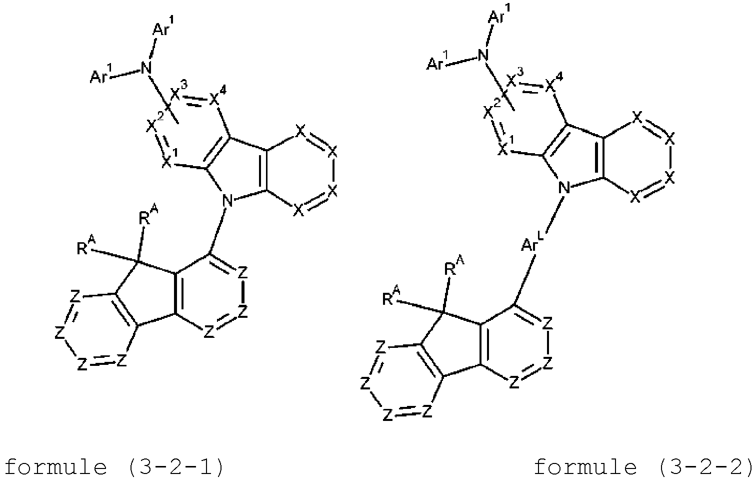 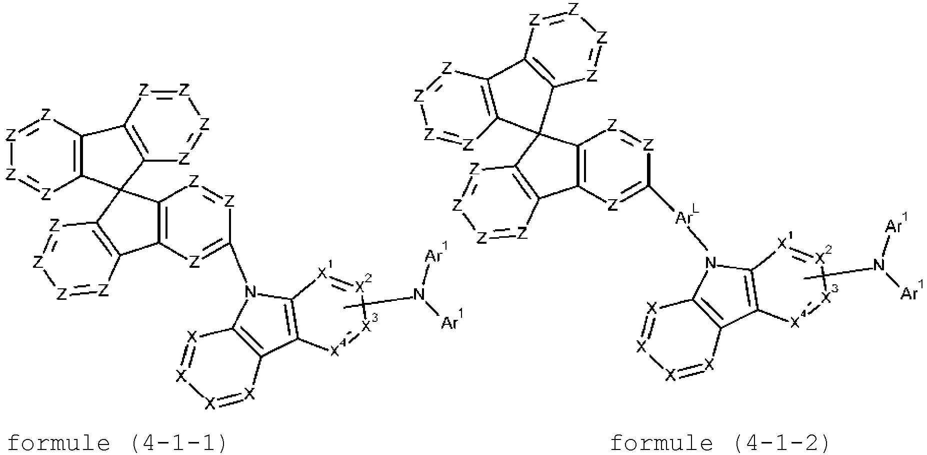 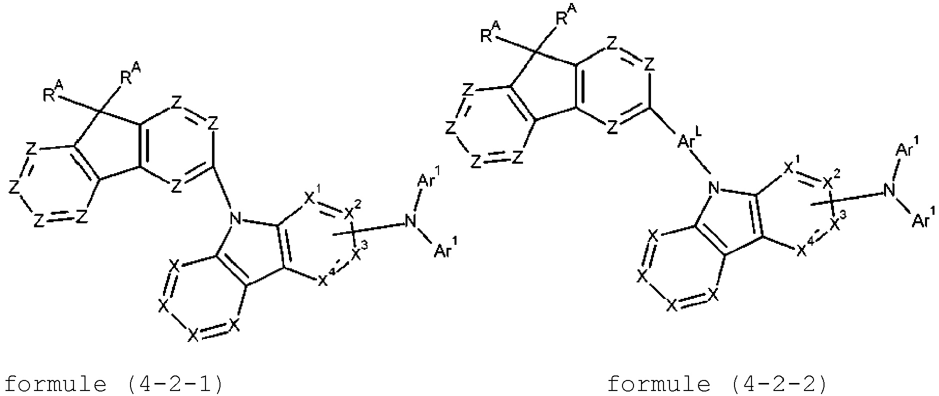 où les symboles ont la même signification que dans la revendication 1.

6. Dispositif électroluminescent organique selon une ou plusieurs des revendications précédentes, **caractérisé en ce que** le composé de formule (1) est choisi parmi les composés de l'une des formules (2-1-1-1) à (4-2-2-4), 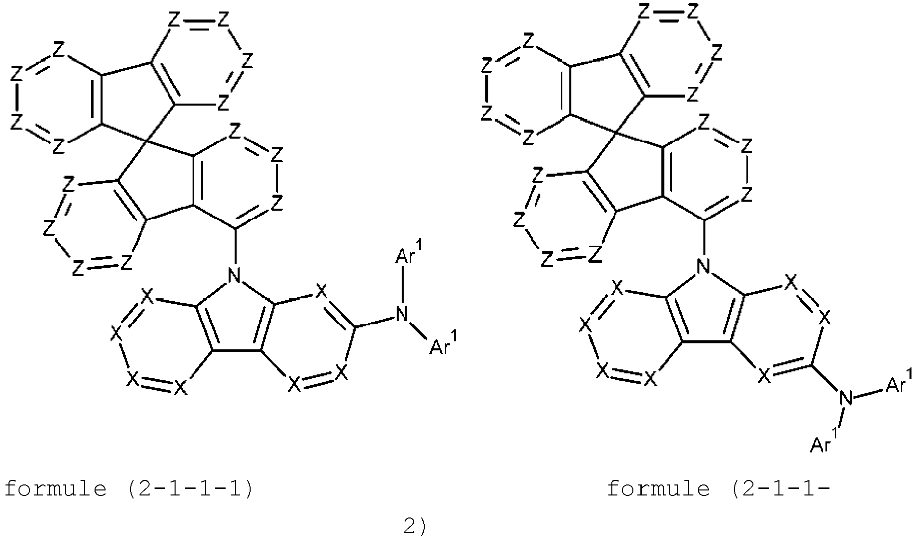 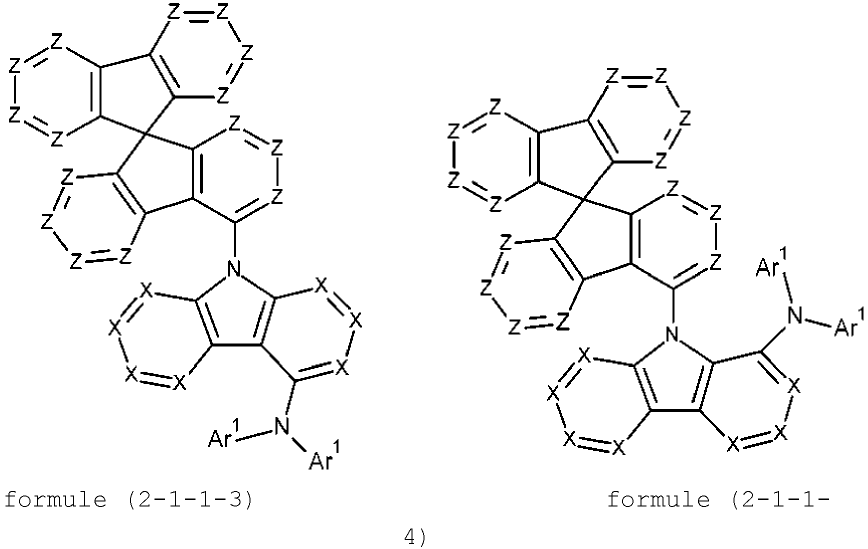 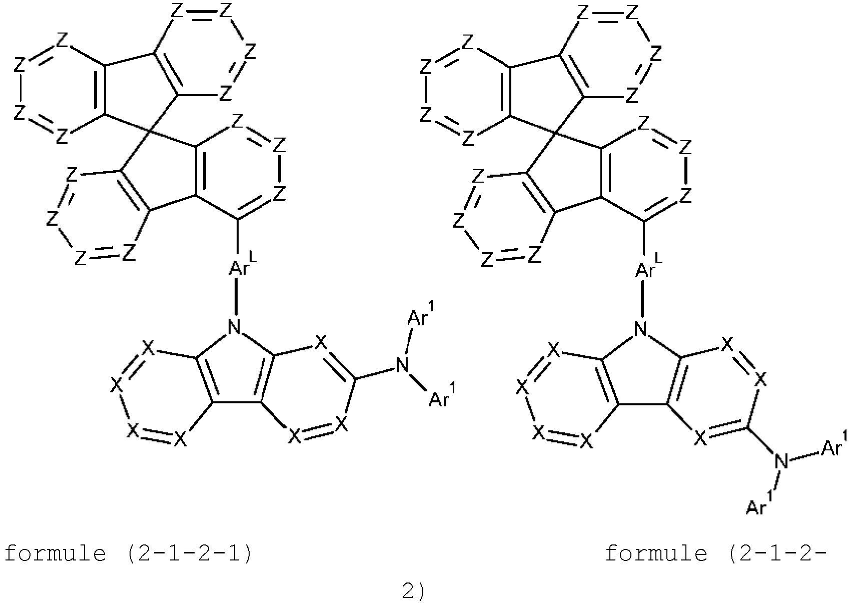 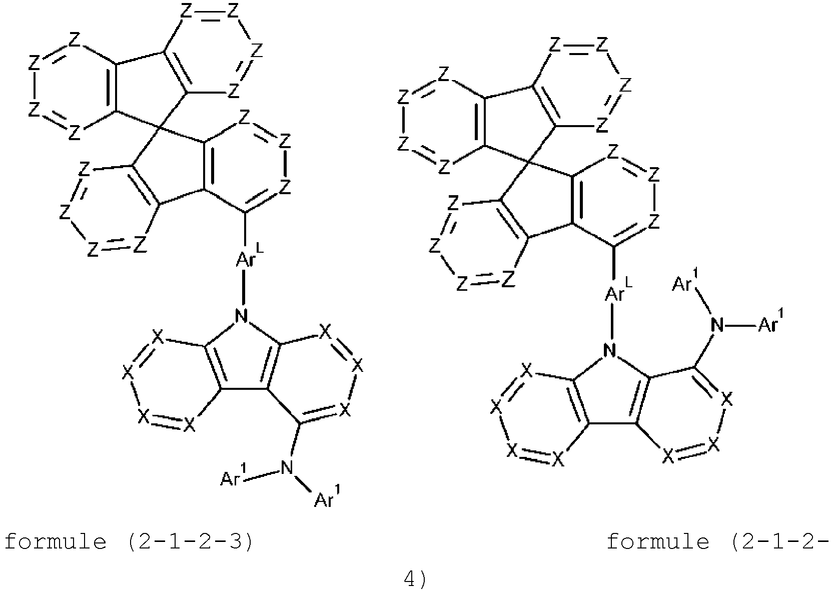 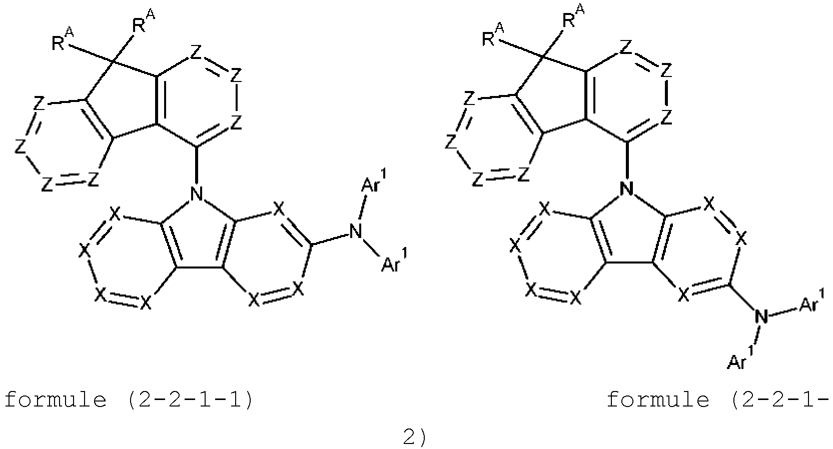 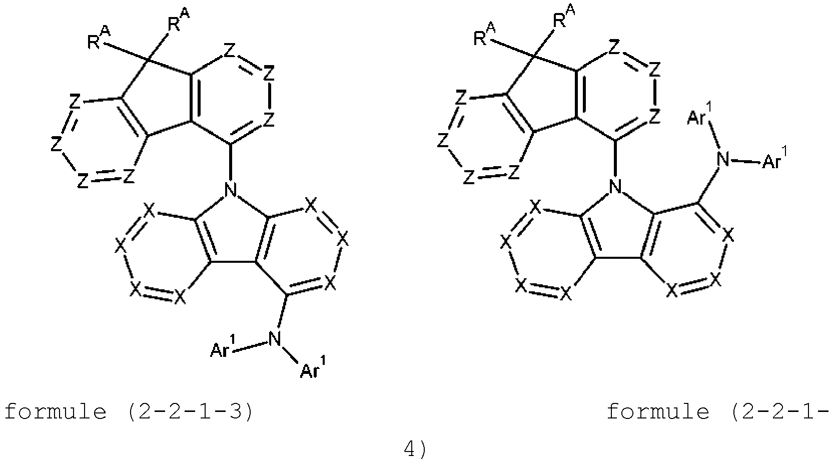 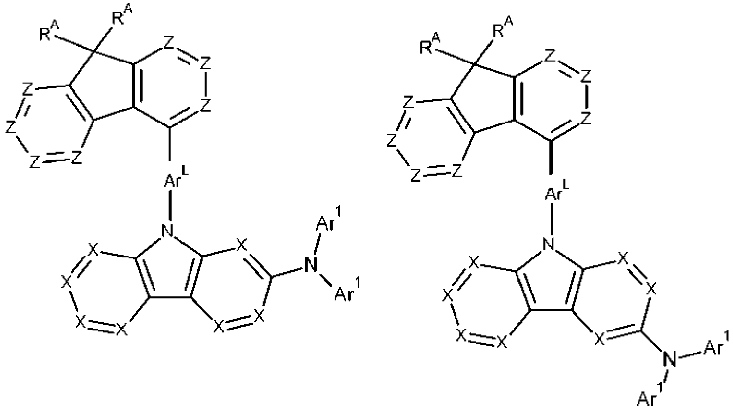 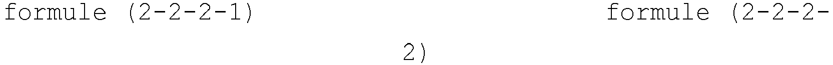 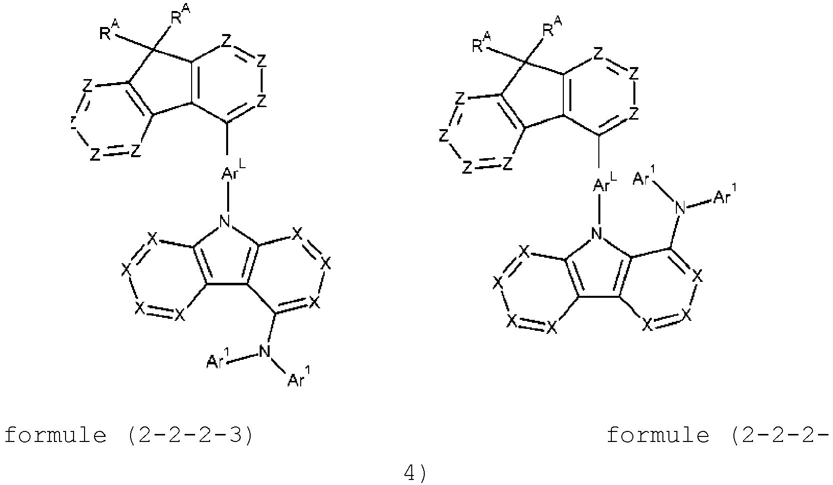 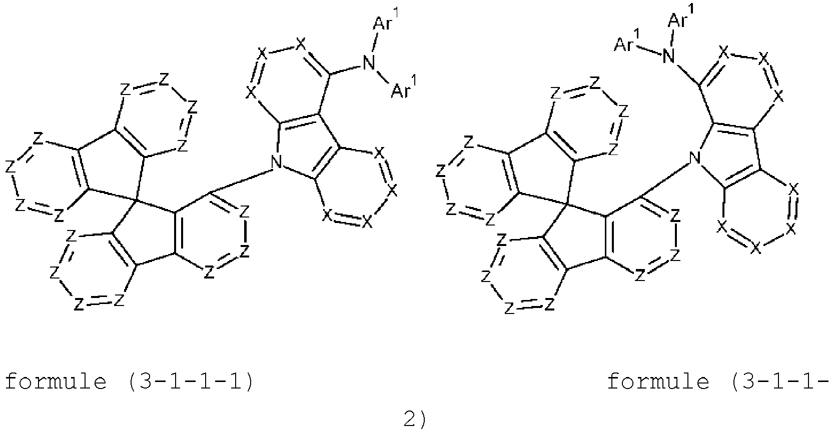 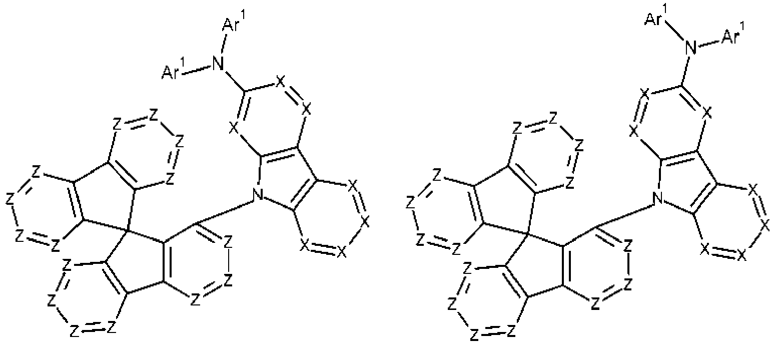 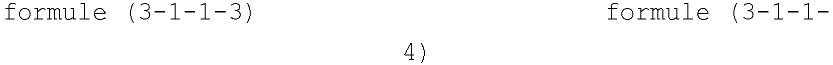 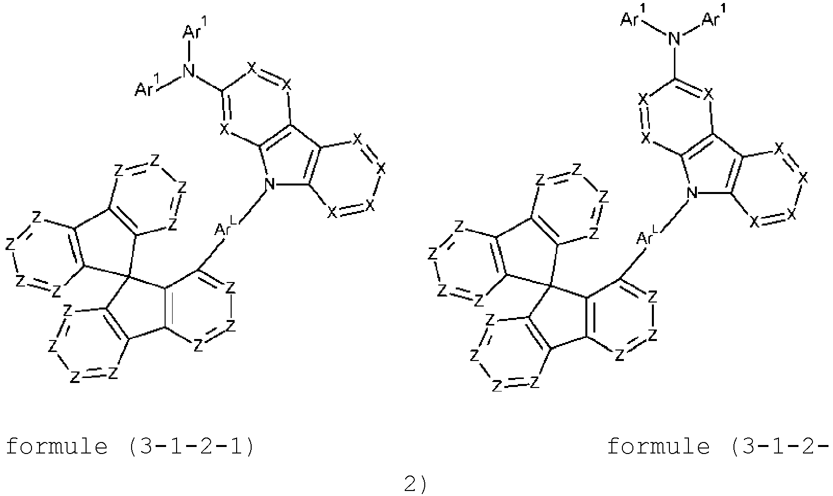 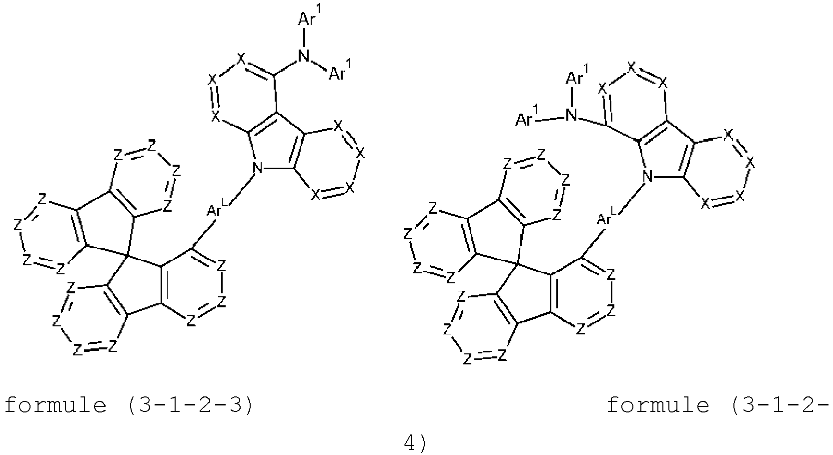 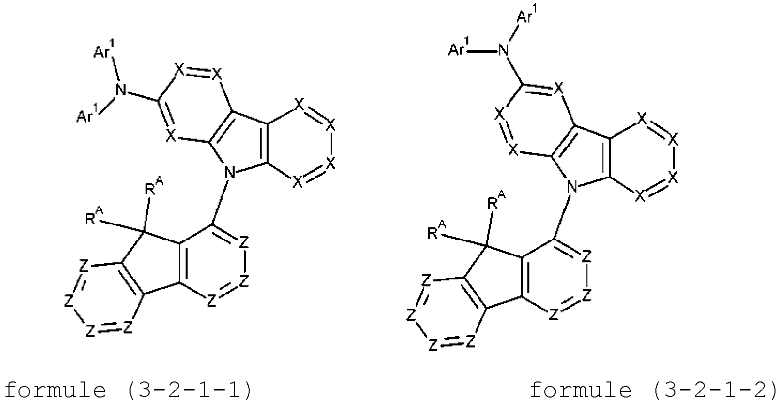 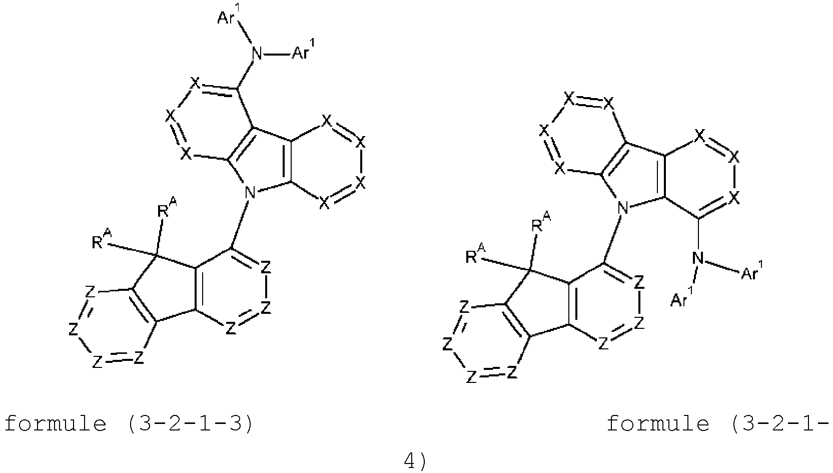 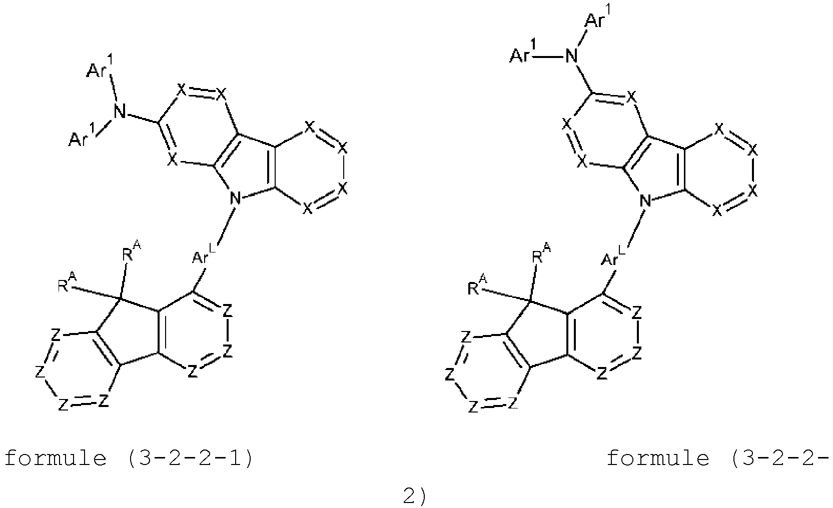 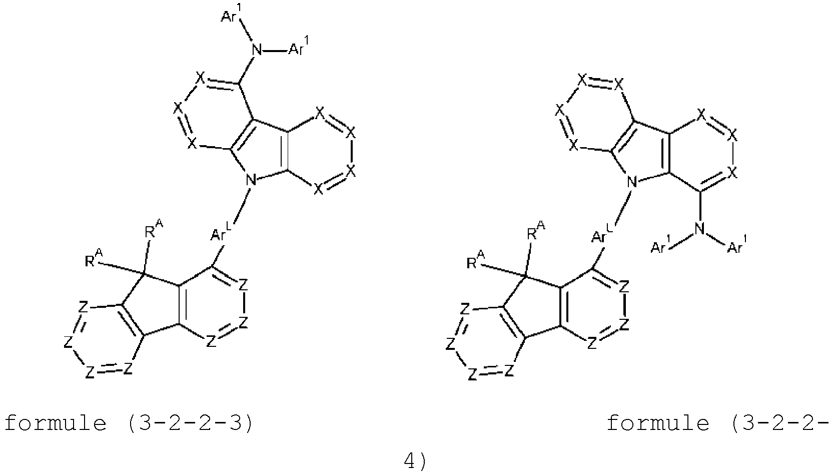 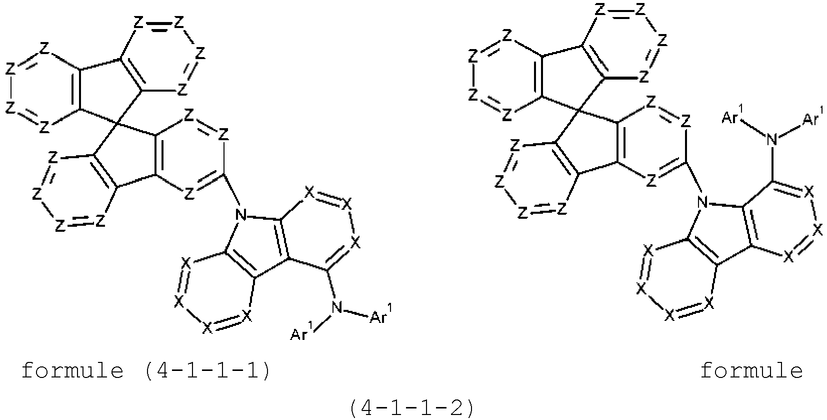 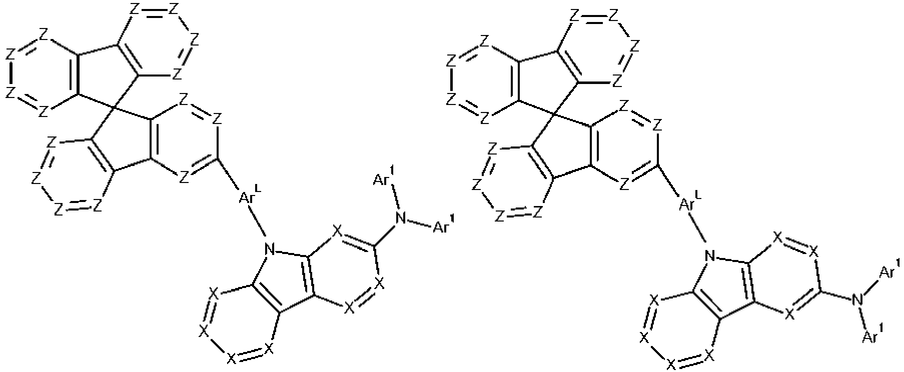 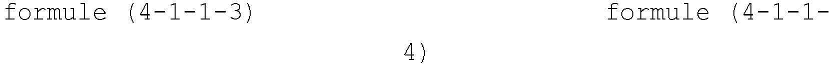 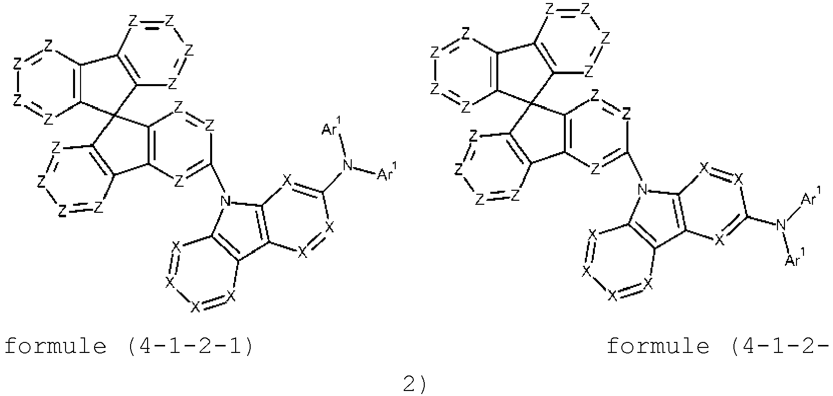 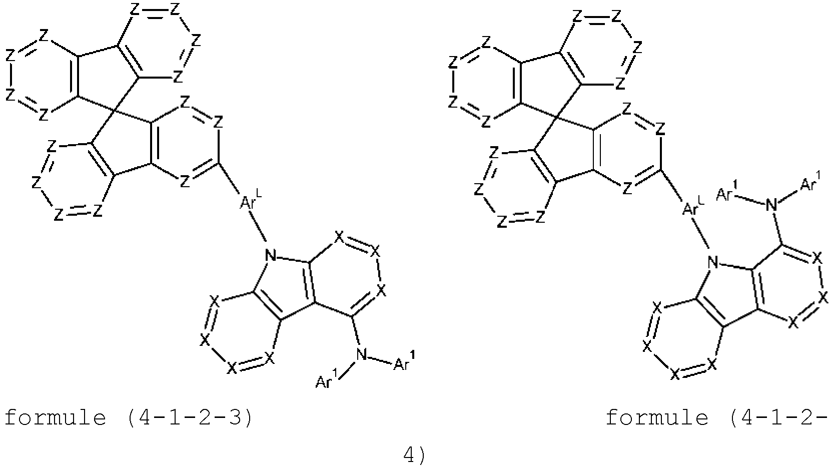 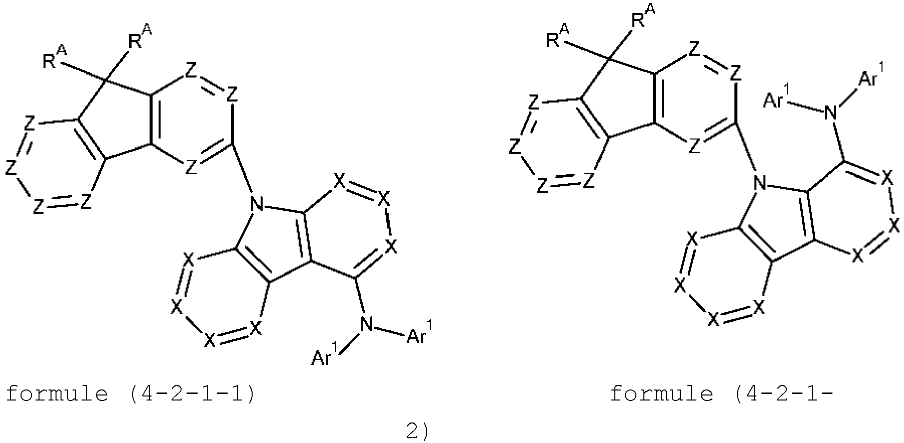 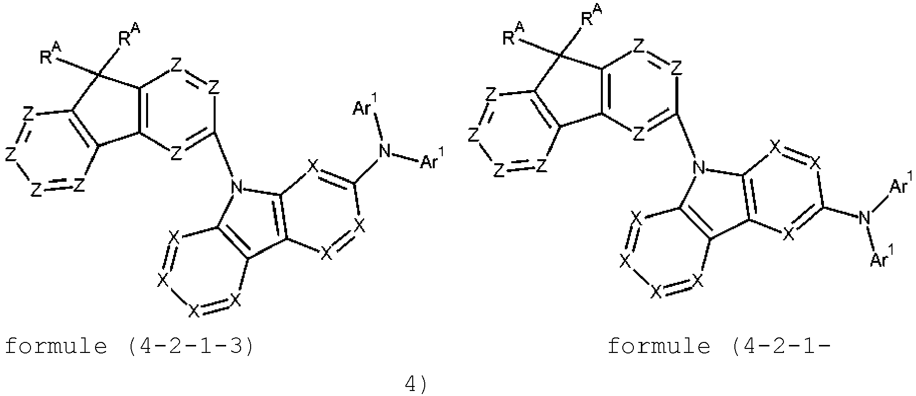 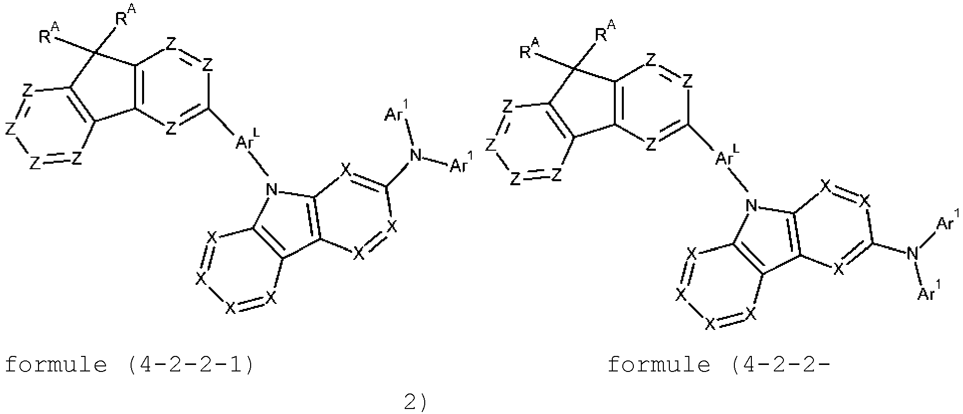 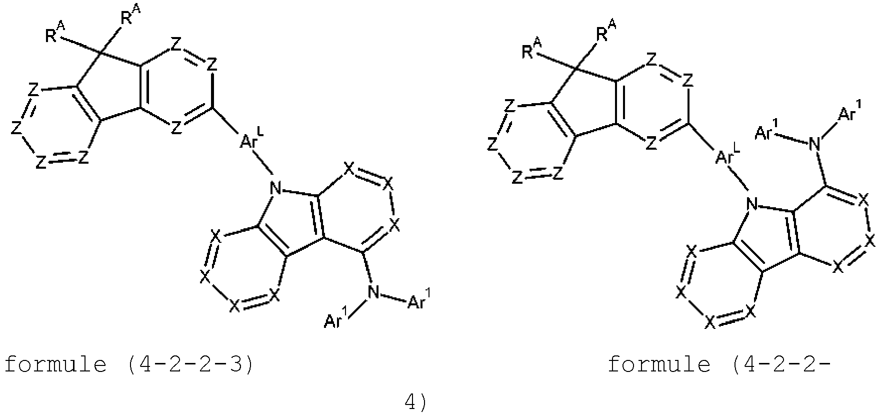 où les symboles ont la même signification que dans la revendication 1.

7. Dispositif électroluminescent organique selon une ou plusieurs des revendications précédentes, **caractérisé en ce que** R^{A}, R^{X} et R^{Z} représentent à chaque occurrence, de manière identique ou différente, H, D, F, un groupe alkyle à chaîne linéaire ayant 1 à 20 atomes C ou un groupe alkyle ramifié ou cyclique ayant 3 à 20 atomes C, chacun pouvant être substitué par un ou plusieurs radicaux R, où dans chaque cas un ou plusieurs CH non adjacents₂ les groupes peuvent être remplacés par RC=CR, C≡C, O ou S et, lorsqu'un ou plusieurs atomes H peuvent être remplacés par D, F, Cl ou CN, un système cyclique aromatique ou hétéroaromatique ayant 5 à 40 atomes de cycle aromatique, qui peut être substitué dans chaque cas par un ou plusieurs radicaux R; où deux radicaux adjacents R^{X}, deux radicaux adjacents R^{Z}, deux radicaux adjacents R^{UN} peut former un système cyclique aliphatique ou un système cyclique aromatique mono- ou polycyclique, qui peut être substitué par un ou plusieurs radicaux R.

8. Dispositif électroluminescent organique selon une ou plusieurs des revendications précédentes, **caractérisé en ce que** R^{X} représente à chaque occurrence, de manière identique ou différente, H, un groupe alkyle à chaîne linéaire ayant 1 à 20 atomes de carbone, dont chacun peut être substitué par un ou plusieurs radicaux R, ou un système cyclique aromatique ayant 5 à 40 atomes de cycle aromatique ou un système cyclique hétéroaromatique ayant 5 à 40 atomes de cycle aromatique, dont chacun peut être substitué par un ou plusieurs radicaux R ; où deux radicaux R^{X} adjacents peuvent former un système cyclique aliphatique ou un système cyclique aromatique mono- ou polycyclique, qui peut être substitué par un ou plusieurs radicaux R.

9. Dispositif électroluminescent organique selon une ou plusieurs des revendications précédentes, **caractérisé en ce que** R^{Z} représente à chaque occurrence, de manière identique ou différente, H, un groupe alkyle à chaîne linéaire ayant 1 à 20 atomes de carbone ou un groupe alkyle ramifié ayant 3 à 20 atomes de carbone, dont chacun peut être substitué par un ou plusieurs radicaux R, ou un système cyclique aromatique ayant 5 à 40 atomes de cycle aromatique ou un système cyclique hétéroaromatique ayant 5 à 40 atomes de cycle aromatique, dont chacun peut être substitué par un ou plusieurs radicaux R ; où deux radicaux R^{Z} adjacents peuvent former un système cyclique aliphatique ou un système cyclique aromatique mono- ou polycyclique, qui peut être substitué par un ou plusieurs radicaux R.

10. Dispositif électroluminescent organique selon une ou plusieurs des revendications précédentes, **caractérisé en ce que** Ar^{L} est choisi, de manière identique ou différente, parmi benzène, biphényle, terphényle, naphtalène, fluorène, indénofluorène, spirobifluorène, triazine, benzoquinoline, benzoquinazoline, dibenzofurane, dibenzothiophène et carbazole, où chacun des groupes mentionnés ci-dessus peut être substitué par un ou plusieurs radicaux R.

11. Dispositif électroluminescent organique selon une ou plusieurs des revendications précédentes, **caractérisé en ce que** Ar¹ est à chaque occurrence, de manière identique ou différente, choisi parmi phényle, biphényle, terphényle, quaterphényle, naphtyle, fluorényle, benzofluorényle, spirobifluorényle, indénofluorényle, dibenzofuranyle, dibenzothiophényle, carbazolyle, benzofuranyle, benzothiophényle, dibenzofuranyle benzocondensé, dibenzothiophényle benzocondensé, benzimidazo-benzimidazolyle, phényle substitué par naphtyle, phényle substitué par fluorényle, phényle substitué par spirobifluorényle, phényle substitué par dibenzofuranyle, phényle substitué par dibenzothiophényle, phényle substitué par benzimidazobenzimidazolyle et phényle substitué par carbazolyle, dont chacun peut éventuellement être substitué par un ou plusieurs radicaux R.
